# EUROPÄISCHE PATENTSCHRIFT

(11) **EP 0 842 943 B1**
(45) Veröffentlichungstag und Bekanntmachung des Hinweises auf die Patenterteilung: **26.02.2003**
(21) Anmeldenummer: 97119638.1
(22) Anmeldetag: 10.11.1997
(51) Int. Cl.: C07K 5/023, C07K 5/097, C07K 5/117, C07K 14/78, A61K 38/06, A61K 38/07, A61K 38/39

(54) **5-Ring-Heterocyclen als Inhibitoren der Leukozytenadhäsion und VLA-4-Antagonisten**
5-Ring heterocycles as inhibitors of leukocyte adhesion and as VLA-4 antagonists
Hétérocycles à cinq chaînons à titre d'inhibiteurs d'adhésion des leucocytes et d'antagonistes de VLA-4

(30) Priorität: 15.11.1996 DE 19647380
(43) Veröffentlichungstag der Anmeldung: 20.05.1998
(73) Patentinhaber: HOECHST AKTIENGESELLSCHAFT, 65929 Frankfurt am Main (DE)
(72) Erfinder: Stilz, Hans Ulrich, Dr., 65929 Frankfurt (DE); Wehner, Volkmar, Dr., 97657 Sandberg (DE); Knolle, Jochen, Dr., 65830 Kriftel (DE); Bartnik, Eckart, Dr., 65205 Wiesbaden-Delkenheim (DE); Hüls, Christoph, Dr., 55263 Wackernheim (DE)

(56) Entgegenhaltungen:
- EP-A- 0 580 008
- WO-A-95/14008
- WO-A-95/15973
- WO-A-96/22966

## Beschreibung

Gegenstand der vorliegenden Erfindung sind Verbindungen der Formel I 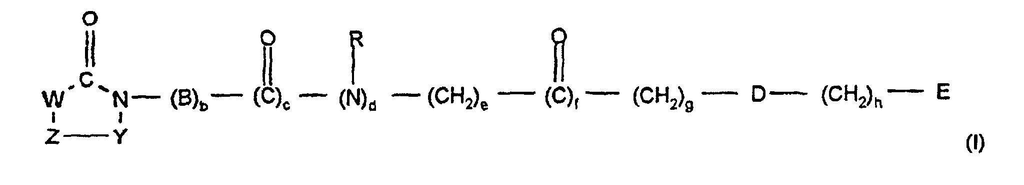 als Inhibitoren der Adhäsion und Migration von Leukozyten und/oder Antagonisten des zur Gruppe der Integrine gehörenden Adhäsionsrezeptors VLA-4. Die Erfindung betrifft die Verwendung von Verbindungen der Formel I und von pharmazeutischen Zubereitungen, die solche Verbindungen enthalten, zur Behandlung oder Prophylaxe von Krankheiten, die durch ein unerwünschtes Ausmaß an Leukozytenadhäsion und/oder Leukozytenmigration verursacht werden oder die damit verbunden sind oder bei denen Zell-Zell- oder Zell-Matrix-Interaktionen eine Rolle spielen, die auf Wechselwirkungen von VLA-4-Rezeptoren mit ihren Liganden beruhen, beispielsweise von Entzündungsprozessen, der rheumatoiden Arthritis oder von allergischen Erkrankungen, ebenso wie die Verwendung von Verbindungen der Formel I zur Herstellung von Arzneimitteln zur Anwendung bei solchen Krankheiten. Ferner betrifft sie neue Verbindungen der Formel I.

Die Integrine sind eine Gruppe von Adhäsionsrezeptoren, die bei Zell-Zellbindenden und Zell-Extrazelluläre Matrix-bindenden Prozessen eine wesentliche Rolle spielen. Sie weisen eine aß-heterodimere Struktur auf und zeigen eine weite zelluläre Verbreitung und ein hohes Maß an evolutiver Konservierung. Zu den Integrinen gehört zum Beispiel der Fibrinogen-Rezeptor auf Thrombozyten, der vor allem mit der RGD-Sequenz des Fibrinogens interagiert, oder der Vitronectin-Rezeptor auf Osteoclasten, der vor allem mit der RGD-Sequenz des Vitronectins oder des Osteopontins interagiert. Man teilt die Integrine in drei Großgruppen ein, die β2-Unterfamilie mit den Vertetern LFA-1, Mac-1 und p150/95, die insbesondere für Zell-Zell-Interaktionen des Immunsystems verantwortlich sind, und die Unterfamilien β1 und β3, deren Vertreter hauptsächlich die Zellanheftung an Komponenten der extrazellulären Matrix vermitteln (Ruoslahti, Annu. Rev. Biochem. 1988, 57, 375). Die Integrine der β1-Unterfamilie, auch VLA-Proteine (very late (activation) antigen) genannt, umfassen mindestens sechs Rezeptoren, die spezifisch mit Fibronektin, Kollagen und/oder Laminin als Liganden interagieren. Innerhalb der VLA-Familie ist das Integrin VLA-4 (α4β1) insofern untypisch, als es hauptsächlich auf lymphoide und myeloide Zellen begrenzt ist und bei diesen verantwortlich ist für Zell-Zell-Interaktionen mit einer Vielzahl von anderen Zellen. VLA-4 vermittelt zum Beispiel die Interaktion von T- und B-Lymphozyten mit dem Heparin II-Bindungsfragment von humanem Plasmafibronektin (FN). Die Bindung von VLA-4 mit dem Heparin II-Bindungsfragment des Plasmafibronektins beruht vor allem auf einer Interaktion mit einer LDVP-Sequenz. Im Unterschied zum Fibrinogen- oder Vitronectin-Rezeptor ist VLA-4 kein typisches RGD-bindendes Integrin (Kilger und Holzmann, J. Mol. Meth. 1995, 73, 347).

Die im Blut zirkulierenden Leukozyten zeigen normalerweise nur eine geringe Affinität zu den vaskulären endothelialen Zellen, die die Blutgefäße auskleiden. Zytokine, die von entzündetem Gewebe abgegeben werden, bewirken die Aktivierung von Endothelzellen und damit die Expression einer Vielzahl von Zelloberflächenantigenen. Diese umfassen zum Beispiel die Adhäsionsmoleküle ELAM-1 (endothelial cell adhesion molecule-1; auch als E-Selektin bezeichnet), das unter anderem Neutrophile bindet, ICAM-1 (intercellular adhesion molecule-1), das mit LFA-1 (leucocyte function-associated antigen 1) auf Leukozyten interagiert, und VCAM-1 (vascular cell adhesion molecule-1), das verschiedene Leukozyten, unter anderem Lymphozyten, bindet (Osbom et al., Cell 1989, 59, 1203). VCAM-1 ist, wie ICAM-1, ein Mitglied der immunglobulin-Gen-Überfamilie. Identifiziert wurde VCAM-1 (zuerst bekannt als INCAM-110) als ein Adhäsionsmolekül, daß auf endothelialen Zellen durch Entzündungs-Zytokine wie TNF und IL-1 und Lipopolysaccharide (LPS) induziert wird. Elices et al. (Cell 1990, 60, 577) zeigten, daß VLA-4 und VCAM-1 ein Rezeptor-Ligand-Paar bilden, das die Anheftung von Lymphozyten an aktiviertes Endothel vermittelt. Die Bindung von VCAM-1 an VLA-4 erfolgt dabei nicht durch eine Interaktion des VLA-4 mit einer RGD-Sequenz, eine solche ist im VCAM-1-nicht enthalten (Bergelson et al., Current Biology 1995, 5, 615). VLA-4 tritt aber auch auf anderen Leukozyten auf, und über den VCAM-1/VLA-4-Adhäsionsmechanismus wird auch die Anheftung von anderen Leukozyten als Lymphozyten vermittelt. VLA-4 repräsentiert somit ein einzelnes Beispiel eines β1-Integrin-Rezeptors, der über die Liganden VCAM-1 bzw. Fibronektin sowohl bei Zell-Zell-Interaktionen als auch bei Zell-Extrazellulärer Matrix-Interaktionen eine wesentliche Rolle spielt.

Die Zytokin-induzierten Adhäsionsmoleküle spielen eine wichtige Rolle bei der Rekrutierung von Leukozyten in extravaskuläre Gewebebereiche. Leukozyten werden in entzündliche Gewebebereiche durch Zelladhäsionsmoleküle rekrutiert, die auf der Oberfläche von endothelialen Zellen exprimiert werden und als Liganden für Leukozyten-Zelloberflächen-Proteine oder -Proteinkomplexe (Rezeptoren) dienen (die Begriffe Ligand und Rezeptor können auch vice versa verwendet werden). Leukozyten aus dem Blut müssen zunächst an endotheliale Zellen anheften, bevor sie in das Synovium auswandern können. Da VCAM-1 an Zellen bindet, die das Integrin VLA-4 (α4β1) tragen, wie Eosinophile, T- und B-Lymphozyten, Monozyten oder auch Neutrophile, kommt ihm und dem VCAM-1/VLA-4-Mechanismus die Funktion zu, derartige Zellen aus dem Blutstrom in Infektionsgebiete und Entzündungsherde zu rekrutieren (Elices et al., Cell 1990, 60, 577; Osbom, Cell 1990, 62, 3; Issekutz et al., J. Exp. Med. 1996, 183, 2175).

Der VCAM-1/VLA-4-Adhäsionsmechanismus wurde mit einer Reihe von physiologischen und pathologischen Prozessen in Verbindung gebracht. VCAM-1 wird außer von Zytokin-induziertem Endothel unter anderem noch von den folgenden Zellen exprimiert: Myoblasten, lymphoiden dendritischen Zellen und Gewebsmakrophagen, rheumatoidem Synovium, Zytokin-stimulierten Neuralzellen, parietalen Epithelzellen der Bowmans Kapsel, dem renalen Tubularepithel, entzündetem Gewebe bei Herz- und Nieren-Transplantat-Abstoßung und von Intestinalgewebe bei Graft versus host-Krankheit. VCAM-1 findet man auch exprimiert auf solchen Gewebearealen des arteriellen Endotheliums, die frühen arteriosklerotischen Plaques eines Kaninchenmodells entsprechen. Zusätzlich wird VCAM-1 follikulären dendritischen Zellen von humanen Lymphknoten exprimiert und findet sich auf Stromazellen des Knochenmarks, zum Beispiel in der Maus. Letzterer Befund weist auf eine Funktion von VCAM-1 in der B-Zell-Entwicklung hin. VLA-4 wird, außer auf Zellen haematopoetischen Ursprunges, auch zum Beispiel auf Melanoma-Zellinien gefunden, und der VCAM-1/VLA-4-Adhäsionsmechanismus wird mit der Metastasierung von solchen Tumoren in Verbindung gebracht (Rice et al., Science 1989, 246, 1303).

Die hauptsächliche Form, in der VCAM-1 in vivo auf endothelialen Zellen vorkommt und die die dominante Form in vivo ist, wird als VCAM-7D bezeichnet und trägt sieben lmmunglobulin-Domänen. Die Domänen 4, 5 und 6 ähneln in ihren Aminosäuresequenzen den Domänen 1, 2 und 3. Die vierte Domäne ist bei einer weiteren, aus sechs Domänen bestehenden Form, hier als VCAM-6D bezeichnet, durch alternatives Splicing entfernt. Auch VCAM-6D kann VLA-4-exprimierende Zellen binden.

Weitere Angaben zu VLA-4, VCAM-1, Integrinen und Adhäsionsproteinen finden sich zum Beispiel in den Artikeln von Kilger und Holzmann, J. Mol. Meth. 1995, 73, 347; Elices, Cell Adhesion in Human Disease, Wiley, Chichester 1995, S. 79; Kuijpers, Springer Semin. Immunopathol. 1995, 16, 379.

Aufgrund der Rolle des VCAM-1/VLA-4-Mechanismus bei Zelladhäsionsprozessen, die von Bedeutung zum Beispiel bei Infektionen, Entzündungen oder Atherosklerose sind, wurde versucht, durch Eingriffe in diese Adhäsionsprozesse Krankheiten zu bekämpfen, insbesondere zum Beispiel Entzündungen (Osborn et al., Cell 1989, 59, 1203). Eine Methode hierzu ist die Verwendung von monoklonalen Antikörpern, die gegen VLA-4 gerichtet sind. Derartige monoklonale Antikörper (mAK), die als VLA-4-Antagonisten die Interaktion zwischen VCAM-1 und VLA-4 blockieren, sind bekannt. So inhibieren zum Beispiel die anti-VLA-4 mAK HP2/1 und HP1/3 die Anheftung von VLA-4 exprimierenden Ramos-Zellen (B-Zell-ähnlichen Zellen) an humane Nabelschnurendothelzellen und an VCAM-1-transfizierte COS-Zellen.

Ebenso inhibiert der anti-VCAM-1 mAK 4B9 die Adhäsion von Ramos-Zellen, Jurkat-Zellen (T-Zell-ähnlichen Zellen) und HL60-Zellen (Granulozyten-ähnlichen Zellen) an COS-Zellen transfiziert mit genetischen Konstrukten, die veranlassen, daß VCAM-6D und VCAM-7D exprimiert werden. In vitro-Daten mit Antikörpern, die gegen die α4-Untereinheit von VLA-4 gerichtet sind, zeigen, daß die Anheftung von Lymphozyten an synoviale Endothelzellen blockiert wird, eine Adhäsion, die bei der rheumatoiden Arthritis eine Rolle spielt (van Dinther-Janssen et al., J. Immunol. 1991, 147, 4207).

In vivo-Versuche haben gezeigt, daß eine experimentelle autoimmune Enzephalomyelitis durch anti-α4 mAK gehemmt werden kann. Die Wanderung von Leukozyten in einen Entzündungsherd wird ebenfalls durch einen monoklonalen Antikörper gegen die α4-Kette von VLA-4 blockiert. Die Beeinflussung des VLA-4-abhängigen Adhäsionsmechanismus mit Antikörpern wurde auch in einem Asthma-Modell untersucht, um die Rolle von VLA-4 bei der Rekrutierung von Leukozyten in entzündetes Lungengewebe zu untersuchen (USSN 07/821,768; EP-A-626 861). Die Gabe von anti-VLA-4-Antikörpern inhibierte die Spätphasenreaktion und die Atemwegsüberreaktion in allergischen Schafen.

Der VLA-4 abhängige Zelladhäsionsmechanismus wurde ebenfalls in einem Primatenmodell der inflammatory bowel disease (IBD) untersucht. In diesem Modell, das der ulcerativen Colitis im Menschen entspricht, ergab die Gabe von anti-VLA-4-Antikörpern eine signifikante Reduktion der akuten Entzündung.

Darüber hinaus konnte gezeigt werden, daß die VLA-4-abhängige Zelladhäsion bei den folgenden klinischen Konditionen einschließlich der folgenden chronischen entzündlichen Prozesse eine Rolle spielt: Rheumatoide Arthritis (Cronstein und Weismann, Arthritis Rheum. 1993, 36, 147; Elices et al., J. Clin. Invest. 1994, 93, 405), Diabetes mellitus (Yang et al., Proc. Natl. Acad. Sci. USA 1993, 90, 10494), systemischer Lupus erythematosus (Takeuchi et al., J. Clin. Invest. 1993, 92, 3008), Allergien vom verzögerten Typ (Typ IV-Allergie) (Elices et al., Clin. Exp. Rheumatol. 1993, 11, S77), multiple Sklerose (Yednock et al., Nature 1992, 356, 63), Malaria (Ockenhouse et al., J. Exp. Med. 1992, 176, 1183), Arteriosklerose (Obrien et al., J. Clin. Invest. 1993, 92, 945), Transplantation (Isobe et al., Transplantation Proceedings 1994, 26, 867-868), verschiedene Malignitäten, zum Beispiel Melanom (Renkonen et al., Am. J. Pathol. 1992, 140, 763), Lymphom (Freedman et al., Blood 1992, 79, 206) und andere (Albelda et al., J. Cell Biol. 1991, 114, 1059).

Eine VLA-4-Blockierung durch geeignete Antagonisten bietet danach effektive therapeutische Möglichkeiten, insbesondere zum Beispiel verschiedene entzündliche Konditionen einschließlich Asthma und IBD zu behandeln. Die besondere Relevanz von VLA-4-Antagonisten für die Behandlung der rheumatoiden Arthritis ergibt sich dabei, wie bereits gesagt, aus der Tatsache, daß Leukozyten aus dem Blut zunächst an endotheliale Zellen anheften müssen, ehe sie in das Synovium auswandern können, und daß bei dieser Anheftung der VLA-4-Rezeptor eine Rolle spielt. Darauf, daß durch Entzündungsagenzien auf endothelialen Zellen VCAM-1 induziert wird (Osborn, Cell 1990, 62, 3; Stoolman, Cell 1989, 56, 907), und auf die Rekrutierung verschiedener Leukozyten in Infektionsgebiete und Entzündungsherde wurde bereits oben eingegangen. T-Zellen adherieren dabei an aktiviertes Endothel hauptsächlich über die LFA-1/ICAM-1- und VLA-4*N*CAM-1-Adhäsionsmechanismen (Springer, Cell 1994,76, 301). Auf den meisten synovialen T-Zellen ist die Bindungskapazität von VLA-4 für VCAM-1 bei der rheumatoiden Arthritis erhöht (Postigo et al., J. Clin. Invest. 1992, 89, 1445). Zusätzlich wurde eine verstärkte Anheftung von synovialen T-Zellen an Fibronektin beobachtet (Laffon et al., J. Clin. Invest. 1991, 88, 546; Morales-Ducret et al., J. Immunol. 1992, 149, 1424). VLA-4 ist also hochreguliert sowohl im Rahmen seiner Expression als auch hinsichtlich seiner Funktion auf T-Lymphozyten der rheumatoiden Synovialmembran. Die Blockierung der Bindung von VLA-4 an seine physiologischen Liganden VCAM-1 und Fibronektin ermöglicht eine effektive Verhinderung oder Linderung von artikulären Entzündungsprozessen. Dies wird auch durch Experimente mit dem Antikörper HP2/1 an Lewis-Ratten mit Adjuvanz-Arthritis bestätigt, bei denen eine effektive Krankheitsprävention beobachtet wurde (Barbadillo et al., Springer Semin. Immunopathol. 1995, 16, 427). VLA-4 stellt also ein wichtiges therapeutisches Zielmolekül dar.

Die oben erwähnten VLA-4-Antikörper und der Einsatz von Antikörpern als VLA-4-Antagonisten sind in den Patentanmeldungen WO-A-93113798, WO-A-93/15764, WO-A-94116094, WO-A-94/17828 und WO-A-95119790 beschrieben. In den Patentanmeldungen WO-A-94115958, WO-A-95/15973, WO-A-96/00581. WO-A-96/06108 und WO-A-96/20216 werden peptidische Verbindungen als VLA-4-Antagonisten beschrieben. Der Einsatz von Antikörpern und peptidischen Verbindungen als Arzneimitteln ist aber mit Nachteilen behaftet, zum Beispiel mangelnder oraler Verfügbarkeit, leichter Abbaubarkeit oder immunoger Wirkung bei längerfristiger Anwendung, und es besteht somit Bedarf nach VLA-4-Antagonisten mit einem günstigen Eigenschaftsprofil für einen Einsatz in der Therapie und Prophylaxe. Bestimmte nichtpeptidische VLA-4-Antagonisten sind bereits in der WO-A-96/22966 beschrieben.

In der WO-A-94/21607 und der WO-A-95/14008 sind substituierte 5-Ring-Heterocyclen beschrieben, in der EP-A-449 079, der EP-A-530 505 (US-A-5 389 614), der WO-A-93/18057, der EP-A-566 919 (US-A-5 397 796), der EP-A-580 008 (US-A-5 424 293) und der EP-A-584 694 (US-A-5 554 594) sind Hydantoinderivate beschrieben, die thrombozytenaggregationshemmende Wirkungen aufweisen. Hinweise auf einen VLA-4-Antagonismus dieser Verbindungen finden sich dort aber nicht Überraschend wurde nun gefunden, daß diese Verbindungen auch die Leukozytenadhäsion hemmen und VLA-4-Antagonisten sind.

Die vorliegende Erfindung betrifft somit die Verwendung von Verbindungen der Formel I, 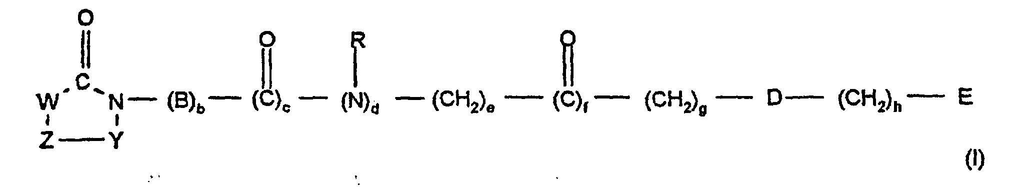 worin
- W: für R¹-A-C(R¹³) oder R¹-A-CH=C steht;
- Y: für eine Carbonyl-, Thiocarbonyl- oder Methylengruppe steht;
- Z: für N(R⁰), Sauerstoff, Schwefel oder eine Methylengruppe steht;
- A: einen zweiwertigen Rest aus der Reihe (C₁-C₆)-Alkylen, (C₃-C₇)-Cycloalkylen, Phenylen, Phenylen-(C₁-C₆)-alkyl, (C₁-C₆)-Alkylen-phenyl, Phenylen-(C₂-C₆)-alkenyl oder einen zweiwertigen Rest eines 5- oder 6-gliedrigen gesättigten oder ungesättigten Ringes, der 1 oder 2 Stickstoffatome enthalten und ein- oder zweifach durch (C₁-C₆)-Alkyl oder doppelt gebundenen Sauerstoff oder Schwefel substituiert sein kann, bedeutet;
- B: einen zweiwertigen Rest aus der Reihe (C₁-C₆)-Alkylen, (C₂-C₆)-Alkenylen, Phenylen, Phenylen-(C₁-C₃)-alkyl, (C₁-C₃)-Alkylen-phenyl bedeutet, wobei der zweiwertige (C₁-C₆)-Alkylen-Rest unsubstituiert oder durch einen Rest aus der Reihe (C₁-C₈)-Alkyl, (C₂-C₈)-Alkenyl, (C₂-C₈)-Alkinyl, (C₃-C₁₀)-Cycloalkyl, (C₃- C₁₀)-Cycloalkyl-(C₁-C₆)-alkyl, gegebenenfalls substituiertes (C₆-C₁₄)-Aryl, im Arylrest gegebenenfalls substituiertes (C₆-C₁₄)-Aryl-(C₁-C₆)-alkyl, gegebenenfalls substituiertes Heteroaryl und im Heteroarylrest gegebenenfalls substituiertes Heteroaryl-(C₁-C₆)-alkyl substituiert sein kann;
- D: für C(R²)(R³), N(R³) oder CH=C(R³) steht;
- E: Tetrazolyl, (R⁸O)₂P(O), HOS(O)₂, R⁹NHS(O)₂ oder R¹⁰CO bedeutet;
- R: Wasserstoff, (C₁-C₈)-Alkyl, (C₃-C₈)-Cycloalkyl, gegebenenfalls substituiertes (C₆- C₁₄)-Aryl oder im Arylrest gegebenenfalls substituiertes (C₆-C₁₄)-Aryl-(C₁-C₈)-alkyl bedeutet;
- R⁰: für Wasserstoff, (C₁-C₈)-Alkyl, (C₃-C₁₂)-Cycloalkyl, (C₃-C₁₂)-Cycloalkyl-(C₁-C₈)-alkyl, (C₆-C₁₂)-Bicycloalkyl, (C₆-C₁₂)-Bicycloalkyl-(C₁-C₈)-alkyl, (C₆-C₁₂)-Tricycloalkyl, (C₆-C₁₂)-Tricycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl, im Heteroarylrest gegebenenfalls substituiertes Heteroaryl-(C₁-C₈)-alkyl, CHO, (C₁-C₈)-Alkyl-CO, (C₃-C₁₂)-Cycloalkyl-CO, (C₃-C₁₂)-Cycloalkyl-(C₁-C₈)-alkyl-CO, (C₆-C₁₂)-Bicycloalkyl-CO, (C₆-C₁₂)-Bicycloalkyl-(C₁-C₈)-alkyl-CO, (C₆-C₁₂)-Tricycloalkyl-CO, (C₈-C₁₂)-Tricycloalkyl-(C₁-C₈)-alkyl-CO, gegebenenfalls substituiertes (C₆-C₁₄)-Aryl-CO, im Arylrest gegebenenfalls substituiertes (C₆-C₁₄)-Aryl-(C₁-C₈)-alkyl-CO, gegebenenfalls substituiertes Heteroaryl-CO, im Heteroarylrest gegebenenfalls substituiertes Heteroaryl-(C₁-C₈)-alkyl-CO, (C₁-C₈)-Alkyl-S(O)ₙ, (C₃-C₁₂)-Cycloalkyl-S(O)ₙ, (C₃-C₁₂)-Cycloalkyl-(C₁-C₈)-alkyl-S(O)ₙ, (C₆-C₁₂)-Bicycloalkyl-S(O)ₙ, (C₆-C₁₂)-Bicycloalkyl-(C₁-C₈)-alkyl-S(O)ₙ, (C₆-C₁₂)-Tricycloalkyl-S(O)Sₙ, (C₆-C₁₂) Tricycloalkyl-(C₁-C₈)-alkyl-S(O)ₙ, gegebenenfalls substituiertes (C₆-C₁₄)-Aryl-S(O)ₙ, im Arylrest gegebenenfalls substituiertes (C₆-C₁₄)-Aryl-(C₁-C₈)-alkyl-S(O)ₙ, gegebenenfalls substituiertes Heteroaryl-S(O)ₙ oder im Heteroarylrest gegebenenfalls substituiertes Heteroaryl-(C₁-C₈)-alkyl-S(O)ₙ steht, wobei n für 1 oder 2 steht;
- R¹: fürX-NH-C(=NH)-(CH₂)ₚ oder X¹ -NH-(CH₂)ₚ steht, wobei p für 0, 1, 2 oder 3 steht;
- X: Wasserstoff, (C₁-C₆)-Alkyl, (C₁-C₆)-Alkylcarbonyl, (C₁-C₆)-Alkoxycarbonyl, (C₁-C₁₈)-Alkylcarbonyloxy-(C₁-C₆)-alkoxycarbonyl, gegebenenfalls substituiertes (C₆-C₁₄)-Arylcarbonyl, gegebenenfalls substituiertes (C₆-C₁₄)-Aryloxycarbonyl, (C₆-C₁₄)-Aryl-(C₁-C₆)-alkoxycarbonyl, das im Arylrest auch substituiert sein kann, (R⁸O)₂P(O), Cyano, Hydroxy, (C₁-C₆)-Alkoxy, (C₆-C₁₄)-Aryl-(C₁-C₆)-alkoxy, das im Arylrest auch substituiert sein kann, oder Amino bedeutet;
- X¹: eine der Bedeutungen von X hat oder R'-NH-C(=N-R") bedeutet, wobei R' und R" unabhängig voneinander die Bedeutungen von X haben;
- R²: Wasserstoff, (C₁-C₈)-Alkyl, gegebenenfalls substituiertes (C₆-C₁₄₎-Aryl, im Arylrest gegebenenfalls substituiertes (C₆-C₁₄)-Aryl-(C₁-C₈)-alkyl oder (C₃-C₈)-Cycloalkyl bedeutet;
- R³: Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)-Cycloalkyl, (C₂-C₈)-Alkenyl, (C₂-C₈)-Alkinyl, (C₂-C₈)-Alkenylcarbonyl, (C₂-C₈)-Alkinylcarbonyl, Pyridyl, R¹¹NH, R⁴CO, COOR⁴_{,} CON(CH₃)R¹⁴, CONHR¹⁴_{,} CSNHR¹⁴_{,} COOR¹⁵_{,} CON(CH₃)R¹⁵ oder CONHR¹⁵ bedeutet;
- R⁴: Wasserstoff oder (C₁-C₂₈)-Alkyl bedeutet, das gegebenenfalls ein- oder mehrfach durch gleiche oder verschiedene Reste R⁴' substituiert sein kann;
- R⁴': Hydroxy, Hydroxycarbonyl, Aminocarbonyl, Mono- oder Di-((C₁-C₁₈)-alkyl)-aminocarbonyl, Amino-(C₂-C₁₈)-alkylaminocarbonyl, Amino-(C₁ -C₃)-alkyl-phenyl-(C₁-C₃)-alkylaminocarbonyl, (C₁-C₁₈)-Alkylcarbonylamino-(C₁-C₃)-alkylphenyl-(C₁-C₃)-alkylaminocarbonyl, (C₁-C₁₈) Alkylcarbonylamino-(C₂-C₁₈)-alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)-Alkoxycarbonyl, gegebenenfalls substituiertes (C₃-C₈)-Cycloalkyl, Halogen, Nitro, Trifluormethyl oder den Rest R⁵ bedeutet;
- R⁵: gegebenenfalls substituiertes (C₆-C₁₄)-Aryl, im Arylrest gegebenenfalls substituiertes (C₆-C₁₄)-Aryl-(C₁-C₈)-alkyl, einen mono- oder bicyclischen 5- bis 12-gliedrigen heterocyclischen Ring, der aromatisch, teilhydriert oder vollständig hydriert sein kann und der ein, zwei oder drei gleiche oder verschiedene Heteroatome aus der Reihe Stickstoff, Sauerstoff und Schwefel enthalten kann, einen Rest R⁶ oder einen Rest R⁶CO- bedeutet, wobei der Aryl- und unabhängig davon der Heterocyclus-Rest ein- oder mehrfach durch gleiche oder verschiedene Reste aus der Reihe (C₁-C₁₈)-Alkyl, (C₁-C₁₈)-Alkoxy, Halogen, Nitro, Amino oder Trifluormethyl substituiert sein können;
- R⁶: für R⁷R⁸N_{,} R⁷O oder R⁷S steht oder eine Aminosäureseitenkette, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, sowie deren Ester und Amide bedeutet, wobei anstelle freier funktioneller Gruppen gegebenenfalls Wasserstoff oder Hydroxymethyl stehen kann und/oder wobei freie funktionelle Gruppen durch in der Peptidchemie übliche Schutzgruppen geschützt sein können;
- R⁷: Wasserstoff, (C₁-C₁₈)-Alkyl, (C₆-C₁₄)-Aryl-(C₁-C₈)-alkyl, (C₁-C₁₈)-Alkylcarbonyl, (C₁-C₁₈)-Alkoxycarbonyl, (C₆-C₁₄)-Arylcarbonyl, (C₆-C₁₄)-Aryl-(C₁-C₈)-alkylcarbonyl oder (C₆-C₁₄)-Aryl-(C₁-C₁₈)-alkyloxycarbonyl, wobei die Alkylgruppen gegebenenfalls durch eine Aminogruppe substituiert und/oder wobei die Arylreste ein- oder mehrfach, vorzugsweise einfach, durch gleiche oder verschiedene Reste aus der Reihe (C₁-C₈)-Alkyl, (C₁-C₈)-Alkoxy, Halogen, Nitro, Amino und Trifluormethyl substituiert sein können, einen natürlichen oder unnatürlichen Aminosäure-, Iminosäure-, gegebenenfalls N-(C₁-C₈)-alkylierten oder N-((C₆-C₁₄₎-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, bedeutet;
- R⁸: Wasserstoff, (C₁-C₁₈)-Alkyl, gegebenenfalls substituiertes (C₆-C₁₄)-Aryl oder (C₆-C₁₄)-Aryl-(C₁-C₈)-alkyl, das im Arylrest auch substituiert sein kann, bedeutet;
- R⁹: Wasserstoff, Aminocarbonyl, (C₁-C₁₈)-Alkylaminocarbonyl, (C₃-C₈)-Cycloalkylaminocarbonyl, gegebenenfalls substituiertes (C₆-C₁₄)-Arylaminocarbonyl, (C₁-C₁₈)-Alkyl, gegebenenfalls substituiertes (C₆-C₁₄)-Aryl oder (C₃-C₈)-Cycloalkyl bedeutet;
- R¹⁰: Hydroxy, (C₁-C₁₈)-Alkoxyl (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
- R¹¹: Wasserstoff, (C₁-C₁₈)-Alkyl, R¹²CO_{,} gegebenenfalls substituiertes (C₆-C₁₄)-Aryl-S(O)₂, (C₁-C₁₈)-Alkyl-S(O)₂, im Arylrest gegebenenfalls substituiertes (C₆-C₁₄)-Aryl-(C₁-C₈)-alkyl oder R⁹NHS(O)₂ bedeutet;
- R¹²: Wasserstoff, (C₁-C₁₈)-Alkyl, (C₂-C₈)-Alkenyl, (C₂-C₈)-Alkinyl, gegebenenfalls substituiertes (C₆-C₁₄)-Aryl, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
- R¹³: Wasserstoff, (C₁-C₆)-Alkyl, im Arylrest gegebenenfalls substituiertes (C₆-C₁₄)-Aryl-(C₁-C₈)-alkyl oder (C₃-C₈)-Cycloalkyl bedeutet;
- R¹⁴: Wasserstoff oder (C₁-C₂₈)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-((C₁-C₁₈)-alkyl)-aminocarbonyl, Amino-(C₂-C₁₈)-alkylaminocarbonyl, Amino-(C₁-C₃)-alkylphenyl-(C₁ -C₃)-alkylaminocarbonyl, (C₁-C₁₈) Alkylcarbonylamino-(C₁-C₃)-alkylphenyl-(C₁-C₃)-alkylaminocarbonyl, (C₁-C₁₈)-Alkylcarbonyl-amino-(C₂-C₁₈)-alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)-Alkoxycarbonyl, gegebenenfalls substituiertes (C₃-C₈)-Cycloalkyl, HOS(O)₂-(C₁-C₃)-alkyl, R⁹NHS(O)₂-(C₁-C₃)-alkyl, (R⁸O)₂P(O)-(C₁-C₃)-alkyl, Tetrazolyl-(C₁-C₃)-alkyl. Halogen, Nitro, Trifluormethyl und R⁵ substituiert sein kann;
- R¹⁵: für R¹⁶-(C₁-C₆)-alkyl oder für R¹⁶ steht;
- R¹⁶: für einen 6- bis 24-gliedrigen bicyclischen oder tricyclischen Rest steht, der gesättigt oder teilweise ungesättigt ist und der auch ein bis vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann;
- b, c, d und f: unabhängig voneinander für 0 oder 1 stehen, aber nicht alle gleichzeitig
- 0: sein können;
- e, g und h: unabhängig voneinander für 0, 1, 2, 3, 4, 5 oder 6 stehen;
in allen ihren stereoisomeren Formen und Mischungen davon in allen Verhältnissen sowie ihrer physiologisch verträglichen Salze zur Herstellung von Arzneimitteln zur Hemmung der Adhäsion und/oder Migration von Leukozyten oder zur Hemmung des VLA-4-Rezeptors, also von Arzneimitteln zur Behandlung oder Prophylaxe von Krankheiten, bei denen die Leukozytenadhäsion und/oder Leukozytenmigration ein unerwünschtes Ausmaß aufweist, oder von Krankheiten, bei denen VLA-4-abhängige Adhäsionsvorgänge eine Rolle spielen, zum Beispiel von Entzündungserkrankungen, sowie die Verwendung der Verbindungen der Formel I bei der Behandlung und Prophylaxe derartiger Krankheiten.

Alkylreste können geradkettig oder verzweigt sein. Dies gilt auch, wenn sie Substituenten tragen oder als Substituenten anderer Reste auftreten, beispielsweise in Alkoxy-, Alkoxycarbonyl- oder Aralkylresten. Entsprechendes gilt für Alkylenreste. Beispiele für geeignete (C₁-C₂₈)-Alkylreste sind: Methyl, Ethyl, Propyl, Butyl, Pentyl, Hexyl, Heptyl, Octyl, Decyl, Undecyl, Dodecyl, Tridecyl, Pentadecyl, Hexadecyl, Heptadecyl, Nonadecyl, Eicosyl, Docosyl, Tricosyl, Pentacosyl, Hexacosyl, Heptacosyl, Octacosyl, Isopropyl, Isobutyl, Isopentyl, Neopentyl, Isohexyl, 3-Methylpentyl, 2,3,5-Trimethylhexyl, sec-Butyl, tert-Butyl, tert-Pentyl. Bevorzugte Alkylreste sind Methyl, Ethyl, Propyl, Isopropyl, Butyl, Isobutyl, sec-Butyl und tert-Butyl. Beispiele für Alkylenreste sind Methylen, Ethylen, Tri-, Tetra-, Penta- und Hexamethylen oder durch einen Alkylrest substituiertes Methylen, zum Beispiel Methylen, das durch eine Methylgruppe, eine Ethylgruppe, eine Isopropylgruppe, eine Isobutylgruppe oder eine tert-Butylgruppe substituiert ist.

Auch Alkenyl- und Alkenylenrest sowie Alkinylreste können geradkettig oder verzweigt sein. Beispiele für Alkenylreste sind Vinyl, 1-Propenyl, 2-Propenyl (=Allyl), Butenyl, 3-Methyl-2-butenyl, für Alkenylenreste Vinylen oder Propenylen, für Alkinylreste Ethinyl, 1-Propinyl oder 2-Propinyl (=Propargyl).

Cycloalkylreste sind insbesondere Cyclopropyl, Cyclobutyl, Cyclopentyl, Cyclohexyl, Cycloheptyl, Cyclooctyl, Cyclononyl, Cyclodecyl, Cycloundecyl und Cyclododecyl, die aber auch durch beispielsweise durch (C₁-C₄)-Alkyl substituiert sein können. Als Beispiele für substituierte Cycloalkylreste seien 4-Methylcyclohexyl und 2,3-Dimethylcyclopentyl genannt. Analoges gilt für Cycloalkylenreste.

Die für R¹⁶ stehenden 6- bis 24-gliedrigen bicyclischen und tricyclischen Reste werden formel durch Abstraktion eines Wasserstoffatoms aus Bicyclen bzw. Tricyclen erhalten. Die zugrunde liegenden Bicyclen und Tricyclen können als Ringglieder nur Kohlenstoffatome enthalten, es kann sich also um Bicycloalkane oder Tricycloalkane handeln, sie können aber auch ein bis vier gleiche oder verschiedene Heteroatome aus der Reihe Stickstoff, Sauerstoff und Schwefel enthalten, es kann sich also um Aza-, Oxa- und Thiabicyclo- und -tricycloalkane handeln. Sind Heteroatome enthalten, so sind bevorzugt ein oder zwei Heteroatome, insbesondere Stickstoff- oder Sauerstoffatome, enthalten. Die Heteroatome können beliebige Positionen im bi- bzw. tricyclischen Gerüst einnehmen, sie können sich in den Brücken oder im Falle von Stickstoffatomen auch an den Brückenköpfen befinden. Sowohl die Bicyclo- und Tricycloalkane als auch ihre Hetero-Analoga können vollständig gesättigt sein oder eine oder mehrere Doppelbindungen enthalten; bevorzugt enthalten sie eine oder zwei Doppelbindungen oder sind insbesondere vollständig gesättigt. Sowohl die Bicyclound Tricycloalkane als auch die Hetero-Analoga und sowohl die gesättigten als auch die ungesättigten Vertreter können unsubstituiert sein oder in beliebigen geeigneten Positionen durch eine oder mehrere Oxogruppen undloder eine oder mehrere gleiche oder verschiedene (C₁-C₄)-Alkylgruppen, zum Beispiel Methyloder Isopropylgruppen, bevorzugt Methylgruppen, substituiert sein. Die freie Bindung des bi- oder tricyclischen Restes kann sich in einer beliebigen Position des Moleküls befinden, der Rest kann also über ein Brückenkopfatom oder ein Atom in einer Brücke gebunden sein. Die freie Bindung kann sich auch in einer beliebigen stereochemischen Position befinden, beispielsweise in einer exo- oder einer endo-Position.

Beispiele für Grundkörper bicyclischer Ringsysteme, von denen sich ein bicyclischer Rest ableiten kann, sind das Norbornan (= Bicyclo[2.2.1]heptan), das Bicyclo[2.2.2]octan und das Bicyclo[3.2.1]octan, Beispiele für Heteroatome enthaltende Systeme, ungesättigte oder substituierte Systeme sind das 7-Azabicylo[2.2.1]heptan, das Bicyclo[2.2.2.]oct-5-en ]oct-5-en und der Campher (= 1,7,7-Trimethyl-2-oxobicyclo[2.2.1]heptan).

Beispiele für Systeme, von denen sich ein tricyclischer Rest ableiten kann, sind das Twistan (= Tricyclo[4.4.0.0^{3,8}]decan), das Adamantan (= Tricyclo[3.3.1.1^{3,7}]decan), das Noradamantan (= Tricyclo[3.3.1.0^{3,7}]nonan), das Tricyclo[2.2.1.0^{2,6}]heptan, das Tricyclo[5.3.2.0^{4,9}]dodecan, das Tricyclo[5.4.0.0^{2,9}]undecan oder das Tricyclo[5.5.1.0^{3,11}]tridecan.

Bevorzugt leiten sich bicyclische oder tricyclische Reste von verbrückten Bicyclen bzw. Tricyclen ab, also von Systemen, in denen Ringe zwei oder mehr als zwei Atome gemeinsam haben. Bevorzugt sind weiterhin auch bicyclische oder tricyclische Reste mit 6 bis 18 Ringgliedern, besonders bevorzugt solche mit 7 bis 12 Ringgliedern.

Im einzelnen besonders bevorzugte bi- und tricyclische Reste sind der 2-Norbornylrest, sowohl derjenige mit der freien Bindung in der exo-Position als auch derjenige mit der freien Bindung in der endo-Position, der 2-Bicyclo[3.2.1]octylrest, der 1-Adamantylrest, der 2-Adamantylrest und der Noradamantylrest, zum Beispiel der 3-Noradamantylrest. Darüber hinaus bevorzugt sind der 1- und der 2-Adamantylrest.

(C₆-C₁₄)-Arylgruppen sind beispielsweise Phenyl, Naphthyl, Biphenylyl, Anthryl oder Fluorenyl, wobei 1-Naphthyl, 2-Naphthyl und insbesondere Phenyl bevorzugt sind. Arylreste, insbesondere Phenylreste, können ein- oder mehrfach, bevorzugt ein-, zwei- oder dreifach, durch gleiche oder verschiedene Reste aus der Reihe (C₁-C₈)-Alkyl, insbesondere (C₁-C₄)-Alkyl, (C₁-C₈)-Alkoxy, insbesondere (C₁-C₄)-Alkoxy, Halogen, Nitro, Amino, Trifluormethyl, Hydroxy, Methylendioxy, Ethylendioxy, Cyano, Hydroxycarbonyl, Aminocarbonyl, (C₁-C₄)-Alkoxycarbonyl, Phenyl, Phenoxy, Benzyl, Benzyloxy, (R⁸O)₂P(O), (R⁸O)₂P(O)-O-, Tetrazolyl substituiert sein. Entsprechendes gilt beispielsweise für Reste wie Aralkyl oder Arylcarbonyl. Aralkylreste sind insbesondere Benzyl sowie 1- und 2-Naphthylmethyl, 2-, 3- und 4-Biphenylylmethyl und 9-Fluorenylmethyl, die auch substituiert sein können. Substituierte Aralkylreste sind beispielsweise durch einen oder mehrere (C₁-C₈)-Alkylreste, insbesondere (C₁-C₄)-Alkylreste, im Arylteil substituiertes Benzyl und Naphthylmethyl, zum Beispiel 2-, 3- und 4-Methylbenzyl, 4-lsobutylbenzyl, 4-tert-Butylbenzyl, 4-Octylbenzyl, 3,5-Dimethylbenzyl, Pentamethylbenzyl, 2-, 3-, 4-, 5-, 6-, 7- und 8-Methyl-1-naphthylmethyl, 1-, 3-, 4-, 5-, 6-, 7- und 8-Methyl-2-naphthylmethyl, durch einen oder mehrere (C₁-C₈)-Alkoxyreste, insbesondere (C₁-C₄)-Alkoxyreste, im Arylteil substituiertes Benzyl und Naphthylmethyl, zum Beispiel 4-Methoxybenzyl, 4-Neopentyloxybenzyl, 3,5-Dimethoxybenzyl, 3,4-Methylendioxybenzyl, 2,3,4-Trimethoxybenzyl, weiter 2-, 3- und 4-Nitrobenzyl, Halobenzyl, zum Beispiel 2-, 3- und 4-Chlor- und 2-, 3-, und 4-Fluorbenzyl, 3,4-Dichlorbenzyl, Pentafluorbenzyl, Trifluormethylbenzyl, zum Beispiel 3- und 4-Trifluormethylbenzyl oder 3,5-Bis(trifluormethyl)benzyl. Substituierte Aralkylreste können aber auch unterschiedliche Substitutenten aufweisen. Beispiele für Pyridyl sind 2-Pyridyl, 3-Pyridyl und 4-Pyridyl.

In monosubstituierten Phenylresten kann sich der Substituent in der 2-, der 3- oder der 4-Position befinden, wobei die 3- und die 4-Position bevorzugt sind. Ist Phenyl zweifach substituiert, können die Substituenten in 1,2-, 1,3- oder 1,4-Position zueinander stehen. Zweifach substituiertes Phenyl kann also in der 2,3-Position, der 2,4-Position, der 2,5-Position, der 2,6-Position, der 3,4-Position oder der 3,5-Position, bezogen auf die Verknüpfungstelle, substituiert sein. Bevorzugt sind in zweifach substituierten Phenylresten die beiden Substituenten in der 3-Position und der 4-Position, bezogen auf die Verknüpfungsstelle, angeordnet. Entsprechendes gilt für Phenylenreste, die zum Beispiel als 1,4-Phenylen oder als 1,3-Phenylen vorliegen können.

Phenylen-(C₁-C₆)-alkyl ist insbesondere Phenylenmethyl und Phenylenethyl. Phenylen-(C₂-C₆)-alkenyl ist insbesondere Phenylenethenyl und Phenylenpropenyl.

Mono- oder bicyclische 5- bis 12-gliedrige heterocyclische Ringe sind beispielsweise Pyrrolyl, Furyl, Thienyl, Imidazolyl, Pyrazolyl, Oxazolyl, Isoxazolyl, Thiazolyl, Isothiazolyl, Tetrazolyl, Pyridyl, Pyrazinyl, Pyrimidinyl, Indolyl, Isoindolyl, Indazolyl, Phthalazinyl, Chinolyl, Isochinolyl, Chinoxalinyl, Chinazolinyl, Cinnolinyl, oder ein benzanelliertes, cyclopenta-, cyclohexa- oder cyclohepta-anelliertes Derivat dieser Reste.

Diese Heterocyclen können, soweit nicht anders angegeben, an einem Stickstoffatom durch (C₁-C₇)-Alkyl, zum Beispiel Methyl oder Ethyl, Phenyl oder Phenyl-(C₁-C₄)-alkyl, zum Beispiel Benzyl, und/oder an einem oder mehreren Kohlenstoffatomen durch (C₁-C₄)-Alkyl, Halogen, Hydroxy, (C₁-C₄)-Alkoxy, zum Beispiel Methoxy, Phenyl-(C₁-C₄)-alkoxy, zum Beispiel Benzyloxy, oder Oxo substituiert sein und aromatisch oder teilweise oder vollständig gesättigt sein. Stickstoffheterocyclen können auch als N-Oxide vorliegen.

Die Reste von aromatischen Heterocyclen, also Heteroarylreste, enthalten bevorzugt einen 5-Ring-Heterocyclus oder 6-Ring-Heterocyclus mit einem, zwei, drei oder vier, insbesondere einem oder zwei, gleichen oder verschiedenen Heteroatomen aus der Reihe Stickstoff, Sauerstoff und Schwefel, der auch anelliert sein kann, beispielsweise benz-anelliert, und der durch einen oder mehrere, zum Beispiel ein, zwei, drei oder vier, gleiche oder verschiedene Substituenten substituiert sein kann. Als Substituenten kommen beispielsweise in Betracht (C₁-C₈)-Alkyl, insbesondere (C₁-C₄)-Alkyl, (C₁-C₈)-Alkoxy, insbesondere (C₁-C₄)-Alkoxy, Halogen, Nitro, Amino, Trifluormethyl, Hydroxy, Methylendioxy, Ethylendioxy, Cyano, Hydroxycarbonyl, Aminocarbonyl, (c₁-C₄)-Alkoxycarbonyl, Phenyl, Phenoxy, Benzyl, Benzyloxy, (R⁸O)₂P(O), (R⁸O)₂P(O)-O- oder Tetrazolyl.

Beispiele für heterocyclische Reste sind 2- oder 3-Pyrrolyl, Phenylpyrrolyl, zum Beispiel 4- oder 5-Phenyl-2-pyrrolyl, 2-Furyl, 3-Furly, 2-Thienyl, 3-Thienyl, 4-Imidazolyl, Methylimidazolyl, zum Beispiel 1-Methyl-2-, -4- oder -5-imidazolyl, 1,3-Thiazol-2-yl, 2-Pyridyl, 3-Pyridyl, 4-Pyridyl, 2-, 3- oder 4-Pyridyl-N-oxid, 2-Pyrazinyl, 2-, 4- oder 5-Pyrimidinyl, 2-, 3- oder 5-Indolyl, substituiertes 2-Indolyl, zum Beispiel 1-Methyl-, 5-Methyl-, 5-Methoxy-, 5-Benzyloxy-, 5-Chlor- oder 4,5-Dimethyl-2-indolyl, 1-Benzyl-2- oder -3-indolyl, 4,5,6,7-Tetrahydro-2-indolyl, Cydohepta[b]-5-pyrrolyl, 2-, 3- oder 4-Chinolyl, 1-, 3- oder 4-Isochinolyl, 1-Oxo-1,2-dihydro-3-isochinolyl, 2-Chinoxalinyl, 2-Benzofuranyl, 2-Benzothienyl, 2-Benzoxazolyl oder Benzothiazolyl. Teilhydrierte oder vollständig hydrierte heterocyclische Ringe sind beispielsweise Dihydropyridinyl, Pyrrolidinyl, zum Beispiel 2-, 3- oder 4-(N-Methylpyrrolidinyl), Piperazinyl, Morpholinyl, Thiomorpholinyl, Tetrahydrothienyl, Benzodioxolanyl.

Halogen steht für Fluor, Chlor, Brom oder Iod, insbesondere für Fluor oder Chlor.

Natürliche oder unnatürliche Aminosäuren können, falls chiral, in der D- oder L-Form vorliegen. Bevorzugt sind α-Aminosäuren. Beispielsweise seien genannt (vgl. Houben-Weyl, Methoden der organischen Chemie, Band 15/1 und 15/2, Georg Thieme Verlag, Stuttgart, 1974):

Aad, Abu, γAbu, ABz, 2ABz, εAca, Ach, Acp, Adpd, Ahb, Aib, βAib, Ala, βAla, ΔAla, Alg, All, Ama, Amt, Ape, Apm, Apr, Arg, Asn, Asp, Asu, Aze, Azi, Bai, Bph, Can, Cit, Cys, (Cys)₂, Cyta, Daad, Dab, Dadd, Dap, Dapm, Dasu, Djen, Dpa, Dtc, Fel, Gln, Glu, Gly, Guv, hAla, hArg, hCys, hGln, hGlu, His, hIle, hLeu, hLys, hMet, hPhe, hPro, hSer, hThr, hTrp, hTyr, Hyl, Hyp, 3Hyp, Ile, Ise, Iva, Kyn, Lant, Lcn, Leu, Lsg, Lys, βLys, ΔLys, Met, Mim, Min, nArg, Nle, Nva, Oly, Orn, Pan, Pec, Pen, Phe, Phg, Pic, Pro, ΔPro, Pse, Pya, Pyr, Pza, Qin, Ros, Sar, Sec, Sem, Ser, Thi, βThi, Thr, Thy, Thx, Tia, Tle, Tly, Trp, Trta, Tyr, Val, tert-Butylglycin (Tbg), Neopentylglycin (Npg), Cyclohexylglycin (Chg), Cyclohexylalanin (Cha), 2-Thienylalanin (Thia), 2,2-Diphenylaminoessigsäure, 2-(p-Tolyl)-2-(p-Tolyl)-2-phenylaminoessigsäure, 2-(p-Chlorphenyl)-aminoessigsäure.

Unter Aminosäureseiten werden Seitenketten von natürlichen oder unnatürlichen Aminosäuren verstanden. Azaaminosäuren sind natürliche oder unnatürliche Aminosäuren, in denen der Zentralbaustein 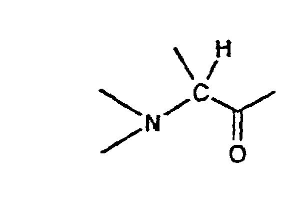 durch 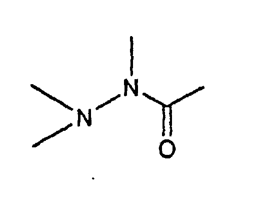 ersetzt ist

Als Rest einer Iminosäure kommen insbesondere Reste von Heterocyclen aus der folgenden Gruppe in Betracht: Pyrrolidin-2-carbonsäure; Piperidin-2-carbonsäure; 1,2,3,4-Tetrahydroisochinolin-3-carbonsäure; -3-carbonsäure; Decahydroisochinolin-3-carbonsäure; Octahydroindol-2-carbonsäure; Decahydrochinolin-2-carbonsäure; Octahydrocyclopenta[b]pyrrol-2-carbonsäure; -carbonsäure; 2-Azabicyclo[2.2.2]octan-3-carbonsäure; 2-Azabicyclo[2.2.1]heptan-3-carbonsäure; 2-Azabicyclo[3.1.O]hexan-3-carbonsäure; 2-Azaspiro[4.4]nonan-3-carbonsäure; 2-Azaspiro[4.5]decan-3-carbonsäure; Spiro(bicyclo[2.2.1]heptan)-2,3-pyrrolidin-5-carbonsäure; Spiro(bicyclo[2.2.2]octan)-2,3-pyrrolidin-5-carbonsäure; 22-Azatricyclo[4.3.0.1^{6,9}]decan-3-carbonsäure; Decahydrocyctohepta[b]pyrrol-2-carbonsäure; Decahydrocycloocta[c]pyrrol-2-carbonsäure; Octahydrocyclopenta[c]pyrrol-2-carbonsäure; Octahydroisoindol-1-carbonsäure; 2,3,3a,4,6a-Hexahydrocyclopenta[b]pyrrol-2-carbonsäure; 2,3,3a,4,5,7a-Hexahydroindol-2-carbonsäure; Tetrahydrothiazol-4-carbonsäure; Isoxazolidin-3-carbonsäure; Pyrazolidin-3-carbonsäure, Hydroxypyrrolidin-2-carbonsäure, die alle gegebenenfalls substituiert sein können (siehe folgende Formein):

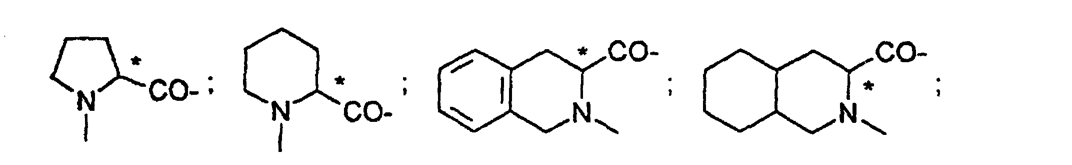

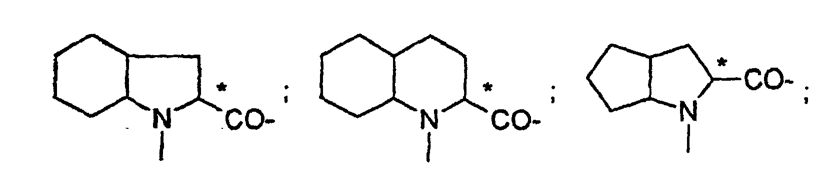

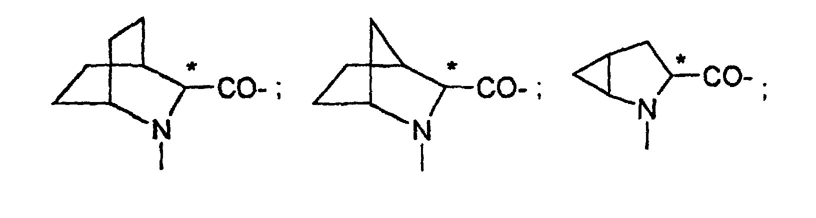

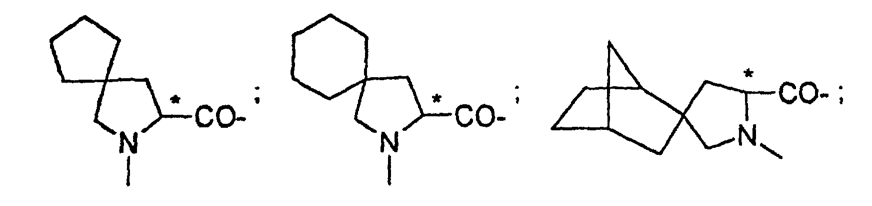

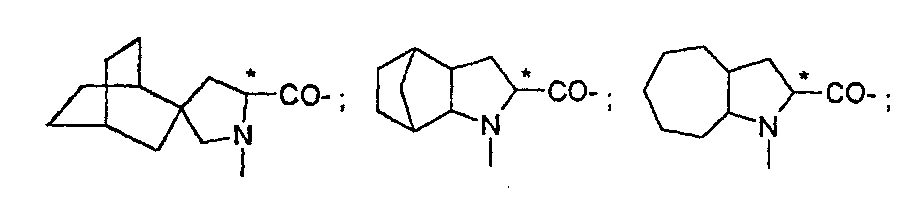

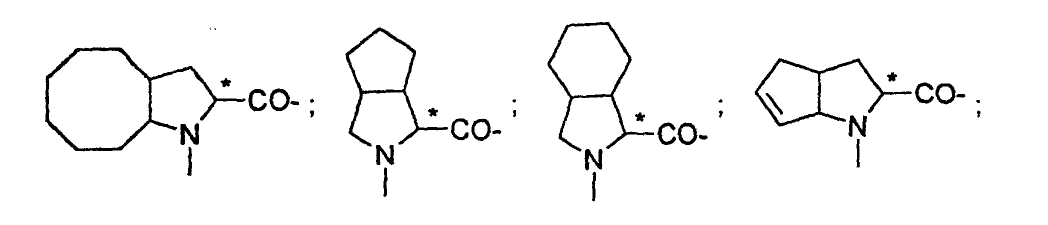

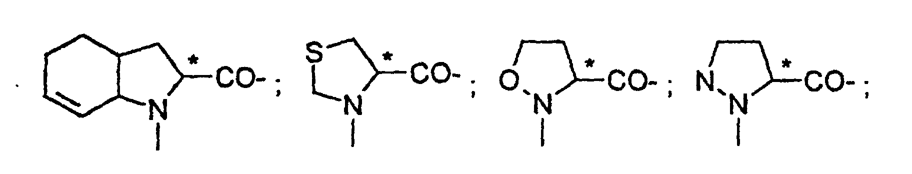

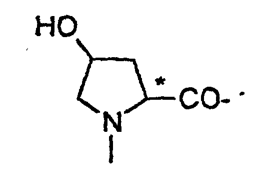

Die oben genannten Resten zugrundeliegenden Heterocyclen sind beispielsweise bekannt aus US-A-4,344,949; US-A 4,374,847; US-A 4,350,704; EP-A 29,488; EP-A 31,741; EP-A 46,953; EP-A 49,605; EP-A 49,658; EP-A 50,800; EP-A 51,020; EP-A 52,870; EP-A 79,022; EP-A 84,164; EP-A 89,637; EP-A 90,341; EP-A 90,362; EP-A 105,102; EP-A 109,020; EP-A 111,873; EP-A 271,865 und EP-A 344,682.

Dipeptide können als Bausteine natürliche oder unnatürliche Aminosäuren, Iminosäuren sowie Azaaminosäuren enthalten. Ferner können die natürlichen oder unnatürlichen Aminosäuren, Iminosäuren, Azaaminosäuren und Dipeptide auch als Ester bzw. Amide vorliegen, wie zum Beispiel als Methylester, Ethylester, Isopropylester, Isobutylester, tert-Butylester, Benzylester, unsubstituiertes Amid, Ethylamid, Semicarbazid oder ω-Amino-(C₂-C₈)-alkylamid.

Funktionelle Gruppen der Aminosäuren, Iminosäuren und Dipeptide können geschützt vorliegen. Geeignete Schutzgruppen wie zum Beispiel Urethanschutzgruppen, Carboxylschutzgruppen und Seitenkettenschutzgruppen sind bei Hubbuch, Kontakte (Merck) 1979, Nr.3, Seiten 14 bis 23, und bei Büllesbach, Kontakte (Merck) 1980, Nr. 1, Seiten 23 bis 35, beschrieben. Insbesondere seien genannt: Aloc, Pyoc, Fmoc, Tcboc, Z, Boc, Ddz, Bpoc, Adoc, Msc, Moc, Z(NO₂), Z(Halₙ), Bobz, Iboc, Adpoc, Mboc, Acm, tert-Butyl, OBzl, ONbzl, OMbzl, Bzl, Mob, Pic, Trt.

Physiologisch verträgliche Salze der Verbindungen der Formel I sind insbesondere pharmazeutisch verwendbare oder nicht-toxische Salze.

Solche Salze werden beispielsweise von Verbindungen der Formel 1, welche saure Gruppen, zum Beispiel Carboxy, enthalten, mit Alkali- oder Erdalkalimetallen gebildet, wie zum Beispiel Na, K, Mg und Ca, sowie mit physiologisch verträglichen organischen Aminen, wie zum Beispiel Triethylamin, Ethanolamin oder Tris-(2-hydroxy-ethyl)-amin.

Verbindungen der Formel I, welche basische Gruppen, zum Beispiel eine Aminogruppe, eine Amidinogruppe oder eine Guanidinogruppe enthalten, bilden mit anorganischen Säuren, wie zum Beispiel Salzsäure, Schwefelsäure oder Phosphorsäure, und mit organischen Carbon- oder Sulfonsäuren, wie zum Beispiel Essigsäure, Citronensäure, Benzoesäure, Maleinsäure, Fumarsäure, Weinsäure, Methansulfonsäure oder p-Toluolsulfonsäure Salze.

Salze können aus den Verbindungen der Formel I nach üblichen, dem Fachmann bekannten Verfahren erhalten werden, beispielsweise durch Vereinigung mit einer organischen oder anorganischen Säure oder Base in einem Lösungsmittel oder Dispergiermittel, oder auch durch Anionenaustausch oder Kationenaustausch aus anderen Salzen.

Die Verbindungen der Formel I können in stereoisomeren Formen vorliegen. Enthalten die Verbindungen der Formel I ein oder mehrere Asymmetriezentren, so können diese unabhängig voneinander die S-Konfiguration oder die R-Konfiguration aufinreisen. Zur Erfindung gehören alle möglichen Stereoisomeren, zum Beispiel Enantiomere und Diastereomere, und Mischungen von zwei oder mehr stereoisomeren Formen, zum Beispiel Mischungen von Enantiomeren und/oder Diastereomeren, in allen Verhältnissen. Enantiomere sind also in enantiomerenreiner Form, sowohl als links- als auch als rechtsdrehende Antipoden, in Form von Racematen und in Form von Mischungen der beiden Enantiomeren in allen Verhältnissen Gegenstand der Erfindung. Bei Vorliegen einer cisltranslsomerie sind sowohl die cis-Form als auch die trans-Form und Mischungen dieser Formen Gegenstand der Erfindung.

Die erfindungsgemäßen Verbindungen der Formel I können darüber hinaus bewegliche Wasserstoffatome enthalten, also in verschiedenen tautomeren Formen vorliegen. Auch alle diese Tautomeren sind Gegenstand der vorliegenden Erfindung. Die vorliegende Erfindung umfaßt weiterhin alle Solvate von Verbindungen der Formel I, zum Beispiel Hydrate oder Addukte mit Alkoholen, sowie Derivate der Verbindungen der Formel I, zum Beispiel Ester, Pro-Drugs und Metabolite, die wie die Verbindungen der Formel I wirken.

Die einzelnen Strukturelemente in der Formel I haben bevorzugt die folgenden Bedeutungen.
W steht bevorzugt für R¹-A-C(R¹³).
A steht bevorzugt für Methylen, Ethylen, Trimethylen, Tetramethylen, Pentamethylen, Cyclohexylen, Phenylen, Phenylenmethyl oder Phenylenethyl.
Y steht bevorzugt für eine Carbonylgruppe.
Z steht bevorzugt für N(R⁰).
B steht bevorzugt für Methylen, Ethylen, Trimethylen, Tetramethylen, Vinylen, Phenylen oder für substituiertes Methylen oder Ethylen. Besonders bevorzugt steht B für einen zweiwertigen Methylenrest oder Ethylenrest (= 1,2-Ethylen), wobei jeder dieser Reste unsubstituiert oder substituiert sein kann, insbesondere für einen unsubstituierten oder substituierten Methylenrest. Ganz besonders bevorzugt sind diese beiden Reste substituiert. Ist ein für B stehender zweiwertiger Methylenrest oder Ethylenrest (= 1,2-Ethylen) substituiert, so ist er bevorzugt substituiert durch einen Rest aus der Reihe (C₁-C₈)-Alkyl, (C₂-C₆)-Alkenyl, (C₂-C₆)-Alkinyl, (C₃-C₈)-Cycloalkyl, insbesondere (C₅-C₆)-Cycloalkyl, (C₃-C₈)-Cycloalkyl-(C₁-C₄)-alkyl, insbesondere (C₅-C₆)-Gycloalkyl-(C₁-C₄)-alkyl, gegebenenfalls substituiertes (C₆-C₁₀)-Aryl, im Arylrest gegebenenfalls substituiertes (C₆-C₁₀)-Aryl-(C₁-C₄)-alkyl, gegebenenfalls substituiertes Heteroaryl oder im Heteroarylrest gegebenenfalls substituiertes Heteroaryl-(C₁-C₄)-alkyl, und er ist besonders bevorzugt substituiert durch (C₁-C₈)-Alkyl, also durch einen geradkettigen oder verzweigten Alkylrest mit 1, 2, 3, 4, 5, 6, 7 und 8 Kohlenstoffatomen.
D steht bevorzugt für C(R²)(R³).
E steht bevorzugt für R¹⁰CO.
R steht bevorzugt für Wasserstoff, (C₁-C₆)-Alkyl oder Benzyl, insbesondere Wasserstoff, Methyl oder Ethyl.
R⁰ steht bevorzugt für (C₁-C₈)-Alkyl, (C₃-C₈)-Cycloalkyl, (C₃-C₈)-Cycloalkyl-(C₁-C₄)-alkyl, gegebenenfalls substituiertes (C₆-C₁₄)-Aryl oder im Arylrest gegebenenfalls substituiertes (C₆-C₁₄)-Aryl-(C₁-C₈)-alkyl. R⁰ steht besonders bevorzugt für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl, ganz besonders bevorzugt für gegebenenfalls substituiertes (C₆-C₁₄)-Aryl oder im Arylrest gegebenenfalls substituiertes (C₆-C₁₄)-Aryl-(C₁-C₈)-alkyl, darüber hinaus bevorzugt für im Arylrest gegebenenfalls substituiertes (C₆-C₁₄)-Aryl-(C₁-C₄)-alkyl. Speziell bevorzugt ist es, wenn R⁰ für unsubstituiertes oder im Arylrest einfach oder mehrfach substituiertes Biphenylylmethyl, Naphthylmethyl oder Benzyl steht.
R¹ steht bevorzugt für X-NH-C(=NH), X-NH-(C=NX)-NH oder X-NH-CH₂.
X und X¹ stehen bevorzugt für Wasserstoff, (C₁-C₆)-Alkylcarbonyl, (C₁-C₆)-Alkoxycarbonyl, (C₁-C₁₈)-Alkylcarbonyloxy-(C₁-C₆)-alkoxycarbonyl oder (C₆-C₁₄)-Aryl-(C₁-C₆)-alkoxycarbonyl, Hydroxy, X¹ zudem für R'-NH-C(=N-R''), wobei R' und R" unabhängig voneinander die bevorzugten Bedeutungen von X haben.
R² steht bevorzugt für Wasserstoff oder (C₁-C₈)-Alkyl.
R³ steht bevorzugt für (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)-Cycloalkyl, (C₂-C₈)-Alkenyl, (C₂-C₈)-Alkinyl, Pyridyl, R¹¹ NH, R⁴CO, COOR⁴, CON(CH₃)R¹⁴, CONHR¹⁴, CSNHR¹⁴, COOR¹⁵, CON(CH₃)R¹⁵ oder CONHR¹⁵, besonders bevorzugt für gegebenenfalls substituiertes (C₆-C₁₄)-Aryl, R¹¹ NH, CON(CH₃)R¹⁴ oder CONHR¹⁴_{.}
R⁴ und R¹⁴ stehen bevorzugt für (C₁-C₈)-Alkyl, das gegebenenfalls wie in der Definition von R⁴ bzw. R¹⁴ angegeben substituiert sein kann.
R¹³ steht bevorzugt für Wasserstoff und insbesondere für (C₁-C₆)-Alkyl, (C₃-C₈)-Cycloalkyl oder Benzyl, wobei ein ganz besonders bevorzugter Alkylrest, für den R¹³ steht, der Methylrest ist.
R¹⁵ steht bevorzugt für R¹⁶-(C₁-C₃)-alkyl oder für R¹⁶, besonders bevorzugt für R¹⁶-(C₁)-alkyl oder R¹⁶. Darüber hinaus bevorzugt steht R¹⁵ dann, wenn R³ für COOR¹⁵ steht, für den exo-2-Norbornylrest, den endo-2-Norbornylrest oder den Bicyclo[3.2.1]octylrest, und steht R¹⁵ dann, wenn R³ für CONHR¹⁵ steht, für den exo-2-Norbornylrest, den endo-2-Norbornylrest, den 3-Noradamantylrest und insbesondere den 1-Adamantylrest, den 2-Adamantylrest, den 1-Adamantylmethylrest oder den 2-Adamantylmethylrest.
R¹⁶ steht bevorzugt für einen 7- bis 12-gliedrigen verbrückten bicyclischen oder tricyclischen Rest, der gesättigt oder teilweise ungesättigt ist und der auch ein bis vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann.
b, c und d stehen unabhängig voneinander für 1.
e, g und h stehen bevorzugt unabhängig voneinander für die Zahlen 0, 1, 2 oder 3.

Für die erfindungsgemäße Verwendung bevorzugte Verbindungen sind solche, in denen in der Formel I gleichzeitig
- W: für R¹-A-C(R¹³) oder R¹-A-CH=C steht;
- Y: für eine Carbonyl-, Thiocarbonyl- oder Methylengruppe steht;
- Z: für N(R⁰), Sauerstoff, Schwefel oder eine Methylengruppe steht;
- A: einen zweiwertigen Rest aus der Reihe (C₁-C₆)-Alkylen, (C₃-C₇)-Cycloalkylen, Phenylen, Phenylen-(C₁-C₆)-alkyl, (C₁-C₆)-Alkylen-phenyl, Phenylen-(C₂-C₆)-alkenyl oder einen zweiwertigen Rest eines 5- oder 6-gliedrigen gesättigten oder ungesättigten Ringes, der 1 oder 2 Stickstoffatome enthalten und ein- oder zweifach durch (C₁-C₆)-Alkyl oder doppelt gebundenen Sauerstoff oder Schwefel substituiert sein kann, bedeutet;
- B: einen zweiwertigen Rest aus der Reihe (C₁-C₆)-Alkylen, (C₂-C₆)-Alkenyten, Phenylen, Phenylen-(C₁-C₃)-alkyl, (C₁-C₃)-Alkylen-phenyl bedeutet;
- D: für C(R²)(R³)_{,} N(R³) oder CH=C(R³) steht;
- E: Tetrazolyl, (R⁸O)₂P(O), HOS(O)₂, R⁹NHS(O)₂ oder R¹⁰CO bedeutet;
- R und R⁰: unabhängig voneinander Wasserstoff, (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl bedeuten;
- R¹: für X-NH-C(=NH)-(CH₂)ₚ oder X¹-NH-(CH₂)ₚ steht, wobei p für 0, 1, 2 oder 3 steht;
- X: Wasserstoff, (C₁-C₆)-Alkyl, (C₁-C₆)-Alkylcarbonyl, (C₁-C₆) Alkoxycarbonyl, (C₁-C₁₈)-Alkylcarbonyloxy-(C₁-C₆)-alkoxycarbonyl, gegebenenfalls substituiertes (C₆-C₁₄)-Arylcarbonyl, gegebenenfalls substituiertes (C₆-C₁₄)-Aryloxycarbonyt, (C₆-C₁₄)-Aryl-(C₁-C₆)-alkoxycarbonyl, das im Arylrest auch substituiert sein kann, (R⁸O)₂P(O), Cyano, Hydroxy, (C₁-C₆)-Alkoxy, (C₆-C₁₄)-Aryl-(C₁-C₆)-alkoxy, das im Arylrest auch substituiert sein kann, oder Amino bedeutet;
- X¹: eine der Bedeutungen von X hat oder R'-NH-C(=N-R") bedeutet, wobei R' und R" unabhängig voneinander die Bedeutungen von X haben;
- R²: Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl oder (C₃-C₈)-Cycloalkyl bedeutet;
- R³: Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)-Cycloalkyl, (C₂-C₈)-Alkenyl, (C₂-C₈)-Alkinyl, (C₂-C₈)-Alkenylcarbonyl, (C₂-C₈)-Alkinylcarbonyl, Pyridyl, R¹¹NH, R⁴CO, COOR⁴, CON(CH₃)R¹⁴, CONHR¹⁴, CSNHR¹⁴, COOR¹⁵, CON(CH₃)R¹⁵ oder CONHR¹⁵ bedeutet;
- R⁴: Wasserstoff oder (C₁-C₂₈)-Alkyl bedeutet, das gegebenenfalls ein- oder mehrfach durch gleiche oder verschiedene Reste R^{4'} substituiert sein kann;
- R^{4'}: Hydroxy, Hydroxycarbonyl, Aminocarbonyl, Mono- oder Di-((C₁-C₁₈)-alkyl)-aminocarbonyl, Amino-(C₂-C₁₈)-alkylaminocarbonyl, Amino-(C₁-C₃)-alkylphenyl-(C₁-C₃)-alkylaminocarbonyl, (C₁-C₁₈)-Alkylcarbonylamino-(C₁-C₃)-alkylphenyl-(C₁-C₃)-alkylaminocarbonyl, (C₁-C₁₈)-Alkylcarbonylamino-(C₂-C₁₈)-alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)-Alkoxycarbonyl, gegebenenfalls substituiertes (C₃-C₈)-Cycloalkyl, Halogen, Nitro, Trifluormethyl oder den Rest R⁵ bedeutet;
- R⁵: gegebenenfalls substituiertes (C₆-C₁₄)-Aryl, im Arylrest gegebenenfalls substituiertes (C₆-C₁₄)-Aryl-(C₁-C₈)-alkyl, einen mono- oder bicyclischen 5- bis 12-gliedrigen heterocyclischen Ring, der aromatisch, teilhydriert oder vollständig hydriert sein kann und der ein, zwei oder drei gleiche oder verschiedene Heteroatame aus der Reihe Stickstoff, Sauerstoff und Schwefel enthalten kann, einen Rest R⁶ oder einen Rest R⁶CO- bedeutet, wobei der Aryl- und unabhängig davon der Heterocyclus-Rest ein- oder mehrfach durch gleiche oder verschiedene Reste aus der Reihe (C₁-C₁₈)-Alkyl, (C₁-C₁₈)-Alkoxy, Halogen, Nitro, Amino oder Trifluormethyl substituiert sein können;
- R⁶: für R⁷R⁸N, R⁷O oder R⁷S steht oder eine Aminosäureseitenkette, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, sowie deren Ester und Amide bedeutet, wobei anstelle freier funktioneller Gruppen gegebenenfalls Wasserstoff oder Hydroxymethyl stehen kann und/oder wobei freie funktionelle Gruppen durch in der Peptidchemie übliche Schutzgruppen geschützt sein können;
- R⁷: Wasserstoff, (C₁-C₁₈) A(kyl, (C₆-C₁₄)-Aryl-(C₁-C₈)-alkyl, (C₁-C₁₈)-Alkylcarbonyl, (C₁-C₁₈)-Alkoxycarbonyl, (C₆-C₁₄)-Arylcarbonyl, (C₆-C₁₄)-Aryl-(C₁-C₈)-alkylcarbonyl oder (C₆-C₁₄)-Aryl-(C₁-C₁₈)-alkyloxycarbonyl, wobei die Alkylgruppen gegebenenfalls durch eine Aminogruppe substituiert und/oder wobei die Arylreste ein- oder mehrfach, vorzugsweise einfach, durch gleiche oder verschiedene Reste aus der Reihe (C₁-C₈)-Alkyl, (C₁-C₈)-Alkoxy, Halogen, Nitro, Amino und Trifluormethyl substituiert sein können, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, bedeutet;
- R⁸: Wasserstoff, (C₁-C₁₈)-Alkyl, gegebenenfalls substituiertes (C₆-C₁₄)-Aryl oder (C₆-C₁₄)-Aryl-(C₁-C₈)-alkyl, -alkyl, das im Arylrest auch substituiert sein kann, bedeutet;
- R⁹: Wasserstoff, Aminocarbonyl, (C₁-C₁₈)-Alkylaminocarbonyl, (C₃-C₈)-Cycloalkylaminocarbonyl, gegebenenfalls substituiertes (C₆-C₁₄)-Arylaminocarbonyl, (C₁-C₁₈)-Alkyl, gegebenenfalls substituiertes (C₆-C₁₄)-Aryl oder (C₃-C₈)-Cycloalkyl bedeutet;
- R¹⁰: Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
- R¹¹: Wasserstoff, (C₁-C₁₈)-Alkyl, R¹²CO, gegebenenfalls substituiertes (C₆-C₁₄)-Aryl-S(O)₂, (C₁-C₁₈)-Alkyl-S(O)₂, -S(O)₂, im Arylrest gegebenenfalls substituiertes (C₆-C₁₄)-Aryl-(C₁-C₈)-alkyl oder R⁹NHS(O)₂ bedeutet;
- R¹²: Wasserstoff, (C₁-C₁₈)-Alkyl, (C₂-C₈)-Alkenyl, (C₂-C₈)-Alkinyl, gegebenenfalls substituiertes (C₆-C₁₄)-Aryl, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
- R¹³: Wasserstoff, (C₁-C₆)-Alkyl, im Arylrest gegebenenfalls substituiertes (C₆-C₁₄)-Aryl-(C₁-C₈)-alkyl oder (C₃-C₈)-Cycloalkyl bedeutet;
- R¹⁴: Wasserstoff oder (C₁-C₂₈)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-((C₁-C₁₈)-alkyl)-aminocarbonyl, Amino-Amino-(C₂-C₁₈)-alkylaminocarbonyl, Amino-(C₁-C₃)-alkylphenyl-(C₁-C₃)-alkylaminocarbonyl, (C₁-C₁₈)-Alkylcarbonylamino-(C₁-C₃)-alkylphenyl-(C₁-C₃)-alkylaminocarbonyl, (C₁-C₁₈)-Alkylcarbonyl-amino-(C₂-C₁₈)-alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)-Alkoxycarbonyl, gegebenenfalls substituiertes (C₃-C₈)-Cycloalkyl, HOS(O)₂-(C₁-C₃)-alkyl, R⁹NHS(O)₂-(C₁-C₃)-alkyl, (R⁸O)₂P(O)-(C₁-C₃)-alkyl, Tetrazolyl-(C₁-C₃)-alkyl, Halogen, Nitro, Trifluormethyl und R⁵ substituiert sein kann;
- R¹⁵: für R¹⁶-(C₁-C₆)-alkyl oder für R¹⁶ steht;
- R¹⁶: für einen 6- bis 24-gliedrigen bicyclischen oder tricyclischen Rest steht, der gesättigt oder teilweise ungesättigt ist und der auch ein bis vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann;
b, c, d und f unabhängig voneinander für 0 oder 1 stehen, aber nicht alle gleichzeitig 0 sein können;
e, g und h unabhängig voneinander für 0, 1, 2, 3, 4, 5 oder 6 stehen;
in allen ihren stereoisomeren Formen und Mischungen davon in allen Verhältnissen, sowie ihre physiologisch verträglichen Salze.

Besonders bevorzugte Verbindungen der Formel I sind solche, worin gleichzeitig
W für R¹-A-CH=C und darin A für einen Phenylenrest steht oder W für R¹-A-C(R¹³) und darin A für einen zweiwertigen Rest aus der Reihe Methylen, Ethylen, Trimethylen, Tetramethylen, Pentamethylen, Cyclohexylen, Phenylen, Phenylenmethyl steht;
B für einen zweiwertigen Rest aus der Reihe Methylen, Ethylen, Trimethylen, Tetramethylen, Vinylen, Phenylen oder für substituiertes Methylen oder Ethylen steht;
E R¹⁰CO bedeutet;
R Wasserstoff, (C₁-C₆)-Alkyl oder Benzyl bedeutet;
R⁰ für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
R¹ für X-NH-C(=NH), X-NH-C(=NX)-NH oder X-NH-CH₂ steht;
X für Wasserstoff, (C₁-C₆)-Alkylcarbonyl, (C₁-C₆)-Alkoxycarbonyl, (C₁-C₈)-Alkylcarbonyloxy-(C₁-C₆)-alkoxycarbonyl, (C₆-C₁₄)-Aryl-(C₁-C₆)-alkoxycarbonyl oder Hydroxy steht;
R² für Wasserstoff oder (C₁-C₈)-Alkyl steht;
R³ für (C₁-C₈)-Alkyl, gegebenenfalls substituiertes (C₆-C₁₄)-Aryl, (C₆-C₁₄)-Aryl-(C₁-C₈)-alkyl, (C₃-C₈)-Cycloalkyl, (C₂-C₈)-Alkenyl, (C₂-C₈)-Alkinyl, Pyridyl, R¹¹NH, R⁴CO, COOR⁴, CONHR¹⁴, CSNHR¹⁴, COOR¹⁵ und CONHR¹⁵ steht;
und e, g und h unabhängig voneinander für die Zahlen 0, 1, 2 oder 3 stehen;
in allen ihren stereoisomeren Formen und Mischungen davon in allen Verhältnissen, sowie ihre physiologisch verträglichen Salze.

Ganz besonders bevorzugte Verbindungen der Formel I sind solche, worin W für R¹-A-C(R¹³) und R¹³ für (C₁-C₆)-Alkyl, im Arylrest gegebenenfalls substituiertes (C₆-C₁₄)-Aryl-(C₁-C₈)-alkyl oder (C₃-C₈)-Cycloalkyl steht, in allen ihren stereoisomeren Formen und Mischungen davon in allen Verhältnissen, sowie ihre physiologisch verträglichen Salze.

Eine Reihe speziell bevorzugter Verbindungen der Formel I bilden solche, worin R³ für gegebenenfalls substituiertes (C₆-C₁₄)-Aryl, für COOR⁴, für R¹¹NH oder für CONHR¹⁴ steht, wobei -NHR¹⁴ für den Rest einer α-Aminosäure, deren ω-Amino-(C₂-C₈)-alkylamid, deren (C₁-C₈)-Alkylester oder deren (C₆-C₁₄)-Aryl-(C₁-C₄)-alkylester steht, in allen ihren stereoisomeren Formen und Mischungen davon in allen Verhältnissen, sowie ihre physiologisch verträglichen Salze. Der für -NHR¹⁴ stehende Rest einer α-Aminosäure wird formal durch Abstraktion eines Wasserstoffatoms von der Aminogruppe der Aminosäure erhalten. Speziell bevorzugt ist es in dieser Reihe, wenn R³ für CONHR¹⁴ steht, wobei -NHR¹⁴ für den Rest der α-Aminosäuren Valin, Lysin, Phenylglycin, Phenylalanin oder Tryptophan oder deren (C₁-C₈)-Alkylester oder (C₆-C₁₄)-Aryl-(C₁-C₄)-alkylester steht.

Darüber hinaus bevorzugte Verbindungen der Formel I in dieser Reihe sind solche, worin gleichzeitig
W für R¹-A-C(R¹³) steht;
Y für eine Carbonylgruppe steht;
Z für N(R⁰) steht;
A für Ethylen, Trimethylen, Tetramethylen, Pentamethylen, Cyclohexylen, Phenylen oder Phenylenmethyl steht;
B für einen unsubstituierten oder substituierten Methylenrest steht;
D für C(R²)(R³) steht;
E für R¹⁰CO steht;
R für Wasserstoff oder (C₁-C₄)-Alkyl, insbesondere Wasserstoff, Methyl oder Ethyl, steht;
R⁰ für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
R¹ für H₂N-C(=NH), H₂N-C(=NH)-NH oder H₂N-CH₂ steht;
R² für Wasserstoff steht;
R³ für den Rest CONHR¹⁴ steht;
R¹⁰ für Hydroxy oder (C₁-C₈)-Alkoxy, bevorzugt (C₁-C₄)-Alkoxy, steht;
R¹³ für (C₁-C₆)-Alkyl, (C₃-C₇)-Cycloalkyl oder Benzyl, insbesondere Methyl, steht;
R¹⁴ für Methyl steht, das durch Hydroxycarbonyl und einen Rest aus der Reihe (C₁-C₄)-Alkyl, Phenyl und Benzyl substituiert ist, oder für Methyl steht, das durch (C₁-C₈)-Alkoxycarbonyl, bevorzugt (C₁-C₄)-Alkoxycarbonyl, und einen Rest aus der Reihe (C₁-C₄)-Alkyl, Phenyl und Benzyl substituiert ist;
b, c und d für 1 stehen und e, f und g für 0 stehen;
h für 1 oder 2, bevorzugt für 1, steht;
in allen ihren stereoisomeren Formen und Mischungen davon in allen Verhältnissen, sowie ihre physiologisch verträglichen Salze.

Steht-NHR¹⁴ für einen (C₁-C₈)-Alkylester einer α-Aminosäure bzw. enthält R¹⁴ einen Alkoxycarbonylrest, so ist der Methyl-, Ethyl-, Isopropyl-, Isobutyl- oder tert-Butylester bevorzugt, steht -NHR¹⁴ für einen (C₆-C₁₄)-Aryl-(C₁-C₄)-alkylester einer α-Aminosäure, so ist der Benzylester bevorzugt.

Eine weitere Reihe speziell bevorzugter Verbindungen der Formel I bilden solche Verbindungen, worin gleichzeitig
W für R¹-A-CH=C und darin A für einen Phenylenrest steht oder W für R¹-A-C(R¹³) und darin A für einen zweiwertigen Rest aus der Reihe Methylen, Ethylen, Trimethyien, Tetramethylen, Pentamethylen, Cyclohexylen, Phenylen, Phenylenmethyl steht;
B für einen zweiwertigen Rest aus der Reihe Methylen, Ethylen, Trimethylen, Tetramethylen, Vinylen, Phenylen oder für substituiertes Methylen oder Ethylen steht;
E R¹⁰ CO bedeutet;
R Wasserstoff oder (C₁-C₆)-Alkyl bedeutet;
R⁰ für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
R¹ für X-NH-C(=NH), X-NH-C(=NX)-NH oder X-NH-CH₂ steht;
X für Wasserstoff, (C₁-C₆)-Alkylcarbonyl, (C₁-C₆)-Alkoxycarbonyl, (C₁-C₈)-Alkylcarbonyloxy-(C₁-C₆)-alkoxycarbonyl, (C₆-C₁₄)-Aryl-(C₁-C₆)-alkoxycarbonyl oder Hydroxy steht;
R² für Wasserstoff oder (C₁-C₈)-Alkyl steht;
R³ für CONHR¹⁵ oder CONHR¹⁴ steht, wobei R¹⁴ hierin für einen unsubstituierten oder durch einen oder mehrere (C₆-C₁₄)-Arylreste substituierten (C₁-C₈)-Alkylrest steht;
R¹⁵ für R¹⁶-(C₁-C₆)-Alkyl oder R¹⁶ steht, wobei R¹⁶ für einen 7- bis 12-gliedrigen verbrückten bicyclischen oder tricyclischen Rest steht, der gesättigt oder teilweise ungesättigt ist und der auch ein bis vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann, und insbesondere R¹⁵ für einen Adamantylrest oder einen Adamantylmethylrest steht;
und e, g und h unabhängig voneinander für die Zahlen 0, 1, 2 oder 3 stehen und b, c und d für 1 stehen;
in allen ihren stereoisomeren Formen und Mischungen davon in allen Verhältnissen, sowie ihre physiologisch verträglichen Salze.

Darüber hinaus bevorzugte Verbindungen der Formel I in dieser Reihe sind solche, worin gleichzeitig
W für R¹-A-C(R¹³) steht;
Y für eine Carbonylgruppe steht;
Z für N(R⁰) steht;
A für Ethylen, Trimethylen, Tetramethylen, Pentamethylen, Cyclohexylen, Phenylen oder Phenylenmethyl steht;
B für einen unsubstituierten oder substituierten Methylenrest steht;
D für C(R²)(R³) steht;
E für R¹⁰CO steht;
R für Wasserstoff oder (C₁-C₄)-Alkyl, insbesondere Wasserstoff, Methyl oder Ethyl, steht;
R⁰ für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
R¹ für H₂N-C(=NH), H₂N-C(=NH)-NH oder H₂N-CH₂ steht;
R² für Wasserstoff steht;
R³ für CONHR¹⁵ oder CONHR¹⁴ steht, wobei R¹⁴ hierin für einen unsubstituierten oder durch einen oder mehrere (C₆-C₁₀)-Arylreste substituierten (C₁-C₆)-Alkylrest steht;
R¹⁰ für Hydroxy oder (C₁-C₈)-Alkoxy, bevorzugt (C₁-C₄)-Alkoxy, steht;
R¹³ für (C₁-C₆)-Alkyl, (C₃-C₇)-Cycloalkyl oder Benzyl, insbesondere Methyl, steht;
R¹⁵ für einen Adamantylrest oder einen Adamantylmethylrest steht;
b, c und d für 1 stehen und e, f und g für 0 stehen;
h für 1 oder 2, bevorzugt für 1, steht;
in allen ihren stereoisomeren Formen und Mischungen davon in allen Verhältnissen, sowie ihre physiologisch verträglichen Salze.

Weiterhin bilden eine Reihe speziell bevorzugter Verbindungen der Formel I solche, worin gleichzeitig
W für R¹-A-C(R¹³) steht;
Y für eine Carbonylgruppe steht;
Z für N(R⁰) steht;
A für Ethylen, Trimethylen, Tetramethylen, Pentamethylen, Cyclohexylen, Phenylen, Phenylenmethyl steht;
B für einen unsubstituierten oder substiuierten Methylenrest oder Ethylenrest steht;
D für C(R²)(R³) steht;
E für R¹⁰CO steht;
R für Wasserstoff oder (C₁-C₄)-Alkyl, insbesondere Wasserstoff, Methyl oder Ethyl, steht;
R⁰ für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
R¹ für H₂N-C(=NH), H₂N-C(=NH)-NH oder H₂N-CH₂ steht;
R² für Wasserstoff steht;
R³ für einen unsubstituierten Phenylrest oder Naphthylrest, einen durch einen, zwei oder drei gleiche oder verschiedene Reste aus der Reihe (C₁-C₄)-Alkyl, (C₁-C₄)-Alkoxy, Hydroxy, Halogen, Trifluormethyl, Nitro, Methylendioxy, Ethylendioxy, Hydroxycarbonyl, (C₁-C₄)-Alkoxycarbonyl, Aminocarbonyl, Cyan, Phenyl, Phenoxy, Benzyl und Benzyloxy substituierten Phenylrest oder Naphthylrest, einen Pyridylrest, einen (C₁-C₄)-Alkylrest, einen (C₂-C₄)-Alkenylrest, einen (C₂-C₄)-Alkinylrest oder einen (C₅-C₆)-Cycloalkylrest steht, und insbesondere R³ für einen unsubstituierten oder substituierten Phenylrest oder Naphthylrest steht;
R¹⁰ für Hydroxy oder (C₁-C₈)-Alkoxy, insbesondere (C₁-C₄)-Alkoxy, steht und bevorzugt R¹⁰ für einen Rest aus der Reihe Hydroxy, Methoxy, Ethoxy, Propoxy und Isopropoxy steht;
R¹³ für (C₁-C₆)-Alkyl, (C₃-C₇)-Cycloalkyl oder Benzyl, insbesondere Methyl, steht;
b, c und d für 1 stehen und e, f und g für 0 stehen;
h für 1 oder 2, bevorzugt für 1, steht;
in allen ihren stereoisomeren Formen und Mischungen davon in allen Verhältnissen, sowie ihre physiologisch verträglichen Salze.

Schließlich bilden eine Reihe speziell bevorzugter Verbindungen der Formel I solche, worin gleichzeitig
W für R¹-A-C(R¹³) steht;
Y für eine Carbonylgruppe steht;
Z für N(R⁰) steht;
A für Ethylen, Trimethylen, Tetramethylen, Pentamethylen, Cyclohexylen, Phenylen, Phenylenmethyl steht;
B für einen unsubstituierten oder substituierten Methylenrest oder Ethylenrest steht;
D für C(R²)(R³) steht;
E für R¹⁰CO steht;
R für Wasserstoff oder (C₁-C₄)-Alkyl, insbesondere Wasserstoff, Methyl oder Ethyl, steht;
R⁰ für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
R¹ für H₂N-C(=NH), H₂N-C(=NH)-NH oder H₂N-CH₂ steht;
R² für Wasserstoff steht;
R³ für R¹¹NH steht;
R¹⁰ für Hydroxy oder (C₁-C₈)-Alkoxy, insbesondere (C₁-C₄)-Alkoxy, steht und bevorzugt R¹⁰ für einen Rest aus der Reihe Hydroxy, Methoxy, Ethoxy, Propoxy und Isopropoxy steht;
R¹³ für (C₁-C₆)-Alkyl, (C₃-C₇)-Cycloalkyl oder Benzyl, insbesondere Methyl, steht;
b, c, d und e für 1 stehen und f und g für 0 stehen;
h für 0 steht;
in allen ihren stereoisomeren Formen und Mischungen davon in allen Verhältnissen, sowie ihre physiologisch verträglichen Salze.

Ganz speziell bevorzugte Verbindungen der Formel I sind solche, worin ein für die Gruppe B stehender substituierter Methylenrest oder substituierter Ethylenrest als Substituenten einen Rest aus der Reihe (C₁-C₈)-Alkyl, (C₂-C₆)-Alkenyl, (C₂-C₆)-Alkinyl, (C₃-C₈)-Cycloalkyl, insbesondere (C₅-C₆)-Cycloalkyl, (C₃-C₈)-Cycloalkyl-(C₁-C₄)-alkyl, insbesondere (C₅-C₆)-Cycloalkyl-(C₁-C₄)-alkyl, gegebenenfalls substituiertes (C₆-C₁₀)-Aryl, im Arylrest gegebenenfalls substituiertes (C₆-C₁₀)-Aryl-(C₁-C₄)-alkyl, gegebenenfalls substituiertes Heteroaryl und im Heteroarylrest gegebenenfalls substituiertes Heteroaryl-(C₁-C₄)-alkyl trägt, in allen ihren stereoisomeren Formen und Mischungen davon in allen Verhältnissen, sowie ihre physiologisch verträglichen Salze. Noch spezieller bevorzugte Verbindungen der Formel I sind solche, worin B für einen unsubstituierten Methylenrest steht oder für einen Methylenrest steht, der durch einen (C₁-C₈)-Alkylrest, insbesondere durch einen (C₁-C₆)-Alkylrest, substituiert ist, in allen ihren stereoisomeren Formen und Mischungen davon in allen Verhältnissen, sowie ihre physiologisch verträglichen Salze.

Generell sind Verbindungen der Formel I bevorzugt, die an Chiralitätszentren, z.B an dem für D stehenden chiralen Kohlenstoffatom und an dem Zentrum W im 5-Ring-Heterocyclus in der Formel I, eine einheitliche Konfiguration aufweisen.

Die Verbindungen der Formel I können beispielsweise hergestellt werden durch Fragmentkondensation einer Verbindung der Formel II 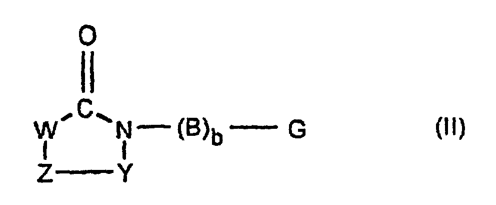 mit einer Verbindung der Formel III, 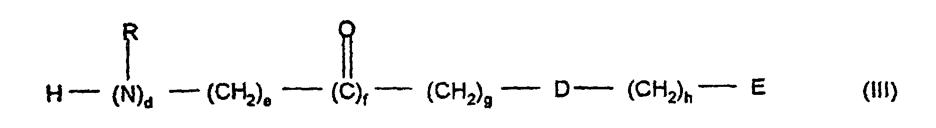 wobei W, Y, Z, B, D, E, R sowie b, d, e, f, g, und h wie oben angegeben definiert sind und G für Hydroxycarbonyl, (C₁-C₆)-Alkoxycarbonyl, aktivierte Carbonsäurederivate, wie Säurechloride oder Aktivester, oder Isocyanato steht.

Zur Kondensation der Verbindungen der Formel II mit denen der Formel III verwendet man vorteilhafterweise die an sich bekannten Kupplungsmethoden der Peptidchemie (siehe zum Beispiel Houben-Weyl, Methoden der Organischen Chemie, Band 15/1 and 15/2, Georg Thieme Verlag, Stuttgart, 1974). Dazu ist es in der Regel nötig, daß vorhandene, nicht reagierende Aminogruppen durch reversible Schutzgruppen während der Kondensation geschützt werden. Gleiches gilt für nicht an der Reaktion beteiligte Carboxylgruppen, die bevorzugt als (C₁-C₆)-Alkyl-, Benzyl- oder tert-Butylester vorliegen. Ein Aminogruppen-Schutz erübrigt sich, wenn die zu generierenden Aminogruppen noch als Nitro- oder Cyanogruppen vorliegen und erst nach der Kupplung zum Beispiel durch Hydrierung gebildet werden. Nach der Kupplung werden die vorhandenen Schutzgruppen in geeigneter Weise abgespalten. Beispielsweise können NO₂-Gruppen (Guanidinoschutz), Benzyloxycarbonylgruppen und Benzylester abhydriert werden. Die Schutzgruppen vom tert-Butyltyp werden sauer abgespalten, während der 9-Fluorenylmethyloxycarbonylrest durch sekundäre Amine entfernt wird. Die Herstellung der Verbindungen der Formel I kann beispielsweise auch erfolgen, indem man die Verbindungen nach üblichen Methoden schrittweise an einer Festphase aufbaut, wie dies weiter unten beispielhaft erläutert ist.

Verbindungen der Formel II, in der W für R¹-A-C(R¹³), Y für eine Carbonylgruppe und Z für NR⁰ steht, können beispielsweise hergestellt werden, indem man zunächst Verbindungen der Formel IV 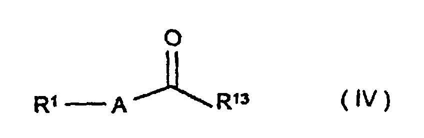 in einer Bucherer-Reaktion zu Verbindungen der Formel V, 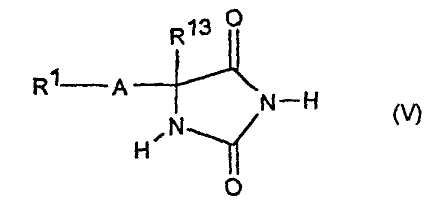 in der ebenso wie in der Formel IV R¹, R¹³ und A wie oben angegeben definiert sind, umsetzt (H. T. Bucherer, V. A. Lieb, J. Prakt. Chem. 141(1934), 5). Verbindungen der Formel VI, 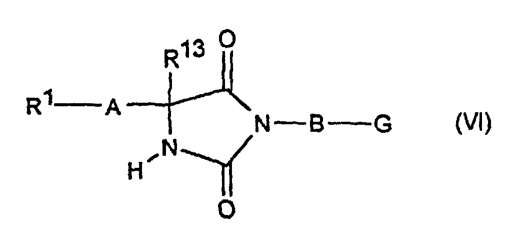 in der R¹, R¹³, A, B und G wie oben angegeben definiert sind, können dann erhalten werden, indem man die Verbindungen der Formel V beispielsweise zunächst mit einem alkylierenden Reagenz umsetzt, das den Rest -B-G in das Molekül einführt. Die Umsetzung von Verbindungen der Formel VI mit einem zweiten Reagenz der Formel R⁰-LG, in der R⁰ die oben angegebenen Bedeutungen hat und LG eine nucleophil substituierbare Abgangsgruppe, zum Beispiel Halogen, insbesondere Chlor oder Brom, (C₁-C₄)-Alkoxy, gegebenenfalls substituiertes Phenoxy oder eine heterocyclische Abgangsgruppe wie zum Beispiel Imidazolyl, darstellt, führt zu den entsprechenden Verbindungen der Formel II. Diese Umsetzungen können analog bekannten, dem Fachmann geläufigen Methoden durchgeführt werden. Je nach dem Einzelfall kann es hierbei, wie bei allen Schritten in der Synthese der Verbindungen der Formel 1, angebracht sein, funktionelle Gruppen die zu Nebenreaktionen oder unerwünschten Reaktionen führen könnten, durch eine dem Syntheseproblem angepaßte Schutzgruppenstrategie temporär zu blockieren, was dem Fachmann bekannt ist.

Steht W für R¹-A-CH=C, so kann dieses Strukturelement beispielsweise eingeführt werden, indem analog bekannten Methoden ein Aldehyd mit einem 5-Ring-Heterocyclus kondensiert wird, der eine Methylengruppe in der der Gruppe W entsprechenden Position enthält.

Verbindungen der Formel I, in denen der 5-Ring-Heterocyclus einen Dioxo- oder Thioxo-oxo-substituierten Imidazolidinring darstellt, in dem W für R¹-A-C(R¹³) steht, können auch wie folgt erhalten werden:
Durch Reaktion von α-Aminosäuren oder N-substituierten α-Aminosäuren oder bevorzugt deren Ester, zum Beispiel der Methyl-, Ethyl-, tert-Butyl- oder Benzylester, beispielsweise einer Verbindung der Formel VII, 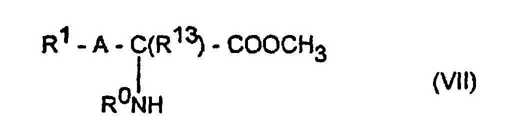 worin R⁰, R¹, R¹³ und A wie oben angegeben definiert sind, mit einem Isocyanat oder Isothiocyanat beispielsweise der Formel VIII, 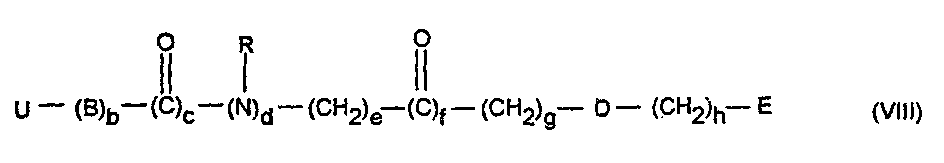 worin B, D, E und R sowie b, c, d, e, f, g und h wie oben angegeben definiert sind und U für Isocyanato oder Isothiocyanato steht, erhält man Harnstoff- oder Thioharnstoffderivate, beispielsweise der Formel IX, 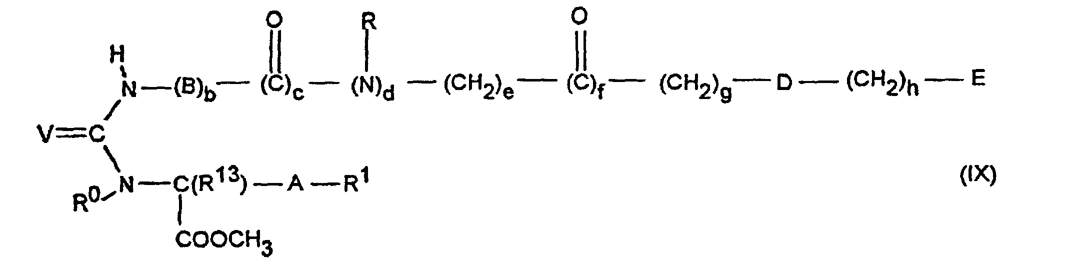 für die die oben angegebenen Definitionen gelten und in der V Sauerstoff oder Schwefel bedeutet, und die durch Erhitzen mit Säure unter Verseifung der Esterfunktionen zu Verbindungen der Formel Ia 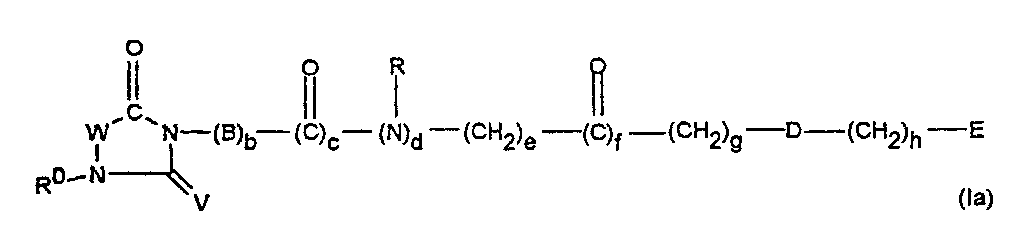 cyclisiert werden, in der V Sauerstoff oder Schwefel bedeutet, W für R¹-A-C(R¹³) steht und für die ansonsten die oben angegebenen Bedeutungen gelten. Die Cyclisierung der Verbindungen der Formel IX zu den Verbindungen der Formel Ia kann auch durch Behandlung mit Basen in inerten Lösungsmittel durchgeführt werden, zum Beispiel durch Behandlung mit Natriumhydrid in einem aprotischen Lösungsmittel wie Dimethylformamid.

Während der Cyclisierung können Guanidinogruppen durch Schutzgruppen, zum Beispiel NO₂, blockiert werden. Aminogruppen können in geschützer Form oder noch als NO₂- oder Cyanofunktion vorliegen, die später zur Aminogruppe reduziert oder im Falle der Cyanogruppe auch in die Formamidinogruppe umgewandelt werden kann.

Verbindungen der Formel I, in denen der 5-Ring-Heterocyclus einen Dioxo- oder Thioxo-oxo-substituierten Imidazolidinring darstellt, in dem W für R¹-A-C(R¹³) und c für 1 steht, können auch erhalten werden, indem man eine Verbindung der Formel VII mit einem Isocyanat oder Isothiocyanat der Formel X 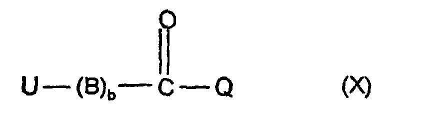 umsetzt, in der B, U und b wie oben für die Formel VIII angegeben definiert sind und Q eine Alkoxygruppe, zum Beispiel eine (C₁-C₄)-Alkoxygruppe wie Methoxy, Ethoxy oder tert-Butoxy, eine (C₆-C₁₄)-Aryloxygruppe, zum Beispiel Phenoxy, oder eine (C₆-C₁₄)-Aryl-(C₁-C₄)-alkoxygruppe, zum Beispiel Benzyloxy, bedeutet. Dabei wird eine Verbindung der Formel XI 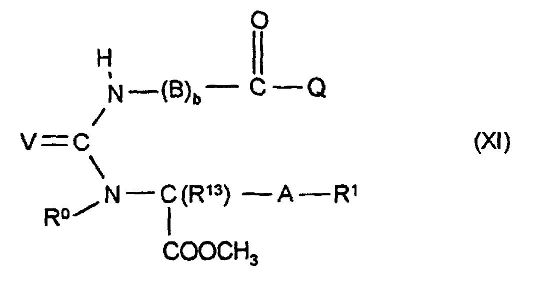 erhalten, in der A, B, V, Q, R⁰, R¹, R¹³ und b wie oben für die Formeln IX und X angegeben definiert sind, die dann unter dem Einfluß einer Säure oder einer Base, wie oben für die Cyclisierung der Verbindungen der Formel IX beschrieben, zu einer Verbindung der Formel XII, 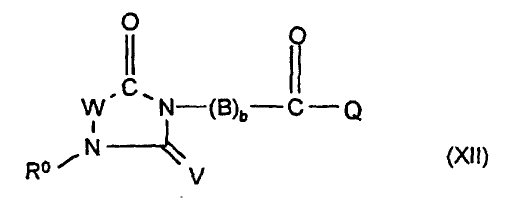 in der B, Q, V, W, R⁰ und b wie oben für die Formeln Ia und X angegeben definiert sind, cyclisiert wird. Aus der Verbindung der Formel XII wird dann durch Hydrolyse der Gruppe CO-Q zur Carbonsäure COOH und nachfolgende Kupplung mit einer Verbindung der Formel III, wie oben für die Kupplung der Verbindungen der Formeln II und III beschrieben, eine Verbindung der Formel Ia erhalten. Auch hier können während der Cyclisierung funktionelle Gruppen in geschützter Form vorliegen oder in Form von Vorstufen vorliegen, zum Beispiel Guanidinogruppen durch NO₂ blockiert werden oder Aminogruppen in geschützer Form oder noch als NO₂- oder Cyanofunktion vorliegen, die später zur Aminogruppe reduziert oder im Falle der Cyanogruppe auch in die Formamidinogruppe umgewandelt werden kann.

Eine weitere Methode zur Herstellung von Verbindungen der Formel la ist beispielsweise die Umsetzung von Verbindungen der Formel XIII, 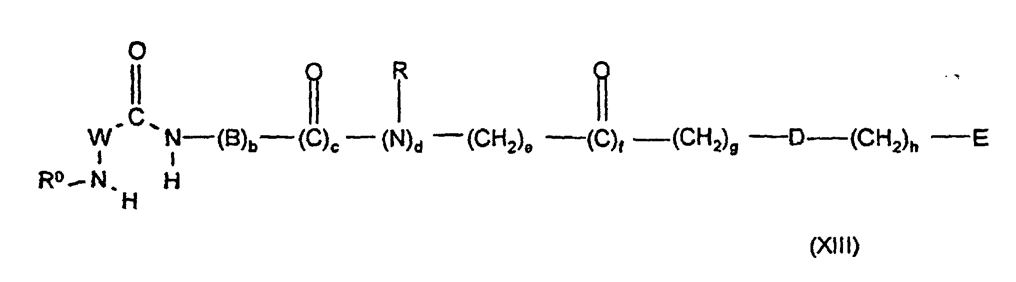 in der W für R¹-A-C(R¹³) steht und für die ansonsten die oben angegebenen Definitionen gelten, mit Phosgen, Thiophosgen oder entsprechenden Äquivalenten (analog S. Goldschmidt und M. Wick, Liebigs Ann. Chem. 575 (1952), 217-231 und C. Tropp, Chem. Ber. 61 (1928), 1431-1439).

Die Überführung der Aminofunktion in die Guanidinofunktion kann mit folgenden Reagenzien durchgeführt werden:
1. O-Methylisoharnstoff (S.Weiss und H. Krommer, Chemiker Zeitung 98 (1974), 617-618)
2. S S-Methylisothioharnstoff (R.F. Borne, M.L.Forrester und I.W. Waters, J. Med. Chem. 20 (1977), 771-776)
3. Nitro-S-methylisothioharnstoff (L.S. Hafner und R.E. Evans, J. Org. Chem. 24 (1959) 57)
4. Formamidinosulfonsäure (K. Kim, Y.-T. Lin und H.S. Mosher, Tetrah. Lett. 29 (1988), 3183-3186)
5. 3,5-Dimethyl-1-pyrazolyl-formamidinium-nitrat (F.L. Scott, D.G. O'Donovan und J. Reilly, J. Amer. Chem. Soc. 75 (1953), 4053-4054)
6. N,N'-Di-tert-butyloxycarbonyl-S-methyl-isothioharnstoff (R. J. Bergeron und J. S. McManis, J. Org. Chem. 52 (1987), 1700-1703)
7. N-Alkoxycarbonyl-, N,N'-Dialkoxycarbonyl-, N-Alkylcarbonyl- und N,N'-dialkylcarbonyl-S-methyl-isothioharnstoff (H. Wollweber, H. Kölling, E. Niemers, A. Widdig, P. Andrews, H.-P. Schulz und H. Thomas, Arzneim. Forsch./Drug Res. 34 (1984), 531-542).

Amidine können aus den entsprechenden Cyanoverbindungen durch Anlagerung von Alkoholen (zum Beispiel Methanol oder Ethanol) in saurem wasserfreiem Medium (zum Beispiel Dioxan, Methanol oder Ethanol) und anschließende Aminolyse, zum Beispiel Behandlung mit Ammoniak in Alkoholen wie zum Beispiel Isopropanol, Methanol oder Ethanol hergestellt werden (G. Wagner, P. Richter und Ch. Garbe, Pharmazie 29 (1974), 12-55). Eine weitere Methode, Amidine herzustellen, ist die Anlagerung von H₂S an die Cyanogruppe, gefolgt von einer Methylierung des entstandenen Thioamids und anschließender Umsetzung mit Ammoniak (DDR-Patent Nr. 235 866).

Hinsichtlich der Herstellung der Verbindungen der Formel I wird weiterhin vollinhaltlich Bezug genommen auf folgende Schriften: WO-A-94/21607, WO-A-95194008, EP-A-449 079, EP-A-530 505 (US-A-5 389 614), WO-A-93/18057, EP-A-566 919 (US-A-5 397 796), EP-A-580 008 (US-A-5 424 293) und EP-A-584 694 (US-A-5 554 594) sowie WO-A-96/33976.

Die Verbindungen der Formel I sind Antagonisten des Adhäsionsrezeptors VLA-4. Sie haben die Fähigkeit, Zell-Zell- und Zell-Matrix-Interaktionsprozesse zu inhibieren, bei denen Wechselwirkungen zwischen VLA-4 mit seinen Liganden eine Rolle spielen. Die Wirksamkeit der Verbindungen der Formel I kann beispielsweise in einem Assay nachgewiesen werden, in dem die Bindung von Zellen, die den VLA-4-Rezeptor aufweisen, zum Beispiel von Leukozyten, an Liganden dieses Rezeptors gemessen wird, zum Beispiel an VCAM-1, das dafür vorteilhafterweise auch gentechnisch hergestellt werden kann. Einzelheiten eines solchen Assay sind weiter unten beschrieben. Insbesondere vermögen die Verbindungen der Formel I die Adhäsion und die Migration von Leukozyten inhibieren, etwa die Anheftung von Leukozyten an endotheliale Zellen, die - wie oben erläutert - über den VCAM-1/VLA-4-Adhäsionsmechanismus gesteuert wird.

Die Verbindungen der Formel I und ihre physiologisch verträglichen Salze eignen sich daher zur Behandlung und Prophylaxe von Krankheiten, die auf der Wechselwirkung zwischen dem VLA-4-Rezeptor und seinen Liganden beruhen oder durch eine Hemmung dieser Wechselwirkung beeinflußt werden können, und insbesondere eignen sie sich für die Behandlung und Prophylaxe von Krankheiten, die zumindest teilweise durch ein unerwünschtes Ausmaß an Leukozytenadhäsion und/oder Leukozytenmigration verursacht werden oder damit verbunden sind, und zu deren Vorbeugung, Linderung oder Heilung die Adhäsion und/oder Migration von Leukozyten verringert werden soll. Sie können somit zum Beispiel bei entzündlichen Erscheinungen unterschiedlichster Ursache als Entzündungshemmer eingesetzt werden. Anwendung finden die Verbindungen der Formel I gemäß der vorliegenden Erfindung beispielsweise zur Behandlung oder Prophylaxe der rheumatoiden Arthritis, der inflammatorischen bowel disease (ulcerativen Colitis), des systemischen Lupus erythematosus oder zur Behandlung oder Prophylaxe von inflammatorischen Erkrankungen des zentralen Nervensystems, wie zum Beispiel der multiplen Sklerose, zur Behandlung oder Prophylaxe von Asthma oder von Allergien, zum Beispiel Allergien vom verzögerten Typ (Typ IV-Allergie), weiterhin zur Behandlung oder Prophylaxe von cardiovaskulären Erkrankungen, der Arteriosklerose, von Restenosen, zur Behandlung oder Prophylaxe von Diabetes, zur Verhinderung der Schädigung von Organtransplantaten, zur Hemmung von Tumorwachstum oder Tumormetastasierung bei verschiedenen Malignitäten, zur Therapie der Malaria, sowie von weiteren Krankheiten, bei denen eine Blockierung des Integrins VLA-4 und/oder eine Beeinflussung der Leukozytenaktivität zur Vorbeugung, Linderung oder Heilung angebracht erscheint. Die Verbindungen der Formel I und ihre Salze können weiterhin für diagnostische Zwecke, zum Beispiel bei in vitro-Diagnosen, und als Hilfsmittel in biochemischen Untersuchungen eingesetzt werden, bei denen eine VLA-4-Blockierung oder eine Beeinflussung von Zell-Zell- oder Zell-Matrix-Interaktionen angestrebt wird.

Die Verbindungen der Formel I und ihre physiologisch verträglichen Salze können erfindungsgemäß am Tier, bevorzugt am Säugetier, und insbesondere am Menschen als Arzneimittel zur Therapie oder Prophylaxe verabreicht werden. Sie können für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der Formel I und/oder ihrer physiologisch verträglichen Salze neben üblichen, pharmazeutisch einwandfreien Trägerstoffen und/oder Zusatzstoffen enthalten. Gegenstand der vorliegenden Erfindung ist auch die Verwendung von pharmazeutischen Zubereitungen, die eine oder mehrere Verbindungen der Formel I und/oder ihre physiologisch verträglichen Salze enthalten, für die oben genannten erfindungsgemäßen Verwendungen der Verbindungen der Formel I. Die pharmazeutischen Zubereitungen enthalten normalerweise etwa 0,5 bis 90 Gew.-% der therapeutisch wirksamen Verbindungen der Formel I und/oder ihrer physiologisch verträglichen Salze.

Die Arzneimittel können oral, zum Beispiel in Form von Pillen, Tabletten, Filmtabletten, Dragees, Granulaten, Hart- und Weichgelatinekapseln, Lösungen, Sirupen, Emulsionen oder Suspensionen verabreicht werden. Die Verabreichung kann aber auch rektal, zum Beispiel in Form von Suppositorien, oder parenteral, zum Beispiel in Form von Injektions- oder Infusionslösungen, Mikrokapseln oder Rods, oder perkutan, zum Beispiel in Form von Salben oder Tinkturen, oder auf anderem Wege, zum Beispiel in Form von Nasalsprays oder Aerosolmischungen, erfolgen.

Die Herstellung der erfindungsgemäß einzusetzenden pharmazeutischen Präparate erfolgt in an sich bekannter Weise, wobei neben der oder den Verbindungen der Formel I und/oder ihren physiologisch verträglichen Salze pharmazeutisch inerte anorganische und/oder organische Trägerstoffe verwendet werden. Für die Herstellung von Pillen, Tabletten, Dragees und Hartgelatinekapseln kann man zum Beispiel Lactose, Maisstärke oder Derivate davon, Talk, Stearinsäure oder deren Salze etc. verwenden. Trägerstoffe für Weichgelatinekapseln und Suppositorien sind zum Beispiel Fette, Wachse, halbfeste und flüssige Polyole, natürliche oder gehärtete Öle etc. Als Trägerstoffe für die Herstellung von Lösungen, zum Beispiel Injektionslösungen, oder von Emulsionen oder Sirupen eignen sich zum Beispiel Wasser, Alkohole, Glycerin, Polyole, Saccharose, Invertzucker, Glukose, pflanzliche Öle etc. Als Trägerstoffe für Mikrokapseln, Implantate oder Rods eignen sich zum Beispiel Mischpolymerisate aus Glykolsäure und Milchsäure.

Die pharmazeutischen Präparate können neben den Wirkstoffen und Trägerstoffen noch Zusatzstoffe, wie zum Beispiel Füllstoffe, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungsmittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der Formel I und/oder ihre physiologisch verträglichen Salze enthalten. Ferner können sie neben mindestens einer Verbindung der Formel I undloder ihren physiologisch verträglichen Salzen noch einen oder mehrere andere therapeutisch oder prophylaktisch wirksame Stoffe enthalten, zum Beispiel Stoffe mit entzündungshemmender Wirkung.

Die Dosis kann innerhalb weiter Grenzen variieren und ist in jedem einzelnen Fall den individuellen Gegebenheiten anzupassen. Im allgemeinen ist bei der oralen Verabreichung eine Tagesdosis von etwa 0,01 bis 100 mg/kg, vorzugsweise 0,1 bis 10 mg/kg, insbesondere 0,3 bis 2 mg/kg Körpergewicht zur Erzielung wirksamer Ergebnisse angemessen. Bei intravenöser Applikation beträgt die Tagesdosis im allgemeinen etwa 0,01 bis 50 mg/kg, vorzugsweise 0,01 bis 10 mg/kg Körpergewicht. Die Tagesdosis kann, insbesondere bei der Applikation größerer Mengen, in mehrere, zum Beispiel 2, 3, oder 4, Teilverabreichungen aufgeteilt werden. Gegebenenfalls kann es, je nach individuellem Verhalten, erforderlich werden, von der angegebenen Tagesdosis nach oben oder nach unten abzuweichen. Pharmazeutische Präparate enthalten normalerweise 0,2 bis 500 mg, vorzugsweise 1 bis 100 mg Wirkstoff der Formel I und/oder dessen physiologisch verträgliche Salze pro Dosis.

Bestimmte Verbindungen der Formel I sind im Stand der Technik noch nicht offenbart. Gegenstand der vorliegenden Erfindung sind auch diese neuen Verbindungen per se.

Die vorliegende Erfindung betrifft somit zum einen auch Verbindungen der Formel Ib per se, 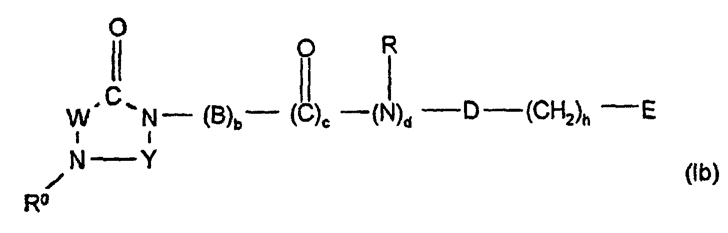 worin
- W: für R¹-A-CH oder R¹-A-CH=C steht;
- Y: für eine Carbonyl-, Thiocarbonyl- oder Methylengruppe steht;
- A: einen zweiwertigen Rest aus der Reihe (C₁-C₆)-Alkylen, (C₃-C₇)-Cycloalkylen, Phenylen, Phenylen-(C₁-C₆)-alkyl, (C₁-C₆)-Alkylen-phenyl, Phenylen-(C₂-C₆)-alkenyl oder einen zweiwertigen Rest eines 5- oder 6-gliedrigen gesättigten oder ungesättigten Ringes, der 1 oder 2 Stickstoffatome enthalten und ein- oder zweifach durch (C₁-C₆)-Alkyl oder doppelt gebundenen Sauerstoff oder Schwefel substituiert sein kann, bedeutet;
- B: einen zweiwertigen Rest aus der Reihe (C₁-C₆)-Alkylen, (C₂-C₆)-Alkenylen, Phenylen, Phenylen-Phenylen-(C₁-C₃)-alkyl, (C₁-C₃)-Alkylen-phenyl bedeutet;
- D: für C(R²)(R³) steht;
- E: Tetrazolyl, (R⁸O)₂P(O), HOS(O)₂, R⁹NHS(O)₂ oder R¹⁰CO bedeutet;
- R: Wasserstoff, (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl bedeutet;
- R⁰: (C₇-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl bedeutet;
- R¹: für X-NH-C(=NH)-(CH₂)ₚ oder X¹-NH-(CH₂)ₚ steht, wobei p für die Zahlen 0, 1, 2 oder 3 steht;
- X: Wasserstoff, (C₁-C₆)-Alkyl, (C₁-C₆)-Alkylcarbonyl, (C₁-C₆)-Alkoxycarbonyl, (C₁-C₁₈)-Alkylcarbonyloxy-(C₁-C₆)-alkoxycarbonyl, gegebenenfalls substituiertes (C₆-C₁₄)-Arylcarbonyl, gegebenenfalls substituiertes (C₆-C₁₄)-Aryloxycarbonyl, (C₆-C₁₄)-Aryl-(C₁-C₆)-alkoxycarbonyl, das im Arylrest auch substituiert sein kann, (R⁸O)₂P(O), Cyano, Hydroxy, (C₁-C₆)-Alkoxy, (C₆-C₁₄)-Aryl-(C₁-C₆)-alkoxy, das im Arylrest auch substituiert sein kann, oder Amino bedeutet;
- X¹: eine der Bedeutungen von X hat oder R'-NH-C(=N-R'') bedeutet, wobei R' und R" unabhängig voneinander die Bedeutungen von X haben;
- R²: Wasserstoff oder Phenyl bedeutet;
- R³: Wasserstoff, COOR⁴, CON(CH₃)R⁴ oder CONHR⁴ bedeutet;
- R⁴: Wasserstoff oder (C₁-C₂₈)-Alkyl bedeutet, das gegebenenfalls ein- oder mehrfach durch gleiche oder verschiedene Reste R^{4'} substituiert sein kann;
- R^{4'}: Hydroxy, Hydroxycarbonyl, Aminocarbonyl, Mono- oder Di-((C₁-C₁₈)-alkyl)-aminocarbonyl, Amino-(C₂-C₁₈)-alkylaminocarbonyl, Amino-(C₁-C₃)-alkyl-phenyl-(C₁-C₃)-alkylaminocarbonyl, (C₁-C₁₈)-Alkylcarbonylamino-(C₁-C₃)-alkylphenyl-(C₁-C₃)-alkylaminocarbonyl, (C₁-C₁₈)-Alkylcarbonylamino-(C₂-C₁₈)-alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)-Alkoxycarbonyl, gegebenenfalls substituiertes (C₃-C₈)-Cycloalkyl, Halogen, Nitro, Trifluormethyl oder den Rest R⁵ bedeutet;
- R⁵: gegebenenfalls substituiertes (C₆-C₁₄)-Aryl, im Arylrest gegebenenfalls substituiertes (C₆-C₁₄)-Aryl-(C₁-C₈)-alkyl, einen mono- oder bicyclischen 5- bis 12-gliedrigen heterocyclischen Ring, der aromatisch, teilhydriert oder vollständig hydriert sein kann und der ein, zwei oder drei gleiche oder verschiedene Heteroatome aus der Reihe Stickstoff, Sauerstoff und Schwefel enthalten kann, einen Rest R⁶ oder einen Rest R⁶CO- bedeutet, wobei der Aryl- und unabhängig davon der Heterocyclus-Rest ein- oder mehrfach durch gleiche oder verschiedene Reste aus der Reihe (C₁-C₁₈)-Alkyl, (C₁-C₁₈)-Alkoxy, Halogen, Nitro, Amino oder Trifluormethyl substituiert sein können;
- R⁶: für R⁷R⁸N, R⁷O oder R⁷S steht oder eine Aminosäureseitenkette, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, sowie deren Ester und Amide bedeutet, wobei anstelle freier funktioneller Gruppen gegebenenfalls Wasserstoff oder Hydroxymethyl stehen kann und/oder wobei freie funktionelle Gruppen durch in der Peptidchemie übliche Schutzgruppen geschützt sein können;
- R⁷: Wasserstoff, (C₁-C₁₈)-Alkyl, (C₆-C₁₄)-Aryl-(C₁-C₈)-alkyl, (C₁-C₁₈)-Alkylcarbonyl, (C₁-C₁₈)-Alkoxycarbonyl, (C₆-C₁₄)-Arylcarbonyl, (C₆-C₁₄)-Aryl-(C₁-C₈)-alkylcarbonyl oder (C₆-C₁₄)-Aryl-(C₁-C₁₈)-alkyloxycarbonyl, wobei die Alkylgruppen gegebenenfalls durch eine Aminogruppe substituiert undloder wobei die Arylreste ein- oder mehrfach, vorzugsweise einfach, durch gleiche oder verschiedene Reste aus der Reihe (C₁-C₈)-Alkyl, (C₁-C₈)-Alkoxy, Halogen, Nitro, Amino und Trifluormethyl substituiert sein können, einen natürlichen oder unnatürlichen Aminosäure-, Iminosäure-, gegebenenfalls N- (C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, bedeutet;
- R⁸: Wasserstoff, (C₁-C₁₈)-Alkyl, gegebenenfalls substituiertes (C₆-C₁₄)-Aryl oder (C₆-C₁₄)-Aryl oder (C₆-C₁₄)-Aryl-(C₁-C₈)-alkyl, das im Arylrest auch substituiert sein kann, bedeutet;
- R⁹: Wasserstoff, Aminocarbonyl, (C₁-C₁₈)-Alkylaminocarbonyl, (C₃-C₈)-Cycloalkylaminocarbonyl, gegebenenfalls substituiertes (C₆-C₁₄)-Arylaminocarbonyl, (C₁-C₁₈)-Alkyl, gegebenenfalls substituiertes (C₆-C₁₄)-Aryl oder (C₃-C₈)-Cycloalkyl bedeutet;
- R¹⁰: Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-Alkyl)-amino bedeutet;
- b, c, und d: unabhängig voneinander für 0 oder 1 stehen können, aber nicht alle gleichzeitig 0 sein können;
- h: für die Zahlen 0, 1, 2, 3, 4, 5 oder 6 steht;
in allen ihren stereoisomeren Formen und Mischungen davon in allen Verhältnissen, sowie ihre physiologisch verträglichen Salze.

Alle obigen Erläuterungen zur Formel I, zum Beispiel hinsichtlich Alkylresten, Arylresten, usw. gelten auch für die Verbindungen der Formel Ib entsprechend.

Auch die obigen bevorzugten Bedeutungen gelten hier entsprechend.

Die obigen Erläuterungen zur Herstellung der Verbindungen der Formel I und deren Verwendung gelten ebenso auch für die Verbindungen der Formel Ib. Diese Verbindungen sind naturgemäß ebenfalls Inhibitoren der Leukozytenadhäsion und/oder Antagonisten des VLA-4-Rezeptors sind und zur Behandlung und Prophylaxe von Krankheiten geeignet sind, die durch ein unerwünschtes Ausmaß an Leukozytenadhäsion und/oder Leukozytenmigration verursacht werden oder damit verbunden sind oder bei denen Zell-Zell- oder Zell-Matrix-Interaktionen eine Rolle spielen, die auf Wechselwirkungen von VLA-4-Rezeptoren mit ihren Liganden beruhen, beispielsweise von Entzündungsprozessen. Gegenstand der vorliegenden Erfindung sind weiterhin die Verbindungen der Formel lb zur Verwendung als Arzneimittel sowie pharmazeutische Präparate, die eine oder mehrere Verbindungen der Formel Ib und/oder ihre physiologisch verträglichen Salze neben pharmazeutisch einwandfreien Trägerstoffen und/oder Zusatzstoffen enthalten, wobei auch für diese pharmazeutischen Präparate wiederum die obigen Erläuterungen gelten.

Bestimmte Verbindungen der Formel I sind im Stand der Technik nicht explizit offenbart und stellen eine Auswahl aus der von der WO-A-95/14008 umfaßten Vielfalt von Verbindungen dar. Gegenstand der vorliegenden Erfindung sind auch diese neuen Verbindungen per se. Die vorliegende Erfindung betrifft somit zum anderen auch Verbindungen der Formel Ic per se, 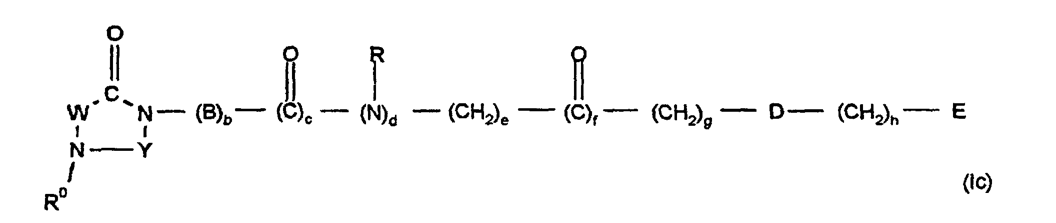 worin
- W: für R¹-A-C(R¹³) steht;
- Y: für eine Carbonyl-, Thiocarbonyl- oder Methylengruppe steht;
- A: einen Phenylenrest steht;
- B: einen zweiwertigen Rest aus der Reihe (C₁-C₆)-Alkylen, (C₂-C₆)-Alkenylen, Phenylen, Phenylen-(C₁-C₃)-alkyl, (C₁-C₃)-Alkylen-phenyl bedeutet;
- D: für C(R²)(R³)_{,} N(R³) oder CH=C(R³) steht;
- E: Tetrazolyl, (R⁸O)₂P(O), HOS(O)₂, R⁹NHS(O)₂ oder R¹⁰CO bedeutet;
- R: Wasserstoff, (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl bedeutet;
- R⁰: für im Arylrest gegebenenfalls substituiertes (C₆-C₁₄)-Aryl-(C₁-C₈)-alkyl steht;
- R¹: für X-NH-C(=NH)-(CH₂)ₚ oder X¹-NH-(CH₂)ₚ steht, wobei p für 0, 1, 2 oder 3 steht;
- X: Wasserstoff, (C₁-C₆)-Alkyl, (C₁-C₆)-Alkylcarbonyl, (C₁-C₆)-Alkoxycarbonyl, (C₁-C₁₈)-Alkylcarbonyloxy-(C₁-C₆)-alkoxycarbonyl, gegebenenfalls substituiertes (C₆-C₁₄)-Arylcarbonyl, gegebenenfalls substituiertes (C₆-C₁₄)-Aryloxycarbonyl, (C₆-C₁₄)-Aryl-(C₁-C₆)-alkoxycarbonyl, das im Arylrest auch substituiert sein kann, (R₈O)₂P(O), Cyano, Hydroxy, (C₁-C₆)-Alkoxy, (C₆-C₁₄)-Aryl-(C₁-C₆)-alkoxy, das im Arylrest auch substituiert sein kann, oder Amino bedeutet;
- X¹: eine der Bedeutungen von X hat oder R'-NH-C(=N-R") bedeutet, wobei R' und R" unabhängig voneinander die Bedeutungen von X haben;
- R²: Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl oder (C₃-C₈)-Cycloalkyl bedeutet;
- R³: Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)-Cycloalkyl, (C₂-C₈)-Alkenyl, (C₂-C₈)-Alkinyl, (C₂-C₈)-Alkenylcarbonyl, (C₂-C₈)-Alkinylcarbonyl, Pyridyl, R¹¹NH, R⁴CO, COOR⁴, CON(CH₃)R¹⁴, CONHR¹⁴, CSNHR¹⁴, COOR¹⁵, CON(CH₃)R¹⁵ oder CONHR¹⁵ bedeutet;
- R⁴: Wasserstoff oder (C₁-C₂₈)-Alkyl bedeutet, das gegebenenfalls ein- oder mehrfach durch gleiche oder verschiedene Reste R^{4'} substituiert sein kann;
- R^{4'}: Hydroxy, Hydroxycarbonyl, Aminocarbonyl, Mono- oder Di-((C₁-C₁₈)-alkyl)-aminocarbonyl, Amino-(C₂-C₁₈)-alkylaminocarbonyl, Amino-(C₁-C₃)-alkyl-phenyt-(C₁-C₃)-alkylaminocarbonyl, (C₁-C₁₈)-Alkylcarbonylamino-(C₁-C₃)-alkylphenyl-(C₁-C₃)-alkylaminocarbonyl, (C₁-C₁₈)-Alkylcarbonylamino-(C₂-C₁₈)-alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)-Alkoxycarbonyl, gegebenenfalls substituiertes (C₃-C₈)-Cycloalkyl, Halogen, Nitro, Trifluormethyl oder den Rest R⁵ bedeutet;
- R⁵: gegebenenfalls substituiertes (C₆-C₁₄)-Aryl, im Arylrest gegebenenfalls substituiertes (C₆-C₁₄)-Aryl-(C₁-C₈)-alkyl, einen mono- oder bicyclischen 5- bis 12-gliedrigen heterocyclischen Ring, der aromatisch, teilhydriert oder vollständig hydriert sein kann und der ein, zwei oder drei gleiche oder verschiedene Heteroatome aus der Reihe Stickstoff, Sauerstoff und Schwefel enthalten kann, einen Rest R⁶ oder einen Rest R₆CO- bedeutet, wobei der Aryl- und unabhängig davon der Heterocyclus-Rest ein- oder mehrfach durch gleiche oder verschiedene Reste aus der Reihe (C₁-C₁₈)-Alkyl, (C₁-C₁₈)-Alkoxy, Halogen, Nitro, Amino oder Trifluormethyl substituiert sein können;
- R⁶: für R⁷R⁸N_{,} R⁷O oder R⁷S steht oder eine Aminosäureseitenkette, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, sowie deren Ester und Amide bedeutet, wobei anstelle freier funktioneller Gruppen gegebenenfalls Wasserstoff oder Hydroxymethyl stehen kann und/oder wobei freie funktionelle Gruppen durch in der Peptidchemie übliche Schutzgruppen geschützt sein können;
- R⁷: Wasserstoff, (C₁-C₁₈)-Alkyl, (C₆-C₁₄)-Aryl-(C₁-C₈)-alkyl, (C₁-C₁₈)-Alkylcarbonyl, (C₁-C₁₈)-Alkoxycarbonyl, (C₆-C₁₄)-Arylcarbonyl, (C₆-C₁₄)-Aryl-(C₁-C₈)-alkylcarbonyl oder (C₆-C₁₄)-Aryl-(C₁-C₁₈)-alkyloxycarbonyl, wobei die Alkylgruppen gegebenenfalls durch eine Aminogruppe substituiert und/oder wobei die Arylreste ein- oder mehrfach, vorzugsweise einfach, durch gleiche oder verschiedene Reste aus der Reihe (C₁-C₈)-Alkyl, (C₁-C₈)-Alkoxy, Halogen, Nitro, Amino und Trifluormethyl substituiert sein können, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, bedeutet;
- R⁸: Wasserstoff, (C₁-C₁₈)-Alkyl, gegebenenfalls substituiertes (C₆-C₁₄)-Aryl oder (C₆-C₁₄)-Aryl-(C₁-C₈)-alkyl, das im Arylrest auch substituiert sein kann, bedeutet;
- R⁹: Wasserstoff, Aminocarbonyl, (C₁-C₁₈)-Alkylaminocarbonyl, (C₃-C₈)-Cycloalkylaminocarbonyl, gegebenenfalls substituiertes (C₆-C₁₄)-Arylaminocarbonyl, (C₁-C₁₈)-Alkyl, gegebenenfalls substituiertes (C₆-C₁₄)-Aryl oder (C₃-C₈)-Cycloalkyl bedeutet;
- R¹⁰: Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
- R¹¹: Wasserstoff, (C₁-C₁₈)-Alkyl, R¹²CO, gegebenenfalls substituiertes (C₆-C₁₄)-Aryl-S(O)₂, (C₁-C₁₈)-Alkyl-S(O)₂, im Arylrest gegebenenfalls substituiertes (C₆-C₁₄)-Aryl-(C₁-C₈)-alkyl oder R⁹NHS(O)₂ bedeutet;
- R¹²: Wasserstoff, (C₁-C₁₈)-Alkyl, (C₂-C₈)-Alkenyl, (C₂-C₈)-Alkinyl, gegebenenfalls substituiertes (C₆-C₁₄)-Aryl, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
- R¹³: (C₁-C₆)-Alkyl, im Arylrest gegebenenfalls substituiertes (C₆-C₁₄)-Aryl-(C₁-C₈)-alkyl oder (C₃-C₈)-Cycloalkyl bedeutet;
- R¹⁴: Wasserstoff oder (C₁-C₂₈)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-((C₁-C₁₈)-alkyl)-aminocarbonyl, Amino-(C₂-C₁₈)-alkylaminocarbonyl, Amino-(C₁-C₃)-alkylphenyl-(C₁-C₃)-alkylaminocarbonyl, (C₁-C₁₈)-Alkylcarbonylamino-(C₁-C₃)-alkylphenyl-(C₁-C₃)-alkylaminocarbonyl, (C₁-C₁₈)-Alkylcarbonyl-amino-(C₂-C₁₈)-alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)-Alkoxycarbonyl, gegebenenfalls substituiertes (C₃-C₈)-Cycloalkyl, HOS(O)₂-(C₁-C₃)-alkyl, R⁹NHS(O)₂-(C₁-C₃)-alkyl, (R⁸O)₂P(O)-(C₁-C₃)-alkyl, Tetrazolyl-(C₁-C₃)-alkyl, Halogen, Nitro, Trifluormethyl und R⁵ substituiert sein kann;
- R¹⁵: für R¹⁶-(C₁-C₆)-alkyl oder für R¹⁶ steht;
- R¹⁶: für einen 6- bis 24-gliedrigen bicyclischen oder tricyclischen Rest steht, der gesättigt oder teilweise ungesättigt ist und der auch ein bis vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann;
b, c, d und f unabhängig voneinander für 0 oder 1 stehen, aber nicht alle gleichzeitig 0 sein können;
e, g und h unabhängig voneinander für 0, 1, 2, 3, 4, 5 oder 6 stehen;
in allen ihren stereoisomeren Formen und Mischungen davon in allen Verhältnissen, sowie ihre physiologisch verträglichen Salze.

Alle obigen Erläuterungen zur Formel I, zum Beispiel hinsichtlich Alkylresten, Arylresten, usw. gelten auch für die Verbindungen der Formel Ic entsprechend. Auch die obigen bevorzugten Bedeutungen gelten hier entsprechend. Besonders bevorzugt stehen in den Verbindungen der Formel Ic zudem unabhängig voneinander b für 1, c für 1, d für 1, f für 0 und g für 0. e und h stehen besonders bevorzugt unabhängig voneinander für 0 oder 1. Besonders bevorzugt ist es auch, wenn Y in den Verbindungen der Formel Ic für eine Carbonylgruppe steht.

Die obigen Erläuterungen zur Herstellung der Verbindungen der Formel I und deren Verwendung gelten ebenso auch für die Verbindungen der Formel Ic. Diese Verbindungen sind naturgemäß ebenfalls Inhibitoren der Leukozytenadhäsion und/oder Antagonisten des VLA-4-Rezeptors sind und zur Behandlung und Prophylaxe von Krankheiten geeignet sind, die durch ein unerwünschtes Ausmaß an Leukozytenadhäsion und/oder Leukozytenmigration verursacht werden oder damit verbunden sind oder bei denen Zell-Zell- oder Zell-Matrix-Interaktionen eine Rolle spielen, die auf Wechselwirkungen von VLA-4-Rezeptoren mit ihren Liganden beruhen, beispielsweise von Entzündungsprozessen. Gegenstand der vorliegenden Erfindung sind weiterhin die Verbindungen der Formel Ic zur Verwendung als Arzneimittel sowie pharmazeutische Präparate, die eine oder mehrere Verbindungen der Formel Ic und/oder ihre physiologisch verträglichen Salze neben pharmazeutisch einwandfreien Trägerstoffen und/oder Zusatzstoffen enthalten, wobei auch für diese pharmazeutischen Präparate wiederum die obigen Erläuterungen gelten.

Weiterhin sind im Stand der Technik explizit noch keine Verbindungen der Formel I offenbart, in der b für 1 steht und B für einen substituierten Alkylenrest steht. Gegenstand der vorliegenden Erfindung sind somit auch Verbindungen der Formel Id per se, 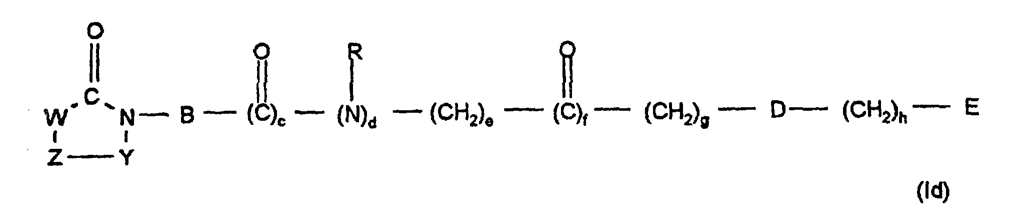 worin
- W: für R¹-A-C(R¹³) oder R¹-A-CH=C steht;
- Y: für eine Carbonyl-, Thiocarbonyl- oder Methylengruppe steht;
- Z: für N(R⁰) steht;
- A: einen zweiwertigen Rest aus der Reihe (C₁-C₆)-Alkylen, (C₃-C₇)-Cycloalkylen, Phenylen, Phenylen-(C₁-C₆)-alkyl, (C₁-C₆)-Alkylen-phenyl, Phenylen-(C₂-C₆)-alkenyl oder einen zweiwertigen Rest eines 5- oder 6-gliedrigen gesättigten oder ungesättigten Ringes, der 1 oder 2 Stickstoffatome enthalten und ein- oder zweifach durch (C₁-C₆)-Alkyl oder doppelt gebundenen Sauerstoff oder Schwefel substituiert sein kann, bedeutet;
- B: einen zweiwertigen (C₁-C₆)-Alkylen-Rest bedeutet, der durch einen Rest aus der Reihe (C₁-C₈)-Alkyl, (C₂-C₈)-Alkenyl, (C₂-C₈)-Alkinyl, (C₃-C₁₀)-Cycloalkyl, (C₃-C₁₀)-Cycloalkyl-(C₁-C₆)-alkyl, gegebenenfalls substituiertes (C₆-C₁₄)-Aryl, im Arylrest gegebenenfalls substituiertes (C₆-C₁₄)-Aryl-(C₁-C₆)-alkyl, gegebenenfalls substituiertes Heteroaryl und im Heteroarylrest gegebenenfalls substituiertes Heteroaryl-(C₁-C₆)-alkyl substituiert ist;
- D: für C(R²)(R³), N(R³) oder CH=C(R³) steht;
- E: Tetrazolyl, (R⁸O)₂P(O), HOS(O)₂, R⁹NHS(O)₂ oder R¹⁰CO bedeutet;
- R: Wasserstoff, (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl bedeutet;
- R⁰: für Wasserstoff, (C₁-C₈)-Alkyl, (C₃-C₁₂)-Cycloalkyl, (C₃-C₁₂)-Cycloalkyl-(C₁-C₈)-alkyl, (C₆-C₁₂)-Bicycloalkyl, (C₆-C₁₂)-Bicycloalkyl-(C₁-C₈)-alkyl, (C₆-C₁₂)-Tricycloalkyl, (C₆-C₁₂)-Tricycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl, im Heteroarylrest gegebenenfalls substituiertes Heteroaryl-(C₁-C₈)-alkyl, CHO, (C₁-C₈)-Alkyl-CO, (C₃-C₁₂)-Cycloalkyl-CO, (C₃-C₁₂)-Cycloalkyl-(C₁-C₈)-alkyl-CO, (C₆-C₁₂)-Bicycloalkyl-CO, (C₆-C₁₂)-Bicycloalkyl-(C₁-C₈)-alkyl-CO, (C₆-C₁₂)-Tricycloalkyl-CO, (C₆-C₁₂)-Tricycloalkyl-(C₁-C₈)-alkyl-CO, gegebenenfalls substituiertes (C₆-C₁₄)-Aryl-CO, im Arylrest gegebenenfalls substituiertes (C₆-C₁₄)-Aryl-(C₁-C₈)-alkyl-CO, gegebenenfalls substituiertes Heteroaryl-CO, im Heteroarylrest gegebenenfalls substituiertes Heteroaryl-(C₁-C₈)-alkyl-CO, (C₁-C₈)-Alkyl-S(O)ₙ, (C₃-C₁₂)-Cycloalkyl-S(O)ₙ, (C₃-C₁₂)-Cycloalkyl-(C₁-C₈)-alkyl-S(O)ₙ, (C₆-C₁₂)-Bicycloalkyl-S(O)ₙ, (C₆-C₁₂)-Bicycloalkyl-(C₁-C₈)-alkyl-S(O)ₙ, (C₆-C₁₂)-Tricycloalkyl-S(O)ₙ, (C₆-C₁₂)-Tricycloalkyl-(C₁-C₈)-alkyl-S(O)ₙ, gegebenenfalls substituiertes (C₆-C₁₄)-Aryl-S(O)ₙ, im Arylrest gegebenenfalls substituiertes (C₆-C₁₄)-Aryl-(C₁-C₈)-alkyl-S(O)ₙ, gegebenenfalls substituiertes Heteroaryl-S(O)ₙ oder im Heteroarylrest gegebenenfalls substituiertes Heteroaryl-(C₁-C₈)-alkyl-S(O)ₙ steht, wobei n für 1 oder 2 steht;
- R¹: für X-NH-C(=NH)-(CH₂)ₚ oder X¹-NH-(CH₂)ₚ steht, wobei p für 0, 1, 2 oder 3 steht;
- X: Wasserstoff, (C₁-C₆)-Alkyl, (C₁-C₆)-Alkylcarbonyl, (C₁-C₆)-Alkoxycarbonyl, (C₁-C₁₈)-Alkylcarbonyloxy-(C₁-C₆)-alkoxycarbonyl, gegebenenfalls substituiertes (C₆-C₁₄)-Arylcarbonyl, gegebenenfalls substituiertes (C₆-C₁₄)-Aryloxycarbonyl, (C₆-C₁₄)-Aryl-(C₁-C₆)-alkoxycarbonyl, das im Arylrest auch substituiert sein kann, (R⁸O)₂P(O), Cyano, Hydroxy, (C₁-C₆)-Alkoxy, (C₆-C₁₄)-Aryl-(C₁-C₆)-alkoxy, das im Arylrest auch substituiert sein kann, oder Amino bedeutet;
- X¹: eine der Bedeutungen von X hat oder R'-NH-C(=N-R'') bedeutet, wobei R' und R" unabhängig voneinander die Bedeutungen von X haben;
- R²: Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl oder (C₃-C₈)-Cycloalkyl bedeutet;
- R³: Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)-Cycloalkyl, (C₂-C₈)-Alkenyl, (C₂-C₈)-Alkinyl, (C₂-C₈)-Alkenylcarbonyl, (C₂-C₈)-Alkinylcarbonyl, Pyridyl, R¹¹NH, R⁴CO, COOR⁴, CON(CH₃)R¹⁴, CONHR¹⁴, CSNHR¹⁴, COOR¹⁵, CON(CH₃)R¹⁵ oder CONHR¹⁵ bedeutet;
- R⁴: Wasserstoff oder (C₁-C₂₈)-Alkyl bedeutet, das gegebenenfalls ein- oder mehrfach durch gleiche oder verschiedene Reste R^{4'} substituiert sein kann;
- R^{4'}: Hydroxy, Hydroxycarbonyl, Aminocarbonyl, Mono- oder Di-((C₁-C₁₈)-alkyl)-aminocarbonyl, Amino-(C₂-C₁₈)-alkylaminocarbonyl, Amino-(C₁-C₃)-alkyl-phenyl-(C₁-C₃)-alkylaminocarbonyl, (C₁-C₁₈)-Alkylcarbonylamino-(C₁-C₃)-alkylphenyl-(C₁-C₃)-alkylaminocarbonyl, (C₁-C₁₈)-Alkylcarbonylamino-(C₂-C₁₈)-alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)-Alkoxycarbonyl, gegebenenfalls substituiertes (C₃-C₈)-Cycloalkyl, Halogen, Nitro, Trifluormethyl oder den Rest R⁵ bedeutet;
- R⁵: gegebenenfalls substituiertes (C₆-C₁₄)-Aryl, im Arylrest gegebenenfalls substituiertes (C₆-C₁₄)-Aryl-(C₁-C₈)-alkyl, einen mono- oder bicyclischen 5- bis 12-gliedrigen heterocyclischen Ring, der aromatisch, teilhydriert oder vollständig hydriert sein kann und der ein, zwei oder drei gleiche oder verschiedene Heteroatome aus der Reihe Stickstoff, Sauerstoff und Schwefel enthalten kann, einen Rest R⁶ oder einen Rest R⁶CO- bedeutet, wobei der Aryl- und unabhängig davon der Heterocyclus-Rest ein- oder mehrfach durch gleiche oder verschiedene Reste aus der Reihe (C₁-C₁₈)-Alkyl, (C₁-C₁₈)-Alkoxy, Halogen, Nitro, Amino oder Trifluormethyl substituiert sein können;
- R⁶: für R⁷R⁸N, R⁷O oder R⁷S steht oder eine Aminosäureseitenkette, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, sowie deren Ester und Amide bedeutet, wobei anstelle freier funktioneller Gruppen gegebenenfalls Wasserstoff oder Hydroxymethyl stehen kann und/oder wobei freie funktionelle Gruppen durch in der Peptidchemie übliche Schutzgruppen geschützt sein können;
- R⁷: Wasserstoff, (C₁-C₁₈)-Alkyl, (C₆-C₁₄)-Aryl-(C₁-C₈)-alkyl, (C₁-C₁₈)-Alkylcarbonyl, (C₁-C₁₈)-Alkoxycarbonyl, (C₆-C₁₄)-Arylcarbonyl, (C₆-C₁₄)-Aryl-(C₁-C₈)-alkylcarbonyl oder (C₆-C₁₄)-Aryl-(C₁-C₁₈)-alkyloxycarbonyl, wobei die Alkylgruppen gegebenenfalls durch eine Aminogruppe substituiert und/oder wobei die Arylreste ein- oder mehrfach, vorzugsweise einfach, durch gleiche oder verschiedene Reste aus der Reihe (C₁-C₈)-Alkyl, (C₁-C₈)-Alkoxy, Halogen, Nitro, Amino und Trifluormethyl substituiert sein können, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, bedeutet;
- R⁸: Wasserstoff, (C₁-C₁₈)-Alkyl, gegebenenfalls substituiertes (C₆-C₁₄)-Aryl oder (C₆-C₁₄)-Aryl-(C₁-C₈)-alkyl, das im Arylrest auch substituiert sein kann, bedeutet;
- R⁹: Wasserstoff, Aminocarbonyl, (C₁-C₁₈)-Alkylaminocarbonyl, (C₃-C₈)-Cycloalkylaminocarbonyl, gegebenenfalls substituiertes (C₆-C₁₄)-Arylaminocarbonyl, (C₁-C₁₈)-Alkyl, gegebenenfalls substituiertes (C₆-C₁₄)-Aryl oder (C₃-C₈)-Cycloalkyl bedeutet;
- R¹⁰: Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
- R¹¹: Wasserstoff, (C₁-C₁₈)-Alkyl, R¹²CO, gegebenenfalls substituiertes (C₆-C₁₄)-Aryl-S(O)₂, (C₁-C₁₈)-Alkyl-S(O)₂, im Arylrest gegebenenfalls substituiertes (C₆-C₁₄)-Aryl-(C₁-C₈)-alkyl oder R⁹NHS(O)₂ bedeutet;
- R¹²: Wasserstoff, (C₁-C₁₈)-Alkyl, (C₂-C₈)-Alkenyl, (C₂-C₈)-Alkinyl, gegebenenfalls substituiertes (C₆-C₁₄)-Aryl, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
- R¹³: Wasserstoff, (C₁-C₆)-Alkyl, im Arylrest gegebenenfalls substituiertes (C₆-C₁₄)-Aryl-(C₁-C₈)-alkyl oder (C₃-C₈)-Cycloalkyl bedeutet;
- R¹⁴: Wasserstoff oder (C₁-C₂₈)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-((C₁-C₁₈)-alkyl)-aminocarbonyl, Amino-(C₂-C₁₈)-alkylaminocarbonyl, Amino-(C₁-C₃)-alkylphenyl-(C₁-C₃)-alkylaminocarbonyl, (C₁-C₁₈)-Alkylcarbonylamino-(C₁-C₃)-alkylphenyl-(C₁-C₃)-alkylaminocarbonyl, (C₁-C₁₈)-Alkylcarbonyl-amino-(C₂-C₁₈)-alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)-Alkoxycarbonyl, gegebenenfalls substituiertes (C₃-C₈)-Cycloalkyl, HOS(O)₂-(C₁-C₃)-alkyl, R⁹NHS(O)₂-(C₁-C₃)-alkyl, (R⁸O)₂P(O)-(C₁-C₃)-alkyl, Tetrazolyl-(C₁-C₃)-alkyl, Halogen, Nitro, Trifluormethyl und R⁵ substituiert sein kann;
- R¹⁵: für R¹⁶-(C₁-C₆)-alkyl oder für R¹⁶ steht;
- R¹⁶: für einen 6- bis 24-gliedrigen bicyclischen oder tricyclischen Rest steht, der gesättigt oder teilweise ungesättigt ist und der auch ein bis vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann;
c, d und f unabhängig voneinander für 0 oder 1 stehen, aber nicht alle gleichzeitig 0 sein können;
e, g und h unabhängig voneinander für 0, 1, 2, 3, 4, 5 oder 6 stehen;
in allen ihren stereoisomeren Formen und Mischungen davon in allen Verhältnissen, sowie ihre physiologisch verträglichen Salze.

Alle obigen Erläuterungen zur Formel I, zum Beispiel hinsichtlich Alkylresten, Arylresten, usw. gelten auch für die Verbindungen der Formel Id entsprechend. Auch die obigen bevorzugten Bedeutungen gelten hier entsprechend. Besonders bevorzugt stehen in den Verbindungen der Formel Id zudem unabhängig voneinander c für 1, d für 1, f für 0 und g für 0. e und h stehen besonders bevorzugt unabhängig voneinander für 0 oder 1. Hinsichtlich der Gruppe B gilt für die Verbindungen der Formel Id zudem folgendes.

Der für die Gruppe B stehende (C₁-C₆)-Alkylenrest ist in den Verbindungen der Formel Id bevorzugt ein (C₁-C₄)-Alkylenrest, besonders bevorzugt ein Methylenrest oder ein Ethylenrest (=1,2-Ethylen), ganz besonders bevorzugt ein Methylenrest. Der Substituent an der Gruppe B kann zum einen einen Cyclus enthalten, wenn es sich um einen Substituenten aus der Reihe (C₃-C₁₀)-Cycloalkyl, (C₃-C₁₀)-Cycloalkyl-(C₁-C₆)-alkyl, gegebenenfalls substituiertes (C₆-C₁₄)-Aryl, im Arylrest gegebenenfalls substituiertes (C₆-C₁₄)-Aryl-(C₁-C₆)-alkyl, gegebenenfalls substituiertes Heteroaryl und im Heteroarylrest gegebenenfalls substituiertes Heteroaryl-(C₁-C₆)-alkyl handelt, und kann zum anderen acyclisch sein, wenn es sich um einen Substituenten aus der Reihe (C₁-C₈)-Alkyl, (C₂-C₈)-Alkenyl und (C₂-C₈)-Alkinyl handelt. Diese acyclischen Substituenten können jeder 2, 3, 4, 5, 6, 7 oder 8 Kohlenstoffatome oder im Falle des gesättigten Alkylrests auch 1 Kohlenstoffatom enthalten. Im Falle der Alkenylreste und Alkinylreste kann sich die Doppelbindung oder Dreifachbindung in einer beliebigen Position befinden und im Falle der Doppelbindung cis-Konfiguration oder trans-Konfiguration aufweisen. Wie oben erläutert, können diese Alkylreste, Alkenylreste und Alkinylreste geradkettig oder verzweigt sein.

Als Beispiele für Substituenten, die der für B stehende (C₁-C₆)-Alkylenrest tragen kann, seien genannt Methyl, Ethyl, n-Propyl, n-Butyl, n-Pentyl, n-Hexyl, n-Heptyl, n-Octyl, Isopropyl, Isobutyl, Isopentyl, Isohexyl, sec-Butyl, tert-Butyl, tert-Pentyl, Neopentyl, Neohexyl, 3-Methylpentyl, 2-Ethylbutyl, Vinyl, Allyl, 1-Propenyl, 2-Butenyl. 3-Butenyl, 3-Methyl-2-butenyl, Ethinyl, 1-Propinyl, 2-Propinyl, 6-Hexinyl, Phenyl, Benzyl, 1-Phenylethyl, 2-Phenylethyl, 3-Phenylpropyl, 4-Biphenylylmethyl, Cyclopropyl, Cyclopropylmethyl, Cyclopentyl, Cyclohexyl, Cyclohexylmethyl, 2-Cyclohexylethyl, 3-Cyclooctylpropyl, 2-Pyridyl, 3-Pyridyl, 4-Pyridyl, 4-Pyridylmethyl, 2-(4-Pyridyl)ethyl, 2-Furylmethyl, 2-Thienylmethyl, 3-Thienylmethyl oder 2-(3-Indolyl)ethyl.

Bevorzugte Verbindungen der Formel Id sind solche, worin gleichzeitig
- W: für R¹-A-C(R¹³) steht;
- Y: für eine Carbonylgruppe steht;
- Z: für N(R⁰) steht;
- A: einen zweiwertigen Rest aus der Reihe (C₃-C₇)-Cycloalkylen, Phenylen, Phenylen-(C₁-C₆)-alkyl, (C₁-C₆)-Alkylen-phenyl oder einen zweiwertigen Rest eines 5- oder 6-gliedrigen gesättigten oder ungesättigten Ringes, der 1 oder 2 Stickstoffatome enthalten und ein- oder zweifach durch (C₁-C₆)-Alkyl oder doppelt gebundenen Sauerstoff oder Schwefel substituiert sein kann, bedeutet;
- B: einen zweiwertigen Methylenrest oder Ethylenrest bedeutet, der durch einen Rest aus der Reihe (C₁-C₈)-Alkyl, (C₂-C₈)-Alkenyl, (C₂-C₈)-Alkinyl, (C₃-C₁₀)-Cycloalkyl, (C₃-C₁₀)-Cycloalkyl-(C₁-C₆)-alkyl, gegebenenfalls substituiertes (C₆-C₁₄)-Aryl, im Arylrest gegebenenfalls substituiertes (C₆-C₁₄)-Aryl-(C₁-C₆)-alkyl, gegebenenfalls substituiertes Heteroaryl und im Heteroarylrest gegebenenfalls substituiertes Heteroaryl-(C₁-C₆)-alkyl substituiert ist;
- D: für C(R²)(R³) steht;
- E: Tetrazolyl oder R¹⁰CO bedeutet;
- R: Wasserstoff oder (C₁-C₈)-Alkyl bedeutet;
- R⁰: für Wasserstoff, (C₁-C₈)-Alkyl, (C₃-C₁₂)-Cycloalkyl, (C₃-C₁₂)-Cycloalkyl-(C₁-C₈)-alkyl, (C₆-C₁₂)-Bicycloalkyl, (C₆-C₁₂)-Bicycloalkyl-(C₁-C₈)-alkyl, (C₆-C₁₂)-Tricycloalkyl, (C₆-C₁₂)-Tricycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl, im Heteroarylrest gegebenenfalls substituiertes Heteroaryl-(C₁-C₈)-alkyl, CHO, (C₁-C₈)-Alkyl-CO, (C₃-C₁₂)-Cycloalkyl-CO, (C₃-C₁₂)-Cycloalkyl-(C₁-C₈)-alkyl-CO, (C₆-C₁₂)-Bicycloalkyl-CO, (C₆-C₁₂)-Bicycloalkyl-(C₁-C₈)-alkyl-CO, (C₆-C₁₂)-Tricycloalkyl-CO, (C₆-C₁₂)-Tricycloalkyl-(C₁-C₈)-alkyl-CO, gegebenenfalls substituiertes (C₆-C₁₄)-Aryl-CO, im Arylrest gegebenenfalls substituiertes (C₆-C₁₄)-Aryl-(C₁-C₈)-alkyl-CO, gegebenenfalls substituiertes Heteroaryl-CO, im Heteroarylrest gegebenenfalls substituiertes Heteroaryl-(C₁-C₈)-Alkyl -CO, (C₁-C₈)-Alkyl-S(O)ₙ, (C₃-C₁₂)-Cycloalkyl-S(O)ₙ, (C₃-C₁₂)-Cycloalkyl-(C₁-C₈)-alkyl-S(O)ₙ, (C₆-C₁₂)-Bicycloalkyl-S(O)ₙ, (C₆-C₁₂)-Bicycloalkyl-(C₁-C₈)-alkyl-S(O)ₙ, (C₆-C₁₂)-Tricycloalkyl-S(O)ₙ, (C₆-C₁₂)-Tricycloalkyl-(C₁-C₈)-alkyl-S(O)ₙ, gegebenenfalls substituiertes (C₆-C₁₄)-Aryl-S(O)ₙ, im Arylrest gegebenenfalls substituiertes (C₆-C₁₄)-Aryl-(C₁-C₈)-alkyl-S(O)ₙ, gegebenenfalls substituiertes Heteroaryl-S(O)ₙ oder im Heteroarylrest gegebenenfalls substituiertes Heteroaryl-(C₁-C₈)-alkyl-S(O)ₙ steht, wobei n für 1 oder 2 steht;
- R¹: für X-NH-C(=NH)-(CH₂)ₚ oder X¹-NH-(CH₂)ₚ steht, wobei p für 0, 1, 2 oder 3 steht;
- X: Wasserstoff, (C₁-C₆)-Alkyl, (C₁-C₆)-Alkylcarbonyl, (C₁-C₆)-Alkoxycarbonyl, (C₁-C₁₈)-Alkylcarbonyloxy-(C₁-C₆)-alkoxycarbonyl, gegebenenfalls substituiertes (C₆-C₁₄)-Arylcarbonyl, gegebenenfalls substituiertes (C₆-C₁₄)-Aryloxycarbonyl, (C₆-C₁₄)-Aryl-(C₁-C₆)-alkoxycarbonyl, das im Arylrest auch substituiert sein kann, Cyano, Hydroxy, (C₁-C₆)-Alkoxy, (C₆-C₁₄)-Aryl-(C₁-C₆)-alkoxy, das im Arylrest auch substituiert sein kann, oder Amino bedeutet;
- X¹: eine der Bedeutungen von X hat oder R'-NH-C(=N-R'') bedeutet, wobei R' und R" unabhängig voneinander die Bedeutungen von X haben;
- R²: Wasserstoff oder (C₁-C₈)-Alkyl bedeutet;
- R³: Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)-Cycloalkyl, (C₂-C₈)-Alkenyl, (C₂-C₈)-Alkinyl, (C₂-C₈)-Alkenylcarbonyl, (C₂-C₈)-Alkinylcarbonyl, Pyridyl, R¹¹NH, CON(CH₃)R¹⁴, CONHR¹⁴, CON(CH₃)R¹⁵ oder CONHR¹⁵ bedeutet;
- R⁵: gegebenenfalls substituiertes (C₆-C₁₄)-Aryl, im Arylrest gegebenenfalls substituiertes (C₆-C₁₄)-Aryl-(C₁-C₈)-alkyl, einen mono- oder bicyclischen 5- bis 12-gliedrigen heterocyclischen Ring, der aromatisch, teilhydriert oder vollständig hydriert sein kann und der ein, zwei oder drei gleiche oder verschiedene Heteroatome aus der Reihe Stickstoff, Sauerstoff und Schwefel enthalten kann, oder einen Rest R⁶CO- bedeutet, wobei der Aryl- und unabhängig davon der Heterocyclus-Rest ein- oder mehrfach durch gleiche oder verschiedene Reste aus der Reihe (C₁-C₈)-Alkyl, (C₁-C₈)-Alkoxy, Halogen, Nitro, Amino oder Trifluormethyl substituiert sein können;
- R⁶: für einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert sein kann, sowie deren Ester und Amide bedeutet, wobei freie funktionelle Gruppen durch in der Peptidchemie übliche Schutzgruppen geschützt sein können;
- R¹⁰: Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
- R¹¹: für R¹²CO, gegebenenfalls substituiertes (C₆-C₁₄)-Aryl-S(O)₂ oder (C₁-C₁₈)-Alkyl-S(O)₂ steht;
- R¹²: Wasserstoff, (C₁-C₁₈)-Alkyl, (C₂-C₈)-Alkenyl, (C₂-C₈)-Alkinyl, gegebenenfalls substituiertes (C₆-C₁₄)-Aryl, (C₁-C₁₈)-Alkoxy, (C₆-C₁₄)-Aryl-(C₁-C₈)-alkoxy, das im Arylrest auch substituiert sein kann oder gegebenenfalls substituiertes (C₆-C₁₄)-Aryloxy bedeutet;
- R¹³: Wasserstoff oder (C₁-C₄)-Alkyl bedeutet;
- R¹⁴: (C₁-C₁₀)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-((C₁-C₁₈)-alkyl)-aminocarbonyl, (C₆-C₁₄)-Aryl-(C₁-C₈)-alkoxycarbonyl, das im Arylrest auch substituiert sein kann, (C₁-C₈)-Alkoxy, (C₁-C₈)-Alkoxycarbonyl, gegebenenfalls substituiertes (C₃-C₈)-Cycloalkyl, Tetrazolyl-(C₁-C₃)-alkyl, -alkyl, Trifluormethyl und R⁵ substituiert sein kann;
- R¹⁵: für R¹⁶-(C₁-C₆)-alkyl oder für R¹⁶ steht;
- R¹⁶: für einen 6- bis 24-gliedrigen bicyclischen oder tricyclischen Rest steht, der gesättigt oder teilweise ungesättigt ist und der auch ein bis vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann;
c und d für 1 stehen und f für 0 steht;
e und h unabhängig voneinander für 0 oder 1 stehen und g für 0 steht;
in allen ihren stereoisomeren Formen und Mischungen davon in allen Verhältnissen, sowie ihre physiologisch verträglichen Salze.

Besonders bevorzugte Verbindungen der Formel Id sind solche, worin der Rest, durch den die Gruppe B substituiert ist, ein (C₁-C₈)-Alkylrest ist, in allen ihren stereoisomeren Formen und Mischungen davon in allen Verhältnissen, sowie ihre physiologisch verträglichen Salze.

Die obigen Erläuterungen zur Herstellung der Verbindungen der Formel I und deren Verwendung gelten ebenso auch für die Verbindungen der Formel Id. Diese Verbindungen sind naturgemäß ebenfalls Inhibitoren der Leukozytenadhäsion und/oder Antagonisten des VLA-4-Rezeptors sind und zur Behandlung und Prophylaxe von Krankheiten geeignet sind, die durch ein unerwünschtes Ausmaß an Leukozytenadhäsion und/oder Leukozytenmigration verursacht werden oder damit verbunden sind oder bei denen Zell-Zell- oder Zell Zell-Matrix-Interaktionen eine Rolle spielen, die auf Wechselwirkungen von VLA-4-Rezeptoren mit ihren Liganden beruhen, beispielsweise von Entzündungsprozessen. Gegenstand der vorliegenden Erfindung sind weiterhin die Verbindungen der Formel Id zur Verwendung als Arzneimittel sowie pharmazeutische Präparate, die eine oder mehrere Verbindungen der Formel Id und/oder ihre physiologisch verträglichen Salze neben pharmazeutisch einwandfreien Trägerstoffen und/oder Zusatzstoffen enthalten, wobei auch für diese pharmazeutischen Präparate wiederum die obigen Erläuterungen gelten.

Im Stand der Technik sind keine Verbindungen der Formel I offenbart, in der R⁰ für einen Acylrest, Sulfonylrest oder Sulfinylrest steht. Gegenstand der vorliegenden Erfindung sind somit weiterhin auch Verbindungen der Formel Ie per se, 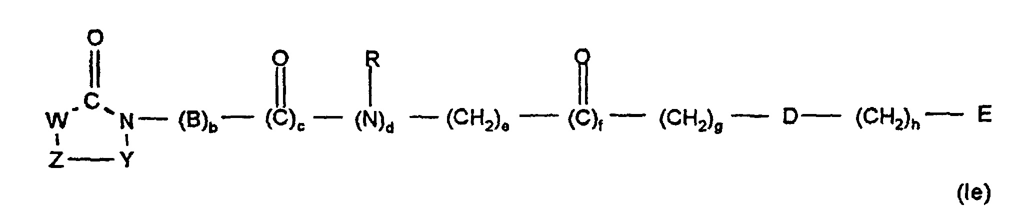 worin
- W: für R¹-A-C(R¹³) oder R¹-A-CH=C steht;
- Y: für eine Carbonyl-, Thiocarbonyl- oder Methylengruppe steht;
- Z: für N(R⁰), Sauerstoff, Schwefel oder eine Methylengruppe steht;
- A: einen zweiwertigen Rest aus der Reihe (C₁-C₆)-Alkylen, (C₃-C₇)-Cycloalkylen, Phenylen, Phenylen-(C₁-C₆)-alkyl, (C₁-C₆)-Alkylen-phenyl, Phenylen-(C₂-C₆)-alkenyl oder einen zweiwertigen Rest eines 5- oder 6-gliedrigen gesättigten oder ungesättigten Ringes, der 1 oder 2 Stickstoffatome enthalten und ein- oder zweifach durch (C₁-C₆)-Alkyl oder doppelt gebundenen Sauerstoff oder Schwefel substituiert sein kann, bedeutet;
- B: einen zweiwertigen Rest aus der Reihe (C₁-C₆)-Alkylen, (C₂-C₆)-Alkenylen, Phenylen, Phenylen-(C₁-C₃)-alkyl, (C₁-C₃)-Alkylen-phenyl bedeutet;
- D: für C(R²)(R³), N(R³) oder CH=C(R³) steht;
- E: Tetrazolyl, (R⁸O)₂P(O), HOS(O)₂, R⁹NHS(O)₂ oder R¹⁰CO bedeutet;
- R: Wasserstoff, (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl bedeutet;
- R⁰: für CHO, (C₁-C₈)-Alkyl-CO, (C₃-C₁₂)-Cycloalkyl-CO, (C₃-C₁₂)-Cycloalkyl-(C₁-C₈)-alkyl-CO, (C₆-C₁₂)-Bicycloalkyl-CO, (C₆-C₁₂)-Bicycloalkyl-(C₁-C₈)-alkyl-CO, (C₆-C₁₂)-Tricycloalkyl-CO, (C₆-C₁₂)-Tricycloalkyl-(C₁-C₈)-alkyl-CO, gegebenenfalls substituiertes (C₆-C₁₄)-Aryl-CO, im Arylrest gegebenenfalls substituiertes (C₆-C₁₄)-Aryl-(C₁-C₈)-alkyl-CO, gegebenenfalls substituiertes Heteroaryl-CO, im Heteroarylrest gegebenenfalls substituiertes Heteroaryl-(C₁-C₈)-alkyl-CO, (C₁-C₈)-Alkyl-S(O)ₙ, (C₃-C₁₂)-Cycloalkyl-S(O)ₙ, (C₃-C₁₂)-Cycloalkyl-(C₁-C₈)-alkyl-S(O)ₙ, (C₆-C₁₂)-Bicycloalkyl-S(O)ₙ, (C₆-C₁₂)-Bicycloalkyl-(C₁-C₈)-alkyl-S(O)ₙ, (C₆-C₁₂)-Tricycloalkyl-S(O)ₙ, (C₆-C₁₂)-Tricycloalkyl-(C₁-C₈)-alkyl-S(O)ₙ, gegebenenfalls substituiertes (C₆-C₁₄)-Aryl-S(O)ₙ, im Arylrest gegebenenfalls substituiertes (C₆-C₁₄)-Aryl-(C₁-C₈)-alkyl-S(O)ₙ, gegebenenfalls substituiertes Heteroaryl-S(O)ₙ oder im Heteroarylrest gegebenenfalls substituiertes Heteroaryl-(C₁-C₈)-alkyl-S(O)ₙ steht, wobei n für 1 oder 2 steht;
- R¹: für X-NH-C(=NH)-(CH₂)ₚ oder X¹-NH-(CH₂)ₚ steht, wobei p für 0, 1, 2 oder 3 steht;
- X: Wasserstoff, (C₁-C₆)-Alkyl, (C₁-C₆)-Alkylcarbonyl, (C₁-C₆)-Alkoxycarbonyl, (C₁-C₁₈)-Alkylcarbonyloxy-(C₁-C₆)-alkoxycarbonyl, gegebenenfalls substituiertes (C₆-C₁₄)-Arylcarbonyl, gegebenenfalls substituiertes (C₆-C₁₄)-Aryloxycarbonyl, (C₆-C₁₄)-Aryl-(C₁-C₆)-alkoxycarbonyl, das im Arylrest auch substituiert sein kann, (R⁸O)₂P(O), Cyano, Hydroxy, (C₁-C₆)-Alkoxy, (C₆-C₁₄)-Aryl-(C₁-C₆)-alkoxy, das im Arylrest auch substituiert sein kann, oder Amino bedeutet;
- X¹: eine der Bedeutungen von X hat oder R'-NH-C(=N-R") bedeutet, wobei R' und R" unabhängig voneinander die Bedeutungen von X haben;
- R²: Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl oder (C₃-C₈)-Cycloalkyl bedeutet;
- R³: Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)-Cycloalkyl, (C₂-C₈)-Alkenyl, (C₂-C₈)-Alkinyl, (C₂-C₈)-Alkenylcarbonyl, (C₂-C₈)-Alkinylcarbonyl, Pyridyl, R¹¹NH, R⁴CO, COOR⁴, CON(CH₃)R¹⁴, CONHR¹⁴, CSNHR¹⁴_{,} COOR¹⁵, CON(CH₃)R¹⁵ oder CONHR¹⁵ bedeutet;
- R⁴: Wasserstoff oder (C₁-C₂₈)-Alkyl bedeutet, das gegebenenfalls ein- oder mehrfach durch gleiche oder verschiedene Reste R⁴' substituiert sein kann;
- R^{4'}: Hydroxy, Hydroxycarbonyl, Aminocarbonyl, Mono- oder Di-((C₁-C₁₈)-alkyl)-aminocarbonyl, Amino-(C₂-C₁₈)-alkylaminocarbonyl, Amino-(C₁-C₃)-alkyl-phenyl-(C₁-C₃)-alkylaminocarbonyl, (C₁-C₁₈)-Alkylcarbonylamino-(C₁-C₃)-alkylphenyl-(C₁-C₃)-alkylaminocarbonyl, (C₁-C₁₈)-Alkylcarbonylamino-(C₂-C₁₈)-alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)-Alkoxycarbonyl, gegebenenfalls substituiertes (C₃-C₈)-Cycloalkyl, Halogen, Nitro, Trifluormethyl oder den Rest R⁵ bedeutet;
- R⁵: gegebenenfalls substituiertes (C₆-C₁₄)-Aryl, im Arylrest gegebenenfalls substituiertes (C₆-C₁₄)-Aryl-(C₁-C₈)-alkyl, einen mono- oder bicyclischen 5- bis 12-gliedrigen heterocyclischen Ring, der aromatisch, teilhydriert oder vollständig hydriert sein kann und der ein, zwei oder drei gleiche oder verschiedene Heteroatome aus der Reihe Stickstoff, Sauerstoff und Schwefel enthalten kann, einen Rest R⁶ oder einen Rest R⁶CO- bedeutet, wobei der Aryl- und unabhängig davon der Heterocyclus-Rest ein- oder mehrfach durch gleiche oder verschiedene Reste aus der Reihe (C₁-C₁₈)-Alkyl, (C₁-C₁₈)-Alkoxy, Halogen, Nitro, Amino oder Trifluormethyl substituiert sein können;
- R⁶: für R⁷R⁸N, R⁷O oder R⁷S steht oder eine Aminosäureseitenkette, einen natürlichen oder unnatürlichen Aminosäure-, lminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu-NH-CH₂- reduziert sein kann, sowie deren Ester und Amide bedeutet, wobei anstelle freier funktioneller Gruppen gegebenenfalls Wasserstoff oder Hydroxymethyl stehen kann und/oder wobei freie funktionelle Gruppen durch in der Peptidchemie übliche Schutzgruppen geschützt sein können;
- R⁷: Wasserstoff, (C₁-C₁₈)-Alkyl, (C₆-C₁₄)-Aryl-(C₁-C₈)-alkyl, (C₁-C₁₈)-Alkylcarbonyl, (C₁-C₁₈)-Alkoxycarbonyl, (C₆-C₁₄)-Arylcarbonyl, (C₆-C₁₄)-Aryl-(C₁-C₈)-alkylcarbonyl oder (C₆-C₁₄)-Aryl-(C₁-C₁₈)-alkyloxycarbonyl, wobei die Alkylgruppen gegebenenfalls durch eine Aminogruppe substituiert und/oder wobei die Arylreste ein- oder mehrfach, vorzugsweise einfach, durch gleiche oder verschiedene Reste aus der Reihe (C₁-C₈)-Alkyl, (C₁-C₈)-Alkoxy, Halogen, Nitro, Amino und Trifluormethyl substituiert sein können, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, bedeutet;
- R⁸: Wasserstoff, (C₁-C₁₈)-Alkyl, gegebenenfalls substituiertes (C₆-C₁₄)-Aryl oder (C₆-C₁₄)-Aryl-(C₁-C₈)-alkyl, das im Arylrest auch substituiert sein kann, bedeutet;
- R⁹: Wasserstoff, Aminocarbonyl, (C₁-C₁₈)-Alkylaminocarbonyl, (C₃-C₈)-Cycloalkylaminocarbonyl, gegebenenfalls substituiertes (C₆-C₁₄)-Arylaminocarbonyl, (C₁-C₁₈)-Alkyl, gegebenenfalls substituiertes (C₆-C₁₄)-Aryl oder (C₃-C₈)-Cycloalkyl bedeutet;
- R¹⁰: Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
- R¹¹: Wasserstoff, (C₁-C₁₈)-Alkyl, R¹²CO, gegebenenfalls substituiertes (C₆-C₁₄)-Aryl-S(O)₂, (C₁-C₁₈)-Alkyl-S(O)₂, im Arylrest gegebenenfalls substituiertes (C₆-C₁₄)-Aryl-(C₁-C₈)-alkyl oder R⁹NHS(O)₂ bedeutet;
- R¹²: Wasserstoff, (C₁-C₁₈)-Alkyl, (C₂-C₈)-Alkenyl, (C₂-C₈)-Alkinyl, gegebenenfalls substituiertes (C₆-C₁₄)-Aryl, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
- R¹³: Wasserstoff, (C₁-C₆)-Alkyl, im Arylrest gegebenenfalls substituiertes (C₆-C₁₄)-Aryl-(C₁-C₈)-alkyl oder (C₃-C₈)-Cycloalkyl bedeutet;
- R¹⁴: Wasserstoff oder (C₁-C₂₈)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-((C₁-C₁₈)-alkyl)-aminocarbonyl, Amino-(C₂-C₁₈)-alkylaminocarbonyl, Amino-(C₁-C₃)-alkylphenyl-(C₁-C₃)-alkylaminocarbonyl, (C₁-C₁₈)-Alkylcarbonylamino-(C₁-C₃)-alkylphenyl-(C₁-C₃)-alkylaminocarbonyl, (C₁-C₁₈)-Alkylcarbonyl-amino-(C₂-C₁₈)-alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)-Alkoxycarbonyl, gegebenenfalls substituiertes (C₃-C₈)-Cycloalkyl, HOS(O)₂-(C₁-C₃)-alkyl, R⁹NHS(O)₂-(C₁-C₃)-alkyl, (R⁸O)₂P(O)-(C₁-C₃)-alkyl, Tetrazolyl-(C₁-C₃)-alkyl, Halogen, Nitro, Trifluormethyl und R⁵ substituiert sein kann;
- R¹⁵: für R¹⁶-(C₁-C₆)-alkyl oder für R¹⁶ steht;
- R¹⁶: für einen 6- bis 24-gliedrigen bicyclischen oder tricyclischen Rest steht, der gesättigt oder teilweise ungesättigt ist und der auch ein bis vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann;
b, c, d und f unabhängig voneinander für 0 oder 1 stehen, aber nicht alle gleichzeitig 0 sein können;
e, g und h unabhängig voneinander für 0, 1, 2, 3, 4, 5 oder 6 stehen;
in allen ihren stereoisomeren Formen und Mischungen davon in allen Verhältnissen, sowie ihre physiologisch verträglichen Salze.

Alle obigen Erläuterungen zur Formel I, zum Beispiel hinsichtlich Alkylresten, Arylresten, usw. gelten auch für die Verbindungen der Formel Ie entsprechend. Auch die obigen bevorzugten Bedeutungen gelten hier entsprechend.

Die obigen Erläuterungen zur Herstellung der Verbindungen der Formel I und deren Verwendung gelten ebenso auch für die Verbindungen der Formel Ie. Diese Verbindungen sind naturgemäß ebenfalls Inhibitoren der Leukozytenadhäsion und/oder Antagonisten des VLA-4-Rezeptors sind und zur Behandlung und Prophylaxe von Krankheiten geeignet sind, die durch ein unerwünschtes Ausmaß an Leukozytenadhäsion und/oder Leukozytenmigration verursacht werden oder damit verbunden sind oder bei denen Zell-Zell- oder Zell-Matrix-Interaktionen eine Rolle spielen, die auf Wechselwirkungen von VLA-4-Rezeptoren mit ihren Liganden beruhen, beispielsweise von Entzündungsprozessen. Gegenstand der vorliegenden Erfindung sind weiterhin die Verbindungen der Formel Ie zur Verwendung als Arzneimittel sowie pharmazeutische Präparate, die eine oder mehrere Verbindungen der Formel Ie und/oder ihre physiologisch verträglichen Salze neben pharmazeutisch einwandfreien Trägerstoffen und/oder Zusatzstoffen enthalten, wobei auch für diese pharmazeutischen Präparate wiederum die obigen Erläuterungen gelten.

### Beispiele

Die Produkte wurden über Massenspektren (MS) und/oder NMR-Spektren identifiziert. Verbindungen, die durch Chromatographie unter Verwendung eines Laufmittels gereinigt wurden, das beispielsweise Essigsäure oder Trifluoressigsäure enthielt, und anschließend gefriergetrocknet wurden, enthielten zum Teil je nach Durchführung der Gefriertrocknung noch die aus dem Laufmittel stammende Säure, sind also teilweise oder vollständig in Form eines Salzes der verwendeten Säure, zum Beispiel in Form des Essigsäuresalzes oder Trifluoressigsäuresalzes, angefallen.

Es bedeuten:
- DMF: N,N-Dimethylformamid
- THF: Tetrahydrofuran
- DCC: N,N'-Dicyclohexylcarbodiimid
- HOBt: 1-Hydroxybenzotriazol
- HOOBt: 3-Hydroxy-4-oxo-3,4-dihydro-1,2,3-benzotriazin

### Beispiel 1:

### ((R,S)-4-(4-(Amino-imino-methyl)-phenyl)-4-methyl-2,5-dioxoimidazolidin-1-yl)-acetyl-L-aspartyl-L-phenylglycin

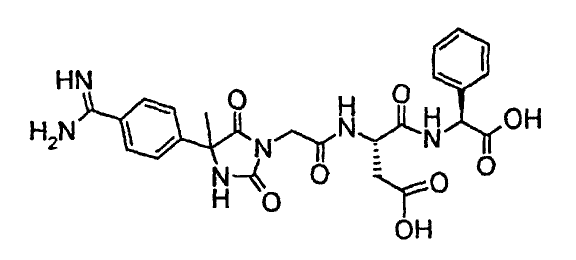

### 1a) (R,S)-4-(4-Cyanophenyl)-4-methyl-2,5-dioxoimidazolidin

20 g (138 mmol) p-Acetylbenzonitril, 115,6 g Ammoniumcarbonat (1,21 mol) und 11,6 g Kaliumcyanid (178 mmol) wurden in 600 ml einer Mischung aus 50 % Ethanol und 50 % Wasser gelöst. Das Gemisch wurde 5 Stunden bei 55 °C gerührt und über Nacht bei Raumtemperatur stehen gelassen. Die Lösung wurde mit 6 N HCl auf einen pH-Wert von 6,3 eingestellt und anschließend zwei Stunden bei Raumtemperatur gerührt. Der Niederschlag wurde abgesaugt, mit Wasser gewaschen und über Phosphorpentoxid im Hochvakuum getrocknet. Ausbeute:
22,33 g (75%).
FAB-MS: 216,1 (M + H)⁺

### 1b) ((R,S)-4-(4-Cyanophenyl)-4-methyl-2,5-dioxoimidazolidin-1-yl)-essigsäure-methylester

1,068 g Natrium (46,47 mmol) wurden unter Stickstoff in 110 ml abs. Methanol gelöst. Die klare Lösung wurde mit 10 g (R,S)-4-(4-Cyanophenyl)-4-methyl-2,5-dioxoimidazolidin (46,47 mmol) versetzt und das Gemisch wurde 2 h unter Rückfluß gekocht. Man gab 7,75 g (46,68 mmol) Kaliumiodid zu und tropfte innerhalb einer Stunde eine Lösung von 4,53 ml Chloressigsäuremethylester (51,3 mmol) in 5 ml Methanol zu. Es wurde 6 Stunden zum Sieden erhitzt, über Nacht bei Raumtemperatur stehen gelassen und eingeengt. Der ölige Rückstand wurde über Kieselgel mit Methylenchlorid/Essigester (9:1) chromatographiert. Ausbeute: 8,81 g (66%).
FAB-MS: 288 (M + H)⁺

### 1c) ((R,S)-4-(4-(Ethoxy-imino-methyl)-phenyl)-4-methyl 2,5-dioxoimidazolidin-1-yl)-essigsäure-methylester-Hydrochlorid

Eine Suspension von 4 g ((R,S)-4-(4-Cyanophenyl)-4-methyl-2,5-diaxoimidazolidin-1-yl)-essigsäure-methylester (13,92 mmol) in 60 ml abs. Ethanol wurde auf 0 °C abgekühlt. Trockenes HCl-Gas wurde in die Suspension eingeleitet, wobei die Temperatur stets unter 10 °C gehalten wurde, bis im IR-Spektrum die Nitril-Bande nicht mehr vorlag. Die ethanolische Lösung wurde mit 200 ml Diethylether versetzt und über Nacht bei 4 °C stehen gelassen. Der Niederschlag wurde abgesaugt und im Hochvakuum getrocknet. Ausbeute: 3,96 g (77 %).
FAB-MS: 334 (M + H)⁺

### 1d) ((R,S)-4-(4-(Amino-imino-methyl)-phenyl)-4-methyl-2,5-dioxoimidazolidin-1-yl)-essigsäure-methylester-Hydrochlorid

3,96 g ((R,S)-4-(4-(Ethoxy-imino-methyl)-phenyl)-4-methyl-2,5-dioxoimidazolidin-1-yl)-essigsäure-methylester-Hydrochlorid (10,7 mmol) wurden in 40 ml Isopropanol suspendiert und mit 11,9 ml einer 2 N Lösung von Ammoniak in Isopropanol versetzt. Das Reaktionsgemisch wurde 2 Stunden bei 50 °C gerührt. Der Ansatz wurde abgekühlt und dann mit 200 ml Diethylether versetzt. Der Niederschlag wurde abgesaugt und im Hochvakuum getrocknet. Ausbeute: 3,27 g (89 %).
FAB-MS: 305 (M + H)⁺

### 1e) ((R,S)-4-(4-(Amino-imino-methyl)-phenyl)-4-methyl-2,5-dioxolmidazolidin-1-yl)-essigsäure-Hydrochlorid

3,27 g ((R,S)-4-(4-(Amino-imino-methyl)-phenyl)-4-methyl-2,5-dioxoimidazolidin-1-yl)-essigsäure-methylester-Hydrochlorid (9,6 mmol) wurden in 50 ml konzentrierter Salzsäure gelöst. Die Lösung wurde 6 Stunden zum Sieden erhitzt und dann eingeengt. Ausbeute: 2,73 g (87 %).
FAB-MS: 291,2 (M + H)⁺

### 1f) ((R,S)-4-(4-(Amino-imina-methyl)-phenyl)-4-methyl-2,5-diaxoimidazolidin-1-yl)-acetyl-L-aspartyl-L-phenylglycin-di-tert-butylester-Hydrochlorid

Zu einer Lösung von 1 g ((R,S)-4-(4-(Amino-imino-methyl)phenyl)-4-methyl-2,5-dioxoimidazolidin-1-yl)-essigsäure-Hydrochlorid (3,06 mmol), 1,27 g H-Asp(OBu^{t})-Phg-Bu^{t} -Hydrochlorid (3,06 mmol) und 413 mg HOBt in 10 ml Dimethylformamid gab man bei 0 °C 673 mg DCC (3,06 mmol). Man ließ eine Stunde bei 0 °C und 4 Stunden bei Raumtemperatur rühren. Anschließend ließ man den Ansatz über das Wochenende im Kühlraum stehen, saugte den Niederschlag ab und engte das Filtrat ein. Zur Reinigung wurde die Substanz über Kieselgel mit Methylenchlorid/Methanol/EisessiglWasser (8,5:1,5:0,15:0,15) chromatographiert. Ausbeute: 920 mg Öl (enthielt noch Essigsäure).
FAB-MS: 651,3 (M + H)⁺

### 1g) ((R,S)-4-(4-(Amino-imino-methyl)-phenyl)4-methyl-2,5-dioxoimidazolidin-1-yl)-acetyl-L-aspartyl-L-phenylglycin

920 mg ((R,S)-4-(4-(Amino-imino-methyl)-phenyl)-4-methyl-2,5-dioxoimidazolidin-1-yl)-acetyl-L-aspartyl-L-phenylglycin-di-tert-butylester-Hydrochlorid wurden in einer Mischung aus 5,4 ml Trifluoressigsäure, 0,6 ml Wasser und 0,6 ml Dimercaptoethan gelöst. Man ließ eine Stunde bei Raumtemperatur stehen und engte im Wasserstrahlvakuum ein. Zur Reinigung wurde die Substanz an Sephadex LH20 mit einer Mischung aus Eisessig, n-Butanol und Wasser chromatographiert. Die Fraktionen mit der reinen Substanz wurden eingeengt. Der Rückstand wurde in Wasser gelöst und gefriergetrocknet. Ausbeute: 390 mg
[α]_{D} = + 1,3 ° (c = 1, in Methanol, 25 °C).
FAB-MS: 539,2 (M + H)⁺

### Beispiel 2:

### ((R,S)-4-(4-(Amino-imino-methyl)-phenyl)-3,4-dimethyl-2,5-dioxoimidazolidin-1-yl)-acetyl-L-aspartyl-L-phenylglycin

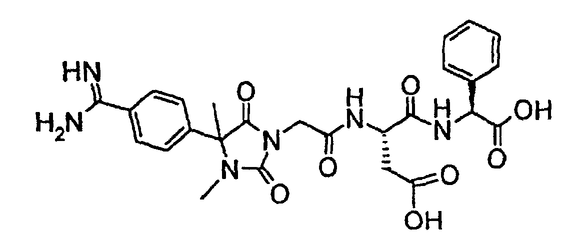

### 2a) ((R,S)-4-(4-Cyanophenyl)-3,4-dimethyl-2,5-dioxoimidazolidin-1-yl)-essigsäure-methylester

3 g ((R,S)-4-(4-Cyanophenyl)-4-methyl-2,5-dioxoimidazolidin-1-yl)-essigsäure-methylester (10,4 mmol) wurden unter Argon in 15 ml wasserfreiem Dimethylformamid gelöst. Im Argon-Gegenstrom wurden 275,5 mg einer Natriumhydrid-Dispersion in Mineralöl (11,4 mmol) zugegeben. Das Reaktionsgemisch wurde 15 Minuten bei Raumtemperatur gerührt. Anschließend versetzet man mit 721 µl Methyliodid (11,4 mmol). Es wurde 4 Stunden bei Raumtemperatur gerührt und dann über Nacht bei Raumtemperatur stehen gelassen. Die Lösung wurde konzentriert. Zur Reinigung wurde die Substanz über Kieselgel mit Methylenchlorid/Essigester (9,5:0,5) chromatographiert. Die Fraktionen mit der reinen Substanz wurden eingeengt. Ausbeute: 2,14 g Öl (68 %).
FAB-MS: 302,2 (M + H)⁺

### 2b) ((R,S)-4-(4-(Ethoxy-imino-methyl)-phenyl)-3,4-dimethyl-2,5-dioxoimidazolidin-1-yl)-essigsäure-methylester-Hydrochlorid

Eine Lösung von 2,56 g ((R,S)-4-(4-Cyanophenyl)-3,4-methyl-2,5-dioxoimidazolidin-1-yl)-essigsäure-methylester (8,5 mmol) in 40 ml abs. Ethanol wurde auf 0 °C abgekühlt. Trockenes HCl-Gas wurde in die Lösung eingeleitet, wobei die Temperatur stets unter 10 °C gehalten wurde, bis im IR-Spektrum die Nitril-Bande nicht mehr vorlag. Die ethanolische Lösung wurde auf 20 ml eingeengt und mit 200 ml Diethylether versetzt. Die Suspension wurde eingeengt und im Hochvakuum getrocknet. Ausbeute: 2,27 g (76 %).
FAB-MS: 348,1 (M + H)⁺

### 2c) ((R,S)-4-(4-(Amino-imino-methyl)-phenyl)-3,4-dimethyl-2,5-dioxoimidazolidin-1-yl)-essigsäure-methylester-Hydrochlorid

2,26 g ((R,S)-4-(4-(Ethoxy-imino-methyl)-phenyl)-3,4-dimethyl-2,5-dioxoimidazolidin-1-yl)-essigsäure-methylester-Hydrochlorid (6,4 mmol) wurden in 25 ml Isopropanol suspendiert und mit 7,2 ml einer 2 N Lösung von Ammoniak in Isopropanol versetzt. Das Reaktionsgemisch wurde 2,5 Stunden bei 50 °C gerührt. Der Ansatz wurde abgekühlt und dann mit 200 ml Diethylether versetzt. Der Niederschlag wurde abgesaugt und im Hochvakuum getrocknet. Ausbeute: 1,03 g (45 %).
FAB-MS: 319,4 (M + H)⁺

### 2d) ((R,S)-4-(4-(Amino-imino-methyl)-phenyl)-3,4-dimethyl-2,5-dioxoimidazolidin-1-yl)-essigsäure-Hydrochlorid

1 g ((R,S)-4-(4-(Amino-imino-methyl)-phenyl)-3,4-dimethyl-2,5-dioxoimidazolidin-1-yl)-essigsäure-methylester-Hydrochlorid (2,8 mmol) wurden in 20 ml konzentrierter Salzsäure gelöst. Die Lösung wurde 6 Stunden zum Sieden erhitzt und dann eingeengt. Ausbeute: 770 mg (81 %).
FAB-MS: 305 (M + H)⁺

### 2e) ((R,S)-4-(4-(Amino-imino-methyl)-phenyl)-3,4-dimethyl-2,5-dioxoimidazolidin-1-yl)-acetyl-L-aspartyl-L-phenylglycin-di-tert-butylester-Hydrochlorid

Zu einer Lösung von 340 mg ((R,S)-4-(4-(Amino-imino-methyl)-phenyl)-3,4-dimethyl-2,5-dioxoimidazolidin-1-yl)-essigsäure-Hydrochlorid (1 mmol), 415 mg H-Asp(OBu^{t})-Phg-OBu^{t} -Hydrochlorid (1 mmol) und 135 mg HOBt in 7 ml Dimethylformamid gab man bei 0 °C 220 mg DCC (1 mmol). Man setzte 0,13 ml N-Ethylmorpholin zu, bis ein pH von 5,0 erreicht war, und ließ eine Stunde bei 0 °C und 2 Stunden bei Raumtemperatur rühren. Anschließend ließ man den Ansatz über das Wochenende im Kühlraum stehen, saugte den Niederschlag ab und engte das Filtrat ein. Zur Reinigung wurde die Substanz an Sephadex LH20 mit einer Mischung aus Eisessig, n-Butanol und Wasser chromatographiert. Die Fraktionen mit der reinen Substanz wurden eingeengt. Der Rückstand wurde in Wasser gelöst und gefriergetrocknet.
Ausbeute: 377 mg (57%).
FAB-MS: 665,2 (M + H)⁺

### 2f) ((R,S)-4-(4-(Amino-imino-methyl)-phenyl)-3,4-dimethyl-2,5-dioxoimidazolidin-1-yl)-acetyl-L-aspartyl-L-phenylglycin

370 mg ((R,S)-4-(4-(Amino-imino-methyl)-phenyl)-3,4-dimethyl-2,5-dioxoimidazolidin-1-yl)-acetyl)-L-aspartyl-L-phenylglycin-di-tert-butylester-Hydrochlorid (0,53 mmol) wurden in einer Mischung aus 3,6 ml Trifluoroessigsäure, 0,4 ml Wasser und 0,4 ml Dimercaptoethan gelöst. Man ließ eine Stunde bei Raumtemperatur stehen und engte im Wasserstrahlvakuum ein. Zur Reinigung wurde die Substanz an Sephadex LH20 mit einer Mischung aus Eisessig, n-Butanol und Wasser chromatographiert. Die Fraktionen mit der reinen Substanz wurden eingeengt. Der Rückstand wurde in Wasser gelöst und gefriergetrocknet. Ausbeute:
210 mg eines weißen Feststoffes (72 %).
[α]_{D} = - 2,8 ° (c =1, in Methanol, 23 °C).
FAB-MS: 553,2 (M + H)⁺

Die Verbindungen der Beispiele 3 bis 17 wurden analog Beispiel 2 hergestellt.

### Beispiel 3:

### ((R,S)-4-(4-(Amino-imino-methyl)-phenyl)-3-ethyl-4-methyl-2,5-dioxoimidazolidin-1-yl)-acetyl-L-aspartyl-L-phenylglycin

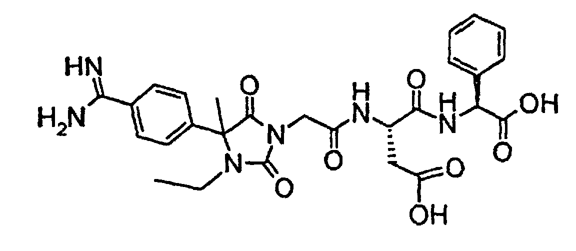

FAB-MS: 567,2 (M + H)⁺

### Beispiel 4:

### ((R,S)-4-(4-(Amino-imino-methyl)-phenyl)-3-benzyl-4-methyl-2,5-dioxoimidazolidin-1-yl)-acetyl-L-aspartyl-L-phenylglycin

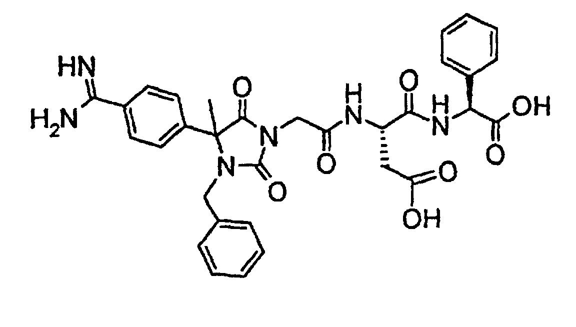

FAB-MS: 629,0 (M + H)⁺

### Beispiel 5:

### ((R,S)-4(4-(Amino-imino-methyl)-phenyl)-3-(4-tert-butyl-benzyl)-4-methyl-2,5-dioxoimidazolidin-1-yl)-acetyl-L-aspartyl-L-phenylglycin

FAB-MS: 685,4 (M + H)⁺

### Beispiel 6:

### ((R,S)-4-(4-(Amino-imino-methyl)-phenyl)-3-(2,3,4,5,6-pentafluorbenzyl)-4-methyl-2,5-dioxoimidazolidin-1-yl)-acetyl-L-aspartyl-L-phenylglycin

FAB-MS: 719,3 (M + H)⁺

### Beispiel 7:

### ((R,S)-4-(4-(Amino-imino-methyl)-phenyl)-3-(4-nitrobenzyl)-4-methyl-2,5-dioxoimidazolidin-1-yl)-acetyl-L-aspartyl-L-phenylglycin

FAB-MS: 674,3 (M + H)⁺

### Beispiel 8:

### ((R, S)-4-(4-(Amino-imino-methyl)-phenyl)-3-(3,5-dimethylbenzyl)4-methyl-2,5-dioxoimidazolidin-1-yl)-acetyl-L-aspartyl-L-phenylglycin

FAB-MS: 657,3 (M + H)⁺

### Beispiel 9:

### ((R,S)-4-(4-(Amino-imino-methyl)-phenyl)-3-((2-naphthyl)-methyl)-4-methyl-2,5-dioxoimidazolidin-1-yl)-acetyl-L-aspartyl-L-phenylglycin

FAB-MS: 679,2 (M + H)⁺

### Beispiel 10:

### ((R,S)-4-(4-(Amino-imino-methyl)-phenyl)-3-((2-(phenylsulfonylmethyl)-benzyl)-4-methyl-2,5-dioxoimidazolidin-1-yl)-acetyl-L-aspartyl-L-phenylglycin

FAB-MS: 783,2 (M + H)⁺

### Beispiel 11:

### ((R,S)-4-(4-(Amino-imino-methyl)-phenyl)-3-((2-biphenylyl}-methyl)-4-methyl-2,5-dioxoimidazolidin-1-yl)-acetyl-L-aspartyl-L-phenylglycin

FAB-MS: 705,2 (M + H)⁺

### Beispiel 12:

### ((R,S)-4-(4-(Amino-imino-methyl)-phenyl)-3-(4-methylbenzyl)-4-methyl-2,5-dioxoimidazolidin-1-yl)-acetyl-L-aspartyl-L-phenylglycin

FAB-MS: 643,3 (M + H)⁺

### Beispiel 13:

### ((R, S)-4-(4-(Amino-imino-methyl)-phenyl)-3-((1-naphthyl)-methyl)-4-methyl-2,5-dioxoinnidazolidin-1-yl)-acetyl-L-aspartyl-L-phenylglycin

FAB-MS: 679,3 (M + H)⁺

### Beispiel 14:

### ((R,S)-4-(4-(Amino-imino-methyl)-phenyl)-3-((4-biphenylyl)-methyl)-4-methyl-2,5-dioxoimidazolidin-1-yl)-acetyl-L-aspartyl-L-phenylglycin

FAB-MS: 705,3 (M + H)⁺

### Beispiel 15:

### ((R,S)-4-(4-(Amino-imino-methyl)-phenyl)-3-(4-trifluormethylbenzyl)-4-methyl-2,5-dioxoimidazolidin-1-yl)-acetyl-L-aspartyl-L-phenylglycin

FAB-MS: 697,3 (M + H)⁺

### Beispiel 16:

### ((R,S)-4-(4-(Amino-imino-methyl)-phenyl)-3-(3,5-bis(trifluormethyl)benzyl)-4-methyl-2,5-dioxoimidazotidin-1-yl)-acetyl-L-aspartyl-L-phenylglycin

FAB-MS: 765,2 (M + H)⁺

### Beispiel 17:

### ((R,S)-4-(4-(Amino-imino-methyl)-phenyl)-3-(pentamethylbenzyl)-4-methyl-2,5-dioxoimidazolidin-1-yl)-acetyl-L-aspartyl-L-phenylglycin

FAB-MS: 699,3 (M + H)⁺

### Beispiel 18:

### ((R,S)-4-(4-(Amino-imino-methyl)-phenyl)-3-((4-biphenylyl)-methyl)-4-methyl-2,5-dioxoimidazolidin-1-yl)-acetyl-L-N-methyl-aspartyl-L-phenylglycin

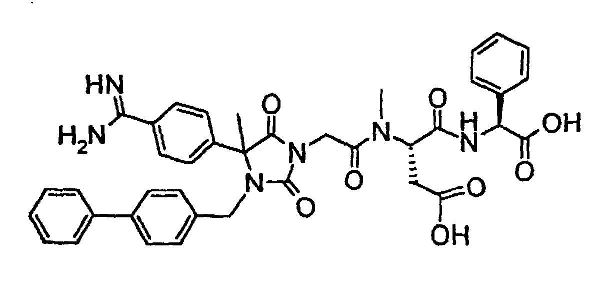

### 18a) ((S)-3-Benzyloxycarbonyl-5-oxo-1,3-oxazolidin-4-yl)-essigsäure-benzylester

3,57 g L-N-Benzyloxycarbonyl-asparaginsäure-β-benzylester (10 mmol) wurden in 300 ml wasserfreiem Toluol gelöst. Es wurden 4,5 g Trioxan (50 mmol), 5,7 mg p-Toluoisulfonsäure (0,03 mmol), sowie 3 Å-Molekularsieb zugegeben. Das Gemisch wurde 30 Minuten unter Rückfluß zum Sieden erhitzt und anschließend im Vakuum eingeengt. Zur Reinigung wurde die Substanz über Kieselgel mit Toluol/Essigester (3:2) chromatographiert. Ausbeute: 2,94 g (80%).
FAB-MS: 370,2 (M + H)⁺

### 18b) L-N-Benzyloxycarbonyl-N-methyl-asparaginsäure-β-benzylester

886 mg ((S)-3-Benzyloxycarbonyl-5-oxo-1,3-oxazolidin-4-yl)-essigsäure-benzylester (2,4 mmol) wurden in 25 ml einer 1:1-Mischung von Methylenchlorid und Triethylsilan gelöst. Man setzte 3 Å-Molekularsieb zu und gab dann portionsweise 2 ml Bortrifluorid-Etherat -Etherat zu. Das Reaktionsgemisch wurde 2 Stunden bei Raumtemperatur gerührt. Die Reaktionslösung wurde mit Methylenchlorid verdünnt und die organische Phase anschließend mit wäßriger Natriumhydrogencarbonat-Lösung ausgeschüttelt. Die organische Phase wurde eingeengt und im Vakuum getrocknet. Ausbeute: 820 mg (92%).
FAB-MS: 394,3 (M + Na)⁺

### 18c) L-N-Benzytoxycarbonyl-N-methyl-aspartyl(β-benzylester)-L-phenylglycin-tert-butylester

197 mg L-N-Benzyloxycarbonyl-N-methyl-asparaginsäure-β-benzylester (0,5 mmol), 122 mg H-Phg-OBu^{t}-Hydrochlorid (0,5 mmol), 164 mg (0,5 mmol) TOTU (O-((cyanoethoxycarbonyl-methylen)amino)-N,N,N',N'-tetramethyluronium-tetrafluoroborat) und 225 µl Diisopropyl-ethylamin (1,5 mmol) und wurden bei 0 °C in 3 ml wasserfreiem Dimethylformamid gelöst. Man ließ 10 Minuten bei 0 °C und 1,5 Stunden bei Raumtemperatur rühren und engte anschließend die Reaktionslösung ein. Der Rückstand wurde zwischen Essigester und 1000 ml einer KHSO₄/K₂SO₄-Lösung (50 g KHSO₄ und 100 g K₂SO₄ in 1000 ml Wasser) verteilt. Die organische Phase wurde dreimal mit einer Natriumhydrogencarbonat-Lösung, mit Wasser und mit gesättigter Natriumchlorid-Lösung gewaschen, über Natriumsulfat getrocknet und im Vakuum eingeengt. Zur Reinigung wurde die Substanz über Kieselgel mit Toluol/Essigester (10:1) chromatographiert. Ausbeute: 214 mg (76%).
FAB-MS: 561,3 (M + H)⁺

### 18d) L-N-Methyl-aspartyl-L-phenylglycin-tert-butylester-Hydrochlorid

2,98 g L-N-Benzyloxycarbony(-N-methyl-aspartyl(β-benzylester)-L-phenylglycin-tert-butylester (5,32 mmol) wurden in 300 ml Methanol gelöst und an der Autobürette unter Zugabe von 2 N methanolischer HCl bei einem pH von 6,5 über Pd/Aktivkohle katalytisch hydriert. Der Katalysator wurde über Kieselgur abgesaugt und das Filtrat eingeengt. Der Rückstand wurde mit Diethylether verrieben, abgesaugt und getrocknet. Ausbeute: 1,623 g (82%).
FAB-MS: 337,3 (M + H)⁺

### 18e) ((R,S)-4-(4-(Amino-imino-methyl)-phenyl)-3-((4-biphenylyl)-methyl)-4-methyl-2,5-dioxoimidazolidin-1-yl)-acetyl-L-N-methyl-aspartyl-L-phenylglycin-tert-butylester

Zu einer Lösung von 123 mg ((R,S)-4-(4-(Amino-imino-methyl)phenyl)-3-((4-biphenyl)-methyl)-4-methyl-2,5-dioxoimidazolidin-1-yl)-essigsäure-Hydrochlorid (0,25 mmol), 84 mg H-(N-Methyl-Asp)-Phg-OBu^{t}-Hydrochlorid (0,25 mmol) und 33,8 mg HOBt (0,25 mmol) in 5 ml Dimethylformamid gab man bei 0 °C 55 mg DCC (0,25 mmol). Man ließ eine Stunde bei 0 °C und 4 Stunden bei Raumtemperatur rühren. Anschließend ließ man den Ansatz über Nacht bei Raumtemperatur stehen, saugte den Niederschlag ab und engte das Filtrat ein. Ausbeute: 270 mg Rohprodukt.
FAB-MS: 775,2 (M + H)⁺

### 18f) ((R,S)-4-(4-(Amino-imino-methyl)-phenyl)-3-((4-biphenylyl)-methyl)-4-methyl-2,5-dioxoimidazolidin-1-yl)-acetyl-L-N-methyl-aspartyl-L-phenylglycin

270 mg ((R,S)-4-(4-(Amino-imino-methyl)-phenyl)-3-((4-biphenylyl)-methyl)-4-methyl-2,5-dioxoimidazolidin-1-yl)-acetyl-L-N-methyl-aspartyl-L-phenylglycin-tert-butylester wurden in einer Mischung aus 2,7 ml Trifluoressigsäure und 0,3 ml Wasser gelöst. Man ließ eine Stunde bei Raumtemperatur stehen und engte im Wasserstrahlvakuum ein. Zur Reinigung wurde die Substanz an Sephadex LH20 mit einer Mischung aus Eisessig, n-Butanol und Wasser chromatographiert. Die Fraktionen mit der reinen Substanz wurden eingeengt. Der Rückstand wurde in Wasser gelöst und gefriergetrocknet. Ausbeute: 30 mg weißer Feststoff (15%).
FAB-MS: 719,0 (M + H)⁺

### Beispiel 19:

### ((R,S)-4-(4-(Amino-imino-methyl)-phenyl)-3-benzyl-4-methyl-2,5-dioxoinnidazolidin-1-yl)-acetyl-L-N-methyl-aspartyl-L-phenylglycin

Die Verbindung des Beispiels 19 wurde analog Beispiel 18 hergestellt.
FAB-MS: 643,2 (M + H)⁺

Die Verbindungen der Beispiele 20 bis 25 wurden analog Beispiel 2 hergestellt.

### Beispiel 20:

### ((R,S)-4-(4-(Amino-imino-methyl)-phenyl)-3,4-dibenzyl-2,5-dioxoimidazolidin-1-yl)-acetyl-L-aspartyl-L-phenylglycin

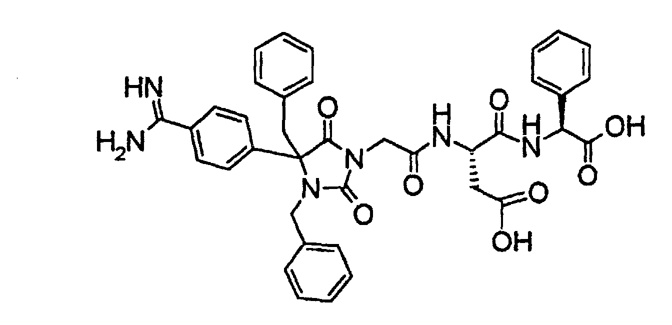

FAB-MS: 705,2 (M + H)⁺

### Beispiel 21:

### ((R,S)-4-(4-(Amino-imino-methyl)-phenyl)-3-benzyl-4-methyl-2,5-dioxoimidazolidin-1-yl)acetyl-L-aspartyl-L-valin

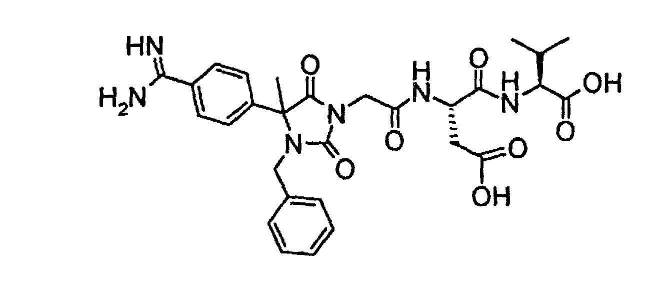

FAB-MS: 595,2 (M + H)⁺

### Beispiel 22:

### ((R,S)-4-(4-(Amino-imino-methyl)-phenyl)-3-benzyl-4-methyl-2,5-dioxoimidazolidin1-yl)-acetyl-L-aspartyl-L-leucin

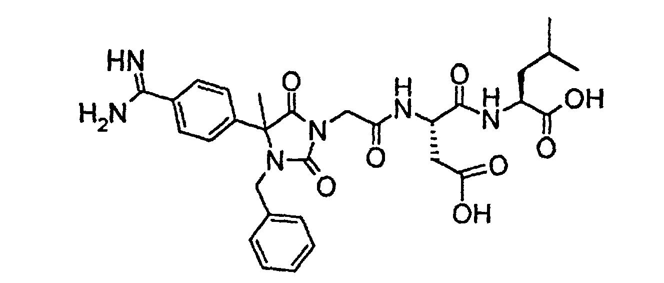

FAB-MS: 609,3 (M + H)⁺

### Beispiel 23:

### ((R,S)-4-(4-(Amino-imino-methyl)-phenyl)-3-benzyl-4-methyl-2,5-dioxoimidazolidin-1-yl)-acetyl-L-aspartyl-L-serin

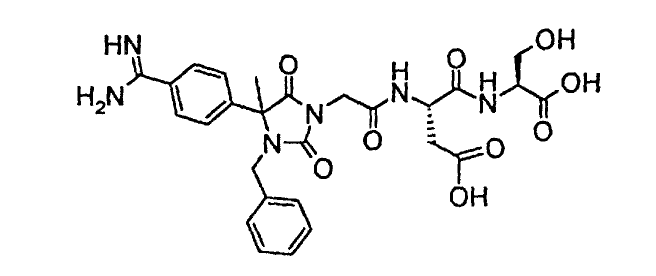

FAB-MS: 583,2 (M + H)⁺

### Beispiel 24:

### ((R,S)-4-(4-(Amino-imino-methyl)-phenyl)-3-benzyl-4-methyl-2,5-dioxoimidazolidin-1-yl)-acetyl-L-aspartyl-L-phenylalanin

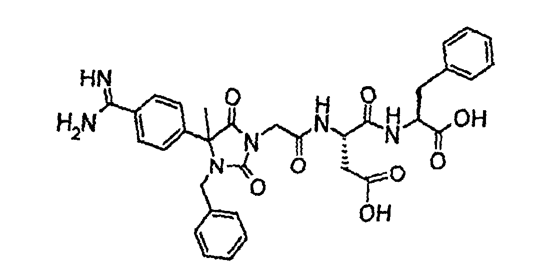

FAB-MS: 643,3 (M + H)⁺

### Beispiel 25:

### ((R,S)-4-(4-(Amino-imino-methyl)-phenyl)-3-benzyl-4-methyl-2,5-dioxoimidazolidin-1-yl)-acetyl-L-aspartyl-L-phenylglycinmethylester

FAB-MS: 643,2 (M + H)⁺

### Beispiel 26:

### ((R,S)-4-(4-(Amino-imino-methyl)-phenyl)-3-benzyl-4-methyl-2,5-dioxoimidazolidin-1-yl)-acetyl-L-aspartyl-(2-adamantyl-amid)

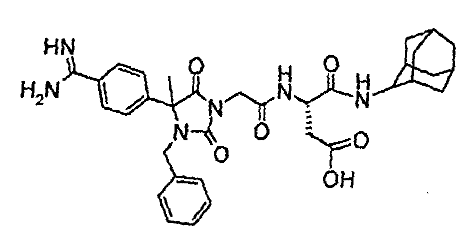

FAB-MS: 629,5 (M + H)⁺

### Beispiel 27:

### ((R,S)-4-(4-(Amino-imino-methyl)-phenyl)-3-benzyl-4-methyl-2,5-dioxoimidazolidin-1-yl)-acetyl-L-aspartyl-(1-adamantyl-amid)

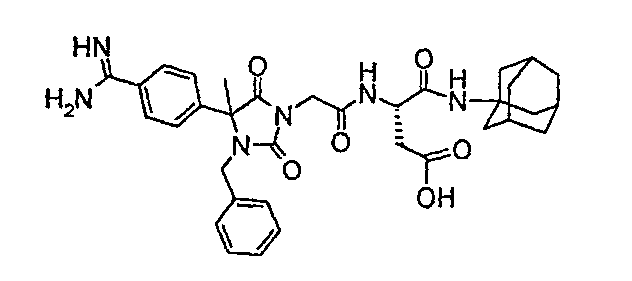

FAB-MS: 629,3 (M + H)⁺

### Beispiel 28:

### ((R,S)-4-(4-(Amino-imino-methyl)-phenyl)-3-benzyl-4-methyl-2,5-dioxoimidazolidin-1-yl)-acetyl-L-aspartyl-((1-adamantylmethyl)-amid)

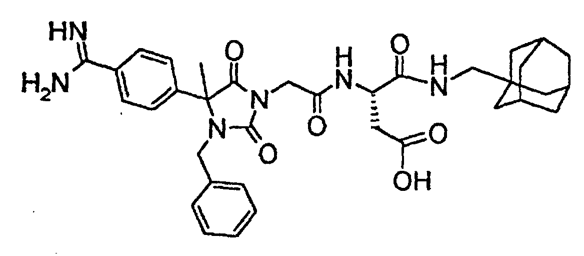

FAB-MS: 643,4 (M + H)⁺

Die Verbindungen der Beispiele 29 und 30 sind Diastereomere. Eine der Verbindungen der Beispiele 29 und 30 weist am Chiralitätszentrum an C-4 des Imidazolidinringes die (S)-Konfiguration auf, die andere die (R)-Konfiguration. Die Verbindungen wurden aus der Verbindung des Beispiels 4 durch Trennung mittels präparativer HPLC (Acetonitril/Wasser/Ammoniumacetat (17:83:0,1)) erhalten.

### Beispiel 29: Diastereomer I

### ((S oder R)-4-(4-(Amino-imino-methyl)-phenyl)-3-benzyl-4-methyl-2,5-dioxoimidazolidin-1-yl)-acetyl-L-aspartyl-L-phenylglycin

FAB-MS: 628,3 (M + H)⁺

### Beispiel 30: Diastereomer II

### ((R oder S)-4-(4-(Amino-imino-methyl)-phenyl)-3-benzyl-4-methyl-2,5-dioxoimidazolidin-1-yl)-acetyl-L-aspartyl-L-phenylglycin

FAB-MS: 628,3 (M + H)⁺

### Beispiel 31:

### (R,S)-3-(((R,S)-4-(4-(Amino-imino-methyl)-phenyl)-3,4-dimethyl-2,5-dioxoimidazolidin-1-yl)-acetylamino)-3-phenylpropionsäure-Hydrochlorid

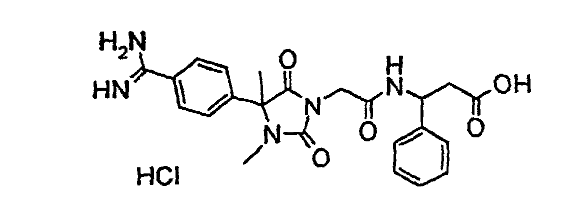

FAB-MS: 452 (M + H)⁺

### Beispiel 32:

### (R,S)-3-(((R,S)-4-(4-(Amino-imino-methyl)-phenyl)-3-ethyl-4-methyl-2,5-dioxoimidazolidin-1-yl)-acetylamino)-3-phenylpropionsäure-Hydrochlorid

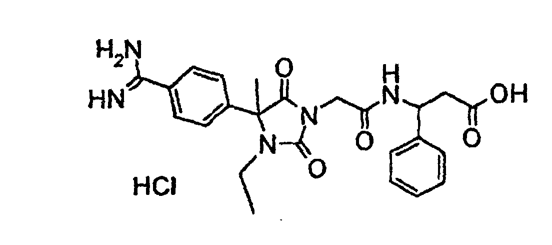

FAB-MS: 466 (M + H)⁺

### Beispiel 33:

### (R,S)-3-(((R,S)-4-(4-(Amino-imino-methyl)-phenyl)-3-benzyl-4-methyl-2,5-dioxoimidazolidin-1-yl)-acetylamino)-3-phenylpropionsäure-Hydrochlorid

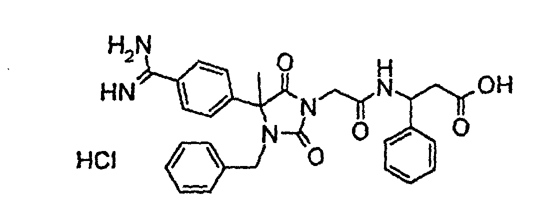

FAB-MS: 528,3 (M + H)⁺

### Beispiel 34:

### 3-(((R,S)-4-(4-(Amino-imino-methyl)-phenyl)-3-benzyl-4-methyl-2,5-diaxolmidazolidin-1-yl)-acetylamino)-propionsäure-Hydrochlorid

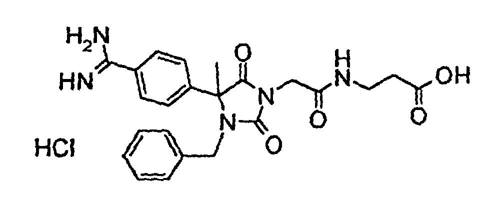

FAB-MS: 452,3 (M + H)⁺

### Beispiel 35:

### ((S)-4-(3-Aminopropyl)-3-ethyl-2,5-dioxoimidazolidin-1-yl)-acetyl-L-aspartyl-L-phenylglycin

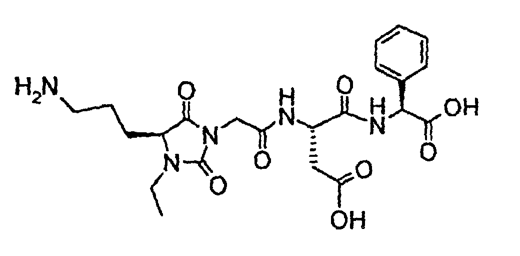

Die Verbindung wurde analog den Schritten a) bis e) des Beispiels 36 hergestellt und der der Verbindung des Beispiels 36e) entsprechende tert-Butylester mit Trifluoressigsäure gespalten.
FAB-MS: 492,6 (M + H)⁺

### Beispiel 36:

### ((S)-4-(3-Guanidinopropyl)-3-ethyl-2,5-dioxoimidazolidin-1-yl)-acetyl-L-aspartyl-D-phenylglycin

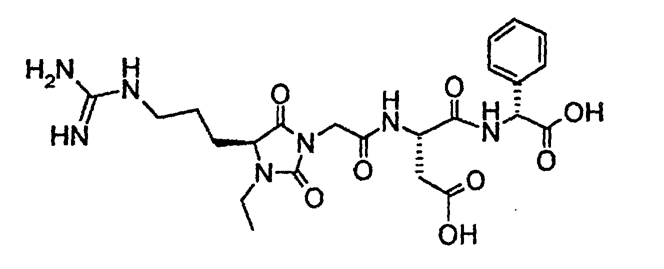

### 36a) ((S)-4-(3-Benzyloxycarbonylaminopropyl)-2,5-dioxoimidazolidin-1-yl)-essigsäure-ethylester

52 g (185,7 mmol) H-Orn(Z)-OCH₃ und 24,15 ml (185,7 mmol) Ethylmorpholin wurden in 500 ml DMF gelöst und auf 0 °C abgekühlt. Man ließ dann unter Rühren 23,77 ml (185,7 mmol) lsocyanato-essigsäure-ethylester zutropfen und über Nacht auf Raumtemperatur kommen. Zur Aufarbeitung wurde das DMF im Vakuum entfernt und der Rückstand in 500 ml Essigester aufgenommen. Die Essigesterlösung wurde mehrfach mit Wasser gewaschen, die Essigesterphase über Nacht auf 0 °C abgekühlt und das ausgefallene Produkt abfiltriert. Es wurde anschließend nochmals aus Essigester umkristallisiert. Ausbeute: 55,4 g (79%).
CI-MS: 378 (M + H)⁺

### 36b) ((S)-4-(3-Benzyloxycarbonylaminopropyl)-3-ethyl-2,5-dioxoimidazolidin-1-yl)-essigsäure-ethylester

6,74 g (17,8 mmol) ((S)-4-(3-Benzyloxycarbonylaminopropyl)-2,5-dioxoimidazolidin-l-yl)-essigsäure-ethylester wurden in 40 ml DMF auf 0 °C abgekühlt. Danach wurden portionsweise 0,49 g (20 mmol) Natriumhydrid zugegeben. Anschließend versetzte man mit 2,18 g (20 mmol) Ethylbromid und ließ über Nacht auf Raumtemperatur kommen. Nach Beendigung der Reaktion wurde das Lösungsmittel im Vakuum entfernt und das Rohprodukt durch Chromatographie an Kieselgel mit Methylenchlorid/Methanol/Essigsäure/Wasser (99:1:0,1:0,1) aufgetrennt. Ausbeute:
5,4 g (75%).
ES-MS: 406 (M + H)⁺

### 36c) ((S)-4-(3-Benzyloxycarbonylaminopropyl)-3-ethyl-2,5-dioxoimidazotidin-1-yl)-essigsäure

2,025 g (5 mmol) ((S)-4-(3-Benzyloxycarbonylaminopropyl)-3-ethyl-2,5-dioxoimidazolidin-1-yl)-essigsäure-ethylester wurden in 15 ml Ethanol gelöst und mit 50 ml einer 0,1 N LiOH-Lösung versetzt. Man ließ 4 Tage bei Raumtemperatur rühren. Nach Beendigung der Reaktion wurde mit 200 ml Wasser versetzt und mit Zitronensäure auf pH 3 gestellt. Man extrahierte die wäßrige Phase mit Essigester, wusch die organische Phase mehrmals mit Wasser und engte ein. Der Rückstand wurde an Kieselgel mit Methylenchlorid/Methanol (8:3) chromatographiert.
Ausbeute: 810 mg (40%).
ES-MS: 378,3 (M + H)⁺

### 36d) ((S)-4-(3-Benzyloxycarbonylaminopropyl)-3-ethyl-2,5-dioxoimidazolidin-1-yl)-acetyl-L-aspartyl-D-phenylglycin-di-tert-butylester

810 mg (2,15 mmol) ((S)-4-(3-Benzyloxycarbonylaminopropyl)-3-ethyl-2,5-dioxoimidazolidin-1-yl)-essigsäure wurden in 10 ml DMF mit 87 mg (2,36 mmol) DCC und 290 mg (2,15 mmol) HOBt versetzt und 30 Minuten gerührt. Anschließend wurden 928 mg (2,15 mmol) H-AspH-Asp(OBu^{t})-D-Phg-OBu^{t} und 280 µl (2,15 mmol) N-Ethylmorpholin zugegeben. Man ließ über Nacht reagieren. Nach Beendigung der Reaktion wurde das Lösungsmittel im Vakuum entfernt, der Rückstand in Methylenchlorid aufgenommen und der ausgefallene Dicyclohexylharnstoff abfiltriert. Die Lösung wurde eingeengt und der Rückstand an Kieselgel mit Methylenchlorid/Methanol/Essigsäure/Wasser (97:3:0,1:0,1) chromatographiert.
Ausbeute: 1,5 g (94%).
ES-MS: 738,4 (M + H)⁺

### 36e) ((S)-4-(3-Aminopropyl)-3-ethyl-2,5-dioxoimidazolidin-1-yl)-acetyl-L-aspartyl-D-phenylglycin-di-tert-butylester

1,5 g (2,03 mmol) ((S)-4-(3-Benzyloxycarbonylaminopropyl)-3-ethyl-2,5-dioxoimidazolidin-1-yl)-acetyl-L-aspartyl-D-phenylglycin-di-tert-butylester wurden in 70 ml Methanol gelöst, mit 0,5 g 10 %igem PalladiumlAktivkohle-Katalysator versetzt und hydriert. Nach Beendigung der Reaktion wurde der Katalysator abgesaugt, das Filtrat eingeengt und der Rückstand im Vakuum getrocknet.
Ausbeute: 1,2 g (92%).
MS: 604,3 (M + H)⁺

### 36f) ((S)-4-(3-Guanidinopropyl)-3-ethyl-2,5-dioxoimidazolidin-1-yl)-acetyl-L-aspartyl-D-phenylglycin-di-tert-butylester

1 g (1,56 mmol) ((S)-4-(3-Aminopropyl)-3-ethyl-2,5-dioxoimidazolidin-1-yl)-acetyl-L-aspartyl-D-phenylglycin-di-tert-butylester wurden in 17 ml DMF gelöst. Zu der Lösung gab man 0,228 g (1,56 mmol) 1H-Pyrazol-1-carboxamidin-Hydrochlorid und 0,8 ml (4,68 mmol) Diisopropyl-ethylamin hinzu und ließ über Nacht reagieren. Nach Beendigung der Reaktion wurde das Lösungsmittel im Vakuum entfernt und der Rückstand durch Chromatographie an Kieselgel mit Methylenchlorid/Methanol/Essigsäure/Wasser (95:5:0,5:0,5) gereinigt. Ausbeute:
0,68 g (68%).
ES-MS: 646,4 (M + H)⁺

### 36g) ((S)-4-(3-Guanidinopropyl)-3-ethyl-2,5-dioxoimidazolidin-1-yl)-acetyl-L-aspartyl-D-phenylglycin

0,68 g (1,05 mmol) ((S)-4-(3-Guanidinopropyl)-3-ethyl-2,5-dioxoimidazolidin-1-yl)-acetyl-L-aspartyl-D-phenylglycin-di-tert-butylester wurden in 10 ml einer Mischung aus Trifluoressigsäure und Wasser (95:5) gelöst. Nach 30 Minuten wurde die Reaktionslösung im Vakuum eingeengt und der Rückstand in 100 ml Wasser aufgenommen. Anschließend wurde mit Amberlite IRA 93/45 in das Essigsäuresalz umgewandelt und durch Chromatographie über Sephadex G25 mit 1 M Essigsäure gereinigt. Ausbeute: 0,181 g (32%).
FAB-MS: 534,3 (M + H)⁺

### Beispiel 37:

### ((S)-4-(3-Aminopropyl)-3-benzyl-2,5-dioxoimidazolidin-1-yl)-acetyl-L-aspartyl-L-phenylglycin

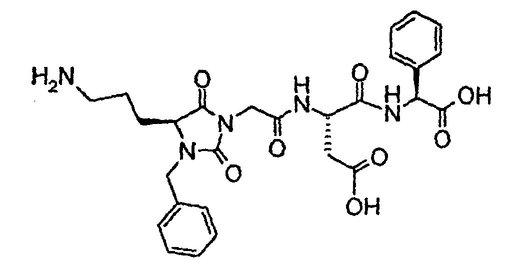

Die Verbindung des Beispiels 37 wurde analog Beispiel 35 hergestellt.
FAB-MS: 553,6 (M + H)⁺

Die Verbindungen der Beispiele 38 bis 40 wurden analog Beispiel 36 hergestellt.

### Beispiel 38:

### ((S)-4-(3-Guanidinopropyl)-3-benzyl-2, 5-dioxoimidazolidin-1-yl)-acetyl-L-aspartyl-L-phenylglycin

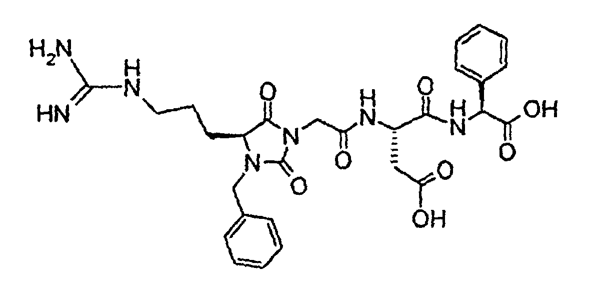

FAB-MS: 596,4 (M + H)⁺

### Beispiel 39:

### ((S)-4-(3-Guanidinopropyl)-3-benzyl-2,5-dioxoimidazolidin-1-yl)-acetyl-D-aspartyl-L-phenylglycin-L-tyrosin-Hydrochlorid

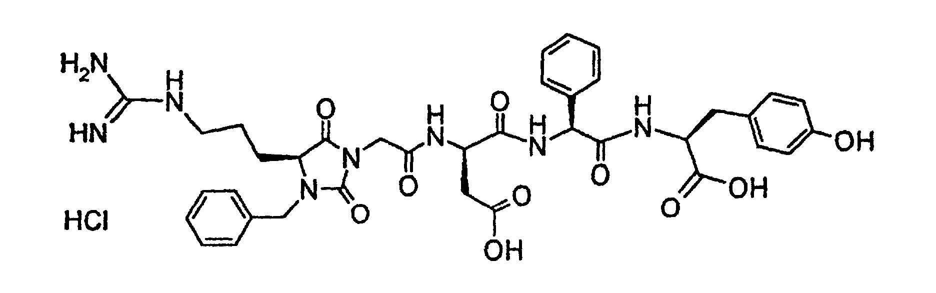

FAB-MS: 792,4 (M + H)⁺

### Beispiel 40:

### ((S)-4-(3-Guanidinopropyl)-3-benzyl-2,5-dioxoimidazolidin-1-yl)-acetyl-D-glutamyl-L-phenylglycin-L-tyrosin-Hydrochlorid

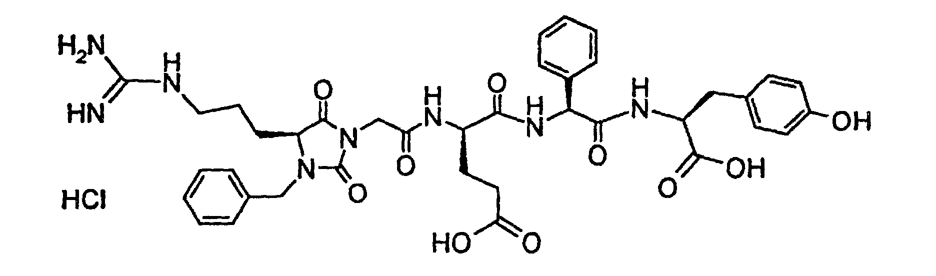

FAB-MS: 806,4 (M + H)⁺

### Beispiel 41:

### ((S)-4-(4-(Amino-imino-methyl)-phenyl)-3-((2-naphthyl)-methyl)-4-methyl-2,5-dioxoimidazolidin-1-yl)-acetyl-L-aspartyl-L-phenylglycin

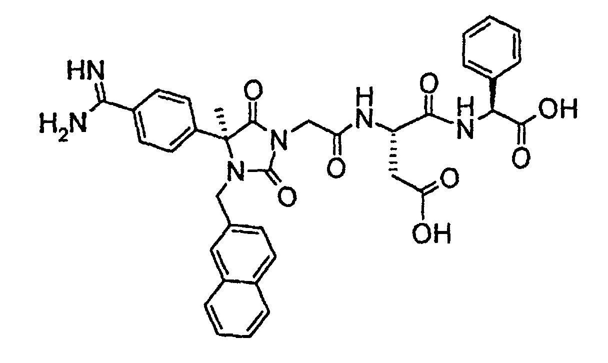

Die Verbindung wurde hergestellt durch Trennung von ((R,S)-4-(4-(Amino-iminomethyl)-phenyl)-3-((2-naphthyl)-methyl)-4-methyl-2,5-dioxoimidazolidin-1-yl)-acetyl-L-aspartyl-L-phenylglycin (siehe Beispiel 9) mittels MPLC über Kieselgel.
ES(+)-MS: 679,3 (M+H)⁺

### Beispiel 42:

### (R,S)-3-(((R,S)-4-(4-(Amino-imino-methyl)-phenyl)-3-benzyl-4-methyl-2,5-dioxoimidazolidin-1-yl)-acetylamino)-2-(4-methoxy-benzyl)-propionsäure

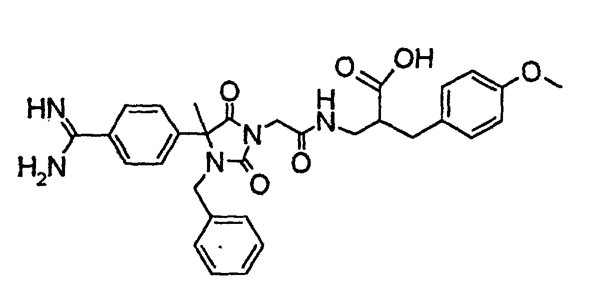

ES(+)-MS: 572,3 (M+H)⁺

### Beispiel 43:

### (R,S)-3-(((R,S)-4-(4-(Amino-imino-methyl)-phenyl)-3-benzyl-4-methyl-2,5-dioxoimidazolidin-1-yl)-acetylamino)-2-phenyl-propionsäure

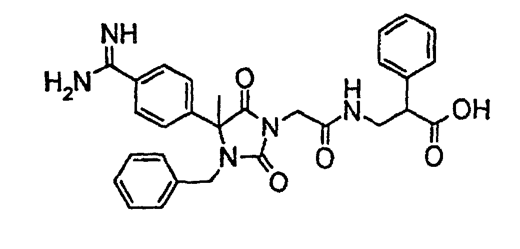

ES(+)-MS: 528,3 (M+H)⁺

### Beispiel 44:

### (R,S)-3-(((R,S)-4-(4-(Amino-imino-methyl)-phenyl)-3-benzyl-4-methyl-2,5-dioxoimidazolidin-1-yl)-acetylamino)-2-((1-naphthyl)-methyl)-propionsäure

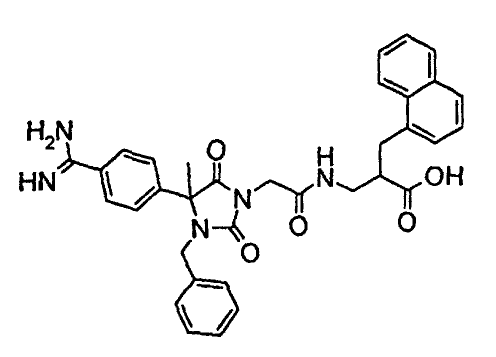

ES(+)-MS: 592,4 (M+H)⁺

### Beispiel 45:

### (R,S)-3-(((R,S)-4-(4-(Amino-imino-methyl)-phenyl)-3-benzyl-4-methyl-2,5-dioxoimidazolidin-1-yl)-acetylamino)-2-(4-tert-butyl-benzyl)-propionsäure

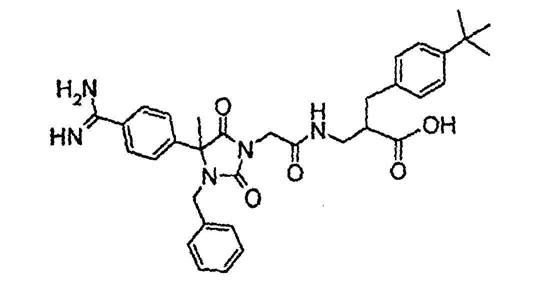

ES(+)-MS: 598,4 (M+H)⁺

### Beispiel 46:

### (S)-3-(((R,S)-4-(4-(Amino-imino-methyl)-phenyl)-3-benzyl-4-methyl-2,5-dioxoimidazolidin-1-yl)-acetylamino)-2-benzyloxycarbonylamino-propionsäure

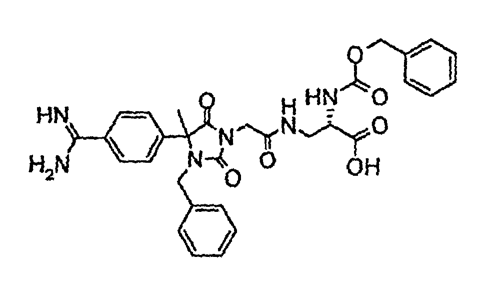

### 46a) (S)-3-Amino-2-benzyloxycarbonylamino-propionsäure-tert-butylester

10 (42 mmol) (S)-3-Amino-2-benzyloxycarbonylamino-propionsäure wurden in einem Gemisch aus 100 ml Dioxan, 100 ml Isobutylen und 8 ml konz. H₂SO₄ 3 Tage bei 20 atm N₂-Druck im Autoklaven geschüttelt. Überschüssiges Isobutylen wurde abgeblasen und zur verbleibenden Lösung wurden 150 ml Diethylether und 150 ml gesättigte NaHCO₃-Lösung gegeben. Die Phasen wurden getrennt und die wäßrige Phase wurde 2 x mit je 100 ml Diethylether extrahiert. Die vereinigten organischen Phasen wurden mit 2 x 100 ml Wasser gewaschen und über Na₂SO₄ getrocknet. Nach Entfernen des Lösungsmittels im Vakuum erhielt rear: 9,58 g (78 %) Produkt als blaßgelbes Öl.

### 46b) (S)-3-(((R,S)-4-(4-(Amino-imino-methyl)-phenyl)-3-benzyl-4-methyl-2,5-dioxoimidazolidin-1-yl)-acetylamino)-2-benzyloxycarbonylamino-propionsäure-tert-butylester

208 mg (0,5 mmol) ((R,S)-4-(4-(Ammino-imino-methyl)phenyl)-3-benzyl-4-methyl-2,5-dioxoimidazolidin-1-yl)-essigsäure-Hydrochlorid (hergestellt analog Beispiel 2a unter Verwendung von Benzylbromid an Stelle von Methyliodid und anschließenden Folgereaktionen, siehe Beispiele 2b - 2d) und 81,5 mg (0,5 mmol) HOOBt wurden in 5 ml DMF suspendiert und bei 0 °C mit 110 mg (0,55 mmol) DCC versetzt. Man rührte 1 h bei 0 °C und 1 h bei RT und gab anschließend 147 mg (0,5 mmol) (S)-3-Amino-2-benzyloxycarbonylamino-propionsäure-tert-butylester zu, rührte 2 h bei Raumtemperatur und ließ über Nacht bei Raumtemperatur stehen. Das Lösungsmittel wurde im Vakuum entfernt und der Rückstand mit Dichlormethan/Methanol/Eisessig/Wasser (9:1:0,1:0,1) über Kieselgel chromatographiert. Nach Einengen und Gefriertrocknen erhielt man 225 mg (69 %) (S)-3-(((R,S)-4-(4-(Amino-imino-methyl)-phenyl)-3-benzyl-4-methyl-2,5-dioxoimidazolidin-1-yl)-acetylamino)-2-benzyloxycarbonylamino-propionsäure-tert-butylester als farblosen Feststoff.

### 46c) (S)-3-(((R,S)-4-(4-(Amino-imino-methyl)-phenyl)-3-benzyl-4-methyl-2,5-dioxoimidazolidin-1-yl)-acetylamino)-2-benzyloxycarbonylamino-propionsäure

220 mg (0,33 mmol) (S)-3-(((R,S)-4-(4-(Amino-imino-methyl)-phenyl)-3-benzyl-4-methyl-2,5-dioxoimidazolidin-1-yl)-acetylamino)-2-benzyloxycarbonylamino-propionsäure-tert-butylester wurden in 5 ml 90 %iger Trifluoressigsäure 1 h bei Raumtemperatur stehen gelassen. Nach Einengen im Vakuum wurde der Rückstand mit Dichlormethan/Methanol/Eisessig/Wasser (8:2:0,2:0,2) über Kieselgel chromatographiert. Nach Einengen der Produktfraktionen und Gefriertrocknen erhielt man 155 mg (78 %) (S)-3-(((R,S)-4-(4-(Amino-imino-methyl)-phenyl)-3-benzyl-4-methyl-2,5-dioxoimidazolidin-1-yl)-acetylamino)-2-benzyloxycarbonylamino-propionsäure als farblosen Feststoff. FAB-MS: 601,3 (M+H)⁺

### Beispiel 47:

### (R)-3-(((R,S)-4-(4-(Amino-imino-methyl)-phenyl)-3-((2-naphthyl)-methyl)-4-methyl-2,5-dioxoimidazolidin-1-yl)-acetylamino)-2-benzyloxycarbonylamino-propionsäure

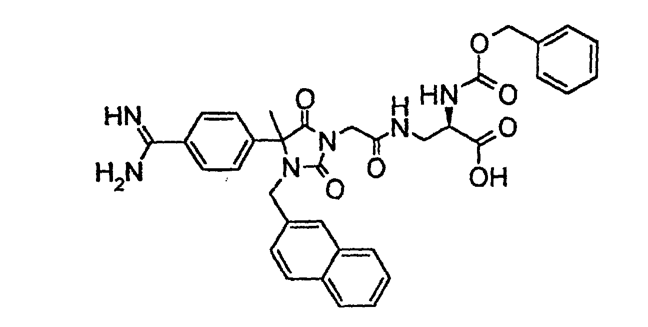

Die Synthese erfolgte analog Beispiel 46 unter Verwendung von (R)-3-Amino-2-benzyloxycarbonylamino-tert-butylester an Stelle des (S)-Isomeren und 2-Brommethylnaphthalin an Stelle von Benzylbromid.
ES(+)-MS: 651,3 (M+H)⁺

### Beispiel 48:

### (S)-3-(((R,S)-4-(4-(Amino-imino-methyl)-phenyl)-3-benzyl-4-methyl-2,5-dioxoimidazolidin-1-yl)-acetyl-N-methylamino)-2-benzyloxycarbonylaminopropionsäure

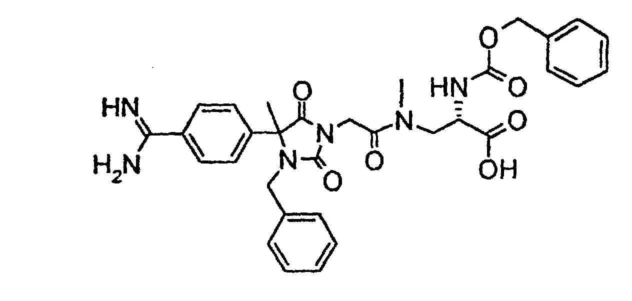

Die Synthese erfolgte analog Beispiel 46 unter Verwendung von (S)-2-Benzyloxycarbonylamino-3-(N-methyl-amino)-propionsäure-tert-butylester (hergestellt aus (S)-3-Amino-2-benzyloxycarbonylamino-propionsäure-tert-butylester analog S.C. Miller, T.S. Scanlan, J. Am. Chem. Soc. 1997, 119, 2301).
ES(+)-MS: 615,3 (M+H)⁺

### Beispiel 49:

### ((R,S)-4-(4-(Amino-imino-methyl)-phenyl)-3-benzyl-4-methyl-2,5-dioxoimidazolidin-1-yl)-acetyl-L-asparaginsäure

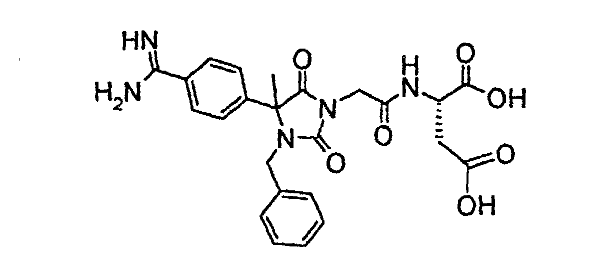

ES(+)-MS: 496,1 (M+H)⁺

### Beispiel 50:

### ((R,S)-2-((S)-4-(4-(Amino-imino-methyl)-phenyl)-3-benzyl-4-methyl-2,5-dioxoimidazolidin-1-yl)-2-(2-methylpropyl)-acetyl)-L-aspartyl-L-phenylglycin

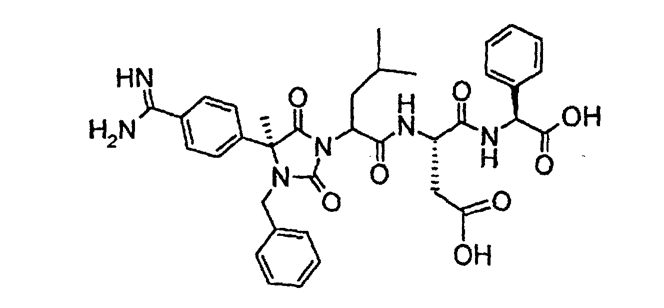

### 50a) (S)-4-(4-Cyano-phenyl)-4-methyl-2,5-dioxoimidazolidin (50.1)

6 g (22,2 mmol) (S)-4-(4-Bromo-phenyl)-4-methyl-2,5-dioxoimidazolidin und 9 g (100,2 mmol) Kupfer(I)cyanid wurden in 57 ml DMF 5 h unter Rückfluß erhitzt. Nach Abkühlen auf Raumtemperatur wurde das Gemisch mit Wasser und Essigester versetzt und unter Eiskühlung mit 2 N Salzsäure auf einen pH-Wert von 2 gestellt. Nach Filtration wurde die Wasserphase 2 x mit Essigester extrahiert. Die vereinigten organischen Phasen wurden mit Wasser und gesättigter Natriumchlorid-Lösung gewaschen, über Natriumsulfat getrocknet und nach Filtration im Vakuum eingeengt. Das Rohprodukt wurde mit Dichlormethan/Methanol (95:5) über Kieselgel chromatographiert. Nach Einengen der Produktfraktionen erhielt man 2,74 g (57 %) (50.1).

### 50b) (R,S)-2-((S)-4-(4-Cyano-phenyl)-4-methyl-2,5-dioxoimidazolidin-1-yl)-4-methyl-pentansäure-tert-butylester (50.2)

Man gab zu einer Lösung von 1 g (4,64 mmol) (50.1) in 15 ml absolutem DMF unter Argon 128 mg (5,35 mmol) Natriumhydrid, ließ 2 h bei Raumtemperatur rühren, gab 1,23 g (4,9 mmol) D,L-2-Brom-4-methylpentansäure-tert-butylester (50.7) (Herstellung siehe 50g) zu und rührte 4 h bei Raumtemperatur. Nach Zugabe von weiteren 40 mg Natriumhydrid ließ man das Gemisch 3 Tage bei Raumtemperatur stehen, entfernte das Lösungsmittel im Vakuum und verteilte den Rückstand zwischen Essigester und Wasser. Man stellte mit gesättigter KHSO₄/K₂SO₄-Lösung einen pH-Wert von 4 ein, trennte die Phasen und extrahierte die wäßrige Phase 2 x mit Essigester. Die vereinigten organischen Phasen wurden über Natriumsulfat getrocknet, das Trockenmittel wurde abfiltriert und das Filtrat im Vakkum eingeengt. Der Rückstand wurde mit Heptan/Essigester (2:1, dann 1:2), über Kieselgel filtriert. Nach Einengen der Produktfraktionen erhielt man 666 mg (37 %) (50.2).

### 50c) (R,S)-2-((S)-4-(4-Cyano-phenyl)-3-benzyl-4-methyl-2,5-dioxoimidazolidin-1-yl)-4-methyl-pentansäure-tert-butylester (50.3)

Man gab zu einer Lösung von 990 mg (2,56 mmol) (50.2) in absolutem DMF unter Argon bei 0 °C 74 mg (3,07 mmol) Natriumhydrid, ließ 1 h bei Raumtemperatur rühren, gab 334 µl (2,81 mmol) Benzylbromid zu und ließ 1,5 h bei Raumtemperatur rühren. Das Lösungsmittel wurde im Vakuum entfernt, der Rückstand zwischen Wasser und Essigester verteilt und nach Phasentrennung die Wasserphase mit Essigester extrahiert. Die vereinigten organischen Phasen wurden über Natriumsulfat getrocknet, das Trockenmittel wurde abfiltriert und das Filtrat im Vakuum eingeengt. Man erhielt 1,22 g (100 %) (50.3).

### 50d) (R,S)-2-((S)-4-(4-(Amino-hydroximino-methyl)-phenyl)-3-benzyl-4-methyl-2,5-dioxoimidazolidin-1-yl)-4-methyl-pentansäure-tert-butylester (50.4)

Man gab zu einer Lösung von 1,21 g (2,54 mmol) (50.3) in 30 ml Ethanol 353 mg (6,08 mmol) Hydroxylammoniumchlorid und 1,05 ml (7,62 mmol) Triethylamin und erhitzte das Gemisch 2 h unter Rückfluß. Das Lösungsmittel wurde im Vakuum entfernt, der Rückstand in Wasser/Essigester aufgenommen und nach Phasentrennung die Wasserphase 2 x mit Essigester extrahiert. Die vereinigten organischen Phasen wurden über Natriumsulfat getrocknet. Das Trockenmittel wurde ab filtriert und das Lösungsmittel im Vakuum entfernt, Man erhielt 1,16 g (90 %) (50.4).

### 50e) (R,S)-2-((S)-4-(4-(Amino-imino-methyl)-phenyl)-3-benzyl-4-methyl-2,5-dioxoimidazolidin-1-yl)-4-methyl-pentansäure-Hydrochlorid (50.5)

Eine Lösung von 850 mg (1,67 mmol) (50.4) in 50 ml Essigsäure wurde über Raney-Nickel hydriert. Nach 2 h wurde der Katalysator abfiltriert, das Filtrat im Vakuum eingeengt, der Rückstand in 10 %iger Essigsäure gelöst, die Lösung gefriergetrocknet und der Rückstand in 10 ml 90 %iger Trifluoressigsäure gelöst. Nach 15 min bei Raumtemperatur wurde die Trifluoressigsäure im Vakuum entfernt, der Rückstand 2 x mit Toluol eingeengt, mit 0,5 N Salzsäure versetzt und gefriergetrocknet. Man erhielt 700 mg (87 %) (50.5).

### 50f) ((R,S)-2-((S)-4-(4-(Amino-imino-methyl)-phenyl)-3-benzyl-4-methyl-2,5-dioxoimidazolidin-1-yl)-2-(2-methylpropyl)-acetyl)-L-aspartyl-L-phenylglycin (50.6)

Zu einer Lösung von 200 mg (0,422 mmol) (50.5) und 175 mg (0,422 mmol) H-Asp(O^{t}Bu)-Phg-O^{t}Bu-Hydrochloridin 20 ml absolutem DMF wurden 138 mg (0,422 mmol) TOTU (O-[Cyan(ethoxycarbonyl)methylenamino]-1,1,3,3-tetramethyluroniumtetrafluoroborat) und 216 µl (1,26 mmol) Diisopropyl-ethylamin gegeben. Nach 2 h Rühren bei Raumtemperatur wurde das Reaktionsgemisch im Vakuum eingeengt, der Rückstand in Essigester aufgenommen und die organische Phase 2 x mit gesättigter NaHCO₃-Lösung und Wasser gewaschen. Nach Trocknen über Natriumsulfat, Filtration und Einengen des Filtrats im Vakuum erhielt man 320 mg Rohprodukt, das mit Dichlormethan/Methanol/Eisessig/Wasser (9:1:0,1:0,1) über Kieselgel chromatographiert wurde. Nach Einengen der Produktfraktionen wurde der Rückstand in 5 ml 90%iger Trifluoressigsäure gelöst, nach 15 min bei Raumtemperatur die Trifluoressigsäure im Vakuum entfernt, der so erhaltene Rückstand 2 x mit Toluol eingeengt und der Rückstand schließlich in 20 %iger Essigsäure gelöst und gefriergetrocknet. Man erhielt 132 mg (46 %) (50.6).
ES(+)-MS: 685,4 (M+H)⁺

### 50g) Synthese von D,L-2-Brom-4-methylpentansäure-tert-butylester (50.7)

Man gab zu einer Lösung von 2,5 g (12,8 mmol) D,L-2-Brom-4-methylpentansäure in 80 ml Chloroform und 80 ml tert-Butylacetat 1,96 ml konzentrierte Schwefelsäure und 0,515 ml Oleum (20 %ig) und ließ das Gemisch 3 h bei Raumtemperatur rühren. Man stellte durch Zugabe von 10 %iger NaHCO₃-Lösung einen pH Wert von 4 ein. Die wäßrige Phase wurde abgetrennt und 2 x mit Dichlormethan extrahiert. Die vereinigten organischen Phasen wurden über Natriumsulfat getrocknet. Nach Filtration und Einengen des Filtrats im Vakuum erhielt man 2,62 g (82 %) (50.7).

### Beispiele 51 und 52:

Die Verbindungen der Beispiele 51 und 52 sind Diastereomere. Sie wurden erhalten durch Trennung des Diastereomerengemisches (50.6) aus Beispiel 50 mittels präparativer HPLC (RP-18; Eluens A/B = 60:40; A Wasser/0,1 1 % Trifluoressigsäure; B = 80 % Acetonitril/20 % Wasser/0,1 % Trifluoressigsäure). Eine der Verbindungen der Beispiele 51 und 52 weist am Chiralitätszentrum in der 2-(2 2-(2-Methylpropyl)-acetyl-Einheit -Einheit (R)-Konfiguration auf, die andere (S)-Konfiguration.

### Beispiel 51:

### ((R oder S)-2-((S)-4-(4-(Amino-imino-methyl)-phenyl)-3-benzyl-4-methyl-2,5-dioxoimidazolidin-1-yl)-2-(2-methylpropyl)-acetyl)-L-aspartyl-L-phenylglycin

ES(+)-MS: 685,4 (M+H)⁺

### Beispiel 52:

### ((S oder R)-2-((S)-4-(4-(Amino-imino-methyl)-phenyl)-3-benzyl-4-methyl-2,5-dioxoimidazolidin-1-yl)-2-(2-methylpropyl)-acetyl)-L-aspartyl-L-phenylglycin

ES(+)-MS: 685,4 (M+H)⁺

### Beispiel 53:

### (R,S)-3-((R,S)-2-((S)-4-(4-(Amino-imino-methyl)-phenyl)-3-benzyl-4-methyl-2,5-dioxoimidazolidin-1-yl)-2-(2-methylpropyl)-acetylamino)-3-(2,4-dimethoxy-phenyl)-propionsäure

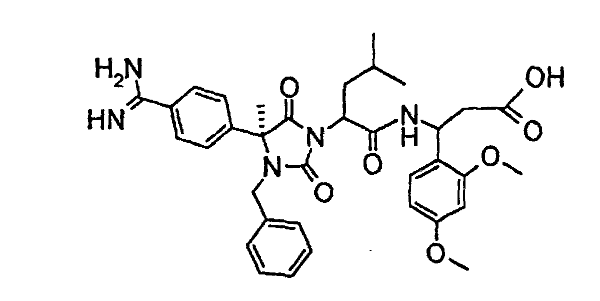

Die Verbindung wurde hergestellt durch Kupplung von (R,S) 2-((S)-4-(4-(Aminoimino-methyl)-phenyl)-3-benzyl-4-methyl-2,5-dioxoimidazolidin-1-yl)-4-methylpentansäure-Hydrochlorid (50.5) und (R,S)-3-Amino-3-(2,4-dimethoxy-phenyl)-propionsäure-tert-butylester-Hydrochlorid und anschließende Spaltung des tert-Butylesters wie in Beispiel 50 beschrieben.
ES(+)-MS: 644,4 (M+H)⁺

3-Substituierte β-Aminosäuren und β-Aminosäureester, wie sie im Beispiel 53 und anderen Beispielen eingesetzt wurden, wurden nach der folgenden allgemeinen Synthesevorschrift für die Herstellung von 3-substituierten β-Aminosäuren und β-Aminosäureestern erhalten.

Racemische 3-substituierte β-Aminosäuren wurden analog W. M. Radionow, E.A.Postovskaya, J. Am. Chem. Soc. 1929, 51, 841 (siehe auch: Houben-Weyl, Methoden der Organischen Chemie, Band XI/2, Georg Thieme Verlag, Stuttgart, 1958, S. 497) hergestellt. Aus diesen Säuren wurden nach literaturbekannten Verfahren die Methylester bzw. Ethylester hergestellt. Die tert-Butylester der 3-substituierten β-Aminosäuren wurden aus diesen Säuren hergestellt, indem sie zunächst in die β-Benzyloxycarbonylamino-säuren -säuren überführt wurden. Aus diesen wurden dann nach der folgenden allgemeinen Synthesevorschrift die tert-Butylester hergestellt: Zu 1 mmol der β-Benzyloxycarbonylamino-carbonsäure -carbonsäure in 13 ml absolutem Dichlormethan wurden 1,5 mmol Oxalylchlorid gegeben. Nach 4 h Rühren bei Raumtemperatur wurden das Reaktionsgemisch eingeengt und zum Rückstand wurden 6,5 ml tert-Butanol gegeben. Es wurde 1 h bei Raumtemperatur gerührt und das Reaktionsgemisch im Vakuum eingeengt. Der Rückstand wurde in Essigester aufgenommen und 2 x mit gesättigter NaHCO₃-Lösung und Wasser extrahiert. Die organische Phase wurde über Natriumsulfat getrocknet und nach Filtration das Lösungsmittel im Vakuum entfernt. Zur Herstellung der β-Aminosäure-tert-butylester-Hydrochloride wurde anschließend die Benzyloxycarbonylgruppe über 10 % Pd/C in Methanol/HCl hydrogenolytisch abgespalten.

Enantiomerenreine 3-substituierte β-Aminosäureester wurden analog S.G. Davis, O. Ichihara, Tetrahedron Asymmetry, 1991, 2, 183; S.G. Davis, N.M.Garrido, O. Ichihara, I.A.S. Walters, J. Chem. Soc., Chem. Commun., 1993, 1153; S.G. Davis, I.A.S. Walters, J. Chem. Soc., Perkin Trans. 1, 1994, 1129 hergestellt.

### Beispiel 54:

### (R,S)-3-((R,S)-2-((S)-4-(4-(Amino-imino-methyl)-phenyl)-3-benzyl-4-methyl-2,5-dioxoimidazolidin-1-yl)-2-(2-methylpropyl)-acetylamino)-3-(3,4-methylendioxyphenyl)-propionsäure

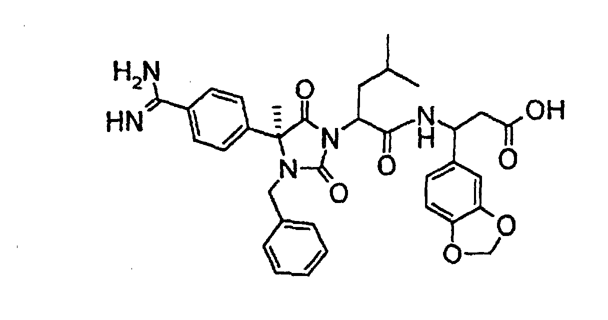

Die Verbindung wurde hergestellt durch Umsetzung von (50.5) mit (R,S)-3-Amino-3-(3,4-methylendioxy-phenyl)-propionsäure-tert-butylester-Hydrochlorid und anschließende Spaltung des tert-Butylesters wie in Beispiel 50 beschrieben.
ES(+)-MS: 628,4 (M+H)⁺

Analog zu Beispiel 54 wurden die Beispiele 55 bis 60 durchgeführt.

### Beispiel 55:

### (R,S)-3-(((R,S)-4-(4-(Amino-imino-methyl)-phenyl)-3-benzyl-4-methyl-2,5-dioxoimidazolidin-1-yl)-acetylamino)-3-(1-naphthyl)-propionsäure

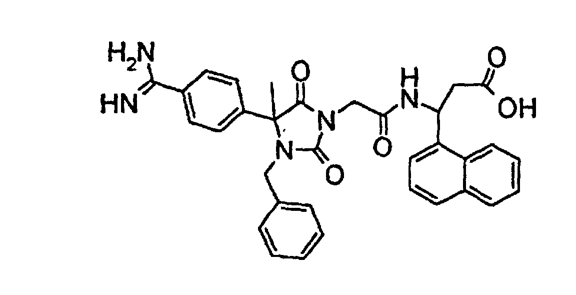

ES(+)-MS: 578,3 (M+H)⁺

### Beispiel 56:

### (R,S)-3-(((R,S)-4-(4-(Amino-imino-methyl)-phenyl)-3-benzyl-4-methyl-2,5-dioxoimidazolidin-1-yl)-acetylamino)-buttersäure

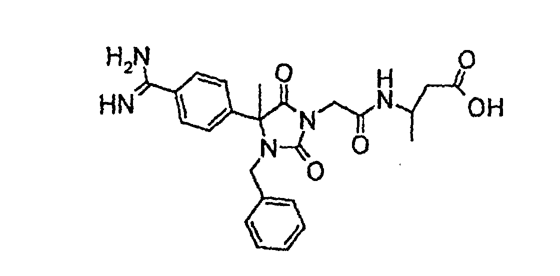

ES(+)-MS: 466,2 (M+H)⁺

### Beispiel 57:

### (R,S)-3-(((R,S)-4-(4-(Amino-imino-methyl)-phenyl)-3-benzyl-4-methyl-2,5-dioxoimidazolidin-1-yl)-acetylamino)-3-(3,4-dimethoxy-phenyl)-propionsäure

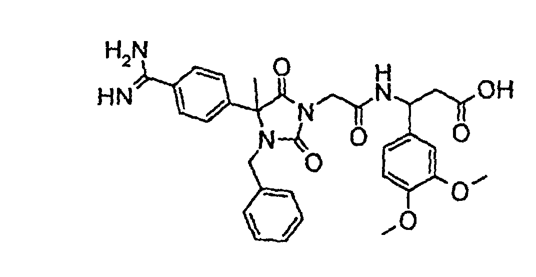

FAB-MS: 588,3 (M+H)⁺

### Beispiel 58:

### (R,S)-3-(((R,S)-4-(4-(Amino-imino-methyl)-phenyl-3-benzyl-4-methyl-2, 5-dioxoimidazolidin-1-yl)-acetylamino)-3-(3,4-ethylendioxy-phenyl)-propionsäure

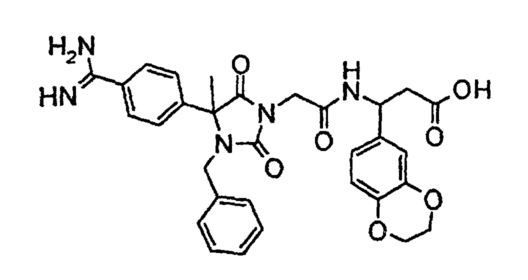

ES(+)-MS: 586,2 (M+H)⁺

### Beispiel 59:

### (R,S)-3-(((R,S)-4-(4-(Amino-imino-methyl)-phenyl)-3-benzyl-4-methyl-2,5-dioxoimidazolidin-1-yl)-acetylamino)-3-(3,5-dimethoxy-phenyl)-propionsäure

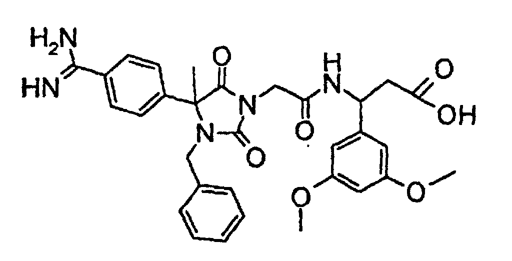

ES(+)-MS: 588,2 (M+H)⁺

### Beispiel 60:

### (R,S)-3-(((R,S)-4-(4-(Amino-imino-methyl)-phenyl)-3-benzyl-4-methyl-2,5-dioxoimidazolidin-1-yl)-acetylamino)-3-(3-methoxy-phenyl)-propionsäure

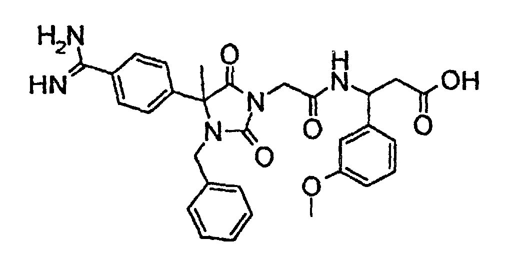

ES(+)-MS: 558,2 (M+H)⁺

### Beispiel 61:

### (R,S)-3-(((R,S)-4-(4-(Amino-imino-methyl)-phenyl)-3-benzyl-4-methyl-2,5-dioxoimidazolidin-1-yl)-acetylamino)-3-(2-methoxy-phenyl)-propionsäure

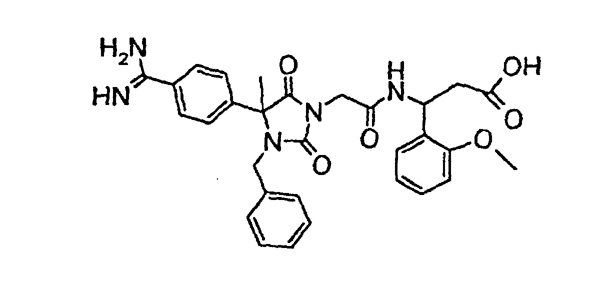

ES(+)-MS: 558,2 (M+H)⁺

### Beispiel 62:

### ((R,S)-2-((R,S)-4-(4-(Amino-imino-methyl)-phenyl)-3-benzyl-4-methyl-2,5-dioxaimidazolidin-1-yl)-2-methyl-acetyl)-L-aspartyl-L-phenylglycin

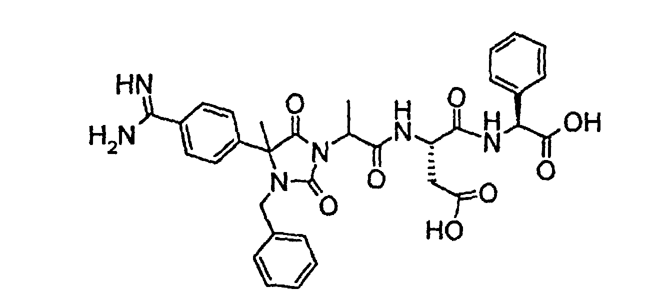

Die Verbindung wurde analog Beispiel 50 hergestellt, indem man racemisches (50.1) mit (R,S)-2-Brom-propionsäureethylester umsetzte und vor der Peptidkupplung mit H-Asp(O^{t}Bu)-Phg-O^{t}Bu x HCI den Ethylester mit 6 N Salzsäure spaltete.
ES(+)-MS: 643,3 (M+H)⁺

### Beispiel 63:

### ((S)-4-(4-(Amino-imino-methyl)-phenyl)-3-benzyl-4,methyl-2,5-dioxoimidazolidin-1-yl)-acetyl-L-aspartyl-L-phenylglycin

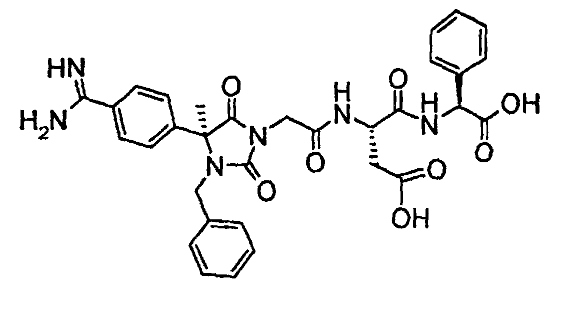

Die Verbindung wurde analog Beispiel 50 hergestellt, indem man (50.1) mit 2-Bromessigsäureethylester umsetzte und vor der Peptidkupplung mit H-Asp(OtBu)-Phg-O^{t}Bu x HCl den Ethylester mit 6 N Salzsäure spaltete.
ES(+)-MS: 629,3 (M+H)⁺

### Beispiel 64:

### (3-((R,S)-4-(4-(Amino-imino-methyl)-phenyl)-3-benzyl-4-methyl-2,5-dioxoimidazolidin-1-yl)-propionyl)-L-aspartyl-L-phenylglycin

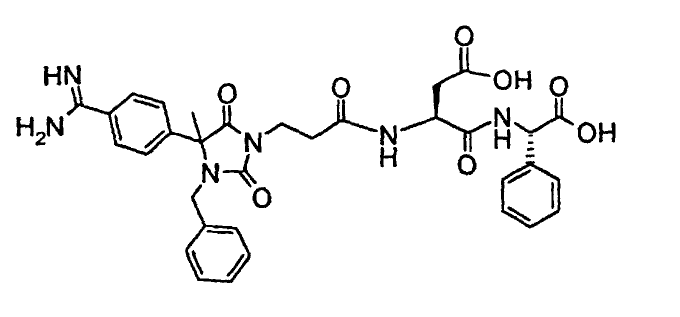

Die Verbindung wurde analog Beispiel 50 hergestellt, indem man racemisches (50.1) mit 3-Brom-propionsäureethylester umsetzte und vor der Peptidkupplung mit H-Asp(O^{t}Bu)-Phg-O^{t}Bu x HCl den Ethylester mit 6 N Salzsäure spaltete.
ES(+)-MS: 643,3 (M+H)⁺

### Beispiel 65:

### ((R,S)-4-(4-(Amino-imino-methyl)-phenyl)-3-benzyl-4-methyl-2,5-dioxoimidazolidin-1-yl)-acetyl-L-aspartyl-L-N-methyl-phenylglycin

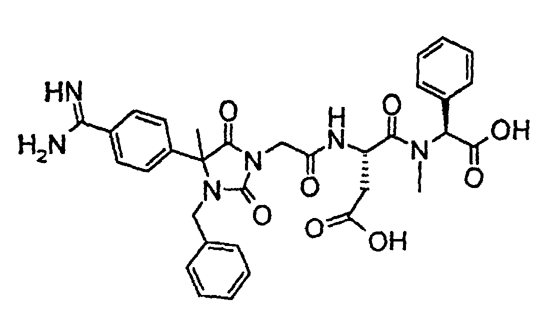

Die Verbindung wurde analog Beispiel 63 erhalten, wobei man racemisches (50.1) einsetzte und an Stelle von H-Asp(O^{t}Bu)-Phg-O^{t}Bu x HCI mit H-Asp(O^{t}Bu)-(N-Methyl-Phg)-O^{t}Bu x HCI kuppelte.
ES(+)-MS: 643,3 (M+H)⁺

### Beispiel 66:

### (R,S)-3-(((R,S)-4-(4-(Amino-imino-methyl)-phenyl)-3-benzyl-4-methyl-2,5-dioxoimidazolidin-1-yl)-acetyl-(N-methyl-amino))-3-phenyl-propionsäure

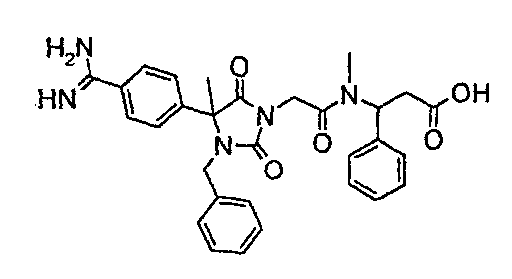

ES(+)-MS: 542,3 (M+H)⁺

### Beispiel 67:

### (S)-3-(((R,S)-4-(4-(Amino-imino-methyl)-phenyl)-3-benzyl-4-methyl-2,5-dioxoimidazolidin-1-yl)-acetylamino)-2-(4-trifluormethyl-benzyloxycarbonylamino)-propionsäure

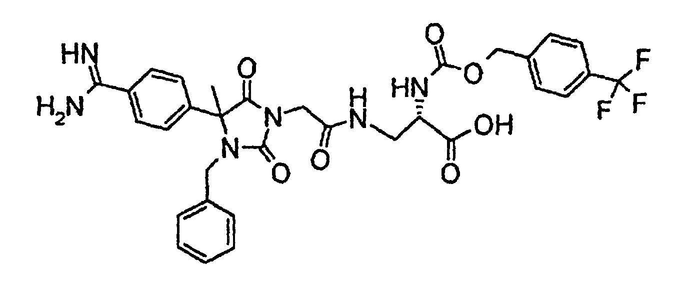

Man gab zu einer Lösung von 300 mg (0,644 mmol) (S)-3-(((R,S)-4-(4-(Amino-imino-methyl)-phenyl)-3-benzyl-4-methyl-2,5-dioxoimidazolidin-1-yl)-acetylamino)-2-amino-propionsäure-di-Hydrochlorid (hergestellt aus (S)-3-(((R,S)-4-(4-(Amino-imino-methyl)-phenyl)-3-benzyl-4-methyl-2,5-dioxoimidazolidin-1-yl)-acetylamino)-2-benzyloxycarbonylamino-propionsäure-tert-butylester durch hydrogenolytische Spaltung der Benzyloxycarbonylgruppe und Spaltung des tert-Butylesters mit 6 N Salzsäure) 0,22 ml (1,29 mmol) Diisopropyl-ethylamin und 204 mg (0,644 mmol) N-(4-Tifluormethyl-benzyloxycarbonyloxy)-succinimidund ließ das Gemisch 4 h bei Raumtemperatur rühren. Das Lösungsmittel wurde im Vakuum entfernt und der Rückstand mit Dichlormethan/Methanol/Essigsäure/Wasser (9:1:0,1:0,1) und Methanol über Kieselgel chromatographiert. Die Produktfraktionen wurden eingeengt und mit 40 %iger Essigsäure über Sephadex LH20 chromatographiert. Man erhielt nach Einengen der Produktfraktionen und Gefriertrocknen 145 mg (37 %) des gewünschten Produktes.
ES(+)-MS: 669,3 (M+H)⁺

### Beispiel 68:

### ((R,S)-4-(4-Aminomethyl-phenyl)-3-benzyl-4-methyl-2,5-dioxoimidazolidin-1-yl)-acetyl-L-aspartyl-L-phenylglycin

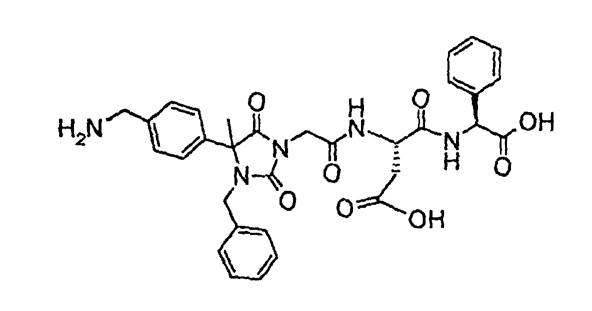

ES(+)-MS: 616,3 (M+H)⁺

### Beispiel 69:

### ((R,S)-4-(4-Guanidinomethyl-phenyl)-3-benzyl-4-methyl-2,5-dioxoimidazolidin-1-yl)-acetyl-L-aspartyl-L-phenylglycin

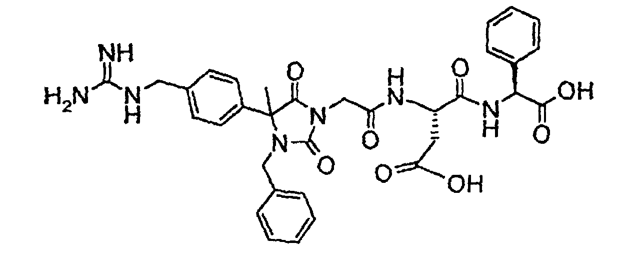

### 69a) ((R,S)-4-(4-Aminomethyl-phenyl)-3-benzyl-4-methyl-2,5-dioxoimidazolidin-1-yl)-essigsäuremethylester

7,55 g (20 mmol) ((R,S)-4-(4-Cyano-phenyl)-3-benzyl-4-methyl-2,5-dioxoimidazolidin-1-yl)-essigsäuremethylester (hergestellt aus racemischem 50.1 durch Reaktion mit 2-Bromessigsäuremethylester und Umsatz des Reaktionsproduktes mit Benzylbromid analog Beispiel 50) wurden in 80 ml eines Gemisches aus Ethanol und 50 %iger Essigsäure 7 h über 1,5 g Pd/C bei 3 atm Wasserstoff-Druck hydriert. Der Katalysator wurde abfiltriert und der Rückstand mit Dichlormethan/Methanol (8:2) über Kieselgel chromatographiert. Nach Einengen der Produktfraktionen erhielt man 7,6 g (100 %) ((R,S)-4-(4-Aminomethyl-phenyl)-3-benzyl-4-methyl-2,5-dioxoimidazolidin-1-yl)-essigsäuremethylester.

### 69b) ((R,S)-4-(4-Aminomethyl-phenyl)-3-benzyl-4-methyl-2,5-dioxoimidazolidin-1-yl)-essigsäure

3,7 g (9,7 mmol) ((R,S)-4-(4-Aminomethyl-phenyl)-3-benzyl-4-methyl-2,5-dioxoimidazolidin-1-yl)-essigsäuremethylester wurden in 80 ml konzentrierter Salzsäure 6 h unter Rückfluß erhitzt. Die Lösung wurde im Vakuum eingeengt, der Rückstand in Wasser aufgenommen, filtriert und das Filtrat gefriergetrocknet. Man erhielt 2,79 g (78 %) ((R,S)-4-(4-Aminomethyl-phenyl)-3-benzyl-4-methyl-2,5-dioxoimidazolidin-1-yl)-essigsäure.

### 69c) ((R,S)-4-(4-Guanidinomethyl-phenyl)-3-benzyl-4-methyl-2,5-dioxoimidazolidin-1-yl)-essigsäure

807 mg (2 mmol) ((R,S)-4-(4-Aminomethyl-phenyl)-3-benzyl-4-methyl-2,5-dioxoimidazolidin-1-yl)-essigsäure in 20 ml absolutem DMF wurden mit 1,02 ml (6 mmol) Diisopropyl-ethylamin und anschließend mit 220 mg (2 mmol) 1-H-Pyrazol-1-carboxamidin x HCl versetzt. Das Reaktionsgemisch wurde 5 h bei 50 °C gerührt und anschließend über Nacht bei Raumtemperatur stehen gelassen. Es wurden erneut 0,2 ml (0,4 mmol) Diisopropyl-ethylamin und 44 mg (0,4 mmol) 1-H-Pyrazol-1-carboxamidin x HCl zugegeben und weitere 6 h bei 50 °C gerührt. Das Reaktionsgemisch wurde im Vakuum eingeengt, der Rückstand 2 x mit Diethylether verrieben, der Diethylether abdekantiert und der Rückstand mit Wasser/Butanol/Essigsäure (43:4,3:3,5) über Sephadex LH 20 chromatographiert. Man erhielt 682 mg (83 %) ((R,S)-4-(4-Guanidinomethyl-phenyl)-3-benzyl-4-methyl-2,5-dioxoimidazolidin-1-yl)-essigsäure

### 69d) ((R,S)-4-(4-Guanidinomethyl-phenyl)-3-benzyl-4-methyl-2,5-dioxoimidazolidin-1-yl)-acetyl-L-aspartyl-L-phenylglycin

Die Verbindung wurde durch Kupplung von ((R,S)-4-(4-Guanidinomethyl-phenyl)-3-benzyl-4-methyl-2,5-dioxoimidazolidin-1-yl)-essigsäure mit H-Asp(O^{t}Bu)-Phg-(O^{t}Bu) x HCI und anschließende Spaltung der tert-Butylester analog Beispiel 2 hergestellt.
ES(+)-MS: 658,3 (M+H)⁺

### Beispiel 70:

### ((R,S)-4-(4-Amino-phenyl)-3-benzyl-4-methyl-2,5-dioxoimidazolidin-1-yl)-acetyl-L-aspartyl-L-phenylglycin

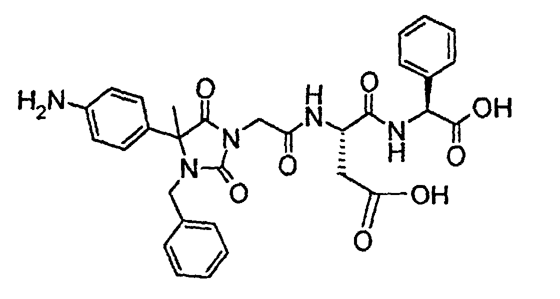

### 70a) ((R,S)-4-(4-Amino-phenyl)-3-benzyl-4-methyl-2,5-dioxoimidazolidin-1-yl)-essigsäure

Eine Lösung von 6,86 g (17,9 mmol) ((R,S)-4-(4-Nitrophenyl)-3-benzyl-4-methyl-2,5-dioxoimidazoüdin-1-yl)-essigsäure (hergestellt aus 4 -Nitrophenyl-methylketon analog der Synthese von ((R, S)-4-(4-Cyano-phenyl)-3-benzyl-4-methyl-2,5-dioxoimidazolidin-1-yl)-essigsäure aus 4-Cyanophenyl-methylketon, vgl. Beispiel 1; zur Einführung der 3-Benzylgruppe siehe Beispiel 50) in 150 ml Methanol wurde 4 h über 10 % Pd/C hydriert. Der Katalysator wurde abfiltriert, das Filtrat im Vakuum eingeengt und der Rückstand mit Diethylether verrieben und anschließend abgesaugt. Man erhielt 3,82 g (60 %) ((R,S)-4-(4-Amino-phenyl)-3-benzyl-4-methyl-2,5-dioxoimidazolidin-1-yl)-essigsäure.

### 70b) ((R,S)-4-(4-Amino-phenyl)-3-benzyl-4-methyl-2,5-dioxoimidazolidin-1-yl)-acetyl-L-aspartyl-L-phenylglycin

Die Verbindung wurde durch Kupplung von ((R,S)-4-(4-Aminophenyl)-3-benzyl-4-methyl-2,5-dioxoimidazolidin-1-yl)-essigsäure mit H-AspH-Asp(O^{t}Bu)-Phg-(O^{t}Bu) x HCI und anschließende Spaltung der tert-Butylester analog Beispiel 2 hergestellt, wobei der Rückstand aus der Trifluoressigsäure-Spaltung -Spaltung mit Diethylether verrieben, abgesaugt und im Hochvakuum getrocknet wurde.
ES(+)-MS: 602,3 (M+H)⁺

### Beispiel 71:

### ((R,S)-4,(4-Guanidino-phenyl)-3-benzyl-4-methyl-2,5-dioxoimidazolidin-1-yl)-acetyl-L-aspartyl-L-phenylglycin

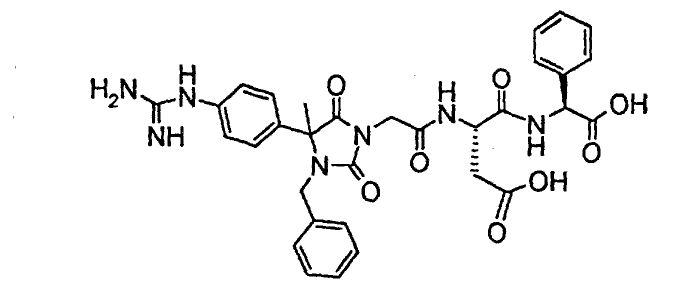

Die Verbindung wurde hergestellt aus ((R,S)-4-(4-Aminophenyl)-3-benzyl-4-methyl-2,5-dioxoimidazolidin-1-yl)-essigsäure (siehe Beispiel 70) nach Überführung der Aminogruppe in die Guanidinogruppe mit 1-H-Pyrazol-1-carboxamidin x HCI (wie in Beispiel 69 beschrieben) und anschließende Kupplung mit H-AsptO^{t}Bu)-Phg-(O^{t}Bu) x HCl und Spaltung der tert-Butylester analog Beispiel 2, wobei der Rückstand aus der Trifluoressigsäure-Spaltung lediglich mit Diethylether verrieben, abgesaugt und im Hochvakuum getrocknet wurde.
ES(+)-MS: 644,3 (M+H)⁺

### Beispiel 72:

### ((R,S)-4-(4-Amino-benzyl)-3-benzyl-4-methyl-2,5-dioxoimidazolidin-1-yl)-acetyl-L-aspartyl-L-phenylglycin

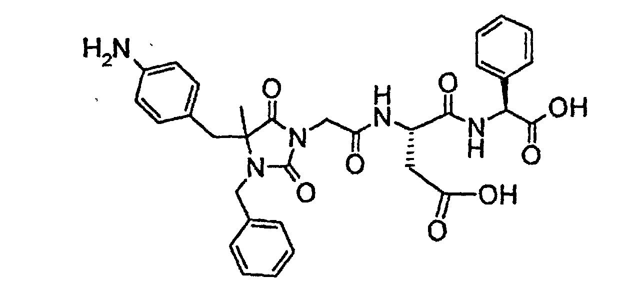

Die Verbindung wurde hergestellt, indem man ((R,S)-4-(4-Aminobenzyl)-3-benzyl-4-methyl-2,5-dioxoimidazolidin-1-yl)-essigsäure, die analog Beispiel 70 aus ((R,S)-4-(4-Nitrobenzyl)-3-benzyl-4-methyl-2,5-dioxoimidazolidin-1-yl)-essigsäure (hergestellt aus 4-Nitrobenzyl-methylketon analog der Synthese von ((R,S)-4-(4-Cyano-phenyl)-3-benzyl-4-methyl-2,5-dioxoimidazolidin-1-yl)-essigsäure aus 4-Cyanophenyl-methylketon, vgl. Beispiel 1; zur Einführung der 3-Benzylgruppe siehe Beispiel 50) synthetisierte, mit H-AspH-Asp(O^{t}Bu)-Phg-(O^{t}Bu) x HCl analog Beispiel 2 kuppelte und nach Spaltung der tert-Butylester mit 90 %iger Trifluoressigsäure den Rückstand mit Wasser/Butanol/Essigsäure (43:4,3:3,5) über Sephadex LH 20 chromatographierte.
ES(+)-MS: 616,3 (M+H)⁺

### Beispiel 73:

### ((R,S)-4-(4-Guanidino-benzyl)-3-benzyl-4-methyl-2,5-dioxoimidazolidin-1-yl)-acetyl-L-aspartyl-L-phenylglycin

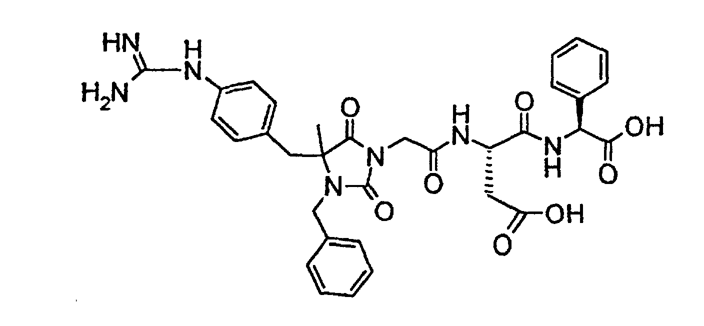

Die Verbindung wurde hergestellt aus ((R,S)-4-(4 Aminobenzyl)-3-benzyl-4-methyl-2,5-dioxoimidazolidin-1-yl)-essigsäure (siehe Beispiel 72) durch Überführung der Aminogruppe in die Guanidinogruppe mit 1-H-Pyrazol-1-carboxamidin x HCI (wie in Beispiel 69 beschrieben), anschließende Kupplung mit H-Asp(O^{t}Bu)-Phg-(O^{t}Bu) x HCI und Spaltung der tert-Butylester analog Beispiel 2, wobei der Rückstand aus der Trifluoressigsäure-Spaltung mit Wasser/Butanol/Essigsäure (43:4,3:3,5) über Sephadex LH 20 chromatographiert wurde.
ES(+)-MS: 658,3 (M+H)⁺

Analog dem Beispiel 67 können auch die Verbindungen der Beispiele 74 und 75 hergestellt werden, indem man zum Beispiel (S)-3-(((R,S)-4-(4-(Amino-imino-methyl)-phenyl)-3-benzyl-4-methyl-2,5-dioxoimidazolidin-1-yl)-acetylamino)-2-amino-propionsäure-di-Hydrochlorid (siehe Beispiel 67) mit den entsprechenden Carbonylchloriden umsetzt. Für die Herstellung kann auch von (S)-2-Amino-3-tert-butoxycarbonylamino-propionsäure-tert-butylester-Hydrochlorid ausgegangen werden. Ebenso können analog dem Beispiel 67 auch andere Benzylcarbamate mit beliebigen Substituenten an dem Benzylring in der Carbamatgruppe hergestellt werden.

### Beispiel 74:

### (S)-3-((R,S)-2-((S)-4-(4-(Amino-imino-methyl)-phenyl)-3-benzyl-4-methyl-2,5-dioxoimidazolidin-1-yl)-2-(2-methylpropyl)-acetylamino)-2-(2-methylpropyloxycarbonylamino)-propionsäure

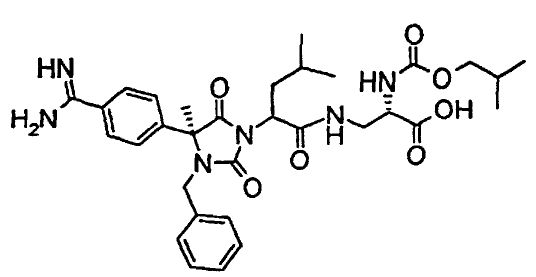

### Beispiel 75:

### (S)-3-((R,S)-2-((S)-4-(4-(Amino-imino-methyl)-phenyl)-3-benzyl-4-methyl-2,5-dioxoimidazolidin-1-yl)-2-(2-methylpropyl)-acetylamino)-2-(2,2-dimethylpropyloxycarbonylamino)-propionsäure

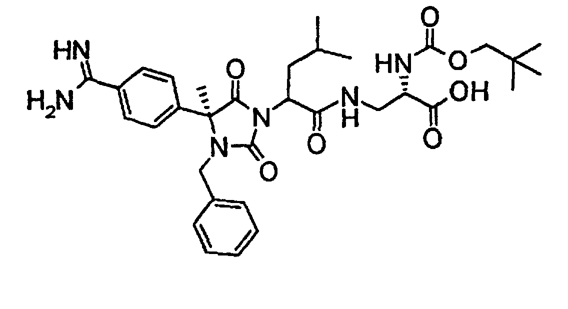

Die Verbindungen der Beispiele 77 bis 79 wurden durch Festphasensynthese nach der in Beispiel 76 angegebenen allgemeinen Vorschrift hergestellt.

### Beispiel 76:

### Festphasensynthese (Allgemeine Vorschrift)

### Allgemeines

Die Synthesen am polymeren Träger wurden nach der Synthesesequenz durchgeführt, die im Schema 1 dargestellt ist. Die Reste R⁵¹ bis R⁵⁵ im Schema 1 haben die Bedeutung der Reste, die sich in der Formel I in der betreffenden Position im Molekül befinden, oder sie können funktionelle Gruppen in geschützter Form oder in Form von Vorstufen enthalten. R⁵¹ entspricht den Resten R¹⁴ und R¹⁵, wobei in diesen Resten vorhandene funktionelle Gruppen in geschützter Form oder in Form von Vorstufen vorliegen können. R⁵² entspricht zusammen mit der CH-Gruppe, an die dieser Rest gebunden ist, der Gruppe B (R⁵² entspricht also einem Substituenten an einer für B stehenden Methylengruppe). R⁵³ entspricht R¹³. R⁵⁴ entspricht der Gruppe R¹-A, wobei darin vorhandene funktionelle Gruppen in geschützter Form oder in Form von Vorstufen vorliegen können, insbesondere zum Beispiel im vorliegenden Fall eine Amidinogruppe in Form der Cyano-Vorstufe vorliegt. R^{54a} entspricht der Gruppe R¹-A. R⁵⁵ entspricht der Gruppe R⁰.

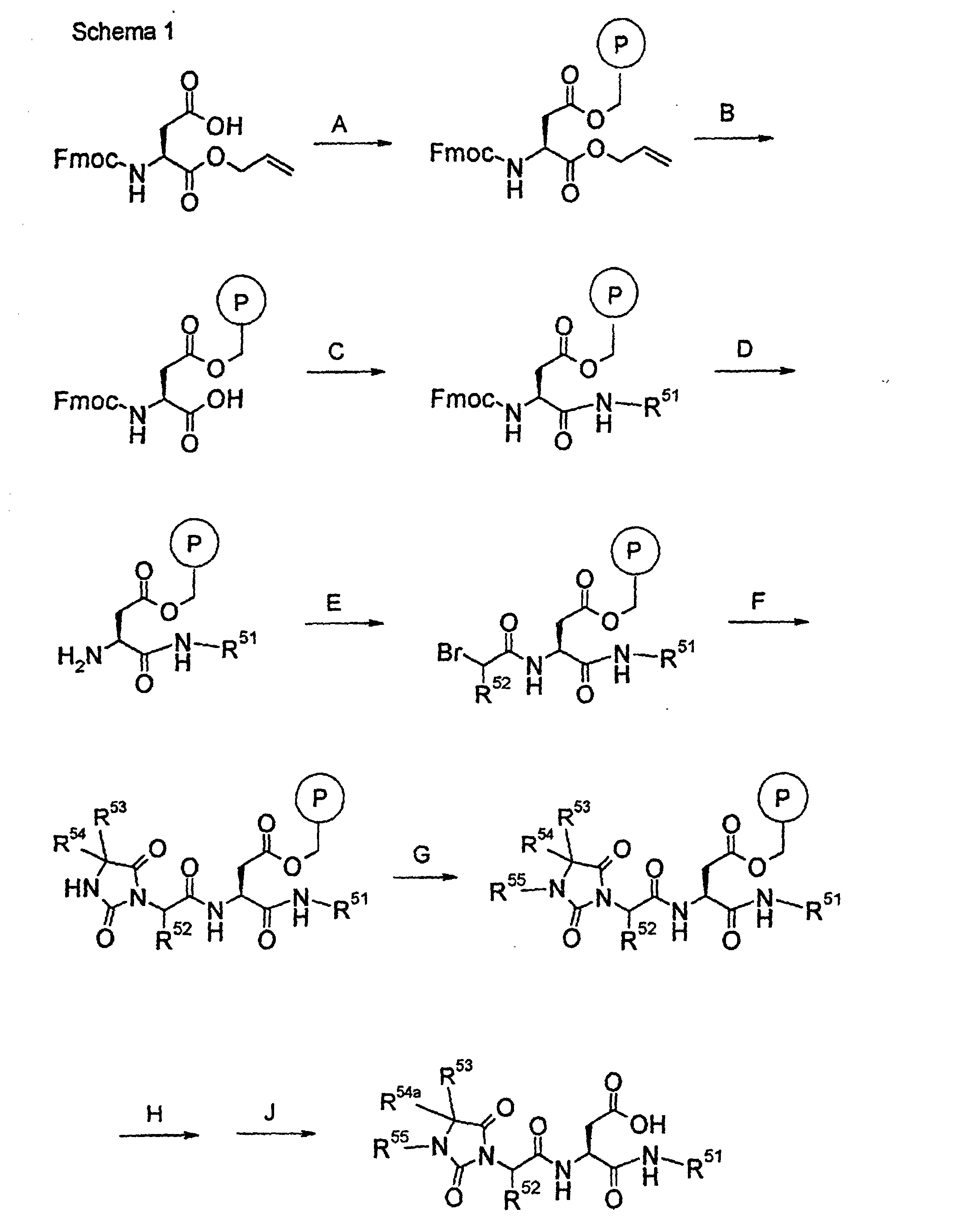

Die Synthese von Zwischenstufen in größerem Maßstab wurde in speziellen Reaktionsgefäßen mit eingelassenen Fritten am Boden des Reaktionsgefäßes durchgeführt, die Synthese der Verbindungen der Formel 1 wurde in Spritzen oder Reaktionsblöcken durchgeführt (Act 496, MultiSynTech). Die Synthesen am Harz wurden durch on bead-Analytik (FT-IR mit ATR-Einheit und MAS-NMR) und Abspaltung einer analytischen Probe vom Harz (HPLC, MS, NMR) verfolgt.

Darstellung des Asparaginsäurebausteines FmocAsp(OH)OAllyl FmocAsp(OtBu)OAllyl (40 g, 88,7 mmol) wurde mit 25 ml Trifluoressigsäure versetzt und 30 min bei Raumtemperatur gerührt. Das Lösungsmittel wurde am Rotationsverdampfer abgezogen. Der Rückstand wurde im Vakuum getrocknet. Man erhielt FmocAsp(OH)OAllyl als gelbes Öl (33,9 g, 97 %).
ES(+)-MS: 395,2 (M+H)⁺

### Anknüpfung an den polymeren Träger (Schritt A in Schema 1)

40 g Wang-Polystyrrolharz (1,1 mmol/g; Bachem) wurden 5 min mit 20 ml DMF bei Raumtemperatur vorgequollen. Nach Zugabe einer Lösung von 26,0 g (1,5 Äquivalente) FmocAsp(OH)OAllyl und 34,3 g (1,5 Äquivalente) 1-Benzotriazolyloxy-tripyrrolidinophosphonium-hexafluorophosphat (PyBOP) und 9,3 ml (1,5 Äquivalente) Diisopropyl-ethylamin in 120 ml DMF wurde das Gemisch 10 h bei 40 °C geschüttelt. Nach beendeter Reaktion wurde die Lösung abgesaugt und das Harz mit DMF gewaschen (5 x 20 ml). Nach Zugabe einer Lösung von Acetanhydrid (10 ml) und Diisopropyl-ethylamin (9,3 ml, 1,5 Äquivalente) in 40 ml DMF wurde das Gemisch erneut für 30 min bei Raumtemperatur geschüttelt. Die Lösung wurde abgesaugt und das Harz nacheinander jeweils dreimal mit 40 ml DMF, Methanol und Dichlormethan gewaschen. Das Harz wurde anschließend im Vakuum getrocknet. Die Bestimmung der Beladung nach der Fmoc-Methode ergab eine Beladung von 0,6 mmol/g

### Abspaltung der Allylgruppe am polymeren Träger (Schritt B)

Das Harz wurde unter Argon 5 min in DMF bei Raumtemperatur vorgequollen. Nach Zugabe von Tetrakis Tetrakis(triphenylphosphin)palladium und N-Methylpyrrolidin (10 Äquivalente) wurde das Gemisch unter Argon 6 h bei 40°C geschüttelt. Nach beendeter Reaktion wurde die Lösung abgesaugt und das Harz nacheinander jeweils dreimal mit DMF, Methanol, Toluol und Dichlormethan gewaschen und anschließend getrocknet.

### Kupplung mit Aminoverbindungen am polymeren Träger (Schritt C)

Das beladene Harz mit freier Carboxylfunktion wurde 5 min in DMF bei Raumtemperatur vorgequollen. Nach Zugabe einer Lösung von HOBt (1,2 Äquivalente), TOTU (1,2 Äquivalente) und Diisopropyl-ethylamin (1,2 Äquivalente) in DMF wurde das Gemisch 30 min bei Raumtemperatur geschüttelt. Die Aminoverbindung (1,2 Äquivalente) wurde gelöst in DMF zugegeben. Die Suspension wurde bei Raumtemperatur bis zur vollständigen Umsetzung geschüttelt (HPLC-Kontrolle). Nach beendeter Reaktion wurde die Lösung abgesaugt und das Harz nacheinander jeweils dreimal mit DMF, Methanol, Toluol und Dichlormethan gewaschen und anschließend getrocknet.

### Abspaltung der Fmoc-Schutzgruppe (Schritt D)

Zur Abspaltung der Fmoc-Schutzgruppe wurde das Harz 5 min in DMF bei Raumtemperatur vorgequollen. Nach Zugabe einer Lösung von DMF/Piperidin (1:1) wurde 20 min bei Raumtemperatur geschüttelt. Die Lösung wurde abgesaugt und der Vorgang wiederholt. Die Abspaltung einer analytischen Probe ergab vollständige Umsetzung nach HPLC/MS Untersuchung. Nach vollständiger Umsetzung wurde das Harz dreimal mit Dichlormethan gewaschen und direkt in die Kupplung eingesetzt.

### Kupplung mit α-Halogencarbonsäuren (Schritt E)

Aus α-Halogencarbonsäuren (5 Äquivalente) wurden durch 30-minütige Reaktion mit Diisopropylcarbodiimid (DIC) (2,4 Äquivalente) in Dichlormethan die symmetrischen Anhydride gebildet. Nach dieser Zeit wurden 2 Äquivalente Diisopropyl-ethylamin zugegeben. Das Gemisch wurde zu dem Harz gegeben und 12 h bei Raumtemperatur geschüttelt. Nach beendeter Reaktion wurde die Lösung abgesaugt und das Harz nacheinander jeweils dreimal mit DMF, Toluol und Dichlormethan gewaschen und anschließend sofort weiter umgesetzt.

Anstatt unter Verwendung der Säuren und von DIC kann die Kupplung auch mit Säurehalogeniden durchgeführt werden. Dazu wird das Harz 5 min mit Dichlormethan bei Raumtemperatur vorgequollen. Die α-Halogencarbonsäurehalogenide (1,5 Äquivalente) werden gelöst in Dichlormethan zugegeben. Nach Zugabe einer katalytischen Menge 4-Dimethylaminopyridin und von Diisopropyl-ethylamin (1 Äquivalent) wird das Gemisch für 8 h bei Raumtemperatur geschüttelt. Nach beendeter Reaktion wird die Lösung abgesaugt und das Harz nacheinander jeweils dreimal mit DMF, Toluol und Dichlormethan gewaschen und anschließend sofort weiter umgesetzt.

### Kupplung der α-Halogenacylverbindungen mit Hydantoinen (Schritt F)

Die 4-Cyanophenylhydantoine (2 Äquivalente) wurden in DMF mit Diazabicycloundecen (DBU) (2 Äquivalente) bei Raumtemperatur aktiviert. Die aktivierte Lösung wurde nach 15 min zu dem in DMF für 5 min vorgequollenen Harz gegeben. Das Gemisch wurde 8 h bei Raumtemperatur geschüttelt. Nach beendeter Reaktion wurde die Lösung abgesaugt und das Harz nacheinander jeweils dreimal mit DMF, Methanol, Toluol und Dichlormethan gewaschen und anschließend getrocknet.

### N-Alkylierung des Hydantoins am polymeren Träger (Schritt G)

Das Harz wurde 5 min in DMF bei Raumtemperatur vorgequollen. Nach Zugabe von Caesiumcarbonat (3 Äquivalente) wurde 30 min bei Raumtemperatur geschüttelt. Nach Zugabe des Alkylierungsmittels (Bromid oder Iodid) wurde 6 h bei 50 °C geschüttelt. Nach beendeter Reaktion wurde die Lösung abgesaugt und das Harz nacheinander jeweils dreimal mit DMF, Methanol/Wasser/DMF(1,5:1,5:7), DMF, Toluol und Dichlormethan gewaschen und anschließend getrocknet.

Anstatt unter Verwendung von Caesiumcarbonat kann die Alkylierung auch mit Phosphazenen durchgeführt werden. Dazu wird das Harz 5 min in DMF bei Raumtemperatur vorgequollen. Nach Zugabe von N'''-tert-Butyl-N,N,N',N',N",N"-hexamethylphosphorimidsäuretriamid(Phosphazen-Base P1-t-Bu) (3 Äquivalente) wird 30 min bei Raumtemperatur geschüttelt. Nach Zugabe des Alkylierungsmittels (Bromid oder Iodid) wird 4 h bei Raumtemperatur geschüttelt. Nach beendeter Reaktion wird die Lösung abgesaugt und das Harz nacheinander jeweils dreimal mit DMF, Toluol und Dichlormethan gewaschen und anschließend getrocknet.

### Herstellung der Amidinogruppe aus der Cyanogruppe am polymeren Träger (Schritt H)

Das Harz wurde mit einer gesättigten Lösung von Schwefelwasserstoff in Pyridin/Triethylamin (2:1) 12 h bei Raumtemperatur geschüttelt. Die Lösung wurde abgesaugt und das Harz nacheinander jeweils dreimal mit Methanol, DMF, Toluol und Dichlormethan gewaschen. Nach Zugabe einer 20-%igen Lösung von Methyliodid in Aceton/Toluol (4:1) wurde weitere 12 h bei Raumtemperatur geschüttelt. Die Lösung wurde abgesaugt und das Harz nacheinander jeweils mit Aceton/Toluol (4:1), DMF, Methanol und Methanol/Toluol (4:1) gewaschen. Nach Zugabe von Ammoniumacetat (10 Äquivalente) in MethanolToluol/Essigsäure (80:16:4) wurde das Gemisch bei 50 °C für 3 h geschüttelt. Nach beendeter Reaktion wurde die Lösung abgesaugt und das Harz nacheinander jeweils dreimal mit DMF, Methanol, Toluol, und Dichlormethan gewaschen und anschließend getrocknet.

### Abspaltung vom Harz (Schritt J)

Zur Abspaltung der Verbindung vom Harz wurde ein Gemisch von Trifluoressigsäure/Dichlormethan (1:1) zum Harz zugegeben. Die Suspension wurde 1 h geschüttelt. Das Harz wurde abfiltriert. Die verbleibende Lösung wurde im Vakuum eingeengt. Der Rückstand wurde durch Kieselgelchromatographie gereinigt (Dichlormethan und Essigester).

### Beispiel 77:

### ((R,S)-4-(4-(Amino-imino-methyl)-phenyl)-3-((2-naphthyl)-methyl)-4-methyl-2,5-dioxoimidazolidin-1-yl)-acetyl-L-aspartyl-L-valin

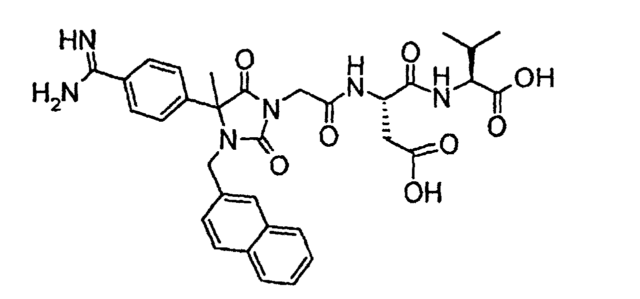

ES(+)-MS: 645,7 (M+H)⁺

### Beispiel 78:

### ((R,S)-4-(4-(Amino-imino-methyl)-phenyl)-3-((2-naphthyl)-methyl)-4-methyl-2,5-dioxoimidazolidin-1-yl)-acetyl-L-aspartyl-L-serin-methylester

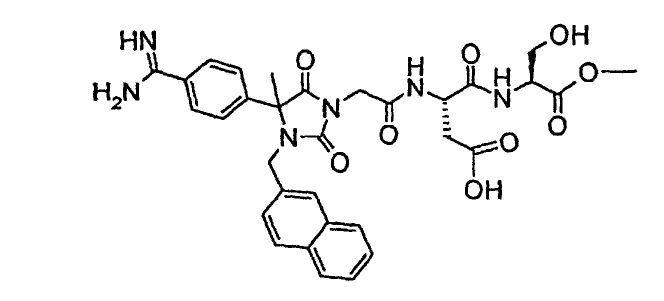

ES(+)-MS: 647,7 (M+H)⁺

### Beispiel 79:

### ((R,S)-4-(4-(Amino-imino-methyl)-phenyl)-3-benzyl-4-methyl-2,5-dioxoimidazolidin-1-yl)-acetyl-L-aspartyl-L-isoleucin

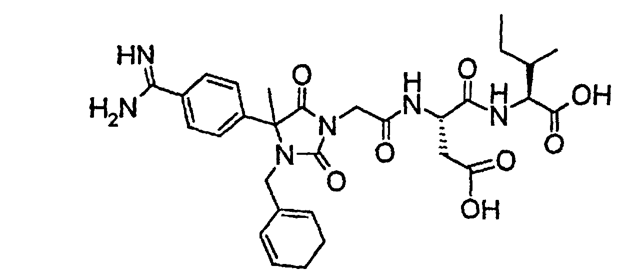

ES(+)-MS: 609,7 (M+H)⁺

### Untersuchung der biologischen Aktivität

Als Testmethode für die Wirksamkeit der Verbindungen der Formel I auf die Interaktion zwischen VCAM-1 und VLA-4 wird ein Assay verwendet, der für diese Interaktion spezifisch ist. Die zellulären Bindungspartner, d. h. die VLA-4-integrine, werden in ihrer natürlichen Form als Oberflächenmoleküle auf humanen U937-Zellen (ATCC CRL 1593), die zur Gruppe der Leukozyten gehören, angeboten. Als spezifische Bindungspartner werden gentechnisch hergestellte rekombinante lösliche Fusionsproteine, bestehend aus der extrazytoplasmatischen Domäne von humanen VCAM-1 und der konstanten Region eines humanen Immunglobulins der Subklasse IgG1, verwendet.

### Testmethode

### Assay zur Messung der Adhäsion von U937-Zellen (ATCC CRL 1593) an hVCAM-1 (1-3)-lgG

1. Herstellung von humanem VCAM-1(1-3)-lgG und humanem CD4-lgG
   Eingesetzt wurde ein genetisches Konstrukt zur Expression der extrazellulären Domäne des humanen VCAM-1, verbunden mit der genetischen Sequenz der schweren Kette des humanen Immunglobulins IgG1 (Hinge, CH2 und CH3 Regionen), von Dr. Brian Seed, Massachusetts General Hospital, Boston, USA. Das lösliche Fusionsprotein hVCAM-1(1-3)-lgG enthielt die drei aminoterminalen extrazellulären Immunglobulin-ähnlichen Domänen des humanen VCAM-1 (Damle und Aruffo, Proc. Natl. Acad. Sci. USA 1991, 88, 6403). CD4-IgG (Zettlmeissl et al., DNA and Cell Biology 1990, 9, 347) diente als Fusionsprotein für negative Kontrollen. Die rekombinanten Proteine wurden als lösliche Proteine nach DEAE/Dextran-vermittelter DNA-Transfektion in COS-Zellen (ATCC CRL1651) gemäß Standardprozeduren exprimiert (Ausubel et al., Current protocols in molecular biology, John Wiley & Sons, Inc., 1994).
2. Assay zur Messung der Adhäsion von U937-Zellen an hVCAM-1(1-3)-lgG
   2.1 96 well-Mikrotitertestplatten (Nunc Maxisorb) wurden mit 100 µl/well einer Ziege-anti-human-lgG-Antikörperlösung (10 µg/ml in 50 mM Tris, pH 9,5) 1 Stunde bei Raumtemperatur inkubiert. Nach Entfernen der Antikörperlösung wurde einmal mit PBS gewaschen.
   2.2 150 µl/well eines Blockierungspuffers (1% BSA in PBS) wurde 0,5 Stunden bei Raumtemperatur auf den Platten inkubiert. Nach Entfernen des Blockierungspuffers wurde einmal mit PBS gewaschen.
   2.3 100 µl pro well eines Zellkulturüberstandes von transfektierten COS-Zellen wurde für 1,5 Stunden bei Raumtemperatur auf den Platten inkubiert. Die COS-Zellen waren mit einem Plasmid transfiziert, welches für die drei N-terminalen Immunglobulin-ähnlichen Domänen des VCAM-1, gekoppelt an den Fc-Teil von humanem lgG₁ (hVCAM-1(1-3)-lgG), codiert. Der Gehalt an hVCAM-1(1-3)-lgG betrug ca. 0,5 -1 µg/ml. Nach Entfernen des Kulturüberstandes wurde einmal mit PBS gewaschen.
   2.4 Die Platten wurden mit 100 µl/well Fc-Rezeptor-Blockpuffer (1 mg/ml γ-Globulin, 100 mM NaCl, 100 µM MgCl₂, 100 µM MnCl₂, 100 µM CaCl₂, 1 mg/ml BSA in 50 mM HEPES, pH 7,5) für 20 Minuten bei Raumtemperatur inkubiert. Nach Entfernen des Fc-Rezeptor-Blockpuffers wurde einmal mit PBS gewaschen.
   2.5 20 µl Bindungspuffer (100 mM NaCl, 100 µM MgCl₂, 100 µM MnCl₂, 100 µM CaCl₂, 1 mg/ml BSA in 50 mM HEPES, pH 7,5) wurden vorgelegt, die zu testenden Substanzen in 10 µl Bindungspuffer zugegeben und für 20 Minuten inkubiert. Als Kontrollen dienten Antikörper gegen VCAM-1 (BBT, Nr. BBA6) und gegen VLA-4 (Immunotech, Nr. 0764).
   2.6 U937-Zellen wurden 20 Minuten in Fc-Rezeptor-Blockpuffer inkubiert und anschließend in einer Konzentration von 1 x 10⁶/ml und in einer Menge von 100 µl pro weil zupipettiert (Endvolumen 125µl/well).
   2.7 Die Platten wurden in einem 45°-Winkel in Stoppuffer (100 mM NaCl, 100 µM MgCl₂, 100 µM MnCl₂, 100 µM CaCl₂ in 25 mM Tris, pH 7,5) langsam eingetaucht und ausgeschlagen. Der Vorgang wurde wiederholt.
   2.8 Anschließend wurden 50 µl/well einer Färbelösung (16,7 µg/ml Hoechst Farbstoff 33258, 4 % Formaldehyd, 0,5 % Triton-X-100 in PBS) 15 Minuten auf den Platten inkubiert.
   2.9 Die Platten wurden ausgeschlagen, in einem 45°-Winkel in Stoppuffer (100 mM NaCl, 100 µM MgCl₂, 100 µM MnCl₂, 100 µM CaCl₂ in 25 mM Tris, pH 7,5) langsam eingetaucht. Der Vorgang wurde wiederholt. Anschließend wurde mit der Flüssigkeit in einem Cytofluorimeter (Millipore) gemessen (Sensitivität: 5, Filter: Anregungswellenlänge: 360 nm, Emissionswellenlänge: 460 nm).

Die Intensität des von den angefärbten U937-Zellen emittierten Lichts ist ein Maß für die Zahl der an der Platte verbliebenen, an das hVCAM-1(1-3)-lgG adhärierten U937-Zellen und somit ein Maß für die Fähigkeit der zugesetzten Testsubstanz, diese Adhäsion zu hemmen. Aus der Hemmung der Adhäsion bei verschiedenen Konzentrationen der Testsubstanz wurde die Konzentration IC₅₀ berechnet, die zu einer Hemmung der Adhäsion um 50 % führt.

Es wurden die folgenden Testergebnisse erhalten:

| Beispiel | U937/VCAM-1 Zelladhäsionstest |
|---|---|
| | lC₅₀ (µM) |
| 1 | 140 |
| 2 | 15 |
| 4 | 1,1 |
| 5 | 15 |
| 6 | 65 |
| 7 | 25 |
| 8 | 3,5 |
| 9 | 0,5 |
| 10 | 47 |
| 11 | 62 |
| 12 | 2,7 |
| 13 | 3,7 |
| 14 | 0,25 |
| 15 | 32 |
| 16 | 30 |
| 17 | 79 |
| 18 | 0,09 |
| 19 | 0,2 |
| 20 | 2,0 |
| 25 | 22 |
| 33 | 45 |
| 34 | 175 |
| 35 | 250 |
| 36 | 250 |
| 37 | 200 |
| 38 | 45 |
| 39 | 8 |
| 40 | 27 |
| 41 | 0,28 |
| 46 | 6,8 |
| 57 | 17,5 |
| 58 | 25 |
| 59 | 27,5 |
| 62 | 0,37 |
| 63 | 0,37 |
| 67 | 2,25 |
| 69 | 4,5 |
| 70 | 3 |
| 71 | 3,25 |
| 73 | 125 |

## Patentansprüche

1. Verwendung von Verbindungen der Formel I, 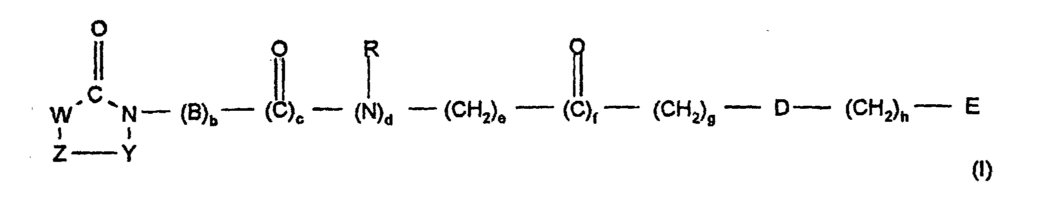 worin
W für R¹-A-C(R¹³) oder R¹-A-CH=C steht;
Y für eine Carbonyl-, Thiocarbonyl- oder Methylengruppe steht;
Z für N(R⁰), Sauerstoff, Schwefel oder eine Methylengruppe steht;
A einen zweiwertigen Rest aus der Reihe (C₁-C₆)-Alkylen, (C₃-C₇)-Cycloalkylen, Phenylen, Phenylen-(C₁-C₆)-alkyl, (C₁-C₆)-Alkylen-phenyl, Phenylen-(C₁-C₆)-alkyl-alkenyl oder einen zweiwertigen Rest eines 5- oder 6-gliedrigen gesättigten oder ungesättigten Ringes, der 1 oder 2 Stickstoffatome enthalten und ein- oder zweifach durch (C₁-C₆)-Alkyl oder doppelt gebundenen Sauerstoff oder Schwefel substituiert sein kann, bedeutet;
B einen zweiwertigen Rest aus der Reihe (C₁-C₆)-Alkylen, (C₂-C₆)-Alkenylen, Phenylen, Phenylen-(C₁-C₃)-alkyl, (C₁-C₃)-Alkylen-phenyl bedeutet, wobei der zweiwertige (C₁-C₆)-Alkylen-Rest unsubstituiert oder durch einen Rest aus der Reihe (C₁-C₈)-Alkyl, (C₂-C₈)-Alkenyl, (C₂-C₈)-Alkinyl, (C₃-C₁₀)-Cycloalkyl, (C₃-C₁₀)-Cycloalkyl-(C₁-C₆)-alkyl, gegebenenfalls substituiertes (C₆-C₁₄)-Aryl, im Arylrest gegebenenfalls substituiertes (C₆-C₁₄)-Aryl-(C₁-C₆)-alkyl, gegebenenfalls substituiertes Heteroaryl und im Heteroarylrest gegebenenfalls substituiertes Heteroaryl-(C₁-C₆)-alkyl substituiert sein kann;
D für C(R²)(R³), N(R³) oder CH=C(R³) steht;
E Tetrazolyl, (R⁸O)₂P(O), HOS(O)₂, R⁹NHS(O)₂ oder R¹⁰CO bedeutet;
R Wasserstoff, (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl bedeutet;
R⁰ für Wasserstoff, (C₁-C₈)-Alkyl, (C₃-C₁₂)-Cycloalkyl, (C₃-C₁₂)-Cycloalkyl-(C₁-C₈)-alkyl, (C₆-C₁₂)-Bicycloalkyl, (C₆-C₁₂)-Bicycloalkyl-(C₁-C₈)-alkyl, (C₆-C₁₂)-Tricycloalkyl, (C₆-C₁₂)-Tricycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl, im Heteroarylrest gegebenenfalls substituiertes Heteroaryl-(C₁-C₈)-alkyl, CHO, (C₁-C₈)-Alkyl-CO, (C₃-C₁₂)-Cycloalkyl-CO, (C₃-C₁₂)-Cycloalkyl-(C₁-C₈)-alkyl-CO, (C₆-C₁₂)-Bicycloalkyl-CO, (C₆-C₁₂)-Bicycloalkyl-(C₁-C₈)-alkyl-CO, (C₆-C₁₂)-Tricycloalkyl-CO, (C₆-C₁₂)-Tricycloalkyl-(C₁-C₈)-alkyl-CO, gegebenenfalls substituiertes (C₆-C₁₄)-Aryl-CO, im Arylrest gegebenenfalls substituiertes (C₆-C₁₄)-Aryl-(C₁-C₈)-alkyl-CO, gegebenenfalls substituiertes Heteroaryl-CO, im Heteroarylrest gegebenenfalls substituiertes Heteroaryl-(C₁-C₈)-alkyl-CO, (C₁-C₈)-Alkyl-S(O)ₙ, (C₃-C₁₂)-Cycloalkyl-S(O)ₙ, (C₃-C₁₂)-Cycloalkyl-(C₁-C₈)-alkyl-S(O)ₙ, (C₆-C₁₂)-Bicycloalkyl-S(O)ₙ, (C₆-C₁₂)-Bicycloalkyl-(C₁-C₈)-alkyl-S(O)ₙ, (C₆-C₁₂)-Tricycloalkyl-S(O)ₙ, (C₆-C₁₂)-Tricycloalkyl-(C₁-C₈)-alkyl-S(O)ₙ, gegebenenfalls substituiertes (C₆-C₁₄)-Aryl-S(O)ₙ, im Arylrest gegebenenfalls substituiertes (C₆-C₁₄)-Aryl-(C₁-C₈)-alkyl-S(O)ₙ, gegebenenfalls substituiertes Heteroaryl-S(O)ₙ oder im Heteroarylrest gegebenenfalls substituiertes Heteroaryl-(C₁-C₈)-alkyl-S(O)ₙ steht, wobei n für 1 oder 2 steht;
R¹ für X-NH-C(=NH)-(CH₂)ₚ oder X¹-NH-(CH₂)ₚ steht, wobei p für 0, 1, 2 oder 3 steht;
X Wasserstoff, (C₁-C₆)-Alkyl, (C₁-C₆)-Alkylcarbonyl, (C₁-C₆)-Alkoxycarbonyl, (C₁-C₁₈)-Alkylcarbonyloxy-(C₁-C₆)-alkoxycarbonyl, gegebenenfalls substituiertes (C₆-C₁₄)-Arylcarbonyl, gegebenenfalls substituiertes (C₆-C₁₄)-Aryloxycarbonyl, (C₆-C₁₄)-Aryl-(C₁-C₆)-alkoxycarbonyl, das im Arylrest auch substituiert sein kann, (R⁸O)₂P(O), Cyano, Hydroxy, (C₁-C₆)-Alkoxy, (C₆-C₁₄)-Aryl-(C₁-C₆)-alkoxy, das im Arylrest auch substituiert sein kann, oder Amino bedeutet;
X¹ eine der Bedeutungen von X hat oder R'-NH-C(=N-R") bedeutet, wobei R' und R" unabhängig voneinander die Bedeutungen von X haben;
R² Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl oder (C₃-C₈)-Cycloalkyl bedeutet;
R³ Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)-Cycloalkyl, (C₂-C₈)-Alkenyl, (C₂-C₈)-Alkinyl, (C₂-C₈)-Alkenylcarbonyl, (C₂-C₈)-Alkinylcarbonyl, Pyridyl, R¹¹NH, R⁴CO, COOR⁴, CON(CH₃)R¹⁴, CONHR¹⁴, CSNHR¹⁴, COOR¹⁵, CON(CH₃)R¹⁵ oder CONHR¹⁵ bedeutet;
R⁴ Wasserstoff oder (C₁-C₂₈)-Alkyl bedeutet, das gegebenenfalls ein- oder mehrfach durch gleiche oder verschiedene Reste R⁴' substituiert sein kann;
R^{4'} Hydroxy, Hydroxycarbonyl, Aminocarbonyl, Mono- oder Di-((C₁-C₁₈)-alkyl)-aminocarbonyl, Amino-(C₂-C₁₈)-alkylaminocarbonyl, Amino-(C₁-C₃)-alkylphenyl-(C₁-C₃)-alkylaminocarbonyl, (C₁-C₁₈)-Alkylcarbonylamino-(C₁-C₃)-alkylphenyl-(C₁-C₃)-alkylaminocarbonyl, (C₁-C₁₈)-Alkylcarbonylamino-(C₂-C₁₈)-alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)-Alkoxycarbonyl, gegebenenfalls substituiertes (C₃-C₈)-Cycloalkyl, Halogen, Nitro, Trifluormethyl oder den Rest R⁵ bedeutet;
R⁵ gegebenenfalls substituiertes (C₆-C₁₄)-Aryl, im Arylrest gegebenenfalls substituiertes (C₆-C₁₄)-Aryl-(C₁-C₈)-alkyl, einen mono- oder bicyclischen 5- bis 12-gliedrigen heterocyclischen Ring, der aromatisch, teilhydriert oder vollständig hydriert sein kann und der ein, zwei oder drei gleiche oder verschiedene Heteroatome aus der Reihe Stickstoff, Sauerstoff und Schwefel enthalten kann, einen Rest R⁶ oder einen Rest R⁶CO- bedeutet, wobei der Aryl- und unabhängig davon der Heterocyclus-Rest ein- oder mehrfach durch gleiche oder verschiedene Reste aus der Reihe (C₁-C₁₈)-Alkyl, (C₁-C₁₈)-Alkoxy, Halogen, Nitro, Amino oder Trifluormethyl substituiert sein können;
R⁶ für R⁷R⁸N, R⁷O oder R⁷S steht oder eine Aminosäureseitenkette, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, sowie deren Ester und Amide bedeutet, wobei anstelle freier funktioneller Gruppen gegebenenfalls Wasserstoff oder Hydroxymethyl stehen kann und/oder wobei freie funktionelle Gruppen durch in der Peptidchemie übliche Schutzgruppen geschützt sein können;
R⁷ Wasserstoff, (C₁-C₁₈)-Alkyl, (C₆-C₁₄)-Aryl-(C₁-C₈)-alkyl,(C₁-C₁₈)-Alkylcarbonyl, (C₁-C₁₈)-Alkoxycarbonyl, (C₆-C₁₄)-Arylcarbonyl, (C₆-C₁₄)-Aryl-(C₁-C₈)-alkylcarbonyl oder (C₆-C₁₄)-Aryl-(C₁-C₁₈)-alkyloxycarbonyl, wobei die Alkylgruppen gegebenenfalls durch eine Aminogruppe substituiert und/oder wobei die Arylreste ein- oder mehrfach, vorzugsweise einfach, durch gleiche oder verschiedene Reste aus der Reihe (C₁-C₈)-Alkyl, (C₁-C₈)-Alkoxy, Halogen, Nitro, Amino und Trifluormethyl substituiert sein können, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu - NH-CH₂- reduziert sein kann, bedeutet;
R⁸ Wasserstoff, (C₁-C₁₈)-Alkyl, gegebenenfalls substituiertes (C₆-C₁₄)-Aryl oder (C₆-C₁₄)-Aryl-(C₁-C₈)-alkyl, das im Arylrest auch substituiert sein kann, bedeutet;
R⁹ Wasserstoff, Aminocarbonyl, (C₁-C₁₈)-Alkylaminocarbonyl, (C₃-C₈)-Cycloalkylaminocarbonyl, gegebenenfalls substituiertes (C₆-C₁₄)-Arylaminocarbonyl, (C₁-C₁₈)-Alkyl, gegebenenfalls substituiertes (C₆-C₁₄)-Aryl oder (C₃-C₈)-Cycloalkyl bedeutet;
R¹⁰ Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
R¹¹ Wasserstoff, (C₁-C₁₈)-Alkyl, R¹²CO, gegebenenfalls substituiertes (C₆-C₁₄)-Aryl-S(O)₂, (C₁-C₁₈)-Alkyl-S(O)₂, im Arylrest gegebenenfalls substituiertes (C₆-C₁₄)-Aryl-(C₁-C₈)-alkyl oder R⁹NHS(O)₂ bedeutet;
R¹² Wasserstoff, (C₁-C₁₈)-Alkyl, (C₂-C₈)-Alkenyl, (C₂-C₈)-Alkinyl, gegebenenfalls substituiertes (C₆-C₁₄)-Aryl, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
R¹³ Wasserstoff, (C₁-C₆)-Alkyl, im Arylrest gegebenenfalls substituiertes (C₆-C₁₄)-Aryl-(C₁-C₈)-alkyl oder (C₃-C₈)-Cycloalkyl bedeutet;
R¹⁴ Wasserstoff oder (C₁-C₂₈)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-((C₁-C₁₈)-alkyl)-aminocarbonyl, Amino-(C₂-C₁₈)-alkylaminocarbonyl, Amino-(C₁-C₃)-alkylphenyl-(C₁-C₃)-alkylaminocarbonyl, (C₁-C₁₈)-Alkylcarbonylamino-(C₁-C₃)-alkylphenyl-(C₁-C₃)-alkylaminocarbonyl, (C₁-C₁₈)-Alkylcarbonyl-amino-(C₂-C₁₈)-alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)-Alkoxycarbonyl, gegebenenfalls substituiertes (C₃-C₈)-Cycloalkyl, HOS(O)₂-(C₁-C₃)-alkyl, R⁹NHS(O)₂-(C₁-C₃)-alkyl, (R⁸O)₂P(O)-(C₁-C₃)-alkyl, Tetrazolyl-(C₁-C₃)-alkyl, Halogen, Nitro, Trifluormethyl und R⁵ substituiert sein kann;
R¹⁵ für R¹⁶-(C₁-C₆)-alkyl oder für R¹⁶ steht;
R¹⁶ für einen 6- bis 24-gliedrigen bicyclischen oder tricyclischen Rest steht, der gesättigt oder teilweise ungesättigt ist und der auch ein bis vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann;
b, c, d und f unabhängig voneinander für 0 oder 1 stehen, aber nicht alle gleichzeitig 0 sein können;
e, g und h unabhängig voneinander für 0, 1, 2, 3, 4, 5 oder 6 stehen;
in allen ihren stereoisomeren Formen und Mischungen davon in allen Verhältnissen sowie ihrer physiologisch verträglichen Salze zur Herstellung von Arzneimitteln zur Hemmung der Adhäsion und/oder Migration von Leukozyten oder zur Hemmung des VLA-4-Rezeptors.

2. Verwendung von Verbindungen der Formel I gemäß Anspruch 1 in allen ihren stereoisomeren Formen und Mischungen davon in allen Verhältnissen sowie von ihren physiologisch verträglichen Salzen, wobei in der Formel I
W für R¹-A-C(R¹³) oder R¹-A-CH=C steht;
Y für eine Carbonyl-, Thiocarbonyl- oder Methylengruppe steht;
Z für N(R⁰), Sauerstoff, Schwefel oder eine Methylengruppe steht;
A einen zweiwertigen Rest aus der Reihe (C₁-C₆)-Alkylen, (C₃-C₇)-Cycloalkylen, Phenylen, Phenylen-(C₁-C₆)-alkyl, (C₁-C₆)-Alkylen-phenyl, Phenylen-(C₂-C₆)-alkenyl oder einen zweiwertigen Rest eines 5- oder 6-gliedrigen gesättigten oder ungesättigten Ringes, der 1 oder 2 Stickstoffatome enthalten und ein- oder zweifach durch (C₁-C₆)-Alkyl oder doppelt gebundenen Sauerstoff oder Schwefel substituiert sein kann, bedeutet;
B einen zweiwertigen Rest aus der Reihe (C₁-C₆)-Alkylen, (C₂-C₆)-Alkenylen, Phenylen, Phenylen-(C₁-C₃)-alkyl, (C₁-C₃)-Alkylen-phenyl bedeutet;
D für C(R²)(R³), N(R³) oder CH=C(R³) steht;
E Tetrazolyl, (R⁸O)₂P(O), HOS(O)₂, R⁹NHS(O)₂ oder R¹⁰CO bedeutet;
R und R⁰ unabhängig voneinander Wasserstoff, (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl bedeuten;
R¹ für X-NH-C(=NH)-(CH₂)ₚ oder X¹-NH-(CH₂)ₚ steht, wobei p für 0, 1, 2 oder 3 steht;
X Wasserstoff, (C₁-C₆)-Alkyl, (C₁-C₆)-Alkylcarbonyl, (C₁-C₆)-Alkoxycarbonyl, (C₁-C₁₈)-Alkylcarbonyloxy-(C₁-C₆)-alkoxycarbonyl, gegebenenfalls substituiertes (C₆-C₁₄)-Arylcarbonyl, gegebenenfalls substituiertes (C₆-C₁₄)-Aryloxycarbonyl, (C₆-C₁₄)-Aryl-(C₁-C₆)-alkoxycarbonyl, das im Arylrest auch substituiert sein kann, (R⁸O)₂P(O), Cyano, Hydroxy, (C₁-C₆)-Alkoxy, (C₆-C₁₄)-Aryl-(C₁-C₆)-alkoxy, das im Arylrest auch substituiert sein kann, oder Amino bedeutet;
X¹ eine der Bedeutungen von X hat oder R'-NH-C(=N-R'') bedeutet, wobei R' und R" unabhängig voneinander die Bedeutungen von X haben;
R² Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl oder (C₃-C₈)-Cycloalkyl bedeutet;
R³ Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)-Cycloalkyl, (C₂-C₈)-Alkenyl, (C₂-C₈)-Alkinyl, (C₂-C₈)-Alkenylcarbonyl, (C₂-C₈)-Alkinylcarbonyl, Pyridyl, R¹¹NH, R⁴CO, COOR⁴, CON(CH₃)R¹⁴, CONHR¹⁴, CSNHR¹⁴, COOR¹⁵, CON(CH₃)R¹⁵ oder CONHR¹⁵ bedeutet;
R⁴ Wasserstoff oder (C₁-C₂₈)-Alkyl bedeutet, das gegebenenfalls ein- oder mehrfach durch gleiche oder verschiedene Reste R^{4'} substituiert sein kann;
R^{4'} Hydroxy, Hydroxycarbonyl, Aminocarbonyl, Mono- oder Di-((C₁-C₁₈)-alkyl)-aminocarbonyl, Amino-(C₂-C₁₈)-alkylaminocarbonyl, Amino-(C₁-C₃)-alkyl-phenyl-(C₁-C₃)-alkylaminocarbonyl, (C₁-C₁₈)-Alkylcarbonylamino-(C₁-C₃)-alkylphenyl-(C₁-C₃)-alkylaminocarbonyl, (C₁-C₁₈)-Alkylcarbonylamino-(C₂-C₁₈)-alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)-Alkoxycarbonyl, gegebenenfalls substituiertes (C₃-C₈)-Cycloalkyl, Halogen, Nitro, Trifluormethyl oder den Rest R⁵ bedeutet;
R⁵ gegebenenfalls substituiertes (C₆-C₁₄)-Aryl, im Arylrest gegebenenfalls substituiertes (C₆-C₁₄)-Aryl-(C₁-C₈)-alkyl, einen mono- oder bicyclischen 5- bis 12-gliedrigen heterocyclischen Ring, der aromatisch, teilhydriert oder vollständig hydriert sein kann und der ein, zwei oder drei gleiche oder verschiedene Heteroatome aus der Reihe Stickstoff, Sauerstoff und Schwefel enthalten kann, einen Rest R⁶ oder einen Rest R⁶CO- bedeutet, wobei der Aryl- und unabhängig davon der Heterocyclus-Rest ein- oder mehrfach durch gleiche oder verschiedene Reste aus der Reihe (C₁-C₁₈)-Alkyl, (C₁-C₁₈)-Alkoxy, Halogen, Nitro, Amino oder Trifluormethyl substituiert sein können;
R⁶ für R⁷R⁸N, R⁷O oder R⁷S steht oder eine Aminosäureseitenkette, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, sowie deren Ester und Amide bedeutet, wobei anstelle freier funktioneller Gruppen gegebenenfalls Wasserstoff oder Hydroxymethyl stehen kann und/oder wobei freie funktionelle Gruppen durch in der Peptidchemie übliche Schutzgruppen geschützt sein können;
R⁷ Wasserstoff, (C₁-C₁₈)-Alkyl, (C₆-C₁₄)-Aryl-(C₁-C₈)-alkyl, (C₁-C₁₈)-Alkylcarbonyl, (C₁-C₁₈)-Alkoxycarbonyl, (C₆-C₁₄)-Arylcarbonyl, (C₆-C₁₄)-Aryl-(C₁-C₈)-alkylcarbonyl oder (C₆-C₁₄)-Aryl-(C₁-C₁₈)-alkyloxycarbonyl, wobei die Alkylgruppen gegebenenfalls durch eine Aminogruppe substituiert und/oder wobei die Arylreste ein- oder mehrfach, vorzugsweise einfach, durch gleiche oder verschiedene Reste aus der Reihe (C₁-C₈)-Alkyl, (C₁-C₈)-Alkoxy, Halogen, Nitro, Amino und Trifluormethyl substituiert sein können, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen DipeptidRest, der im Arylrest auch substituiert und/oder bei dem die Peptidbindung zu -NH-CH₂- reduziert sein kann, bedeutet;
R⁸ Wasserstoff, (C₁-C₁₈)-Alkyl, gegebenenfalls substituiertes (C₆-C₁₄)-Aryl oder (C₆-C₁₄)-Aryl-(C₁-C₈)-alkyl, das im Arylrest auch substituiert sein kann, bedeutet;
R⁹ Wasserstoff, Aminocarbonyl, (C₁-C₁₈)-Alkylaminocarbonyl, (C₃-C₈)-Cycloalkylaminocarbonyl, gegebenenfalls substituiertes (C₆-C₁₄)-Arylaminocarbonyl, (C₁-C₁₈)-Alkyl, gegebenenfalls substituiertes (C₆-C₁₄)-Aryl oder (C₃-C₈)-Cycloalkyl bedeutet;
R¹⁰ Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
R¹¹ Wasserstoff, (C₁-C₁₈)-Alkyl, R¹²CO, gegebenenfalls substituiertes (C₆-C₁₄)-Aryl-S(O)₂, (C₁-C₁₈)-Alkyl-S(O)₂, im Arylrest gegebenenfalls substituiertes (C₆-C₁₄)-Aryl-(C₁-C₈)-alkyl oder R⁹NHS(O)₂ bedeutet;
R¹² Wasserstoff, (C₁-C₁₈)-Alkyl, (C₂-C₈)-Alkenyl, (C₂-C₈)-Alkinyl, gegebenenfalls substituiertes (C₆-C₁₄)-Aryl, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
R¹³ Wasserstoff, (C₁-C₆)-Alkyl, im Arylrest gegebenenfalls substituiertes (C₆-C₁₄)-Aryl-(C₁-C₈)-alkyl oder (C₃-C₈)-Cycloalkyl bedeutet;
R¹⁴ Wasserstoff oder (C₁-C₂₈)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-((C₁-C₁₈)-alkyl)-aminocarbonyl, Amino-(C₂-C₁₈)-alkylaminocarbonyl, Amino-(C₁-C₃)-alkylphenyl-(C₁-C₃)-alkylaminocarbonyl, (C₁-C₁₈)-Alkylcarbonylamino-(C₁-C₃)-alkylphenyl-(C₁-C₃)-alkylaminocarbonyl, (C₁-C₁₈)-Alkylcarbonyl-amino-(C₂-C₁₈)-alkylaminocarbonyl,(C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)-Alkoxycarbonyl, gegebenenfalls substituiertes (C₃-C₈)-Cycloalkyl, HOS(O)₂-(C₁-C₃)-alkyl, R⁹NHS(O)₂-(C₁-C₃)-alkyl, (R⁸O)₂P(O)-(C₁-C₃)-alkyl, Tetrazolyl-(C₁-C₃)-alkyl, Halogen, Nitro, Trifluormethyl und R⁵ substituiert sein kann;
R¹⁵ für R¹⁶-(C₁-C₆)-alkyl oder für R¹⁶ steht;
R¹⁶ für einen 6- bis 24-gliedrigen bicyclischen oder tricyclischen Rest steht, der gesättigt oder teilweise ungesättigt ist und der auch ein bis vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann;
b, c, d und f unabhängig voneinander für 0 oder 1 stehen, aber nicht alle gleichzeitig 0 sein können;
e, g und h unabhängig voneinander für 0, 1, 2, 3, 4, 5 oder 6 stehen.

3. Verwendung von Verbindungen der Formel I gemäß Anspruch 1 in allen ihren stereoisomeren Formen und Mischungen davon in allen Verhältnissen sowie von ihren physiologisch verträglichen Salzen, wobei in der Formel I R⁰ für (C₁-C₈)-Alkyl, (C₃-C₈)-Cycloalkyl, (C₃-C₈)-Cycloalkyl-(C₁-C₄)-alkyl, gegebenenfalls substituiertes (C₆-C₁₄)-Aryl oder im Arylrest gegebenenfalls substituiertes (C₆-C₁₄)-Aryl-(C₁-C₁₈)-alkyl, bevorzugt für unsubstituiertes oder im Arylrest einfach oder mehrfach substituiertes Biphenylylmethyl, Naphthylmethyl oder Benzyl, steht.

4. Verwendung von Verbindungen der Formel I gemäß Anspruch 1 und/oder 3 in allen ihren stereoisomeren Formen und Mischungen davon in allen Verhältnissen sowie von ihren physiologisch verträglichen Salzen, wobei in der Formel I gleichzeitig
W für R¹-A-CH=C und darin A für einen Phenylenrest steht oder W für R¹-A-C(R¹³) und darin A für einen zweiwertigen Rest aus der Reihe Methylen, Ethylen, Trimethylen, Tetramethylen, Pentamethylen, Cyclohexylen, Phenylen, Phenylenmethyl steht;
B für einen zweiwertigen Rest aus der Reihe Methylen, Ethylen, Trimethylen, Tetramethylen, Vinylen, Phenylen oder für substituiertes Methylen oder Ethylen steht;
E R¹⁰CO bedeutet;
R Wasserstoff, (C₁-C₆)-Alkyl oder Benzyl bedeutet;
R⁰ für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
R¹ für X-NH-C(=NH), X-NH-C(=NX)-NH oder X-NH-CH₂ steht;
X für Wasserstoff, (C₁-C₆)-Alkylcarbonyl, (C₁-C₆)-Alkoxycarbonyl, (C₁-C₈)-Alkylcarbonyloxy-(C₁-C₆)-alkoxycarbonyl, (C₆-C₁₄)-Aryl-(C₁-C₆)-alkoxycarbonyl oder Hydroxy steht;
R² für Wasserstoff oder (C₁-C₈)-Alkyl steht;
R³ für (C₁-C₈)-Alkyl, gegebenenfalls substituiertes (C₆-C₁₄)-Aryl, (C₆-C₁₄)-Aryl-(C₁-C₈)-alkyl, (C₃-C₈)-Cycloalkyl, (C₂-C₈)-Alkenyl, (C₂-C₈)-Alkinyl, Pyridyl, R¹¹ NH, R⁴CO, COOR⁴, CONHR¹⁴, CSNHR¹⁴, COOR¹⁵ und CONHR¹⁵ steht;
und e, g und h unabhängig voneinander für die Zahlen 0, 1, 2 oder 3 stehen.

5. Verwendung von Verbindungen der Formel I gemäß einem oder mehreren der Ansprüche 1 bis 4 in allen ihren stereoisomeren Formen und Mischungen davon in allen Verhältnissen sowie von ihren physiologisch verträglichen Salzen, wobei in der Formel I W für R¹-A-C(R¹³) und R¹³ für (C₁-C₆)-Alkyl, im Arylrest gegebenenfalls substituiertes (C₆-C₁₄)-Aryl-(C₁-C₈)-alkyl oder (C₃-C₈)-Cycloalkyl steht.

6. Verwendung von Verbindungen der Formel I gemäß einem oder mehreren der Ansprüche 1 bis 5 in allen ihren stereoisomeren Formen und Mischungen davon in allen Verhältnissen sowie von ihren physiologisch verträglichen Salzen, wobei in der Formel I R³ für gegebenenfalls substituiertes (C₆-C₁₄)-Aryl, für COOR⁴, für R¹¹NH oder für CONHR¹⁴ steht, wobei -NHR¹⁴ für den Rest einer α-Aminosäure, deren ω-Amino-(C₂-C₈)-alkylamid, deren (C₁-C₈)-Alkylester oder deren (C₆-C₁₄)-Aryl-(C₁-C₄)-alkylester, bevorzugt für den Rest der α-Aminosäuren Valin, Lysin, Phenylglycin, Phenylalanin oder Tryptophan oder deren (C₁-C₈)-Alkylester oder deren (C₆-C₁₄)-Aryl-(C₁-C₄)-alkylester, steht.

7. Verwendung von Verbindungen der Formel I gemäß einem oder mehreren der Ansprüche 1 und 3 bis 6 in allen ihren stereoisomeren Formen und Mischungen davon in allen Verhältnissen sowie von ihren physiologisch verträglichen Salzen, wobei in der Formel I gleichzeitig
W für R¹-A-C(R¹³) steht;
Y für eine Carbonylgruppe steht;
Z für N(R⁰) steht;
A für Ethylen, Trimethylen, Tetramethylen, Pentamethylen, Cyclohexylen, Phenylen oder Phenylenmethyl steht;
B für einen unsubstituierten oder substituierten Methylenrest steht;
D für C(R²)(R³) steht;
E für R¹⁰CO steht;
R für Wasserstoff oder (C₁-C₄)-Alkyl, insbesondere Wasserstoff, Methyl oder Ethyl, steht;
R⁰ für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
R¹ für H₂N-C(=NH), H₂N-C(=NH)-NH oder H₂N-CH₂ steht;
R² für Wasserstoff steht;
R³ für den Rest CONHR¹⁴ steht;
R¹⁰ für Hydroxy oder (C₁-C₈)-Alkoxy, bevorzugt (C₁-C₄)-Alkoxy, steht;
R¹³ für (C₁-C₆)-Alkyl, (C₃-C₇)-Cycloalkyl oder Benzyl, insbesondere Methyl, steht;
R¹⁴ für Methyl steht, das durch Hydroxycarbonyl und einen Rest aus der Reihe (C₁-C₄)-Alkyl, Phenyl und Benzyl substituiert ist, oder für Methyl steht, das durch (C₁-C₈)-Alkoxycarbonyl, bevorzugt (C₁-C₄)-Alkoxycarbonyl, und einen Rest aus der Reihe (C₁-C₄)-Alkyl, Phenyl und Benzyl substituiert ist;
b, c und d für 1 stehen und e, f und g für 0 stehen;
h für 1 oder 2, bevorzugt für 1, steht.

8. Verwendung von Verbindungen der Formel I gemäß einem oder mehreren der Ansprüche 1, 3 und 4 in allen ihren stereoisomeren Formen und Mischungen davon in allen Verhältnissen sowie von ihren physiologisch verträglichen Salzen, wobei in der Formel I gleichzeitig
W für R¹-A-CH=C und darin A für einen Phenylenrest steht oder W für R¹-A-C(R¹³) und darin A für einen zweiwertigen Rest aus der Reihe Methylen, Ethylen, Trimethylen, Tetramethylen, Pentamethylen, Cyclohexylen, Phenylen, Phenylenmethyl steht;
B für einen zweiwertigen Rest aus der Reihe Methylen, Ethylen, Trimethylen, Tetramethylen, Vinylen, Phenylen oder für substituiertes Methylen oder Ethylen steht;
E R¹⁰ CO bedeutet;
R Wasserstoff oder (C₁-C₆)-Alkyl bedeutet;
R⁰ für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
R¹ für X-NH-C(=NH), X-NH-C(=NX)-NH oder X-NH-CH₂ steht;
X für Wasserstoff, (C₁-C₆)-Alkylcarbonyl, (C₁-C₆)-Alkoxycarbonyl, (C₁-C₈)-Alkylcarbonyloxy-(C₁-C₆)-alkoxycarbonyl, (C₆-C₁₄)-Aryl-(C₁-C₆)-alkoxycarbonyl oder Hydroxy steht;
R² für Wasserstoff oder (C₁-C₈)-Alkyl steht;
R³ für CONHR¹⁵ oder CONHR¹⁴ steht, wobei R¹⁴ hierin für einen unsubstituierten oder durch einen oder mehrere (C₆-C₁₄)-Arylreste substituierten (C₁-C₈)-Alkylrest steht;
R¹⁵ für R¹⁶-(C₁-C₆)-Alkyl oder R¹⁶ steht, wobei R¹⁶ für einen 7- bis 12-gliedrigen verbrückten bicyclischen oder tricyclischen Rest steht, der gesättigt oder teilweise ungesättigt ist und der auch ein bis vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann, und insbesondere R¹⁵ für einen Adamantylrest oder einen Adamantylmethylrest steht;
und e, g und h unabhängig voneinander für die Zahlen 0, 1, 2 oder 3 stehen und b, c und d für 1 stehen.

9. Verwendung von Verbindungen der Formel I gemäß einem oder mehreren der Ansprüche 1, 3, 4, 5 und 8 in allen ihren stereoisomeren Formen und Mischungen davon in allen Verhältnissen sowie von ihren physiologisch verträglichen Salzen, wobei in der Formel I gleichzeitig
W für R¹-A-C(R¹³) steht;
Y für eine Carbonylgruppe steht;
Z für N(R⁰) steht;
A für Ethylen, Trimethylen, Tetramethylen, Pentamethylen, Cyclohexylen, Phenylen oder Phenylenmethyl steht;
B für einen unsubstituierten oder substituierten Methylenrest steht;
D für C(R²)(R³) steht;
E für R¹⁰CO steht;
R für Wasserstoff oder (C₁-C₄)-Alkyl, insbesondere Wasserstoff, Methyl oder Ethyl, steht;
R⁰ für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
R¹ für H₂N-C(=NH), H₂N-C(=NH)-NH oder H₂N-CH₂ steht;
R² für Wasserstoff steht;
R³ für CONHR¹⁵ oder CONHR¹⁴ steht, wobei R¹⁴ hierin für einen unsubstituierten oder durch einen oder mehrere (C₆-C₁₀)-Arylreste substituierten (C₁-C₆)-Alkylrest steht;
R¹⁰ für Hydroxy oder (C₁-C₈)-Alkoxy, bevorzugt (C₁-C₄)-Alkoxy, steht;
R¹³ für (C₁-C₆)-Alkyl, (C₃-C₇)-Cycloalkyl oder Benzyl, insbesondere Methyl, steht;
R¹⁵ für einen Adamantylrest oder einen Adamantylmethylrest steht;
b, c und d für 1 stehen und e, f und g für 0 stehen;
h für 1 oder 2, bevorzugt für 1, steht.

10. Verwendung von Verbindungen der Formel I gemäß einem oder mehreren der Ansprüche 1, 3, 4 und 5 in allen ihren stereoisomeren Formen und Mischungen davon in allen Verhältnissen sowie von ihren physiologisch verträglichen Salzen, wobei in der Formel I gleichzeitig
W für R¹-A-C(R¹³) steht;
Y für eine Carbonylgruppe steht;
Z für N(R⁰) steht;
A für Ethylen, Trimethylen, Tetramethylen, Pentamethylen, Cyclohexylen, Phenylen, Phenylenmethyl steht;
B für einen unsubstituierten oder substiuierten Methylenrest oder Ethylenrest steht;
D für C(R²)(R³) steht;
E für R¹⁰CO steht;
R für Wasserstoff oder (C₁-C₄)-Alkyl, insbesondere Wasserstoff, Methyl oder Ethyl, steht;
R⁰ für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
R¹ für H₂N-C(=NH), H₂N-C(=NH)-NH oder H₂N-CH₂ steht;
R² für Wasserstoff steht;
R³ für einen unsubstituierten Phenylrest oder Naphthylrest, einen durch einen, zwei oder drei gleiche oder verschiedene Reste aus der Reihe (C₁-C₄)-Alkyl, (C₁-C₄)-Alkoxy, Hydroxy, Halogen, Trifluormethyl, Nitro, Methylendioxy, Ethylendioxy, Hydroxycarbonyl, (C₁-C₄)-Alkoxycarbonyl, Aminocarbonyl, Cyan, Phenyl, Phenoxy, Benzyl und Benzyloxy substituierten Phenylrest oder Naphthylrest, einen Pyridylrest, einen (C₁-C₄)-Alkylrest, einen (C₂-C₄)-Alkenylrest, einen (C₂-C₄)-Alkinylrest oder einen (C₅-C₆)-Cycloalkylrest steht, und insbesondere R³ für einen unsubstituierten oder substituierten Phenylrest oder Naphthylrest steht;
R¹⁰ für Hydroxy oder (C₁-C₈)-Alkoxy, insbesondere (C₁-C₄)-Alkoxy, steht und bevorzugt R¹⁰ für einen Rest aus der Reihe Hydroxy, Methoxy, Ethoxy, Propoxy und Isopropoxy steht;
R¹³ für (C₁-C₆)-Alkyl, (C₃-C₇)-Cycloalkyl oder Benzyl, insbesondere Methyl, steht;
b, c und d für 1 stehen und e, f und g für 0 stehen;
h für 1 oder 2, bevorzugt für 1, steht.

11. Verwendung von Verbindungen der Formel I gemäß einem oder mehreren der Ansprüche 1, 3, 4 und 5 in allen ihren stereoisomeren Formen und Mischungen davon in allen Verhältnissen sowie von ihren physiologisch verträglichen Salzen, wobei in der Formel I gleichzeitig
W für R¹-A-C(R¹³) steht;
Y für eine Carbonylgruppe steht;
Z für N(R⁰) steht;
A für Ethylen, Trimethylen, Tetramethylen, Pentamethylen, Cyclohexylen, Phenylen, Phenylenmethyl steht;
B für einen unsubstituierten oder substituierten Methylenrest oder Ethylenrest steht;
D für C(R²)(R³) steht;
E für R¹⁰CO steht;
R für Wasserstoff oder (C₁-C₄)-Alkyl, insbesondere Wasserstoff, Methyl oder Ethyl, steht;
R⁰ für (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl steht;
R¹ für H₂N-C(=NH), H₂N-C(=NH)-NH oder H₂N-CH₂ steht;
R² für Wasserstoff steht;
R³ für R¹¹NH steht;
R¹⁰ für Hydroxy oder (C₁-C₈)-Alkoxy, insbesondere (C₁-C₄)-Alkoxy, steht und bevorzugt R¹⁰ für einen Rest aus der Reihe Hydroxy, Methoxy, Ethoxy, Propoxy und Isopropoxy steht;
R¹³ für (C₁-C₆)-Alkyl, (C₃-C₇)-Cycloalkyl oder Benzyl, insbesondere Methyl, steht;
b, c, d und e für 1 stehen und f und g für 0 stehen;
h für 0 steht.

12. Verwendung von Verbindungen der Formel I gemäß einem oder mehreren der Ansprüche 1 und 3 bis 11 in allen ihren stereoisomeren Formen und Mischungen davon in allen Verhältnissen sowie von ihren physiologisch verträglichen Salzen, worin ein für die Gruppe B stehender substituierter Methylenrest oder substituierter Ethylenrest als Substituenten einen Rest aus der Reihe (C₁-C₈)-Alkyl, (C₂-C₆)-Alkenyl, (C₂-C₆)-Alkinyl, (C₃-C₈)-Cycloalkyl, insbesondere (C₅-C₆)-Cycloalkyl, (C₃-C₈)-Cycloalkyl-(C₁-C₄)-alkyl, insbesondere (C₅-C₆)-Cycloalkyl-(C₁-C₄)-alkyl, gegebenenfalls substituiertes (C₆-C₁₀)-Aryl, im Arylrest gegebenenfalls substituiertes (C₆-C₁₀)-Aryl-(C₁-C₄)-alkyl, gegebenenfalls substituiertes Heteroaryl und im Heteroarylrest gegebenenfalls substituiertes Heteroaryl-(C₁-C₄)-alkyl trägt.

13. Verwendung von Verbindungen der Formel I gemäß einem oder mehreren der Ansprüche 1 und 3 bis 12 in allen ihren stereoisomeren Formen und Mischungen davon in allen Verhältnissen sowie von ihren physiologisch verträglichen Salzen, worin B für einen unsubstituierten Methylenrest steht oder für einen Methylenrest steht, der durch einen (C₁-C₈)-Alkylrest substituiert ist.

14. Verwendung von Verbindungen der Formel I gemäß einem oder mehreren der Ansprüche 1 bis 13 und/oder von ihren physiologisch verträglichen Salzen zur Herstellung von Arzneimitteln zur Entzündungshemmung.

15. Verwendung von Verbindungen der Formel I gemäß einem oder mehreren der Ansprüche 1 bis 13 und/oder von ihren physiologisch verträglichen Salzen zur Herstellung von Arzneimitteln zur Behandlung oder Prophylaxe der rheumatoiden Arthritis, der inflammatorischen bowel disease, des systemischen Lupus erythematosus oder von inflammatorischen Erkrankungen des zentralen Nervensystems.

16. Verwendung von Verbindungen der Formel I gemäß einem oder mehreren der Ansprüche 1 bis 13 und/oder von ihren physiologisch verträglichen Salzen zur Herstellung von Arzneimitteln zur Behandlung oder Prophylaxe von Asthma oder Allergien.

17. Verwendung von Verbindungen der Formel I gemäß einem oder mehreren der Ansprüche 1 bis 13 und/oder von ihren physiologisch verträglichen Salzen zur Herstellung von Arzneimitteln zur Behandlung oder Prophylaxe von cardiovaskulären Erkrankungen, der Arteriosklerose, von Restenosen oder von Diabetes, zur Verhinderung der Schädigung von Organtransplantaten, zur Hemmung von Tumorwachstum oder Tumormetastasierung oder zur Therapie der Malaria.

18. Verwendung von Verbindungen der Formel I gemäß einem oder mehreren der Ansprüche 1 bis 13 und/oder von ihren physiologisch verträglichen Salzen zur Hemmung der Adhäsion und/oder Migration von Leukozyten oder zur Hemmung des VLA-4-Rezeptors.

19. Verwendung von Verbindungen der Formel I gemäß einem oder mehreren der Ansprüche 1 bis 13 und/oder von ihren physiologisch verträglichen Salzen zur Behandlung oder Prophylaxe von Krankheiten, bei denen die Leukozytenadhäsion und/oder Leukozytenmigration ein unerwünschtes Ausmaß aufweist, oder von Krankheiten, bei denen VLA-4-abhängige Adhäsionsvorgänge eine Rolle spielen, zur Behandlung oder Prophylaxe der rheumatoiden Arthritis, der inflammatorischen bowel disease, des systemischen Lupus erythematosus, von inflammatorischen Erkrankungen des zentralen Nervensystems, von Asthma, von Allergien, von cardiovaskulären Erkrankungen, von Arteriosklerose, von Restenosen, von Diabetes, zur Verhinderung der Schädigung von Organtransplantaten, zur Hemmung von Tumorwachstum oder Tumormetastasierung, zur Therapie der Malaria, oder als Entzündungshemmer.

20. Verbindungen der Formel Ib, 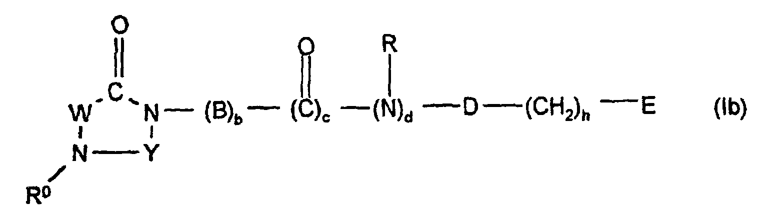 worin
W für R¹-A-CH oder R¹-A-CH=C steht;
Y für eine Carbonyl-, Thiocarbonyl- oder Methylengruppe steht;
A einen zweiwertigen Rest aus der Reihe (C₁-C₆)-Alkylen, (C₃-C₇)-Cycloalkylen, Phenylen, Phenylen-(C₁-C₆)-alkyl,(C₁-C₆)-Alkylen-phenyl, Phenylen-(C₁-C₆)-alkenyl oder einen zweiwertigen Rest eines 5- oder 6-gliedrigen gesättigten oder ungesättigten Ringes, der 1 oder 2 Stickstoffatome enthalten und ein- oder zweifach durch (C₁-C₆)-Alkyl oder doppelt gebundenen Sauerstoff oder Schwefel substituiert sein kann, bedeutet;
B einen zweiwertigen Rest aus der Reihe (C₁-C₆)-Alkylen, (C₂-C₆)-Alkenylen, Phenylen, Phenylen-(C₁-C₃)-alkyl, (C₁-C₃)-Alkylen-phenyl bedeutet;
D für C(R²)(R³) steht;
E Tetrazolyl, (R⁸O)₂P(O), HOS(O)₂, R⁹NHS(O)₂ oder R¹⁰CO bedeutet;
R Wasserstoff, (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl bedeutet;
R⁰ (C₇-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl bedeutet;
R¹ für X-NH-C(=NH)-(CH₂)ₚ oder X¹-NH-(CH₂)ₚ steht, wobei p für die Zahlen 0, 1, 2 oder 3 steht;
X Wasserstoff, (C₁-C₆)-Alkyl, (C₁-C₆)-Alkylcarbonyl, (C₁-C₆)-Alkoxycarbonyl, (C₁-C₁₈)-Alkylcarbonyloxy-(C₁-C₆)-alkoxycarbonyl, gegebenenfalls substituiertes (C₆-C₁₄)-Arylcarbonyl, gegebenenfalls substituiertes (C₆-C₁₄)-Aryloxycarbonyl, (C₆-C₁₄)-Aryl-(C₁-C₆)-alkoxycarbonyl, das im Arylrest auch substituiert sein kann, (R⁸O)₂P(O), Cyano, Hydroxy, (C₁-C₆)-Alkoxy, (C₆-C₁₄)-Aryl-(C₁-C₆)-alkoxy, das im Arylrest auch substituiert sein kann, oder Amino bedeutet;
X¹ eine der Bedeutungen von X hat oder R'-NH-C(=N-R") bedeutet, wobei R' und R" unabhängig voneinander die Bedeutungen von X haben;
R² Wasserstoff oder Phenyl bedeutet;
R³ Wasserstoff, COOR⁴, CON(CH₃)R⁴ oder CONHR⁴ bedeutet;
R⁴ Wasserstoff oder (C₁-C₂₈)-Alkyl bedeutet, das gegebenenfalls ein- oder mehrfach durch gleiche oder verschiedene Reste R^{4'} substituiert sein kann;
R^{4'} Hydroxy, Hydroxycarbonyl, Aminocarbonyl, Mono- oder Di-((C₁-C₁₈)-alkyl)aminocarbonyl, Amino-(C₂-C₁₈)-alkylaminocarbonyl, Amino-(C₁-C₃)-alkylphenyl-(C₁-C₃)-alkylaminocarbonyl, (C₁-C₁₈)-Alkylcarbonylamino-(C₁-C₃)-alkylphenyl-(C₁-C₃)-alkylaminocarbonyl, (C₁-C₁₈)-Alkylcarbonylamino-(C₂-C₁₈)-alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)-Alkoxycarbonyl, gegebenenfalls substituiertes (C₃-C₈)-Cycloalkyl, Halogen, Nitro, Trifluormethyl oder den Rest R⁵ bedeutet;
R⁵ gegebenenfalls substituiertes (C₆-C₁₄)-Aryl, im Arylrest gegebenenfalls substituiertes (C₆-C₁₄)-Aryl-(C₁-C₈)-alkyl, einen mono- oder bicyclischen 5- bis 12-gliedrigen heterocyclischen Ring, der aromatisch, teilhydriert oder vollständig hydriert sein kann und der ein, zwei oder drei gleiche oder verschiedene Heteroatome aus der Reihe Stickstoff, Sauerstoff und Schwefel enthalten kann, einen Rest R⁶ oder einen Rest R⁶CO- bedeutet, wobei der Aryl- und unabhängig davon der Heterocyclus-Rest ein- oder mehrfach durch gleiche oder verschiedene Reste aus der Reihe (C₁-C₁₈)-Alkyl, (C₁-C₁₈)-Alkoxy, Halogen, Nitro, Amino oder Trifluormethyl substituiert sein können;
R⁶ für R⁷R⁸N, R⁷O oder R⁷S steht oder eine Aminosäureseitenkette, einen natürlichen oder unnatürlichen Aminosäure-, Iminosäure-, gegebenenfalls N-(C₁-C₆)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, sowie deren Ester und Amide bedeutet, wobei anstelle freier funktioneller Gruppen gegebenenfalls Wasserstoff oder Hydroxymethyl stehen kann und/oder wobei freie funktionelle Gruppen durch in der Peptidchemie übliche Schutzgruppen geschützt sein können;
R⁷ Wasserstoff, (C₁-C₁₈)-Alkyl, (C₆-C₁₄)-Aryl-(C₁-C₈)-alkyl, (C₁-C₁₈)-Alkylcarbonyl, (C₁-C₁₈)-Alkoxycarbonyl, (C₆-C₁₄)-Arylcarbonyl, (C₆-C₁₄)-Aryl-(C₁-C₈)-alkylcarbonyl oder (C₆-C₁₄)-Aryl-(C₁-C₁₈)-alkyloxycarbonyl, wobei die Alkylgruppen gegebenenfalls durch eine Aminogruppe substituiert und/oder wobei die Arylreste ein- oder mehrfach, vorzugsweise einfach, durch gleiche oder verschiedene Reste aus der Reihe (C₁-C₈)-Alkyl, (C₁-C₈)-Alkoxy, Halogen, Nitro, Amino und Trifluormethyl substituiert sein können, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, bedeutet;
R⁸ Wasserstoff, (C₁-C₁₈)-Alkyl, gegebenenfalls substituiertes (C₆-C₁₄)-Aryl oder (C₆-C₁₄)-Aryl oder (C₆-C₁₄)-Aryl-(C₁-C₈)-alkyl, das im Arylrest auch substituiert sein kann, bedeutet;
R⁹ Wasserstoff, Aminocarbonyl, (C₁-C₁₈)-Alkylaminocarbonyl, (C₃-C₈)-Cycloalkylaminocarbonyl, gegebenenfalls substituiertes (C₆-C₁₄)-Arylaminocarbonyl, (C₁-C₁₈)-Alkyl, gegebenenfalls substituiertes (C₆-C₁₄)-Aryl oder (C₃-C₈)-Cycloalkyl bedeutet;
R¹⁰ Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-Alkyl)-amino bedeutet;
b, c, und d unabhängig voneinander für 0 oder 1 stehen können, aber nicht alle gleichzeitig 0 sein können;
h für die Zahlen 0, 1, 2, 3, 4, 5 oder 6 steht;
in allen ihren stereoisomeren Formen und Mischungen davon in allen Verhältnissen, sowie ihre physiologisch verträglichen Salze.

21. Verbindungen der Formel Ib gemäß Anspruch 20 und/oder ihre physiologisch verträglichen Salze zur Verwendung als Arzneimittel.

22. Pharmazeutisches Präparat, **dadurch gekennzeichnet, daß** es eine oder mehrere Verbindungen der Formel Ib gemäß Anspruch 20 und/oder ihre physiologisch verträglichen Salze neben pharmazeutisch einwandfreien Trägerstoffen und/oder Zusatzstoffen enthält.

23. Verbindungen der Formel Ic, 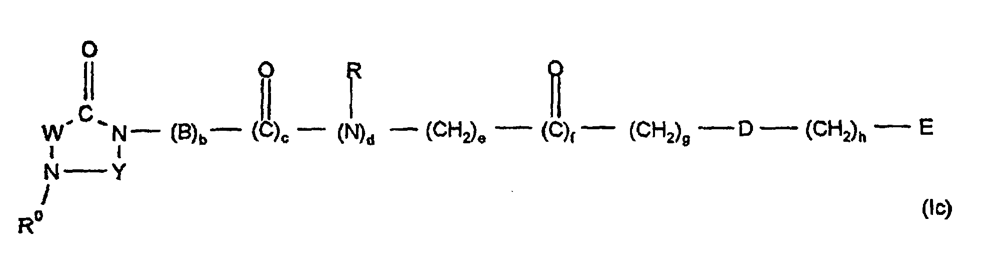 worin
W für R¹-A-C(R¹³) steht;
Y für eine Carbonyl-, Thiocarbonyl- oder Methylengruppe steht;
A einen Phenylenrest steht;
B einen zweiwertigen Rest aus der Reihe (C₁-C₆)-Alkylen, (C₂-C₆)-Alkenylen, Phenylen, Phenylen-(C₁-C₃)-alkyl, (C₁-C₃)-Alkylen-phenyl bedeutet;
D für C(R²)(R³), N(R³) oder CH=C(R³) steht;
E Tetrazolyl, (R⁸O)₂P(O), HOS(O)₂, R⁹NHS(O)₂ oder R¹⁰CO bedeutet;
R Wasserstoff, (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl bedeutet;
R⁰ für im Arylrest gegebenenfalls substituiertes (C₆-C₁₄)-Aryl-(C₁-C₈)-alkyl steht;
R¹ für X-NH-C(=NH)-(CH₂)ₚ oder X¹-NH-(CH₂)ₚ steht, wobei p für 0, 1, 2 oder 3 steht;
X Wasserstoff, (C₁-C₆)-Alkyl, (C₁-C₆)-Alkylcarbonyl, (C₁-C₆)-Alkoxycarbonyl, (C₁-C₁₈)-Alkylcarbonyloxy-(C₁-C₆)-alkoxycarbonyl, gegebenenfalls substituiertes (C₆-C₁₄)-Arylcarbonyl, gegebenenfalls substituiertes (C₆-C₁₄)-Aryloxycarbonyl, (C₆-C₁₄)-Aryl-(C₁-C₆)-alkoxycarbonyl, das im Arylrest auch substituiert sein kann, (R⁸O)₂P(O), Cyano, Hydroxy, (C₁-C₆)-Alkoxy, (C₆-C₁₄)-Aryl-(C₁-C₆)-alkoxy, das im Arylrest auch substituiert sein kann, oder Amino bedeutet;
X¹ eine der Bedeutungen von X hat oder R'-NH-C(=N-R'') bedeutet, wobei R' und R" unabhängig voneinander die Bedeutungen von X haben;
R² Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl oder (C₃-C₈)-Cycloalkyl bedeutet;
R³ Wasserstoff, (C₁-C₈)-Alkenyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)-Cycloalkyl, (C₂-C₈)-Alkenyl, (C₂-C₈)-Alkinyl, (C₂-C₈)-Alkenylcarbonyl, (C₂-C₈)-Alkinylcarbonyl, Pyridyl, R¹¹NH, R⁴CO, COOR⁴, CON(CH₃)R¹⁴, CONHR¹⁴, CSNHR¹⁴, COOR¹⁵, CON(CH₃)R¹⁵ oder CONHR¹⁵ bedeutet;
R⁴ Wasserstoff oder (C₁-C₂₈)-Alkyl bedeutet, das gegebenenfalls ein- oder mehrfach durch gleiche oder verschiedene Reste R^{4'} substituiert sein kann;
R^{4'} Hydroxy, Hydroxycarbonyl, Aminocarbonyl, Mono- oder Di-((C₁-C₁₈)-alkyl)-aminocarbonyl, Amino-(C₂-C₁₈)-alkylaminocarbonyl, Amino-(C₁-C₃)-alkylphenyl-(C₁-C₃)-alkylaminocarbonyl, (C₁-C₁₈)-Alkylcarbonylamino-(C₁-C₃)-alkylphenyl-(C₁-C₃)-alkylaminocarbonyl, (C₁-C₁₈)-Alkylcarbonylamino-(C₂-C₁₈)-alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)-Alkoxycarbonyl, gegebenenfalls substituiertes (C₃-C₈)-Cycloalkyl, Halogen, Nitro, Trifluormethyl oder den Rest R⁵ bedeutet;
R⁵ gegebenenfalls substituiertes (C₆-C₁₄)-Aryl, im Arylrest gegebenenfalls substituiertes (C₆-C₁₄)-Aryl-(C₁-C₈)-alkyl, einen mono- oder bicyclischen 5- bis 12-gliedrigen heterocyclischen Ring, der aromatisch, teilhydriert oder vollständig hydriert sein kann und der ein, zwei oder drei gleiche oder verschiedene Heteroatome aus der Reihe Stickstoff, Sauerstoff und Schwefel enthalten kann, einen Rest R⁶ oder einen Rest R⁶CO- bedeutet, wobei der Aryl- und unabhängig davon der Heterocyclus-Rest ein- oder mehrfach durch gleiche oder verschiedene Reste aus der Reihe (C₁-C₁₈)-Alkyl, (C₁-C₁₈)-Alkoxy, Halogen, Nitro, Amino oder Trifluormethyl substituiert sein können;
R⁶ für R⁷R⁸N, R⁷O oder R⁷S steht oder eine Aminosäureseitenkette, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, sowie deren Ester und Amide bedeutet, wobei anstelle freier funktioneller Gruppen gegebenenfalls Wasserstoff oder Hydroxymethyl stehen kann und/oder wobei freie funktionelle Gruppen durch in der Peptidchemie übliche Schutzgruppen geschützt sein können;
R⁷ Wasserstoff, (C₁-C₁₈)-Alkyl, (C₆-C₁₄)-Aryl-(C₁-C₈)-alkyl, (C₁-C₁₈)-Alkylcarbonyl, (C₁-C₁₈)-Alkoxycarbonyl, (C₆-C₁₄)-Arylcarbonyl, (C₆-C₁₄)-Aryl-(C₁-C₈)-alkylcarbonyl oder (C₆-C₁₄)-Aryl-(C₁-C₁₈)-alkyloxycarbonyl, wobei die Alkylgruppen gegebenenfalls durch eine Aminogruppe substituiert und/oder wobei die Arylreste ein- oder mehrfach, vorzugsweise einfach, durch gleiche oder verschiedene Reste aus der Reihe (C₁-C₈)-Alkyl, (C₁-C₈)-Alkoxy, Halogen, Nitro, Amino und Trifluormethyl substituiert sein können, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, bedeutet;
R⁸ Wasserstoff, (C₁-C₁₈)-Alkyl, gegebenenfalls substituiertes (C₆-C₁₄)-Aryl oder (C₆-C₁₄)-Aryl-(C₁-C₈)-alkyl, das im Arylrest auch substituiert sein kann, bedeutet;
R⁹ Wasserstoff, Aminocarbonyl, (C₁-C₁₈)-Alkylaminocarbonyl, (C₃-C₈)-Cycloalkylaminocarbonyl, gegebenenfalls substituiertes (C₆-C₁₄)-Arylaminocarbonyl, (C₁-C₁₈)-Alkyl, gegebenenfalls substituiertes (C₆-C₁₄)-Aryl oder (C₃-C₈)-Cycloalkyl bedeutet;
R¹⁰ Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
R¹¹ Wasserstoff, (C₁-C₁₈)-Alkyl, R¹²CO, gegebenenfalls substituiertes (C₆-C₁₄)-Aryl-S(O)₂, (C₁-C₁₈)-Alkyl-S(O)₂, im Arylrest gegebenenfalls substituiertes (C₆-C₁₄)-Aryl-(C₁-C₈)-alkyl oder R⁹NHS(O)₂ bedeutet;
R¹² Wasserstoff, (C₁-C₁₈)-Alkyl, (C₂-C₈)-Alkenyl, (C₂-C₈)-Alkinyl, gegebenenfalls substituiertes (C₆-C₁₄)-Aryl, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
R¹³ (C₁-C₁₈)-Alkyl, im Arylrest gegebenenfalls substituiertes (C₆-C₁₄)-Aryl-(C₁-C₈)-alkyl oder (C₃-C₈)-Cycloalkyl bedeutet;
R¹⁴ Wasserstoff oder (C₁-C₂₈)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-((C₁-C₁₈)-alkyl)-aminocarbonyl, Amino-(C₂-C₁₈)-alkylaminocarbonyl, Amino-(C₁-C₃)-alkylphenyl-(C₁-C₃)-alkylaminocarbonyl, (C₁-C₁₈)-Alkylcarbonylamino-(C₁-C₃)-alkylphenyl-(C₁-C₃)-alkylaminocarbonyl, (C₁-C₁₈)-Alkylcarbonyl-amino-(C₂-C₁₈)-alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)-Alkoxycarbonyl, gegebenenfalls substituiertes (C₃-C₈)-Cycloalkyl, HOS(O)₂-(C₁-C₃)-alkyl, R⁹NHS(O)₂-(C₁-C₃)-alkyl, (R⁸O)₂P(O)-(C₁-C₃)-alkyl, Tetrazolyl-(C₁-C₃)-alkyl, Halogen, Nitro, Trifluormethyl und R⁵ substituiert sein kann;
R¹⁵ für R¹⁶-(C₁-C₆)-alkyl oder für R¹⁶ steht;
R¹⁶ für einen 6- bis 24-gliedrigen bicyclischen oder tricyclischen Rest steht, der gesättigt oder teilweise ungesättigt ist und der auch ein bis vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann;
b, c, d und f unabhängig voneinander für 0 oder 1 stehen, aber nicht alle gleichzeitig 0 sein können;
e, g und h unabhängig voneinander für 0, 1, 2, 3, 4, 5 oder 6 stehen;
in allen ihren stereoisomeren Formen und Mischungen davon in allen Verhältnissen, sowie ihre physiologisch verträglichen Salze.

24. Verbindungen der Formel Ic gemäß Anspruch 23 und/oder ihre physiologisch verträglichen Salze zur Verwendung als Arzneimittel.

25. Pharmazeutisches Präparat, **dadurch gekennzeichnet, daß** es eine oder mehrere Verbindungen der Formel Ic gemäß Anspruch 23 und/oder ihre physiologisch verträglichen Salze neben pharmazeutisch einwandfreien Trägerstoffen und/oder Zusatzstoffen enthält.

26. Verbindungen der Formel Id, 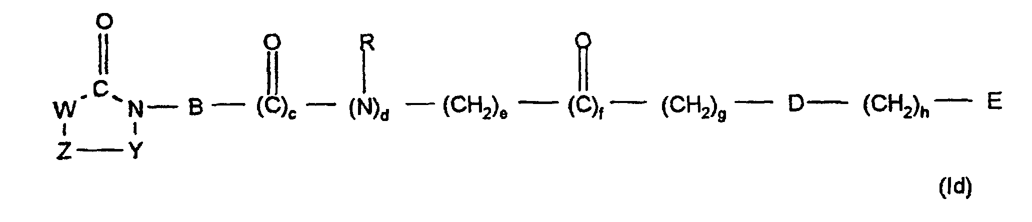 worin
W für R¹-A-C(R¹³) oder R¹-A-CH=C steht;
Y für eine Carbonyl-, Thiocarbonyl- oder Methylengruppe steht;
Z für N(R⁰) steht;
A einen zweiwertigen Rest aus der Reihe (C₁-C₆)-Alkylen, (C₃-C₇)-Cycloalkylen, Phenylen, Phenylen-(C₁-C₆)-alkyl, (C₁-C₆)-Alkylen-phenyl, Phenylen-(C₁-C₆)-alkenyl oder einen zweiwertigen Rest eines 5- oder 6-gliedrigen gesättigten oder ungesättigten Ringes, der 1 oder 2 Stickstoffatome enthalten und ein- oder zweifach durch (C₁-C₆)-Alkyl oder doppelt gebundenen Sauerstoff oder Schwefel substituiert sein kann, bedeutet;
B einen zweiwertigen (C₁-C₆)-Alkylen-Rest bedeutet, der durch einen Rest aus der Reihe (C₁-C₈)-Alkyl, (C₂-C₈)-Alkenyl, (C₂-C₈)-Alkinyl, (C₃-C₁₀)-Cycloalkyl, (C₃-C₁₀)-Cycloalkyl-(C₁-C₆)-alkyl, gegebenenfalls substituiertes (C₆-C₁₄)-Aryl, im Arylrest gegebenenfalls substituiertes (C₆-C₁₄)-Aryl-(C₁-C₆)-alkyl, gegebenenfalls substituiertes Heteroaryl und im Heteroarylrest gegebenenfalls substituiertes Heteroaryl-(C₁-C₆)-alkyl substituiert ist;
D für C(R²)(R³), N(R³) oder CH=C(R³) steht;
E Tetrazolyl, (R⁸O)₂P(O), HOS(O)₂, R⁹NHS(O)₂ oder R¹⁰CO bedeutet;
R Wasserstoff, (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl bedeutet;
R⁰ für Wasserstoff, (C₁-C₈)-Alkyl, (C₃-C₁₂)-Cycloalkyl, (C₃-C₁₂)-Cycloalkyl-(C₁-C₈)-alkyl, (C₆-C₁₂)-Bicycloalkyl, (C₆-C₁₂)-Bicycloalkyl-(C₁-C₈)-alkyl, (C₆-C₁₂)-Tricycloalkyl, (C₆-C₁₂)-Tricycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl, im Heteroarylrest gegebenenfalls substituiertes Heteroaryl-(C₁-C₈)-alkyl, CHO, (C₁-C₈)-Alkyl-CO, (C₃-C₁₂)-Cycloalkyl-CO, (C₃-C₁₂)-Cycloalkyl-(C₁-C₈)-alkyl-CO, (C₆-C₁₂)-Bicycloalkyl-CO, (C₆-C₁₂)-Bicycloalkyl-(C₁-C₈)-alkyl-CO, (C₆-C₁₂)-Tricycloalkyl-CO, (C₆-C₁₂)-Tricycloalkyl-(C₁-C₈)-alkyl-CO, gegebenenfalls substituiertes (C₆-C₁₄)-Aryl-CO, im Arylrest gegebenenfalls substituiertes (C₆-C₁₄)-Aryl-(C₁-C₈)-alkyl-CO, gegebenenfalls substituiertes Heteroaryl-CO, im Heteroarylrest gegebenenfalls substituiertes Heteroaryl-(C₁-C₈)-alkyl-CO, (C₁-C₈)-Alkyl-S(O)ₙ, (C₃-C₁₂)-Cycloalkyl-S(O)ₙ, (C₃-C₁₂)-Cycloalkyl-(C₁-C₈)-alkyl-S(O)ₙ, (C₆-C₁₂)-Bicycloalkyl-S(O)ₙ, (C₆-C₁₂)-Bicycloalkyl-(C₁-C₈)-alkyl-S(O)ₙ, (C₆-C₁₂)-Tricycloalkyl-S(O)ₙ, (C₆-C₁₂)-Tricycloalkyl-(C₁-C₈)-alkyl-S(O)ₙ, gegebenenfalls substituiertes (C₆-C₁₄)-Aryl-S(O)ₙ, im Arylrest gegebenenfalls substituiertes (C₆-C₁₄)-Aryl-(C₁-C₈)-alkyl-S(O)ₙ, gegebenenfalls substituiertes Heteroaryl-S(O)ₙ oder im Heteroarylrest gegebenenfalls substituiertes Heteroaryl-(C₁-C₈)-alkyl-S(O)ₙ steht, wobei n für 1 oder 2 steht;
R¹ für X-NH-C(=NH)-(CH₂)ₚ oder X¹-NH-(CH₂)ₚ steht, wobei p für 0, 1, 2 oder 3 steht;
X Wasserstoff, (C₁-C₆)-Alkyl, (C₁-C₆)-Alkylcarbonyl, (C₁-C₆)-Alkoxycarbonyl, (C₁-C₁₈)-Alkylcarbonyloxy-(C₁-C₆)-alkoxycarbonyl, gegebenenfalls substituiertes (C₆-C₁₄)-Arylcarbonyl, gegebenenfalls substituiertes (C₆-C₁₄)-Aryloxycarbonyl, (C₆-C₁₄)-Aryl-(C₁-C₆)-alkoxycarbonyl, das im Arylrest auch substituiert sein kann, (R⁸O)₂P(O), Cyano, Hydroxy, (C₁-C₆)-Alkoxy, (C₆-C₁₄)-Aryl-(C₁-C₆)-alkoxy, das im Arylrest auch substituiert sein kann, oder Amino bedeutet;
X¹ eine der Bedeutungen von X hat oder R'-NH-C(=N-R'') bedeutet, wobei R' und R" unabhängig voneinander die Bedeutungen von X haben;
R² Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl oder (C₃-C₈)-Cycloalkyl bedeutet;
R³ Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)-Cycloalkyl, (C₂-C₈)-Alkenyl, (C₂-C₈)-Alkinyl, (C₂-C₈)-Alkenylcarbonyl, (C₂-C₈)-Alkinylcarbonyl, Pyridyl, R¹¹NH, R⁴CO, COOR⁴, CON(CH₃)R¹⁴, CONHR¹⁴, CSNHR¹⁴, COOR¹⁵, CON(CH₃)R¹⁵ oder CONHR¹⁵ bedeutet;
R⁴ Wasserstoff oder (C₁-C₂₈)-Alkyl bedeutet, das gegebenenfalls ein- oder mehrfach durch gleiche oder verschiedene Reste R^{4'} substituiert sein kann;
R^{4'} Hydroxy, Hydroxycarbonyl, Aminocarbonyl, Mono-oder Di-((C₁-C₁₈)-alkyl)-aminocarbonyl, Amino-(C₂-C₁₈)-alkylaminocarbonyl, Amino-(C₁-C₃)-alkylphenyl-(C₁-C₃)-alkylaminocarbonyl, (C₁-C₁₈)-Alkylcarbonylamino-(C₁-C₃)-alkylphenyl-(C₁-C₃)-alkylaminocarbonyl, (C₁-C₁₈)-Alkylcarbonylamino-(C₂-C₁₈)-alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)-Alkoxycarbonyl, gegebenenfalls substituiertes (C₃-C₈)-Cycloalkyl, Halogen, Nitro, Trifluormethyl oder den Rest R⁵ bedeutet;
R⁵ gegebenenfalls substituiertes (C₆-C₁₄)-Aryl, im Arylrest gegebenenfalls substituiertes (C₆-C₁₄)-Aryl-(C₁-C₈)-alkyl, einen mono- oder bicyclischen 5- bis 12-gliedrigen heterocyclischen Ring, der aromatisch, teilhydriert oder vollständig hydriert sein kann und der ein, zwei oder drei gleiche oder verschiedene Heteroatome aus der Reihe Stickstoff, Sauerstoff und Schwefel enthalten kann, einen Rest R⁶ oder einen Rest R⁶CO- bedeutet, wobei der Aryl- und unabhängig davon der Heterocyclus-Rest ein- oder mehrfach durch gleiche oder verschiedene Reste aus der Reihe (C₁-C₁₈)-Alkyl, (C₁-C₁₈)-Alkoxy, Halogen, Nitro, Amino oder Trifluormethyl substituiert sein können;
R⁶ für R⁷R⁸N, R⁷O oder R⁷S steht oder eine Aminosäureseitenkette, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, sowie deren Ester und Amide bedeutet, wobei anstelle freier funktioneller Gruppen gegebenenfalls Wasserstoff oder Hydroxymethyl stehen kann und/oder wobei freie funktionelle Gruppen durch in der Peptidchemie übliche Schutzgruppen geschützt sein können;
R⁷ Wasserstoff, (C₁-C₁₈)-Alkyl, (C₆-C₁₄)-Aryl-(C₁-C₈)-alkyl, (C₁-C₁₈)-Alkylcarbonyl, (C₁-C₁₈)-Alkoxycarbonyl, (C₆-C₁₄)-Arylcarbonyl, (C₆-C₁₄)-Aryl-(C₁-C₈)-alkylcarbonyl oder (C₆-C₁₄)-Aryl-(C₁-C₁₈)-alkyloxycarbonyl, wobei die Alkylgruppen gegebenenfalls durch eine Aminogruppe substituiert und/oder wobei die Arylreste ein- oder mehrfach, vorzugsweise einfach, durch gleiche oder verschiedene Reste aus der Reihe (C₁-C₈)-Alkyl, (C₁-C₈)-Alkoxy, Halogen, Nitro, Amino und Trifluormethyl substituiert sein können, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, bedeutet;
R⁸ Wasserstoff, (C₁-C₁₈)-Alkyl, gegebenenfalls substituiertes (C₆-C₁₄)-Aryl oder (C₆-C₁₄)-Aryl-(C₁-C₈)-alkyl, das im Arylrest auch substituiert sein kann, bedeutet;
R⁹ Wasserstoff, Aminocarbonyl, (C₁-C₁₈)-Alkylaminocarbonyl, (C₃-C₈)-Cycloalkylaminocarbonyl, gegebenenfalls substituiertes (C₆-C₁₄)-Arylaminocarbonyl, (C₁-C₁₈)-Alkyl, gegebenenfalls substituiertes (C₆-C₁₄)-Aryl oder (C₃-C₈)-Cycloalkyl bedeutet;
R¹⁰ Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
R¹¹ Wasserstoff, (C₁-C₁₈)-Alkyl, R¹²CO, gegebenenfalls substituiertes (C₆-C₁₄)-Aryl-S(O)₂, (C₁-C₁₈)-Alkyl-S(O)₂, im Arylrest gegebenenfalls substituiertes (C₆-C₁₄)-Aryl-(C₁-C₈)-alkyl oder R⁹NHS(O)₂ bedeutet;
R¹² Wasserstoff, (C₁-C₁₈)-Alkyl, (C₂-C₈)-Alkenyl, (C₂-C₈)-Alkinyl, gegebenenfalls substituiertes (C₆-C₁₄)-Aryl, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
R¹³ Wasserstoff, (C₁-C₆)-Alkyl, im Arylrest gegebenenfalls substituiertes (C₆-C₁₄)-Aryl-(C₁-C₈)-alkyl oder (C₃-C₈)-Cycloalkyl bedeutet;
R¹⁴ Wasserstoff oder (C₁-C₂₈)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-((C₁-C₁₈)-alkyl)-aminocarbonyl, Amino-(C₂-C₁₈)-alkylaminocarbonyl, Amino-(C₁-C₃)-alkylphenyl-(C₁-C₃)-alkylaminocarbonyl, (C₁-C₁₈)-Alkylcarbonylamino-(C₁-C₃)-alkylphenyl-(C₁-C₃)-alkylaminocarbonyl, (C₁-C₁₈)-Alkylcarbonyl-amino-(C₂-C₁₈)-alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)-Alkoxycarbonyl, gegebenenfalls substituiertes (C₃-C₈)-Cycloalkyl, HOS(O)₂-(C₁-C₃)-alkyl, R⁹NHS(O)₂-(C₁-C₃)-alkyl, (R⁸O)₂P(O)-(C₁-C₃)-alkyl, Tetrazolyl-(C₁-C₃)-alkyl, Halogen, Nitro, Trifluormethyl und R⁵ substituiert sein kann;
R¹⁵ für R¹⁶-(C₁-C₆)-alkyl oder für R¹⁶ steht;
R¹⁶ für einen 6- bis 24-gliedrigen bicyclischen oder tricyclischen Rest steht, der gesättigt oder teilweise ungesättigt ist und der auch ein bis vier gleiche oder verschiedene Heteroatome aus der Reihe Stickstoff, Säuerstoff und Schwefel enthalten kann und der auch durch einen oder mehrere gleiche oder verschiedene Substituenten aus der Reihe (C₁-C₄)-Alkyl und Oxo substituiert sein kann;
c, d und f unabhängig voneinander für 0 oder 1 stehen, aber nicht alle gleichzeitig 0 sein können;
e, g und h unabhängig voneinander für 0, 1, 2, 3, 4, 5 oder 6 stehen;
in allen ihren stereoisomeren Formen und Mischungen davon in allen Verhältnissen, sowie ihre physiologisch verträglichen Salze.

27. Verbindungen der Formel Id gemäß Anspruch 26, worin gleichzeitig
W für R¹-A-C(R¹³) steht;
Y für eine Carbonylgruppe steht;
Z für N(R⁰) steht;
A einen zweiwertigen Rest aus der Reihe (C₃-C₇)-Cycloalkylen, Phenylen, Phenylen-(C₁-C₆)-alkyl, (C₁-C₆)-Alkylen-phenyl oder einen zweiwertigen Rest eines 5- oder 6-gliedrigen gesättigten oder ungesättigten Ringes, der 1 oder 2 Stickstoffatome enthalten und ein- oder zweifach durch (C₁-C₆)-Alkyl oder doppelt gebundenen Sauerstoff oder Schwefel substituiert sein kann, bedeutet;
B einen zweiwertigen Methylenrest oder Ethylenrest bedeutet, der durch einen Rest aus der Reihe (C₁-C₈)-Alkyl, (C₂-C₈)-Alkenyl, (C₂-C₈)-Alkinyl, (C₃-C₁₀)-Cycloalkyl, (C₃-C₁₀)-Cycloalkyl-(C₁-C₆)-alkyl, gegebenenfalls substituiertes (C₆-C₁₄)-Aryl, im Arylrest gegebenenfalls substituiertes (C₆-C₁₄)-Aryl-(C₁-C₆)-alkyl, gegebenenfalls substituiertes Heteroaryl und im Heteroarylrest gegebenenfalls substituiertes Heteroaryl-(C₁-C₆)-alkyl substituiert ist;
D für C(R²)(R³) steht;
E Tetrazolyl oder R¹⁰CO bedeutet;
R Wasserstoff oder (C₁-C₈)-Alkyl bedeutet;
R⁰ für Wasserstoff, (C₁-C₈)-Alkyl, (C₃-C₁₂)-Cycloalkyl, (C₃-C₁₂)-Cycloalkyl-(C₁-C₈)-alkyl, (C₆-C₁₂)-Bicycloalkyl, (C₆-C₁₂)-Bicycloalkyl-(C₁-C₈)-alkyl, (C₆-C₁₂)-Tricycloalkyl, (C₆-C₁₂)-Tricycloalkyl-(C₁-C₈)-alkyl, gegebenenfalls substituiertes (C₆-C₁₄)-Aryl, im Arylrest gegebenenfalls substituiertes (C₆-C₁₄)-Aryl-(C₁-C₈)-alkyl, gegebenenfalls substituiertes Heteroaryl, im Heteroarylrest gegebenenfalls substituiertes Heteroaryl-(C₁-C₈)-alkyl, CHO, (C₁-C₈)-Alkyl-CO, (C₃-C₁₂)-Cycloalkyl-CO, (C₃-C₁₂)-Cycloalkyl-(C₁-C₈)-alkyl-CO, (C₆-C₁₂)-Bicycloalkyl-CO, (C₆-C₁₂)-Bicycloalkyl-(C₁-C₈)-alkyl-CO, (C₆-C₁₂)-Tricycloalkyl-CO, (C₆-C₁₂)-Tricycloalkyl-(C₁-C₈)-alkyl-CO, gegebenenfalls substituiertes (C₆-C₁₄)-Aryl-CO, im Arylrest gegebenenfalls substituiertes (C₆-C₁₄)-Aryl-(C₁-C₈)-alkyl-CO, gegebenenfalis substituiertes Heteroaryl-CO, im Heteroarylrest gegebenenfalls substituiertes Heteroaryl-(C₁-C₈)-alkyl-CO, (C₁-C₈)-Alkyl-S(O)ₙ, (C₃-C₁₂)-Cycloalkyl-S(O)ₙ, (C₃-C₁₂)-Cycloalkyl-(C₁-C₈)-alkyl-S(O)ₙ, (C₆-C₁₂)-Bicycloalkyl-S(O)ₙ, (C₆-C₁₂)-Bicycloalkyl-(C₁-C₈)-alkyl-S(O)ₙ, (C₆-C₁₂)-Tricycloalkyl-S(O)ₙ, (C₆-C₁₂)-Tricycloalkyl-(C₁-C₈)-alkyl-S(O)ₙ, gegebenenfalls substituiertes (C₆-C₁₄)-Aryl-S(O)ₙ, im Arylrest gegebenenfalls substituiertes (C₆-C₁₄)-Aryl-(C₁-C₈)-alkyl-S(O)ₙ, gegebenenfalls substituiertes Heteroaryl-S(O)ₙ oder im Heteroarylrest gegebenenfalls substituiertes Heteroaryl-(C₁-C₈)-alkyl-S(O)ₙ steht, wobei n für 1 oder 2 steht;
R¹ für X-NH-C(=NH)-(CH₂)ₚ oder X¹-NH-(CH₂)ₚ steht, wobei p für 0, 1, 2 oder 3 steht;
X Wasserstoff, (C₁-C₆)-Alkyl, (C₁-C₆)-Alkylcarbonyl, (C₁-C₆)-Alkoxycarbonyl, (C₁-C₁₈)-Alkylcarbonyloxy-(C₁-C₆)-alkoxycarbonyl, gegebenenfalls substituiertes (C₆-C₁₄)-Arylcarbonyl, gegebenenfalls substituiertes (C₆-C₁₄)-Aryloxycarbonyl, (C₆-C₁₄)-Aryl-(C₁-C₆)-alkoxycarbonyl, das im Arylrest auch substituiert sein kann, Cyano, Hydroxy, (C₁-C₆)-Alkoxy, (C₆-C₁₄)-Aryl-(C₁-C₆)-alkoxy, das im Arylrest auch substituiert sein kann, oder Amino bedeutet;
X¹ eine der Bedeutungen von X hat oder R'-NH-C(=N-R") bedeutet, wobei R' und R" unabhängig voneinander die Bedeutungen von X haben;
R² Wasserstoff oder (C₁-C₈)-Alkyl bedeutet;
R³ Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)-Cycloalkyl, (C₂-C₈)-Alkenyl, (C₂-C₈)-Alkinyl, (C₂-C₈)-Alkenylcarbonyl, (C₂-C₈)-Alkinylcarbonyl, Pyridyl, R¹¹NH, CON(CH₃)R¹⁴, CONHR¹⁴, CON(CH₃)R¹⁵ oder CONHR¹⁵ bedeutet;
R⁵ gegebenenfalls substituiertes (C₆-C₁₄)-Aryl, im Arylrest gegebenenfalls substituiertes (C₆-C₁₄)-Aryl-(C₁-C₈)-alkyl, einen mono- oder bicyclischen 5- bis 12-gliedrigen heterocyclischen Ring, der aromatisch, teilhydriert oder vollständig hydriert sein kann und der ein, zwei oder drei gleiche oder verschiedene Heteroatome aus der Reihe Stickstoff, Sauerstoff und Schwefel enthalten kann, oder einen Rest R⁶CO- bedeutet, wobei der Aryl- und unabhängig davon der Heterocyclus-Rest ein- oder mehrfach durch gleiche oder verschiedene Reste aus der Reihe (C₁-C₈)-Alkyl, (C₁-C₈)-Alkoxy, Halogen, Nitro, Amino oder Trifluormethyl substituiert sein können;
R⁶ für einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert sein kann, sowie deren Ester und Amide bedeutet, wobei freie funktionelle Gruppen durch in der Peptidchemie übliche Schutzgruppen geschützt sein können;
R¹⁰ Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
R¹¹ für R¹²CO, gegebenenfalls substituiertes (C₆-C₁₄)-Aryl-S(O)₂ oder (C₁-C₁₈)-Alkyl-S(O)₂ steht;
R¹² Wasserstoff, (C₁-C₁₈)-Alkyl, (C₂-C₈)-Alkenyl, (C₂-C₈)-Alkinyl, gegebenenfalls substituiertes (C₆-C₁₄)-Aryl, (C₁-C₁₈)-Alkoxy, (C₆-C₁₄)-Aryl-(C₁-C₈)-alkoxy, das im Arylrest auch substituiert sein kann oder gegebenenfalls substituiertes (C₆-C₁₄)-Aryloxy bedeutet;
R¹³ Wasserstoff oder (C₁-C₄)-Alkyl bedeutet;
R¹⁴ (C₁-C₁₀)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-((C₁-C₁₈)-alkyl)-aminocarbonyl, (C₆-C₁₄)-Aryl-(C₁-C₈)-alkoxycarbonyl, das im Arylrest auch substituiert sein kann, (C₁-C₈)-Alkoxy, (C₁-C₈)-Alkoxycarbonyl, gegebenenfalls substituiertes (C₃-C₈)-Cycloalkyl, Tetrazolyl-(C₁-C₃)-alkyl, Trifluormethyl und R⁵ substituiert sein kann;
R¹⁵ für R¹⁶-(C₁-C₆)-alkyl oder für R¹⁶ steht;
R¹⁶ für einen 6- bis 24-gliedrigen bicyclischen oder tricyclischen Rest steht, der gesättigt oder teilweise ungesättigt ist und der auch ein bis vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann;
c und d für 1 stehen und f für 0 steht;
e und h unabhängig voneinander für 0 oder 1 stehen und g für 0 steht;
in allen ihren stereoisomeren Formen und Mischungen davon in allen Verhältnissen, sowie ihre physiologisch verträglichen Salze.

28. Verbindungen der Formel Id gemäß Anspruch 26 und/oder 27, worin der Rest, durch den die Gruppe B substituiert ist, ein (C₁-C₈)-Alkylrest ist, in allen ihren stereoisomeren Formen und Mischungen davon in allen Verhältnissen, sowie ihre physiologisch verträglichen Salze.

29. Verbindungen der Formel Id gemäß einem oder mehreren der Ansprüche 26 bis 28 und/oder ihre physiologisch verträglichen Salze zur Verwendung als Arzneimittel.

30. Pharmazeutisches Präparat, **dadurch gekennzeichnet, daß** es eine oder mehrere Verbindungen der Formel Id gemäß einem oder mehreren der Ansprüche 26 bis 28 und/oder ihre physiologisch verträglichen Salze neben pharmazeutisch einwandfreien Trägerstoffen und/oder Zusatzstoffen enthält.

31. Verbindungen der Formel le, 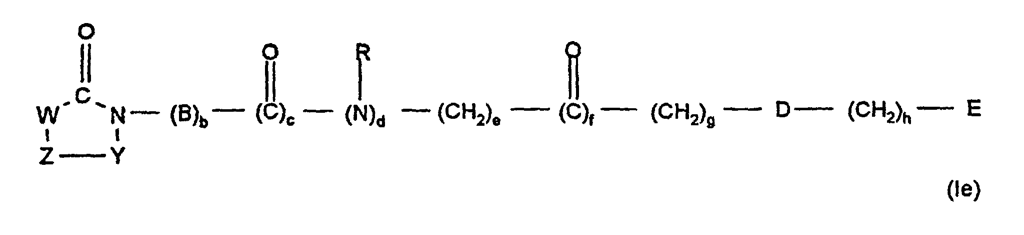 worin
W für R¹-A-C(R¹³) oder R¹-A-CH=C steht;
Y für eine Carbonyl-, Thiocarbonyl- oder Methylengruppe steht;
Z für N(R⁰), Sauerstoff, Schwefel oder eine Methylengruppe steht;
A einen zweiwertigen Rest aus der Reihe (C₁-C₆)-Alkylen, (C₃-C₇)-Cycloalkylen, Phenylen, Phenylen-(C₁-C₆)-alkyl, (C₁-C₆)-Alkylen-phenyl, Phenylen-(C₂-C₆)-alkenyl oder einen zweiwertigen Rest eines 5- oder 6-gliedrigen gesättigten oder ungesättigten Ringes, der 1 oder 2 Stickstoffatome enthalten und ein- oder zweifach durch (C₁-C₆)-Alkyl oder doppelt gebundenen Sauerstoff oder Schwefel substituiert sein kann, bedeutet;
B einen zweiwertigen Rest aus der Reihe (C₁-C₆)-Alkylen, (C₂-C₆)-Alkenylen, Phenylen, Phenylen-(C₁-C₃)-alkyl, (C₁-C₃)-Alkylen-phenyl bedeutet;
D für C(R²)(R³), N(R³) oder CH=C(R³) steht;
E Tetrazolyl, (R⁸O)₂P(O), HOS(O)₂, R⁹NHS(O)₂ oder R¹⁰CO bedeutet;
R Wasserstoff, (C₁-C₈)-Alkyl, (C₃-C₈)-Cycloalkyl, gegebenenfalls substituiertes (C₆-C₁₄)-Aryl oder im Arylrest gegebenenfalls substituiertes (C₆-C₁₄)-Aryl-(C₁-C₈)-alkyl bedeutet;
R⁰ für CHO, (C₁-C₈)-Alkyl-CO, (C₃-C₁₂)-Cycloalkyl-CO, (C₃-C₁₂)-Cycloalkyl-(C₁-C₈)-alkyl-CO, (C₆-C₁₂)-Bicycloalkyl-CO, (C₆-C₁₂)-Bicycloalkyl-(C₁-C₈)-alkyl-CO, (C₆-C₁₂)-Tricycloalkyl-CO, (C₆-C₁₂)-Tricycloalkyl-(C₁-C₈)-alkyl-CO, gegebenenfalls substituiertes (C₆-C₁₄)-Aryl-CO, im Arylrest gegebenenfalls substituiertes (C₆-C₁₄)-Aryl-(C₁-C₈)-alkyl-CO, gegebenenfalls substituiertes Heteroaryl-CO, im Heteroarylrest gegebenenfalls substituiertes Heteroaryl-(C₁-C₈)-alkyl-CO, (C₁-C₈)-Alkyl-S(O)ₙ, (C₃-C₁₂)-Cycloalkyl-S(O)ₙ, (C₃-C₁₂)-Cycloalkyl-(C₁-C₈)-alkyl-S(O)ₙ, (C₆-C₁₂)-Bicycloalkyl-S(O)ₙ, (C₆-C₁₂)-Bicycloalkyl-(C₁-C₈)-alkyl-S(O)ₙ, (C₆-C₁₂)-Tricycloalkyl-S(O)ₙ, (C₆-C₁₂)-Tricycloalkyl-(C₁-C₈)-alkyl-S(O)ₙ, gegebenenfalls substituiertes (C₆-C₁₄)-Aryl-S(O)ₙ, im Arylrest gegebenenfalls substituiertes (C₆-C₁₄)-Aryl-(C₁-C₈)-alkyl-S(O)ₙ, gegebenenfalls substituiertes Heteroaryl-S(O)ₙ oder im Heteroarylrest gegebenenfalls substituiertes Heteroaryl-(C₁-C₈)-alkyl-S(O)ₙ steht, wobei n für 1 oder 2 steht;
R¹ für X-NH-C(=NH)-(CH₂)ₚ oder X¹-NH-(CH₂)ₚ steht, wobei p für 0, 1, 2 oder 3 steht;
X Wasserstoff, (C₁-C₆)-Alkyl, (C₁-C₆)-Alkylcarbonyl, (C₁-C₆)-Alkoxycarbonyl, (C₁-C₁₈)-Alkylcarbonyloxy-(C₁-C₆)-alkoxycarbonyl, gegebenenfalls substituiertes (C₆-C₁₄)-Arylcarbonyl, gegebenenfalls substituiertes (C₆-C₁₄)-Aryloxycarbonyl, (C₆-C₁₄)-Aryl-(C₁-C₆)-alkoxycarbonyl, das im Arylrest auch substituiert sein kann, (R⁸O)₂P(O), Cyano, Hydroxy, (C₁-C₆)-Alkoxy, (C₆-C₁₄)-Aryl-(C₁-C₆)-alkoxy, das im Arylrest auch substituiert sein kann, oder Amino bedeutet;
X¹ eine der Bedeutungen von X hat oder R'-NH-C(=N-R") bedeutet, wobei R' und R" unabhängig voneinander die Bedeutungen von X haben;
R² Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl oder (C₃-C₈)-Cycloalkyl bedeutet;
R³ Wasserstoff, (C₁-C₈)-Alkyl, gegebenenfalls substituiertes (C₆-C₁₄)-Aryl, im Arylrest gegebenenfalls substituiertes (C₆-C₁₄)-Aryl-(C₁-C₈)-alkyl, (C₃-C₈)-Cycloalkyl, (C₂-C₈)-Alkenyl, (C₂-C₈)-Alkinyl, (C₂-C₈)-Alkenylcarbonyl, (C₂-C₈)-Alkinylcarbonyl, Pyridyl, R¹¹NH, R⁴CO, COOR⁴, CON(CH₃)R¹⁴, CONHR¹⁴, CSNHR¹⁴, COOR¹⁵, CON(CH₃)R¹⁵ oder CONHR¹⁵ bedeutet;
R⁴ Wasserstoff oder (C₁-C₂₈)-Alkyl bedeutet, das gegebenenfalls ein- oder mehrfach durch gleiche oder verschiedene Reste R^{4'} substituiert sein kann;
R^{4'} Hydroxy, Hydroxycarbonyl, Aminocarbonyl, Mono- oder Di-((C₁-C₁₈)-alkyl)-aminocarbonyl, Amino-(C₂-C₁₈)-alkylaminocarbonyl, Amino-(C₁-C₃)-alkylphenyl-(C₁-C₃)-alkylaminocarbonyl, (C₁-C₁₈)-Alkylcarbonylamino-(C₁-C₃)-alkylphenyl-(C₁-C₃)-alkylaminocarbonyl, (C₁-C₁₈)-Alkylcarbonylamino-(C₂-C₁₈)-alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)-Alkoxycarbonyl, gegebenenfalls substituiertes (C₃-C₈)-Cycloalkyl, Halogen, Nitro, Trifluormethyl oder den Rest R⁵ bedeutet;
R⁵ gegebenenfalls substituiertes (C₆-C₁₄)-Aryl, im Arylrest gegebenenfalls substituiertes (C₆-C₁₄)-Aryl-(C₁-C₈)-alkyl, einen mono- oder bicyclischen 5- bis 12-gliedrigen heterocyclischen Ring, der aromatisch, teilhydriert oder votlständig hydriert sein kann und der ein, zwei oder drei gleiche oder verschiedene Heteroatome aus der Reihe Stickstoff, Sauerstoff und Schwefel enthalten kann, einen Rest R⁶ oder einen Rest R⁶CO- bedeutet, wobei der Aryl- und unabhängig davon der Heterocyclus-Rest ein- oder mehrfach durch gleiche oder verschiedene Reste aus der Reihe (C₁-C₁₈)-Alkyl, (C₁-C₁₈)-Alkoxy, Halogen, Nitro, Amino oder Trifluormethyl substituiert sein können;
R⁶ für R⁷R⁸N, R⁷O oder R⁷S steht oder eine Aminosäureseitenkette, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, sowie deren Ester und Amide bedeutet, wobei anstelle freier funktioneller Gruppen gegebenenfalls Wasserstoff oder Hydroxymethyl stehen kann und/oder wobei freie funktionelle Gruppen durch in der Peptidchemie übliche Schutzgruppen geschützt sein können;
R⁷ Wasserstoff, (C₁-C₁₈)-Alkyl, (C₆-C₁₄)-Aryl-(C₁-C₈)-alkyl, (C₁-C₁₈)-Alkylcarbonyl, (C₁-C₁₈)-Alkoxycarbonyl, (C₆-C₁₄)-Arylcarbonyl, (C₆-C₁₄)-Aryl-(C₁-C₈)-alkylcarbonyl oder (C₆-C₁₄)-Aryl-(C₁-C₁₈)-alkyloxycarbonyl, wobei die Alkylgruppen gegebenenfalls durch eine Aminogruppe substituiert und/oder wobei die Arylreste ein- oder mehrfach, vorzugsweise einfach, durch gleiche oder verschiedene Reste aus der Reihe (C₁-C₈)-Alkyl, (C₁-C₈)-Alkoxy, Halogen, Nitro, Amino und Trifluormethyl substituiert sein können, einen natürlichen oder unnatürlichen Aminosäure-, Iminosäure-, gegebenenfalls N-(C₁-C₈)-alkylierten oder N-((C₆-C₁₄)-Aryl-(C₁-C₈)-alkylierten) Azaaminosäure- oder einen Dipeptid-Rest, der im Arylrest auch substituiert und/oder bei dem die Peptidbindung zu -NH-CH₂- reduziert sein kann, bedeutet;
R⁸ Wasserstoff, (C₁-C₁₈)-Alkyl, gegebenenfalls substituiertes (C₆-C₁₄)-Aryl oder (C₆-C₁₄)-Aryl-(C₁-C₈)-alkyl, das im Arylrest auch substituiert sein kann, bedeutet;
R⁹ Wasserstoff, Aminocarbonyl, (C₁-C₁₈)-Alkylaminocarbonyl, (C₃-C₈)-Cycloalkylaminocarbonyl, gegebenenfalls substituiertes (C₆-C₁₄)-Arylaminocarbonyl, (C₁-C₁₈)-Alkyl, gegebenenfalls substituiertes (C₆-C₁₄)-Aryl oder (C₃-C₈)-Cycloalkyl bedeutet;
R¹⁰ Hydroxy, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
R¹¹ Wasserstoff, (C₁-C₁₈)-Alkyl, R¹²CO, gegebenenfalls substituiertes (C₆-C₁₄)-Aryl-S(O)₂, (C₁-C₁₈)-Alkyl-S(O)₂, im Arylrest gegebenenfalls substituiertes (C₆-C₁₄)-Aryl-(C₁-C₈)-alkyl oder R⁹NHS(O)₂ bedeutet;
R¹² Wasserstoff, (C₁-C₁₈)-Alkyl, (C₂-C₈)-Alkenyl, (C₂-C₈)-Alkinyl, gegebenenfalls substituiertes (C₆-C₁₄)-Aryl, (C₁-C₁₈)-Alkoxy, (C₆-C₁₄)-Aryl-(C₁-C₈)-alkoxy, das im Arylrest auch substituiert sein kann, gegebenenfalls substituiertes (C₆-C₁₄)-Aryloxy, Amino oder Mono- oder Di-((C₁-C₁₈)-alkyl)-amino bedeutet;
R¹³ Wasserstoff, (C₁-C₆)-Alkyl, im Arylrest gegebenenfalls substituiertes (C₆-C₁₄)-Aryl-(C₁-C₈)-alkyl oder (C₃-C₈)-Cycloalkyl bedeutet;
R¹⁴ Wasserstoff oder (C₁-C₂₈)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-((C₁-C₁₈)-alkyl)-aminocarbonyl, Amino-(C₂-C₁₈)-alkylaminocarbonyl, Amino-(C₁-C₃)-alkylphenyl-(C₁-C₃)-alkylaminocarbonyl, (C₁-C₁₈)-Alkylcarbonylamino-(C₁-C₃)-alkylphenyl-(C₁-C₃)-alkylaminocarbonyl, (C₁-C₁₈)-Alkylcarbonyl-amino-(C₂-C₁₈)-alkylaminocarbonyl, (C₆-C₁₄)-Aryl-(C₁-C₈)-alkoxycarbonyl, das im Arylrest auch substituiert sein kann, Amino, Mercapto, (C₁-C₁₈)-Alkoxy, (C₁-C₁₈)-Alkoxycarbonyl, gegebenenfalls substituiertes (C₃-C₈)-Cycloalkyl, HOS(O)₂-(C₁-C₃)-alkyl, R⁹NHS(O)₂-(C₁-C₃)-alkyl, (R⁸O)₂P(O)-(C₁-C₃)-alkyl, Tetrazolyl-(C₁-C₃)-alkyl, Halogen, Nitro, Trifluormethyl und R⁵ substituiert sein kann;
R¹⁵ für R¹⁶-(C₁-C₆)-alkyl oder für R¹⁶ steht;
R¹⁶ für einen 6- bis 24-gliedrigen bicyclischen oder tricyclischen Rest steht, der gesättigt oder teilweise ungesättigt ist und der auch ein bis vier gleiche oder verschiedene Heteroatome aus der Reihe Stickstoff, Sauerstoff und Schwefel enthalten kann und der auch durch einen oder mehrere gleiche oder verschiedene Substituenten aus der Reihe (C₁-C₄)-Alkyl und Oxo substituiert sein kann;
b, c, d und f unabhängig voneinander für 0 oder 1 stehen, aber nicht alle gleichzeitig 0 sein können;
e, g und h unabhängig voneinander für 0, 1, 2, 3, 4, 5 oder 6 stehen;
in allen ihren stereoisomeren Formen und Mischungen davon in allen Verhältnissen, sowie ihre physiologisch verträglichen Salze.

32. Verbindungen der Formel le gemäß Anspruch 31 und/oder ihre physiologisch verträglichen Salze zur Verwendung als Arzneimittel.

33. Pharmazeutisches Präparat, **dadurch gekennzeichnet, daß** es eine oder mehrere Verbindungen der Formel le gemäß Anspruch 31 und/oder ihre physiologisch verträglichen Salze neben pharmazeutisch einwandfreien Trägerstoffen und/oder Zusatzstoffen enthält.

## Claims

1. The use of compounds of the formula I 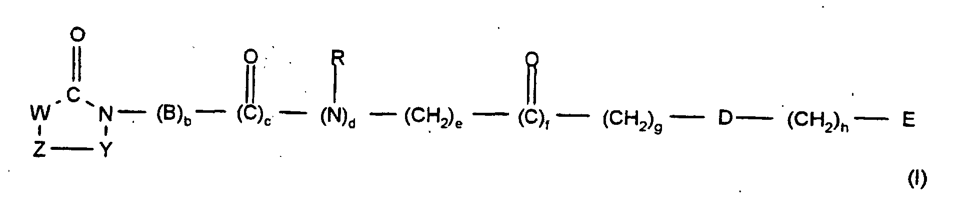 in which
W is R¹-A-C (R¹³) or R¹-A-CH=C;
Y is a carbonyl, thiocarbonyl or methylene group;
Z is N(R⁰), oxygen, sulfur or a methylene group;
A is a bivalent radical from the group consisting of (C₁-C₆)-alkylene, (C₃-C₇)-cycloalkylene, phenylene, phenylene-(C₁-C₆)-alkyl, (C₁-C₆)-alkylenephenyl, phenylene-(C₂-C₆)-alkenyl or a bivalent radical of a 5- or 6-membered saturated or unsaturated ring which can contain 1 or 2 nitrogen atoms and can be mono- or disubstituted by (C₁-C₆)-alkyl or doubly bonded oxygen or sulfur;
B is a bivalent radical from the group consisting of (C₁-C₆)-alkylene, (C₂-C₆)-alkenylene, phenylene, phenylene-(C₁-C₃)-alkyl, (C₁-C₃)-alkylenephenyl, where the bivalent (C₁-C₆)-alkylene radical can be unsubstituted or substituted by a radical from the group consisting of (C₁-C₈)-alkyl, (C₂-C₈)-alkenyl (C₂-C₈)-alkynyl, (C₃-C₁₀)-cycloalkyl, (C₃-C₁₀)-cycloalkyl-(C₁-C₆)-alkyl, optionally substituted (C₆-C₁₄)-aryl, (C₆-C₁₄)-aryl-(C₁-C₆)-alkyl optionally substituted in the aryl radical, optionally substituted heteroaryl and heteroaryl-(C₁-C₆)-alkyl optionally substituted in the heteroaryl radical;
D is C (R²) (R³) , N(R³) or CH=C (R³) ;
E is tetrazolyl, (R⁸O) ₂P (O) , HOS (O) ₂, R⁹NHS (O) ₂ or R¹⁰CO;
R is hydrogen, (C₁-C₈)-alkyl, (C₃-C₈)-cycloalkyl, optionally substituted (C₆-C₁₄)-aryl or (C₆-C₁₄)-aryl-(C₁-C₈)-alkyl optionally substituted in the aryl radical;
R⁰ is hydrogen, (C₁-C₈)-alkyl, (C₃-C₁₂)-cycloalkyl, (C₃-C₁₂)-cycloalkyl-(C₁-C₈)-alkyl, (C₆-C₁₂)-bicycloalkyl, (C₆-C₁₂)-bicycloalkyl-(C₁-C₈)-alkyl, (C₆-C₁₂)-tricycloalkyl, (C₆-C₁₂)-tricycloalkyl-(C₁-C₈)-alkyl, optionally substituted (C₆-C₁₄)-aryl, (C₆-C₁₄)-aryl-(C₁-C₈)-alkyl optionally substituted in the aryl radical, optionally substituted heteroaryl, heteroaryl-(C₁-C₈)-alkyl optionally substituted in the heteroaryl radical, CHO, (C₁-C₈)-alkyl-CO, (C₃-C₁₂)-cycloalkyl-CO, (C₃-C₁₂)-cycloalkyl-(C₁-C₈)-alkyl-CO, (C₆-C₁₂)-bicycloalkyl-CO, (C₆-C₁₂)-bicycloalkyl-(C₁-C₈)-alkyl-CO, (C₆-C₁₂)-tricycloalkyl-CO, (C₆-C₁₂)-tricycloalkyl-(C₁-C₈)-alkyl-CO; optionally substituted (C₆-C₁₄)-aryl-CO, (C₆-C₁₄)-aryl-(C₁-C₈)-alkyl-CO optionally substituted in the aryl radical, optionally substituted heteroaryl-CO, heteroaryl-(C₁-C₈)-alkyl-CO optionally substituted in the heteroaryl radical, (C₁-C₈)-alkyl-S (O)ₙ, (C₃-C₁₂)-cycloalkyl-S (O) ₙ, (C₃-C₁₂)-cycloalkyl-(C₁-C₈)-alkyl-S (O) ₙ, (C₆-C₁₂)-bicycloalkyl-S (O)ₙ, (C₆-C₁₂)-bicycloalkyl-(C₁-C₈)-alkyl-S (O) ₙ, (C₆-C₁₂)-tricycloalkyl-S (O)ₙ, (C₆-C₁₂)-tricycloalkyl-(C₁-C₈)-alkyl-S (O) ₙ, optionally substituted (C₆-C₁₄)-aryl-S (O) ₙ, (C₆-C₁₄)-aryl-(C₁-C₈)-alkyl-S (O) ₙ, optionally substituted in the aryl radical, optionally substituted heteroaryl-S (O)ₙ or heteroaryl-(C₁-C₈)-alkyl-S(O)ₙ, optionally substituted in the heteroaryl radical, where n is 1 or 2;
R¹ is X-NH-C (=NH)-(CH₂) ₚ or X¹-NH-(CH₂) ₚ, where p is 0, 1, 2 or 3;
X is hydrogen, (C₁-C₆)-alkyl, (C₁-C₆)-alkyl carbonyl, (C₁-C₆)-alkoxycarbonyl, (C₁-C₁₈)-alkylcarbonyloxy-(C₁-C₆)-alkoxycarbonyl, optionally substituted (C₆-C₁₄)-arylcarbonyl, optionally substituted (C₆-C₁₄)-aryloxycarbonyl, (C₆-C₁₄)-aryl-(C₁-C₆)-alkoxycarbonyl which can also be substituted in the aryl radical, (R⁸O) ₂P (O) , cyano, hydroxyl, (C₁-C₆)-alkoxy, (C₆-C₁₄)-aryl-(C₁-C₆)-alkoxy which can also be substituted in the aryl radical, or amino;
X¹ has one of the meanings of X or is R'-NH-C(=N-R''), where R' and R" independently of one another have the meanings of X;
R² is hydrogen, (C₁-C₈)-alkyl, optionally substituted (C₆-C₁₄)-aryl, (C₆-C₁₄)-aryl-(C₁-C₈)-alkyl optionally substituted in the aryl radical or (C₃-C₈)-cycloalkyl;
R³ is hydrogen, (C₁-C₈)-alkyl, optionally substituted (C₆-C₁₄)-aryl, (C₆-C₁₄)-aryl-(C₁-C₈)-alkyl optionally substituted in the aryl radical, (C₃-C₈)-cycloalkyl, (C₂-C₈)-alkenyl, (C₂-C₈)-alkynyl, (C₂-C₈)-alkenylcarbonyl, (C₂-C₈)-alkynylcarbonyl, pyridyl, R¹¹NH, R⁴CO, COOR⁴, CON (CH₃) R¹⁴, CONHR¹⁴, CSNHR¹⁴_{,} COOR¹⁵, CON (CH₃) R¹⁵ or CONHR¹⁵;
R⁴ is hydrogen or (C₁-C₂₈)-alkyl which can optionally be mono- or polysubstituted by identical or different radicals R^{4'};
R^{4'} is hydroxyl, hydroxycarbonyl, aminocarbonyl, mono- or di-((C₁-C₁₈)-alkyl) aminocarbonyl, amino-(C₂-C₁₈)-alkylaminocarbonyl, amino-(C₁-C₃)-alkylphenyl-(C₁-C₃)-alkylaminocarbonyl, (C₁-C₁₈)-alkyl carbonyl amino-(C₁-C₃)-alkylphenyl-(C₁-C₃)-alkylaminocarbonyl, (C₁-C₁₈)-alkylcarbonylamino-(C₂-C₁₈)-alkylaminocarbonyl, (C₆-C₁₄)-aryl-(C₁-C₈)-alkoxycarbonyl which can also be substituted in the aryl radical, amino, mercapto, (C₁-C₁₈)-alkoxy, (C₁-C₁₈)-alkoxycarbonyl, optionally substituted (C₃-C₈)-cycloalkyl, halogen, nitro, trifluoromethyl or the radical R⁵;
R⁵ is optionally substituted (C₆-C₁₄)-aryl, (C₆-C₁₄)-aryl-(C₁-C₈)-alkyl optionally substituted in the aryl radical, a mono- or bicyclic 5- to 12-membered heterocyclic ring which can be aromatic, partially hydrogenated or completely hydrogenated and which can contain one, two or three- identical or different from the group consisting of nitrogen, oxygen and sulfur, a radical R⁶ or a radical R⁶CO-, where the aryl and, independently thereof, the heterocyclic radical can be mono- or polysubstituted by identical or different radicals from the group consisting of (C₁-C₁₈)-alkyl, (C₁-C₁₈)-alkoxy, halogen, nitro, amino and trifluoromethyl;
R⁶ is R⁷R⁸N, R⁷O or R⁷S or an amino acid side chain, a natural or unnatural amino acid, imino acid, optionally N-(C₁-C₈)-alkylated or N-((C₆-C₁₄)-aryl-(C₁-C₈)-alkylated) azaamino acid or a dipeptide radical which can also be substituted in the aryl radical and/or in which the peptide bond can be reduced to -NH-CH₂-, and their esters and amides, where instead of free functional groups there can optionally be hydrogen or hydroxymethyl and/or where free functional groups can be protected by protective groups customary in peptide chemistry;
R⁷ is hydrogen, (C₁-C₁₈)-alkyl, (C₆-C₁₄)-aryl-(C₁-C₈)-alkyl, (C₁-C₁₈)-alkylcarbonyl, (C₁-C₁₈)-alkoxycarbonyl, (C₆-C₁₄)-arylcarbonyl, (C₆-C₁₄)-aryl-(C₁-C₈)-alkyl carbonyl or (C₆-C₁₄)-aryl-(C₁-C₁₈)-alkyloxycarbonyl, where the alkyl groups can optionally be substituted by an amino group and/or where the aryl radicals can be mono- or polysubstituted, preferably monosubstituted, by identical or different radicals from the group consisting of (C₁-C₈)-alkyl, (C₁-C₈)-alkoxy, halogen, nitro, amino and trifluoromethyl, a natural or unnatural amino acid, imino acid, optionally N-(C₁-C₈)-alkylated or N-(C₆-C₁₄)-aryl-(C₁-C₈)-alkylated) azaamino acid or a dipeptide radical which can also be substituted in the aryl radical and/or in which the peptide bond can be reduced to -NH-CH₂-;
R⁸ is hydrogen, (C₁-C₁₈)-alkyl, optionally substituted (C₆-C₁₄)-aryl or (C₆-C₁₄)-aryl-(C₁-C₈)-alkyl which can also be substituted in the aryl radical;
R⁹ is hydrogen, aminocarbonyl, (C₁-C₁₈)-alkylamino-carbonyl, (C₃-C₈)-cycloalkylaminocarbonyl, optionally substituted (C₆-C₁₄)-arylamino-carbonyl, (C₁-C₁₈)-alkyl, optionally substituted (C₆-C₁₄)-aryl or (C₃-C₈)-cycloalkyl;
R¹⁰ is hydroxyl, (C₁-C₁₈)-alkoxy, (C₆-C₁₄)-aryl-(C₁-C₈)-alkoxy which can also be substituted in the aryl radical, optionally substituted (C₆-C₁₄)-aryloxy, amino or mono- or di-((C₁-C₁₈)-alkyl) amino;
R¹¹ is hydrogen, (C₁-C₁₈)-alkyl, R¹²CO, optionally substituted (C₆-C₁₄)-aryl-S (O) ₂, (C₁-C₁₈)-alkyl-S (O)₂, (C₆-C₁₄)-aryl-(C₁-C₈)-alkyl optionally substituted in the aryl radical or R⁹NHS(O)₂;
R¹² is hydrogen, (C₁-C₁₈)-alkyl, (C₂-C₈)-alkenyl, (C₂-C₈)-alkynyl, optionally substituted (C₆-C₁₄)-aryl, (C₁-C₁₈)-alkoxy, (C₆-C₁₄)-aryl-(C₁-C₈)-alkoxy which can also be substituted in the aryl radical, optionally substituted (C₆-C₁₄)-aryloxy; amino or mono- or di-((C₁-C₁₈)-alkyl) amino;
R¹³ is hydrogen, (C₁-C₆)-alkyl, (C₆-C₁₄)-aryl-(C₁-C₈)-alkyl optionally substituted in the aryl radical or (C₃-C₈)-cycloalkyl;
R¹⁴ is hydrogen or (C₁-C₂₈)-alkyl which can optionally be mono- or polysubstituted by identical or different radicals from the group consisting of hydroxyl, hydroxycarbonyl, aminocarbonyl, mono- or di-((C₁-C₁₈)-alkyl) aminocarbonyl, amino-(C₂-C₁₈)-alkylaminocarbonyl, amino-(C₁-C₃)-alkylphenyl-(C₁-C₃)-alkylaminocarbonyl , (C₁-C₁₈)-alkylcarbonylamino-(C₁-C₃)-alkylphenyl-(C₁-C₃)-alkylaminocarbonyl, (C₁-C₁₈)-alkylcarbonyl-amino-(C₂-C₁₈)-alkylaminocarbonyl, (C₆-C₁₄)-aryl-(C₁-C₈)-alkoxycarbonyl which can also be substituted in the aryl radical, amino, mercapto, (C₁-C₁₈)-alkoxy, (C₁-C₁₈)-alkoxycarbonyl, optionally substituted (C₃-C₈)-cycloalkyl, HOS (O) ₂-(C₁-C₃)-alkyl, R⁹NHS (O) ₂-(C₁-C₃)-alkyl, (R⁸O)₂P (O)-(C₁-C₃)-alkyl, tetrazolyl-(C₁-C₃)-alkyl, halogen, nitro, trifluoromethyl and R⁵;
R¹⁵ is R¹⁶-(C₁-C₆)-alkyl or R¹⁶;
R¹⁶ is a 6- to 24-membered bicyclic or tricyclic radical which is saturated or partially unsaturated and which can also contain one to four identical or different heteroatoms from the group consisting of nitrogen, oxygen and sulfur and which can also be substituted by one or more identical or different substituents from the group consisting of (C₁-C₄)-alkyl and oxo;
b, c, d and f independently of one another are 0 or 1, but cannot all simultaneously be 0;
e, g and h independently of one another are 0, 1, 2, 3, 4, 5 or 6;
in all their stereoisomeric forms and mixtures thereof in all ratios, and their physiologically tolerable salts for the production of pharmaceuticals for inhibition of the adhesion and/or migration of leucocytes or for inhibition of the VLA-4 receptor.

2. The use of compounds of the formula I as claimed in claim 1 in all their stereoisomeric forms and mixtures thereof in all ratios, and of their physiologically tolerable salts, where in the formula I
W is R¹-A-C (R¹³) or R¹-A-CH=C;
Y is a carbonyl, thiocarbonyl or methylene group;
Z is N(R⁰), oxygen, sulfur or a methylene group;
A is a bivalent radical from the group consisting of (C₁-C₆)-alkylene, (C₃-C₇)-cycloalkylene, phenylene, phenylene-(C₁-C₆)-alkyl, (C₁-C₆)-alkylenephenyl, phenylene-(C₂-C₆)-alkenyl or a bivalent radical of a 5- or 6-membered saturated or unsaturated ring which can contain 1 or 2 nitrogen atoms and can be mono- or disubstituted by (C₁-C₆)-alkyl or doubly bonded oxygen or sulfur;
B is a bivalent radical from the group consisting of (C₁-C₆)-alkylene, (C₂-C₆)-alkenylene, phenylene, phenylene-(C₁-C₃)-alkyl, (C₁-C₃)-alkylene-phenyl;
D is C (R²) (R³) , N(R³) or CH=C(R³);
E is tetrazolyl, (R⁸O) ₂P (O) , HOS(O)₂, R⁹NHS (O) ₂ or R¹⁰CO;
R and R⁰ independently of one another are hydrogen, (C₁-C₈)-alkyl, (C₃-C₈)-cycloalkyl, optionally substituted (C₆-C₁₄)-aryl or (C₆-C₁₄)-aryl-(C₁-C₈)-alkyl optionally substituted in the aryl radical;
R¹ is X-NH-C (=NH)-(CH₂)ₚ or X¹-NH-(CH₂) ₚ, where p is 0, 1, 2 or 3;
X is hydrogen, (C₁-C₆)-alkyl, (C₁-C₆)-alkylcarbonyl, (C₁-C₆)-alkoxycarbonyl, (C₁-C₁₈)-alkylcarbonyloxy-(C₁-C₆)-alkoxycarbonyl, optionally substituted (C₆-C₁₄)-arylcarbonyl, optionally substituted (C₆-C₁₄)-aryloxycarbonyl, (C₆-C₁₄)-aryl-(C₁-C₆)-alkoxycarbonyl which can also be substituted in the aryl radical, (R⁸O)₂P(O), cyano, hydroxyl, (C₁-C₆)-alkoxy, (C₆-C₁₄)-aryl-(C₁-C₆)-alkoxy which can also be substituted in the aryl radical-, or amino;
X³ has one of the meanings of X or is R'-NH-C(=N-R ") where R' and R " independently of one another have the meanings of X;
R³ is hydrogen, (C₁-C₈)- alkyl, optionally substituted (C₆-C₁₄)-aryl, (C₆-C₁₄)-aryl-(C₁-C₈)-alkyl optionally substituted in the aryl radical or (C₃-C₈)-cycloalkyl;
R³ is hydrogen, (C₁-C₈)-alkyl, optionally substituted (C₆-C₁₄)-aryl, (C₆-C₁₄)-aryl-(C₁-C₈)-alkyl, optionally substituted in the aryl radical, (C₃-C₈)-cycloalkyl, (C₂-C₈)-alkenyl, (C₂-C₈)-alkynyl, (C₂-C₈)-alkenylcarbonyl, (C₂-C₈)-alkynylcarbonyl, pyridyl, R¹¹NH, R⁴CO, COOR⁴, CON (CH₃) R¹⁴, CONHR¹⁴, CSNHR¹⁴, COOR¹⁵, CON (CH₃) R¹⁵ or CONHR¹⁵;
R⁴ is hydrogen or (C₁-C₂₈)-alkyl which can optionally be mono- or polysubstituted by identical or different radicals R^{4'};
R^{4'} is hydroxyl, hydroxycarbonyl, aminocarbonyl, mono- or di-((C₁-C₁₈)-alkyl) aminocarbonyl, amino-(C₂-C₁₈)-alkylaminocarbonyl, amino-(C₁-C₃)-alkylphenyl-(C₁-C₃)-alkylaminocarbonyl, (C₁-C₁₈)-alkylcarbonylamino-(C₁-C₃)-alkylphenyl-(C₁-C₃)-alkylaminocarbonyl, (C₁-C₁₈)-alkylcarbonylamino-(C₂-C₁₈)-alkylaminocarbonyl, (C₆-C₁₄)-aryl-(C₁-C₈)-alkoxycarbonyl which can also be substituted in the aryl radical, amino, mercapto, (C₁-C₁₈)-alkoxy, (C₁-C₁₈)-alkoxycarbonyl, optionally substituted (C₃-C₈)-cycloalkyl, halogen, nitro, trifluoromethyl or the radical R⁵;
R⁵ is optionally substituted (C₆-C₁₄)-aryl, (C₆-C₁₄)-aryl-(C₁-C₈)-alkyl optionally substituted in the aryl radical, a mono- or bicyclic 5- to 12-membered heterocyclic ring which can be aromatic, partially hydrogenated or completely hydrogenated and which can contain one, two or three identical or different heteroatoms from the group consisting of nitrogen, oxygen and sulfur, a radical R⁶ or a radical R⁶CO-, where the aryl and, independently thereof, the heterocyclic radical can be mono- or polysubstituted by identical or different radicals from the group consisting of (C₁-C₁₈)-alkyl, (C₁-C₁₈)-alkoxy, halogen, nitro, amino or trifluoromethyl;
R⁶ is R⁷R⁸N, R⁷O or R⁷S or an amino acid side chain, a natural or unnatural amino acid, imino acid, optionally N-(C₁-C₈)-alkylated or N-((C₆-C₁₄)-aryl-(C₁-C₈)-alkylated) azaamino acid or a dipeptide radical which can also be substituted in the aryl radical and/or in which the peptide bond can be reduced to -NH-CH₂-, and their esters and amides, where instead of free functional groups there can optionally be hydrogen or hydroxymethyl and/or where free functional groups can be protected by protective groups customary in peptide chemistry;
R⁷ is hydrogen, (C₁-C₁₈)-alkyl, (C₆-C₁₄)-aryl-(C₁-C₈)-alkyl, (C₁-C₁₈)-alkylcarbonyl, (C₁-C₁₈)-alkoxycarbonyl, (C₆-C₁₄)-arylcarbonyl, (C₆-C₁₄)aryl-(C₁-C₈)-alkyl carbonyl or (C₆-C₁₄)-aryl-(C₁-C₁₈)-alkyloxycarbonyl, where the alkyl groups can optionally be substituted by an amino group and/or where the aryl radicals can be mono- or polysubstituted, preferably monosubstituted, by identical or different radicals from the group consisting of (C₁-C₈)-alkyl, (C₁-C₈)-alkoxy, halogen, nitro, amino and trifluoromethyl, a natural or unnatural amino acid, imino acid, optionallyN-(C₁-C₈)-alkylated or N-((C₆-C₁₄)-aryl-(C₁-C₈)-alkylated) azaamino acid or a dipeptide radical which can also be substituted in the aryl radical and/or in which the peptide bond can be reduced to -NH-CH₂-;
R⁸ is hydrogen, (C₁-C₁₈)-alkyl, optionally substituted (C₆-C₁₄)-aryl or (C₆-C₁₄)-aryl-(C₁-C₈)-alkyl which can also be substituted in the aryl radical;
R⁹ is hydrogen, aminocarbonyl, (C₁-C₁₈)-alkyl-aminocarbonyl, (C₃-C₈)-cycloalkylaminocarbonyl, optionally substituted (C₆-C₁₄)-arylamino-carbonyl, (C₁-C₁₈)-alkyl, optionally substituted (C₆-C₁₄)-aryl or (C₃-C₈)-cycloalkyl;
R¹⁰ is hydroxyl, (C₁-C₁₈)-alkoxy, (C₆-C₁₄)-aryl-(C₁-C₈)-alkoxy which can also be substituted in the aryl radical, optionally substituted (C₆-C₁₄)-aryloxy, amino or mono- or di-((C₁-C₁₈)-alkyl)amino;
R¹¹ is hydrogen, (C₁-C₁₈)-alkyl, R¹²CO, optionally substituted (C₆-C₁₄)-aryl-S (O) ₂, (C₁-C₁₈)-alkyl-S (O) ₂, (C₆-C₁₄)-aryl-(C₁-C₈)-alkyl optionally substituted in the aryl radical or R⁹NHS(O)₂;
R¹² is hydrogen, (C₁-C₁₈)-alkyl, (C₂-C₈)-alkenyl, (C₂-C₈)-alkynyl, optionally substituted (C₆-C₁₄)-aryl, (C₁-C₁₈)-alkoxy, (C₆-C₁₄)-aryl-(C₁-C₈)-alkoxy which can also be substituted in the aryl radical, optionally substituted (C₆-C₁₄)-aryloxy, amino or mono- or di-((C₁-C₁₈)-alkyl)amino;
R¹³ is hydrogen, (C₁-C₆)-alkyl, (C₆-C₁₄)-aryl-(C₁-C₈)-alkyl optionally substituted in the aryl radical or (C₃-C₈)-cycloalkyl;
R¹⁴ is hydrogen or (C₁-C₂₈)-alkyl which can optionally be mono- or polysubstituted by identical or different radicals-from the group consisting of hydroxyl, hydroxycarbonyl, aminocarbonyl, mono- or di-((C₁-C₁₈)-alkyl)-aminocarbonyl, amino-(C₂-C₁₈)-alkylamino-carbonyl, amino-(C₁-C₃)-alkylphenyl-(C₁-C₃)-alkylaminocarbonyl, (C₁-C₁₈)-alkylcarbonylamino-(C₁-C₃)-alkylphenyl-(C₁-C₃)-alkylaminocarbonyl, (C₁-C₁₈)-alkyl carbonyl amino-(C₂-C₁₈)-alkyl-aminocarbonyl, (C₆-C₁₄)-aryl-(C₁-C₈)-alkoxycarbonyl which can also be substiuted in the aryl radical, amino, mercapto, (C₁-C₁₈)-alkoxy, (C₁-C₁₈)-alkoxycarbonyl, optionally substituted (C₃-C₈)-cycloalkyl, HOS (O) ₂-(C₁-C₃)-alkyl, R⁹NHS (O) ₂-(C₁-C₃)-alkyl, (R⁸O) ₂P (O)-(C₁-C₃)-alkyl, tetrazolyl-(C₁-C₃)-alkyl, halogen, nitro, trifluoromethyl and R⁵;
R¹⁵ is R¹⁶-(C₁-C₆)-alkyl or R¹⁶;
R¹⁶ is a 6- to 24-membered bicyclic or tricyclic radical which is saturated or partially unsaturated and which can also contain one to four identical or different heteroatoms from the group consisting of nitrogen, oxygen and sulfur and which can also be substituted by one or more identical or different substituents from the group consisting of (C₁-C₄)-alkyl and oxo;
b, c, d and f independently of one another are 0 or 1, but cannot all simultaneously be 0;
e, g and h independently of one another are 0, 1, 2, 3, 4, 5 or 6.

3. The use of compounds of the formula I as claimed in claim 1 in all their stereoisomeric forms and mixtures thereof in all ratios, and of their physiologically tolerable salts, where in the formula I R⁰ is (C₁-C₈)-alkyl, (C₃-C₈)-cycloalkyl, (C₃-C₈)-cycloalkyl-(C₁-C₄)-alkyl, optionally substituted (C₆-C₁₄)-aryl or (C₆-C₁₄)-aryl-(C₁-C₈)-alkyl optionally substituted in the aryl radical, preferably biphenylylmethyl, naphthylmethyl or benzyl, each of which is unsubstituted or monosubstituted or polysubstituted in the aryl radical.

4. The use of compounds of the formula I as claimed in claim 1 and/or 3 in all their stereoisomeric forms and mixtures thereof in all ratios, and of their physiologically tolerable salts, where in the formula I simultaneously
W is R¹-A-CH=C and therein A is a phenylene radical or W is R¹-A-C (R¹³) and therein A is a bivalent radical from the group consisting of methylene, ethylene, trimethylene, tetramethylene, pentamethylene, cyclohexylene, phenylene, phenylenemethyl;
B is a bivalent radical from the group consisting of methylene, ethylene, trimethylene, tetramethylene, vinylene, phenylene, or is substituted methylene or ethylene;
E is R¹⁰CO;
R is hydrogen, (C₁-C₆)-alkyl or benzyl;
R⁰ is (C₁-C₈)-alkyl, (C₃-C₈)-cycloalkyl, optionally substituted (C₆-C₁₄)-aryl or (C₆-C₁₄)-aryl-(C₁-C₈)-alkyl optionally substituted in the aryl radical;
R¹ is X-NH-C(=NH), X-NH-C (=NX)-NH or X-NH-CH₂ ;
X is hydrogen, (C₁-C₆)-alkyl carbonyl, (C₁-C₆)-alkoxycarbonyl, (C₁-C₈)-alkylcarbonyloxy-(C₁-C₆)-alkoxycarbonyl, (C₆-C₁₄)-aryl-(C₁-C₆)-alkoxy-carbonyl or hydroxyl;
R² is hydrogen or (C₁-C₈)-alkyl;
R³ is (C₁-C₈)-alkyl, optionally substituted (C₆-C₁₄)-aryl, (C₆-C₁₄)-aryl-(C₁-C₈)-alkyl, (C₃-C₈)-cycloalkyl, (C₂-C₈)-alkenyl, (C₂-C₈)-alkynyl, pyridyl, R¹¹NH, R⁴CO, COOR⁴, CONHR¹⁴, CSNHR¹⁴, COOR¹⁵ and CONHR¹⁵;
and e, g and h independently of one another are the numbers 0, 1, 2 or 3.

5. The use of compounds of the formula I as claimed in one or more of claims 1 to 4 in all their stereoisomeric forms and mixtures thereof in all ratios, and of their physiologically tolerable salts, where in the formula I W is R¹-A-C (R¹³) and R¹³ is (C₁-C₆)-alkyl, (C₆-C₁₄)-aryl-(C₁-C₈)-alkyl optionally substituted in the aryl radical or (C₃-C₈)-cycloalkyl .

6. The use of compounds of the formula I as claimed in one or more of claims 1 to 5 in all their stereoisomeric forms and mixtures thereof in all ratios, and of their physiologically tolerable salts, where in the formula I R³ is optionally substituted (C₆-C₁₄)-aryl, COOR⁴, R¹¹NH or CONHR¹⁴, where -NHR¹⁴ is the radical of an α-amino acid, its ω-amino-(C₂-C₈)-alkylamide, its (C₁-C₈)-alkyl ester or its (C₆-C₁₄)-aryl-(C₁-C₄)-alkyl ester, preferably the radical of the α-amino acids valine, lysine, phenylglycine, phenylalanine or tryptophan or their (C₁-C₈)-alkyl esters or (C₆-C₁₄)-aryl-(C₁-C₄)-alkyl esters.

7. The use of compounds of the formula I as claimed in one or more of claims 1 and 3 to 6 in all their stereoisomeric forms and mixtures thereof in all ratios, and of their physiologically tolerable salts, where in the formula I simultaneously
W is R¹-A-C (R¹³)
Y is a carbonyl group;
Z is N (R⁰);
A is ethylene, trimethylene, tetramethylene, pentamethylene, cyclohexylene, phenylene or phenylenemethyl;
B is an unsubstituted or substituted methylene radical;
D is C (R²) (R³);
E is R¹⁰CO;
R is hydrogen or (C₁-C₄)-alkyl, in particular hydrogen, methyl or ethyl;
R⁰ is (C₁-C₈)-alkyl, (C₃-C₈)-cycloalkyl, optionally substituted (C₆-C₁₄)-aryl or (C₆-C₁₄)-aryl-(C₁-C₈)-alkyl optionally substituted in the aryl radical;
R¹ is H₂N-C (=NH) , H₂N-C (=NH)-NH or H₂N-CH₂;
R² is hydrogen;
R³ is the radical CONHR¹⁴;
R¹⁰ is hydroxyl or (C₁-C₈)-alkoxy, preferably (C₁-C₄)-alkoxy;
R¹³ is (C₁-C₆)-alkyl, (C₃-C₇)-cycloalkyl or benzyl, in particular methyl;
R¹⁴ is methyl which is substituted by hydroxycarbonyl and a radical from the group consisting of (C₁-C₄)-alkyl, phenyl and benzyl, or is methyl which is substituted by (C₁-C₈)-alkoxycarbonyl, preferably (C₁-C₄)-alkoxycarbonyl, and a radical from the group consisting of (C₁-C₄)-alkyl, phenyl and benzyl;
b, c and d are 1 and e, f and g are 0; h is 1 or 2; preferably 1.

8. The use of compounds of the formula I as claimed in one or more of claims 1, 3 and 4 in all their stereoisomeric forms and mixtures thereof in all ratios, and of their physiologically tolerable salts, where in the formula I simultaneously W is R¹-A-CH=C and therein A is a phenylene radical or W is R¹-A-C (R¹³) and therein A is a bivalent radical from the group consisting of methylene, ethylene, trimethylene, tetramethylene, pentamethylene, cyclohexylene, phenylene, phenylenemethyl;
B is a bivalent radical from the group consisting of methylene, ethylene, trimethylene, tetramethylene, vinylene, phenylene or is substituted methylene or ethylene;
E is R¹⁰ CO;
R is hydrogen or (C₁-C₆)-alkyl;
R⁰ is (C₁-C₈)-alkyl, (C₃-C₈)-cycloalkyl, optionally substituted (C₆-C₁₄)-aryl or (C₆-C₁₄)-aryl-(C1-C₈)-alkyl optionally substituted in the aryl radical;
R¹ is X-NH-C(=NH), X-NH-C (=NX)-NH or X-NH-CH₂;
X is hydrogen, (C₁-C₆)-alkylcarbonyl, (C₁-C₆)-alkoxycarbonyl, (C₁-C₈)-alkylcarbonyloxy-(C₁-C₆)-alkoxycarbonyl, (C₆-C₁₄)-aryl-(C₁-C₆)-alkoxy-carbonyl or hydroxyl;
R² is hydrogen or (C₁-C₈)-alkyl;
R³ is CONHR¹⁵ or CONHR¹⁴ where R¹⁴ herein is a (C₁-C₈)-alkyl radical which is unsubstituted or substituted by one or more (C₆-C₁₄)-aryl radicals;
R¹⁵ is R¹⁶-(C₁-C₆)-alkyl or R¹⁶, where R¹⁶ is a 7- to 12-membered bridged bicyclic or tricyclic radical which is saturated or partially unsaturated and which can also contain one to four identical or different heteroatoms from the group consisting of nitrogen, oxygen and sulfur and which can also be substituted by one or more identical or different substituents from the group consisting of (C₁-C₄)-alkyl and oxo, and in particular R¹⁵ is an adamantyl radical or an adamantylmethyl radical;
and e, g and h independently of one another are the numbers 0, 1, 2 or 3 and b, c and d are 1.

9. The use of compounds of the formula I as claimed in one or more of claims 1, 3, 4, 5 and 8 in all their stereoisomeric forms and mixtures thereof in all ratios, and of their physiologically tolerable salts, where in the formula I simultaneously
W is R¹-A-C(R¹³);
Y is a carbonyl group;
Z is N (R⁰);
A is ethylene, trimethylene, tetramethylene, pentamethylene, cyclohexylene, phenylene or phenylenemethyl;
B is an unsubstituted or substituted methylene radical;
D is C(R²) (R³);
E is R¹⁰CO;
R is hydrogen or (C₁-C₄)-alkyl, in particular hydrogen, methyl or ethyl;
R⁰ is (C₁-C₈)-alkyl, (C₃-C₈)-cycloalkyl, optionally substituted (C₆-C₁₄)-aryl or (C₆-C₁₄)-aryl-(C₁-C₈)-alkyl optionally substituted in the aryl radical;
R¹ is H₂N-C (=NH) , H₂N-C (=NH)-NH or H₂N-CH₂;
R² is hydrogen;
R³ is CONHR¹⁵ or CONHR¹⁴ where R¹⁴ herein is a (C₁-C₆)-alkyl radical which is unsubstituted or substituted by one or more (C₆-C₁₀)-aryl radicals;
R¹⁰ is hydroxyl or (C₁-C₈)-alkoxy, preferably (C₁-C₄)-alkoxy;
R¹³ is (C₁-C₆)-alkyl, (C₃-C₇)-cycloalkyl or benzyl, in particular methyl;
R¹⁵ is an adamantyl radical or an adamantylmethyl radical;
b, c and d are 1 and e, f and g are 0;
h is 1 or 2, preferably 1.

10. The use of compounds of the formula I as claimed in one or more of claims 1, 3, 4 and 5 in all their stereoisomeric forms and mixtures thereof in all ratios, and of their physiologically tolerable salts, where in the formula I simultaneously
W is R¹-A-C(R¹³);
Y is a carbonyl group;
Z is N(R⁰);
A is ethylene, trimethylene, tetramethylene, pentamethylene, cyclohexylene, phenylene, phenylenemethyl;
B is an unsubstituted or substituted methylene radical or ethylene radical; D is C (R²) (R³)
E is R¹⁰CO;
R is hydrogen or (C₁-C₄)-alkyl, in particular hydrogen, methyl or ethyl;
R⁰ is (C₁-C₈)-alkyl, (C₃-C₈)-cycloalkyl, optionally substituted (C₆-C₁₄)-aryl or (C₆-C₁₄)-aryl-(C₁-C₈)-alkyl which is optionally substituted in the aryl radical;
R¹ is H₂N-C (=NH) , H₂N-C (=NH)-NH or H₂N-CH₂;
R² is hydrogen;
R³ is an unsubstituted phenyl radical or naphthyl radical, a phenyl radical or naphthyl radical substituted by one, two or three identical or different radicals from the group consisting of (C₁-C₄)-alkyl, (C₁-C₄)-alkoxy, hydroxyl, halogen, trifluoromethyl; nitro, methylenedioxy, ethylenedioxy, hydroxycarbonyl, (C₁-C₄)-alkoxycarbonyl, aminocarbonyl, cyano, phenyl, phenoxy, benzyl and benzyloxy, a pyridyl radical, a (C₁-C₄)-alkyl radical, a (C₂-C₄)-alkenyl radical, a (C₂-C₄)-alkynyl radical or a (C₅-C₆)-cycloalkyl radical, and in particular R³ is an unsubstituted or substituted phenyl radical or naphthyl radical;
R¹⁰ is hydroxyl or (C₁-C₈)-alkoxy, in particular (C₁-C₄)-alkoxy, and preferably R¹⁰ is a radical from the group consisting of hydroxyl, methoxy, ethoxy, propoxy and isopropoxy;
R¹³ is (C₁-C₆)-alkyl, (C₃-C₇)-cycloalkyl or benzyl, in particular methyl;
b, c and d are 1 and e, f and g are 0;
h is 1 or 2, preferably 1.

11. The use of compounds of the formula I as claimed in one or more of claims 1, 3, 4 and 5 in all their stereoisomeric forms and mixtures thereof in all ratios, and of their physiologically tolerable salts, where in the formula I simultaneously
W is R¹-A-C(R¹³);
Y is a carbonyl group;
Z is N(R⁰);
A is ethylene, trimethylene, tetramethylene, pentamethylene, cyclohexylene, phenylene, phenylenemethyl;
B is an unsubstituted or substituted methylene radical or ethylene radical;
D is C (R²)(R³);
E is R¹⁰CO;
R is hydrogen or (C₁-C₄)-alkyl, in particular hydrogen, methyl or ethyl;
R⁰ is (C₁-C₈)-alkyl, (C₃-C₈)-cycloalkyl, optionally substituted (C₆-C₁₄)-aryl or (C₆-C₁₄)-aryl-(C₁-C₈)-alkyl optionally substituted in the aryl radical;
R¹ is H₂N-C (=NH) , H₂N-C (=NH)-NH or H₂N-CH₂;
R² is hydrogen;
R³ is R¹¹NH;
R¹⁰ is hydroxyl or (C₁-C₈)-alkoxy, in particular (C₁-C₄)-alkoxy, and preferably R¹⁰ is a radical from the group consisting of hydroxyl, methoxy, ethoxy, propoxy and isopropoxy;
R¹³ is (C₁-C₆)-alkyl, (C₃-C₇)-cycloalkyl or benzyl, in particular methyl;
b, c, d and e are 1 and f and g are 0;
h is 0.

12. The use of compounds of the formula I as claimed in one or more of claims 1 and 3 to 11 in all their stereoisomeric forms and mixtures thereof in all ratios, and of their physiologically tolerable salts, in which a substituted methylene radical or substituted ethylene radical representing the group B carries as a substituent a radical from the group consisting of (C₁-C₈)-alkyl, (C₂-C₆)-alkenyl, (C₂-C₆)-alkynyl, (C₃-C₈)-cycloalkyl, in particular (C₅-C₆)-cycloalkyl, (C₃-C₈)-cycloalkyl-(C₁-C₄)-alkyl, in particular (C₅-C₆)-cycloalkyl-(C₁-C₄)-alkyl, optionally substituted (C₆-C₁₀)-aryl, (C₆-C₁₀)-aryl-(C₁-C₄)-alkyl optionally substituted in the aryl radical, optionally substituted heteroaryl and heteroaryl-(C₁-C₄)-alkyl optionally substituted in the heteroaryl radical.

13. The use of compounds of the formula I as claimed in one or more of claims 1 and 3 to 12 in all their stereoisomeric forms and mixtures thereof in all ratios, and of their physiologically tolerable salts, in which B is an unsubstituted methylene radical or a methylene radical which is substituted by a (C₁-C₈)-alkyl radical.

14. The use of compounds of the formula I as claimed in one or more of claims 1 to 13 and/or of their physiologically tolerable salts for the production of pharmaceuticals for inhibiting inflammation.

15. The use of compounds of the formula I as claimed in one or more of claims 1 to 13 and/or of their physiologically tolerable salts for the production of pharmaceuticals for the treatment or prophylaxis of rheumatoid arthritis, inflammatory bowel disease, systemic lupus erythematosus or inflammatory disorders of the central nervous system.

16. The use of compounds of the formula I as claimed in one or more of claims 1 to 13 and/or of their physiologically tolerable salts for the production of pharmaceuticals for the treatment or prophylaxis of asthma or allergies.

17. The use of compounds of the formula I as claimed in one or more of claims 1 to 13 and/or of their physiologically tolerable salts for the production of pharmaceuticals for the treatment or prophylaxis of cardiovascular disorders, arteriosclerosis, restenoses or diabetes, for the prevention of damage to organ transplants, for the inhibition of tumor growth or tumor metastasis or for the therapy of malaria.

18. The use of compounds of the formula I as claimed in one or more of claims 1 to 13 and/or of their physiologically tolerable salts for the inhibition of the adhesion and/or migration of- leucocytes or for the inhibition of the VIA-4 receptors.

19. The use of compounds of the formula I as claimed in one or more of claims 1 to 13 and/or of their physiologically tolerable salts for the treatment or prophylaxis of illnesses in which leucocyte adhesion and/or leucocyte migration exhibits an undesired extent, or of illnesses in which VLA-4-dependent adhesion processes play a part, for the treatment or prophylaxis of rheumatoid arthritis, inflammatory bowel disease, systemic lupus erythematosus, inflammatory disorders of the central nervous system, asthma, allergies, cardiovascular disorders, arteriosclerosis, restenoses, diabetes, for the prevention of damage to organ transplants, for the inhibition of tumor growth or tumor metastasis, for the therapy of malaria, or as inhibitors of inflammation.

20. A compound of the formula Ib 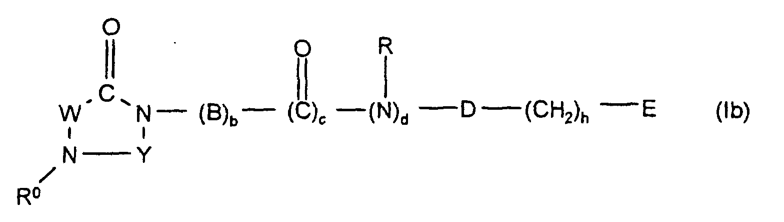 in which
W is R¹-A-CH or R¹-A-CH=C;
Y is a carbonyl, thiocarbonyl or methylene group;
A is a bivalent radical from the group consisting of (C₁-C₆)-alkylene, (C₃-C₇)-cycloalkylene, phenylene, phenylene-(C₁-C₆)-alkyl, (C₁-C₆)-alkylenephenyl, phenylene-(C₂-C₆)-alkenyl or a bivalent radical of a 5- or 6-membered saturated or unsaturated ring which can contain 1 or 2 nitrogen atoms and can be mono- or disubstituted by (C₁-C₆)-alkyl or doubly bonded oxygen or sulfur;
B is a bivalent radical from the group consisting of (C₁-C₆)-alkylene, (C₂-C₆)-alkenylene, phenylene, phenylene-(C₁-C₃)-alkyl, (C₁-C₃)-alkylenephenyl;
D is C(R²)(R³);
E is tetrazolyl, (R⁸O)₂P(O), HOS(O)₂, R⁹NHS(O)₂ or R¹⁰CO;
R is hydrogen, (C₁-C₈)-alkyl, (C₃-C₈)-cycloalkyl, optionally substituted (C₆-C₁₄)-aryl or (C₆-C₁₄)-aryl-(C₁-C₈)-alkyl optionally substituted in the aryl radical;
R⁰ is (C₇-C₈)-alkyl, (C₃-C₈)-cycloalkyl, optionally substituted (C₆-C₁₄)-aryl or (C₆-C₁₄)-aryl-(C₁-C₈)-alkyl optionally substituted in the aryl radical;
R¹ is X-NH-C (=NH)-(CH₂)ₚ or X¹-NH-(CH₂)ₚ where p is the numbers 0, 1, 2 or 3;
X is hydrogen, (C₁-C₆)-alkyl, (C₁-C₆)-alkylcarbonyl, (C₁-C₆)-alkoxycarbonyl, (C₁-C₁₈)-alkylcarbonyloxy-(C₁-C₆)-alkoxycarbonyl, optionally substituted (C₆-C₁₄)-arylcarbonyl, optionally substituted (C₆-C₁₄)-aryloxycarbonyl, (C₆-C₁₄)-aryl-(C₁-C₆)-alkoxycarbonyl which can also be substituted in the aryl radical, (R⁸O)₂P(O) , cyano, hydroxyl, (C₁-C₆)-alkoxy, (C₆-C₁₄)-aryl-(C₁-C₆)-alkoxy which can also be substituted in the aryl radical, or amino;
X¹ has one of the meanings of X or is R'-NH-C(=N-R "), where R' and R'' independently of one another have the meanings of X;
R² is hydrogen or phenyl;
R³ is hydrogen, COOR⁴, CON (CH₃) R⁴ or CONHR⁴;
R⁴ is hydrogen or (C₁-C₂₈)-alkyl which can optionally be mono- or polysubstituted by identical or different radicals R^{4'};
R^{4'} is hydroxyl, hydroxycarbonyl, aminocarbonyl, mono- or di-((C₁-C₁₈)-alkyl) aminocarbonyl, amino-(C₂-C₁₈)-alkyl aminocarbonyl, amino-(C₁-C₃)-alkylphenyl-(C₁-C₃)-alkylaminocarbonyl, (C₁-C₁₈)-alkylcarbonylamino-(C₁-C₃)-alkylphenyl-(C₁-C₃)-alkylaminocarbonyl, (C₁-C₁₈)-alkylcarbonylamino-(C₂-C₁₈)-alkylaminocarbonyl, (C₆-C₁₄)-aryl-(C₁-C₈)-alkoxycarbonyl which can also be substituted in the aryl radical, amino, mercapto, (C₁-C₁₈)-alkoxy, (C₁-C₁₈)-alkoxycarbonyl, optionally substituted (C₃-C₈)-cycloalkyl, halogen, nitro, trifluoromethyl or the radical R⁵;
R⁵ is optionally substituted (C₆-C₁₄)-aryl, (C₆-C₁₄)-aryl-(C₁-C₈)-alkyl optionally substituted in the aryl radical, a mono- or bicyclic 5- to 12-membered heterocyclic ring which can be aromatic, partially hydrogenated or completely hydrogenated and which can contain one, two or three identical or different heteroatoms from the group consisting of nitrogen, oxygen and sulfur, a radical R⁶ or a radical R⁶CO-, where the aryl and, independently thereof, the heterocyclic radical can be mono- or polysubstituted by identical or different radicals from the group consisting of (C₁-C₁₈)-alkyl, (C₁-C₁₈)-alkoxy, halogen, nitro, amino and trifluoromethyl;
R⁶ is R⁷R⁸N, R⁷O or R⁷S or an amino acid side chain, a natural or unnatural amino acid, imino acid, optionally N-(C₁-C₈)-alkylated or N-((C₆-C₁₄)-aryl-(C₁-C₈)-alkylated) azaamino acid or a dipeptide radical which can also-be substituted in the aryl radical and/or in which the peptide bond can be reduced to -NH-CH₂-, and their esters and amides, where instead of free functional groups there can optionally be hydrogen or hydroxymethyl and/or where free functional groups can be protected by protective groups customary in peptide chemistry;
R⁷ is hydrogen, (C₁-C₁₈)-alkyl, (C₆-C₁₄)-aryl-(C₁-C₈)-alkyl, (C₁-C₁₈)-alkyl carbonyl, (C₁-C₁₈)-alkoxycarbonyl, (C₆-C₁₄)-aryl carbonyl, (C₆-C₁₄)-aryl-(C₁-C₈)-alkylcarbonyl or (C₆-C₁₄)-aryl-(C₁-C₁₈)-alkyloxycarbonyl, where the alkyl groups can optionally be substituted by an amino group and/or where the aryl radicals can be mono- or polysubstituted, preferably monosubstituted, by identical or different radicals from the group consisting of (C₁-C₈)-alkyl, (C₁-C₈)-alkoxy, halogen, nitro, amino and trifluoromethyl, a natural or unnatural amino acid, imino acid, optionally N-(C₁-C₈)-alkylated or N-((C₆-C₁₄)-aryl-(C₁-C₈)-alkylated) azaamino acid or a dipeptide radical which can also be substituted in the aryl radical and/or in which the peptide bond can be reduced to -NH-CH₂-.
R⁸ is hydrogen, (C₁-C₁₈)-alkyl, optionally substituted (C₆-C₁₄)-aryl or (C₆-C₁₄)-aryl or (C₆-C₁₄)-aryl-(C₁-C₈)-alkyl which can also be substituted in the aryl radical;
R⁹ is hydrogen, aminocarbonyl, (C₁-C₁₈)-alkyl-aminocarbonyl, (C₃-C₈)-cycloalkylaminocarbonyl, optionally substituted (C₆-C₁₄)-arylamino-carbonyl, (C₁-C₁₈)-alkyl, optionally substituted (C₆-C₁₄)-aryl or (C₃-C₈)-cycloalkyl ;
R¹⁰ is hydroxyl, (C₁-C₁₈)-alkoxy, (C₆-C₁₄)-aryl-(C₁-C₈)-alkoxy which can also be substituted in the aryl radical, optionally substituted (C₆-C₁₄)-aryloxy, amino or mono- or di-((C₁-C₁₈)-alkyl)amino;
b, c and d independently of oneanother can be 0 or 1, but cannot all simultaneously be 0;
h is the numbers 0, 1, 2, 3, 4, 5 or 6;
in all its stereoisomeric forms and mixtures thereof in all ratios, or its physiologically tolerable salts.

21. A compound of the formula Ib as claimed in claim 20 and/or its physiologically tolerable salts for use as a pharmaceutical.

22. A pharmaceutical preparation, which comprises one or more compounds of the formula Ib as claimed in claim 20 and/or their physiologically tolerable salts in addition to pharmaceutically innocuous excipients and/or additives.

23. A compound of the formula Ic 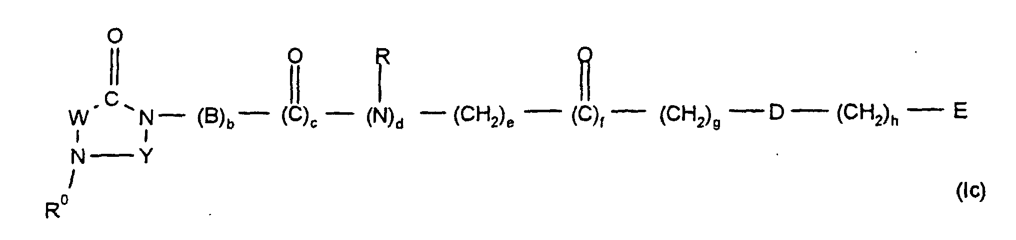 in which
W is R¹-A-C(R¹³);
Y is a carbonyl, thiocarbonyl or methylene group;
A is a phenylene radical;
B is a bivalent radical from the group consisting of (C₁-C₆)-alkylene, (C₂-C₆)-alkenylene, phenylene, phenylene-(C₁-C₃)-alkyl, (C₁-C₃)-alkylenephenyl;
D is C(R²)(R³), N(R³) or CH=C (R³);
E is tetrazolyl, (R⁸O)₂P(O) , HOS(O)₂, R⁹NHS(O)₂ or R¹⁰CO;
R is hydrogen, (C₁-C₈)-alkyl, (C₃-C₈)-cycloalkyl, optionally substituted (C₆-C₁₄)-aryl or (C₆-C₁₄)-aryl-(C₁-C₈)-alkyl optionally substituted in the aryl radical;
R⁰ is (C₆-C₁₄)-aryl-(C₁-C₈)-alkyl optionally substituted in the aryl radical;
R¹ is X-NH-C (=NH)-(CH₂)ₚ or X¹-NH-(CH₂) ₚ, where p is 0, 1, 2 or 3;
X is hydrogen, (C₁-C₆)-alkyl, (C₁-C₆)-alkylcarbonyl, (C₁-C₆)-alkoxycarbonyl, (C₁-C₁₈)-alkylcarbonyloxy-(C₁-C₆)-alkoxycarbonyl, optionally substituted (C₆-C₁₄)-arylcarbonyl, optionally substituted (C₆-C₁₄)-aryloxycarbonyl, (C₆-C₁₄)-aryl-(C₁-C₆)-alkoxycarbonyl which can also be substituted in the aryl radical, (R⁸O)₂P(O) , cyano, hydroxyl, (C₁-C₆)-alkoxy, (C₆-C₁₄)-aryl-(C₁-C₆)-alkoxy which can also be substituted in the aryl radical, or amino;
X¹ has one of the meanings of X or is R'-NH-C(=N-R''), where R' and R'' independently of one another have the meanings of X;
R² is hydrogen, (C₁-C₈)-alkyl optionally substituted (C₆-C₁₄)-aryl, (C₆-C₁₄)-aryl-(C₁-C₈)-alkyl optionally substituted in the aryl radical or (C₃-C₈)-cycloalkyl;
R³ is hydrogen, (C₁-C₈)-alkyl, optionally substituted (C₆-C₁₄)-aryl, (C₆-C₁₄)-aryl-(C₁-C₈)-alkyl optionally substituted in the aryl radical, (C₃-C₈)-cycloalkyl, (C₂-C₈)-alkenyl, (C₂-C₈)-alkynyl, (C₂-C₈)-alkenylcarbonyl, (C₂-C₈)-alkynylcarbonyl, pyridyl, R¹¹NH, R⁴CO, COOR⁴, CON (CH₃) R¹⁴, CONHR¹⁴, CSNHR¹⁴, COOR¹⁵, CON (CH₃) R¹⁵ or CONHR¹⁵;
R⁴ is hydrogen or (C₁-C₂₈)-alkyl which can optionally be mono- or polysubstituted by identical or different radicals R^{4'};
R^{4'} is hydroxyl, hydroxycarbonyl, aminocarbonyl, mono- or di-((C₁-C₁₈)-alkyl) aminocarbonyl, amino-(C₂-C₁₈)-alkylaminocarbonyl, amino-(C₁-C₃)-alkylphenyl-(C₁-C₃)-alkylaminocarbonyl, (C₁-C₁₈)-alkylcarbonylamino-(C₁-C₃)-alkylphenyl-(C₁-C₃)-alkylaminocarbonyl, (C₁-C₁₈)-alkylcarbonylamino-(C₂-C₁₈)-alkylaminocarbonyl, (C₆-C₁₄)-aryl-(C₁-C₈)-alkoxycarbonyl which can also be substituted in the aryl radical, amino, mercapto, (C₁-C₁₈)-alkoxy, (C₁-C₁₈)-alkoxycarbonyl, optionally substituted (C₃-C₈)-cycloalkyl, halogen, nitro, trifluoromethyl or the radical R⁵ ;
R⁵ is optionally substituted (C₆-C₁₄)-aryl, (C₆-C₁₄)-aryl-(C₁-C₈)-alkyl optionally substituted in the aryl radical, a mono- or bicyclic 5- to 12-membered heterocyclic ring which can be aromatic, partially hydrogenated or completely hydrogenated and which can contain one, two or three identical or different heteroatoms from the group consisting of nitrogen, oxygen and sulfur, a radical R⁶ or a radical R⁶CO-, where the aryl and, independently thereof, the heterocyclic radical can be mono- or polysubstituted by identical or different radicals from the group consisting of (C₁-C₁₈)-alkyl, (C₁-C₁₈)-alkoxy, halogen, nitro, amino or trifluoromethyl;
R⁶ is R⁷R⁸N, R⁷O or R⁷S or an amino acid side chain, a natural or unnatural amino acid, imino acid, optionally N-(C₁-C₈)-alkylated or N-((C₆-C₁₄)-aryl-(C₁-C₈)-alkylated) azaamino acid or a dipeptide radical which can also-be substituted in the aryl radical and/or in which the peptide bond can be reduced to -NH-CH₂-, and their esters and amides, where instead of free functional groups there can optionally be hydrogen or hydroxymethyl and/or where free functional groups can be protected by protective groups customary in peptide chemistry;
R⁷ is hydrogen, (C₁-C₁₈)-alkyl, (C₆-C₁₄)-aryl-(C₁-C₈)-alkyl, (C₁-C₁₈)-alkyl carbonyl, (C₁-C₁₈)-alkoxycarbonyl, (C₆-C₁₄)-aryl carbonyl, (C₆-C₁₄)-aryl-(C₁-C₈)-alkylcarbonyl or (C₆-C₁₄)-aryl-(C₁-C₁₈)-alkyloxycarbonyl, where the alkyl groups can optionally be substituted by an amino group and/or where the aryl radicals can be mono- or polysubstituted, preferably monosubstituted, by identical or different radicals from the group consisting of (C₁-C₈)-alkyl, (C₁-C₈)-alkoxy, halogen, nitro, amino and trifluoromethyl, a natural or unnatural amino acid, imino acid, optionally N-(C₁-C₈)-alkylated or N-((C₆-C₁₄)-aryl-(C₁-C₈)-alkylated) azaamino acid or a dipeptide radical which can also be substituted in the aryl radical and/or in which the peptide bond can be reduced to -NH-CH₂-;
R⁸ is hydrogen, (C₁-C₁₈)-alkyl, optionally substituted (C₆-C₁₄)-aryl or (C₆-C₁₄)-aryl-(C₁-C₈)-alkyl which can also be substituted in the aryl radical;
R⁹ is hydrogen, aminocarbonyl, (C₁-C₁₈)-alkylaminocarbonyl , (C₃-C₈)-cycloalkylaminocarbonyl, optionally substituted (C₆-C₁₄)-arylaminocarbonyl, (C₁-C₁₈)-alkyl, optionally substituted (C₆-C₁₄)-aryl or (C₃-C₈)-cycloalkyl;
R¹⁰ is hydroxyl, (C₁-C₁₈)-alkoxy, (C₆-C₁₄)-aryl-(C₁-C₈)-alkoxy which can also be substituted in the aryl radical, optionally substituted (C₆-C₁₄)-aryloxy, amino or mono- or di-((C₁-C₁₈)-alkyl) amino;
R¹¹ is hydrogen, (C₁-C₁₈)-alkyl, R¹²CO, optionally substituted (C₆-C₁₄)-aryl-S (O)₂, (C₁-C₁₈)-alkyl-S (O)₂, (C₆-C₁₄)-aryl-(C₁-C₈)-alkyl optionally substituted in the aryl radical or R⁹NHS(O)₂;
R¹² is hydrogen, (C₁-C₁₈)-alkyl, (C₂-C₈)-alkenyl, (C₂-C₈)-alkynyl, optionally substituted (C₆-C₁₄)-aryl, (C₁-C₁₈)-alkoxy, (C₆-C₁₄)-aryl-(C₁-C₈)-alkoxy which can also be substituted in the aryl radical, optionally substituted (C₆-C₁₄)-aryloxy, amino or mono- or di-((C₁-C₁₈)-alkyl)amino;
R¹³ is (C₁-C₆)-alkyl, (C₆-C₁₄)-aryl-(C₁-C₈)-alkyl optionally substituted in the aryl radical or (C₃-C₈)-cycloalkyl;
R¹⁴ is hydrogen or (C₁-C₂₈)-alkyl which can optionally be mono- or polysubstituted by identical or different radicals from the group consisting of hydroxyl, hydroxycarbonyl, aminocarbonyl, mono- or di-((C₁-C₁₈)-alkyl)-aminocarbonyl, amino-(C₂-C₁₈)-alkylamino-carbonyl, amino-(C₁-C₃)-alkylphenyl-(C₁-C₃)-alkylaminocarbonyl, (C₁-C₁₈)-alkylcarbonylamino-(C₁-C₃)-alkylphenyl-(C₁-C₃)-alkylaminocarbonyl, (C₁-C₁₈)-alkylcarbonylamino-(C₂-C₁₈)-alkylamino-carbonyl, (C₆-C₁₄)-aryl-(C₁-C₈)-alkoxycarbonyl which can also be substituted in the aryl radical, amino, mercapto, (C₁-C₁₈)-alkoxy, (C₁-C₁₈)-alkoxycarbonyl, optionally substituted (C₃-C₈)-cycloalkyl, HOS (O) ₂-(C₁-C₃)-alkyl, R⁹NH(O) ₂-(C₁-C₃)-alkyl, (R⁸O) ₂P (O)-(C₁-C₃)-alkyl, tetrazolyl-(C₁-C₃)-alkyl, halogen, nitro, trifluoromethyl and R⁵;
R¹⁵ is R¹⁶-(C₁-C₆)-alkyl or R¹⁶;
R¹⁶ is a 6- to 24-membered bicyclic or tricyclic radical which is saturated or partially unsaturated and which can also contain one to four identical or different heteroatoms from the group consisting of nitrogen, oxygen and sulfur and which can also be substituted by one or more identical or different substituents from the group consisting of (C₁-C₄)-alkyl and oxo;
b, c, d and f independently of one another are 0 or 1, but cannot all simultaneously be 0;
e, g and h independently of one another are 0, 1, 2, 3, 4, 5 or 6;
in all its stereoisomeric forms and mixtures thereof in all ratios, or its physiologically tolerable salts.

24. A compound of the formula Ic as claimed in claim 23 and/or its physiologically tolerable salts for use as a pharmaceutical.

25. A pharmaceutical preparation, which comprises one or more compounds of the formula Ic as claimed in claim 23 and/or their physiologically tolerable salts in addition to pharmaceutically innocuous excipients and/or additives.

26. A compound of the formula Id 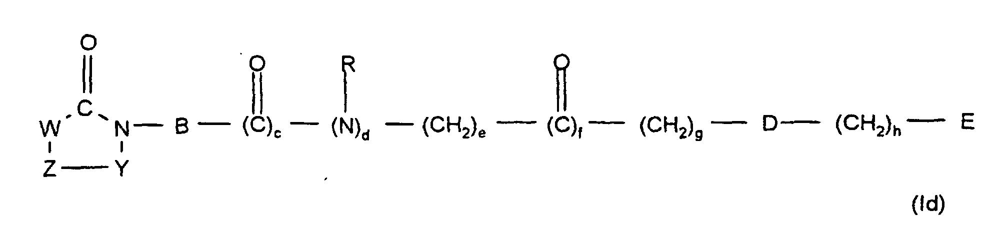 in which
W is R¹-A-C(R¹³) or R¹-A-CH=C;
Y is a carbonyl, thiocarbonyl or methylene group;
Z is N (R⁰) ;
A is a bivalent radical from the group consisting of (C₁-C₆)-alkylene, (C₃-C₇)-cycloalkylene, phenylene, phenylene-(C₁-C₆)-alkyl, (C₁-C₆)-alkylenephenyl, phenylene-(C₂-C₆)-alkenyl or a bivalent radical of a 5- or 6-membered saturated or unsaturated ring which can contain 1 or 2 nitrogen atoms and can be mono- or disubstituted by (C₁-C₆)-alkyl or doubly bonded oxygen or sulfur;
B is a bivalent (C₁-C₆)-alkylene radical which is substituted by a radical from the group consisting of (C₁-C₈)-alkyl, (C₂-C₈)-alkenyl, (C₂-C₈)-alkynyl, (C₃-C₁₀)-cycloalkyl, (C₃-C₁₀)-cycloalkyl-(C₁-C₆)-alkyl, optionally substituted (C₆-C₁₄)-aryl, (C₆-C₁₄)-aryl-(C₁-C₆)-alkyl optionally substituted in the aryl radical, optionally substituted heteroaryl and heteroaryl-(C₁-C₆)-alkyl optionally substituted in the heteroaryl radical;
D is C(R²)(R³) , N(R³) or CH=C(R³);
E is tetrazolyl, (R⁸O)₂P(O) , HOS(O)₂, R⁹NHS(O)₂ or R¹⁰CO;
R is hydrogen, (C₁-C₈)-alkyl, (C₃-C₈)-cycloalkyl, optionally substituted (C₆-C₁₄)-aryl or (C₆-C₁₄)-aryl-(C₁-C₈)-alkyl optionally substituted in the aryl radical;
R⁰ is hydrogen, (C₁-C₈)-alkyl, (C₃-C₁₂)-cycloalkyl, (C₃-C₁₂)-cycloalkyl-(C₁-C₈)-alkyl, (C₆-C₁₂)-bicycloalkyl, (C₆-C₁₂)-bicycloalkyl-(C₁-C₈)-alkyl, (C₆-C₁₂)-tricycloalkyl, (C₆-C₁₂)-tricycloalkyl-(C₁-C₈)-alkyl, optionally substituted (C₆-C₁₄)-aryl, (C₆-C₁₄)-aryl-(C₁-C₈)-alkyl optionally substituted in the aryl radical, optionally substituted heteroaryl, heteroaryl-(C₁-C₈)-alkyl optionally substituted in the heteroaryl radical, CHO, (C₁-C₈)-alkyl-CO, (C₃-C₁₂)-cycloalkyl-CO, (C₃-C₁₂)-cycloalkyl-(C₁-C₈)-alkyl-CO, (C₆-C₁₂)-bicycloalkyl-CO, (C₆-C₁₂)-bicycloalkyl-(C₁-C₈)-alkyl-CO, (C₆-C₁₂)-tricycloalkyl-CO, (C₆-C₁₂)-tricycloalkyl-(C₁-C₈)-alkyl-CO, optionally substituted (C₆-C₁₄)-aryl-CO, (C₆-C₁₄)-aryl-(C₁-C₈)-alkyl-CO optionally substituted in the aryl radical; optionally substituted heteroaryl-CO, heteroaryl-(C₁-C₈)-alkyl-CO optionally substituted in the heteroaryl radical, (C₁-C₈)-alkyl-S (O)ₙ, (C₃-C₁₂)-cycloalkyl-S (O) ₙ, (C₃-C₁₂)-cycloalkyl-(C₁-C₈)-alkyl-S (O) ₙ, (C₆-C₁₂)-bicycloalkyl-S(O)ₙ, (C₆-C₁₂)-bicycloalkyl-(C₁-C₈)-alkyl-S (O)ₙ, (C₆-C₁₂)-tricycloalkyl-S (O)ₙ, (C₆-C₁₂)-tricycloalkyl-(C₁-C₈)-alkyl-S (O)ₙ, optionally substituted (C₆-C₁₉)-aryl-S (O)ₙ, (C₆-C₁₄)-aryl-(C₁-C₈)-alkyl-S (O) ₙ optionally substituted in the aryl radical, optionally substituted heteroaryl- S(O)ₙ or heteroaryl-(C₁-C₈)-alkyl-S (O) ₙ optionally substituted in the heteroaryl radical, where n is 1 or 2;
R¹ is X-NH-C (=NH)-(CH₂)ₚ or X¹-NH-(CH₂)ₚ, where p is 0, 1, 2 or 3;
X is hydrogen, (C₁-C₆)-alkyl, (C₁-C₆)-alkylcarbonyl, (C₁-C₆)-alkoxycarbonyl, (C₁-C₁₈)-alkylcarbonyloxy-(C₁-C₆)-alkoxycarbonyl, optionally substituted (C₆-C₁₄)-arylcarbonyl, optionally substituted (C₆-C₁₄)-aryloxycarbonyl, (C₆-C₁₄)-aryl-(C₁-C₆)-alkoxycarbonyl which can also be substituted in the aryl radical, (R⁸O)₂P(O), cyano, hydroxyl, (C₁-C₆)-alkoxy, (C₆-C₁₄)-aryl-(C₁-C₆)-alkoxy which can also be substituted in the aryl radical, or amino;
X¹ has one of the meanings of X or is R'-NH-C(=N-R "), where R' and R'' independently of one another have the meanings of X;
R² is hydrogen, (C₁-C₈)-alkyl, optionally substituted (C₆-C₁₄)-aryl, (C₆-C₁₄)-aryl-(C₁-C₈)-alkyl optionally substituted in the aryl radical or (C₃-C₈)-cycloalkyl;
R³ is hydrogen, (C₁-C₈)-alkyl, optionally substituted (C₆-C₁₄)-aryl, (C₆-C₁₄)-aryl-(C₁-C₈)-alkyl optionally substituted in the aryl radical, (C₃-C₈)-cycloalkyl, (C₂-C₈)-alkenyl, (C₂-C₈)-alkynyl, (C₂-C₈)-alkenylcarbonyl, (C₂-C₈)-alkynylcarbonyl, pyridyl, R¹¹NH, R⁴CO, COOR⁴, CON(CH₃)R¹⁴, CONHR¹⁴, CSNHR¹⁴, COOR¹⁵, CON(CH₃)R¹⁵ or CONHR¹⁵;
R⁴ is hydrogen or (C₁-C₂₈)-alkyl which can optionally be mono- or polysubstituted by identical or different radicals R^{4'};
R^{4'} is hydroxyl, hydroxycarbonyl, aminocarbonyl, mono- or di-((C₁-C₁₈)-alkyl) amino-(C₂-C₁₈)-alkylaminocarbonyl, amino-(C₁-C₃)-alkylphenyl-(C₁-C₃)-alkylaminocarbonyl, (C₁-C₁₈)-alkylcarbonylamino-(C₁-C₃)-alkylphenyl-(C₁-C₃)-alkylaminocarbonyl, (C₁-C₁₈)-alkyl-carbonylamino-(C₂-C₁₈)-alkylaminocarbonyl, (C₆-C₁₄)-aryl-(C₁-C₈)-alkoxycarbonyl which can also be substituted in the aryl radical, amino, mercapto, (C₁-C₁₈)-alkoxy, (C₁-C₁₈)-alkoxy-carbonyl, optionally substituted (C₃-C₈)-cyclo-alkyl, halogen, nitro, trifluoromethyl or the radical R⁵;
R⁵ is optionally substituted (C₆-C₁₄)-aryl, (C₆-C₁₄)-aryl-(C₁-C₈)-alkyl optionally substituted in the aryl radical, a mono- or bicyclic 5- to 12-membered heterocyclic ring which can be aromatic, partially hydrogenated or completely hydrogenated and which can contain one, two or three identical or different heteroatoms from the group consisting of nitrogen, oxygen and sulfur, a radical R⁶ or a radical R⁶CO-, where the aryl, and independently thereof, the heterocyclic radical can be mono- or polysubstituted by identical or different radicals from the group consisting of (C₁-C₁₈)-alkyl, (C₁-C₁₈)-alkoxy, halogen, nitro, amino and trifluoromethyl;
R⁶ is R⁷R⁸N, R⁷O or R⁷S or an amino acid side chain, a natural or unnatural amino acid, imino acid, optionally N-(C₁-C₈)-alkylated or N-((C₆-C₁₄)-aryl-(C₁-C₈)-alkylated) azaamino acid or a dipeptide radical which can also be substituted in the aryl radical and/or in which the peptide bond can be reduced to -NH-CH₂-, and their esters and amides, where instead of free functional groups there can optionally be hydrogen or hydroxymethyl and/or where free functional groups can be protected by protective groups customary in peptide chemistry;
R⁷ is hydrogen, (C₁-C₁₈)-alkyl, (C₆-C₁₄)-aryl-(C₁-C₈)-alkyl, (C₁-C₁₈)-alkylcarbonyl, (C₁-C₁₈)-alkoxycarbonyl, (C₆-C₁₄)-arylcarbonyl, (C₆-C₁₄)-aryl-(C₁-C₈)-alkylcarbonyl or (C₆-C₁₄)-aryl-(C₁-C₁₈)-alkyloxycarbonyl, where the alkyl groups can optionally be substituted by an amino group and/or where the aryl radicals can be mono- or polysubstituted, preferably monosubstituted, by identical or different radicals from the group consisting of (C₁-C₈)-alkyl, (C₁-C₈)-alkoxy, halogen, nitro, amino and trifluoromethyl, a natural or unnatural amino acid, imino acid, optionally N-(C₁-C₈)-alkylated or N-((C₆-C₁₄)-aryl-(C₁-C₈)-alkylated) azaamino acid or a dipeptide radical which can also be substituted in the aryl radical and/or in which the peptide bond can be reduced to -NH-CH₂-;
R⁸ is hydrogen, (C₁-C₁₈)-alkyl, optionally substituted (C₆-C₁₄)-aryl or (C₆-C₁₄)-aryl-(C₁-C₈)-alkyl which can also be substituted in the aryl radical;
R⁹ is hydrogen, aminocarbonyl, (C₁-C₁₈)-alkyl-aminocarbonyl, (C₃-C₈)-cycloalkylaminocarbonyl, optionally substituted (C₆-C₁₄)-arylamino-carbonyl, (C₁-C₁₈)-alkyl, optionally substituted (C₆-C₁₄)-aryl or (C₃-C₈)-cycloalkyl;
R¹⁰ is hydroxyl, (C₁-C₁₈)-alkoxy, (C₆-C₁₄)-aryl-(C₁-C₈)-alkoxy which can also be substituted in the aryl radical, optionally substituted (C₆-C₁₄)-aryloxy, amino or mono- or di-((C₁-C₁₈)-alkyl)amino;
R¹¹ is hydrogen, (C₁-C₁₈)-alkyl, R¹²CO, optionally substituted (C₆-C₁₄)-aryl-S(O)₂, (C₁-C₁₈)-alkyl-S(O)₂, (C₆-C₁₄)-aryl-(C₁-C₈)-alkyl optionally substituted in the aryl radical or R⁹NHS(O)₂;
R¹² is hydrogen, (C₁-C₁₈)-alkyl, (C₂-C₈)-alkenyl, (C₂-C₈)-alkynyl, optionally substituted (C₆-C₁₄)-aryl, (C₁-C₁₈)-alkoxy, (C₆-C₁₄)-aryl-(C₁-C₈)-alkoxy which can also be substituted in the aryl radical, optionally substituted (C₆-C₁₄)-aryloxy, amino or mono- or di-((C₁-C₁₈)-alkyl)amino;
R¹³ is hydrogen, (C₁-C₆)-alkyl, (C₆-C₁₄)-aryl-(C₁-C₈)-alkyl optionally substituted in the aryl radical or (C₃-C₈)-cycloalkyl;
R¹⁴ is hydrogen or (C₁-C₂₈)-alkyl which can optionally be mono- or polysubstituted by identical or different radicals from the group consisting of hydroxyl, hydroxycarbonyl, amino-carbonyl, mono- or di-((C₁-C₁₈)-alkyl)-aminocarbonyl, amino-(C₂-C₁₈)- alkylamino-carbonyl, amino-(C₁-C₃)-alkylphenyl-(C₁-C₃)-alkylaminocarbonyl, (C₁-C₁₈)-alkylcarbonylamino-(C₁-C₃)-alkylphenyl-(C₁-C₃)-alkylaminocarbonyl, (C₁-C₁₈)-alkylcarbonyjamino-(C₂-C₁₈)-alkylamino-carbonyl, (C₆-C₁₄)-aryl-(C₁-C₈)-alkoxycarbonyl which can also be substituted in the aryl radical, amino, mercapto, (C₁-C₁₈)-alkoxy, (C₁-C₁₈)-alkoxycarbonyl, optionally substituted (C₃-C₈)-cycloalkyl, HOS (O) ₂-(C₁-C₃)-alkyl, R⁹NHS (O) ₂-(C₁-C₃)-alkyl, (R⁸O) ₂P (O)-(C₁-C₃)-alkyl, tetrazolyl-(C₁-C₃)-alkyl, halogen, nitro, trifluoromethyl and R⁵;
R¹⁵ is R¹⁶-(C₁-C₆)-alkyl or R¹⁶;
R¹⁶ is a 6- to 24-membered bicyclic or tricyclic radical which is saturated or partially unsaturated and which can also contain one to four identical or different heteroatoms from the group consisting of nitrogen, oxygen and sulfur and which can also be substituted by one or more identical or different substituents from the group consisting of (C₁-C₄)-alkyl and oxo;
c, d and f independently of one another are 0 or 1, but cannot all simultaneously be 0;
e, g and h independently of one another are 0, 1, 2, 3, 4, 5 or 6;
in all its stereoisomeric forms and mixtures thereof in all ratios, or its physiologically tolerable salts.

27. A compound of the formula Id as claimed in claim 26, in which simultaneously
W is R¹-A-C(R¹³)
Y is a carbonyl group;
Z is N (R⁰) ;
A is a bivalent radical from the group consisting of (C₃-C₇)-cycloalkylene, phenylene, phenylene-(C₁-C₆)-alkyl, (C₁-C₆)-alkylenephenyl or a bivalent radical of a 5- or 6-membered saturated or unsaturated ring which can contain 1 or 2 nitrogen atoms and can be mono- or disubstituted by (C₁-C₆)-alkyl or doubly bonded oxygen or sulfur;
B is a bivalent methylene radical or ethylene radical which is substituted by a radical from the group consisting of (C₁-C₈)-alkyl, (C₂-C₈)-alkenyl, (C₂-C₈)-alkynyl, (C₃-C₁₀)-cycloalkyl, (C₃-C₁₀)-cycloalkyl-(C₁-C₆)-alkyl, optionally substituted (C₆-C₁₄)-aryl, (C₆-C₁₄)-aryl-(C₁-C₆)-alkyl optionally substituted in the aryl radical, optionally substituted heteroaryl and heteroaryl-(C₁-C₆)-alkyl optionally substituted in the heteroaryl radical;
D is C(R²)(R³);
E is tetrazolyl or R¹⁰CO;
R is hydrogen or (C₁-C₈)-alkyl;
R⁰ is hydrogen, (C₁-C₈)-alkyl, (C₃-C₁₂)-cycloalkyl, (C₃-C₁₂)-cycloalkyl-(C₁-C₈)-alkyl, (C₆-C₁₂)-bicycloalkyl, (C₆-C₁₂)-bicycloalkyl-(C₁-C₈)-alkyl, (C₆-C₁₂)-tricycloalkyl, (C₆-C₁₂)-tricycloalkyl-(C₁-C₈)-alkyl, optionally substituted (C₆-C₁₄)-aryl, (C₆-C₁₄)-aryl-(C₁-C₈)-alkyl optionally substituted in the aryl radical, optionally substituted heteroaryl, heteroaryl -(C₁-C₈)-alkyl optionally substituted in the heteroaryl radical, CHO, (C₁-C₈)-alkyl-CO, (C₃-C₁₂)-cycloalkyl-CO, (C₃-C₁₂)-cycloalkyl-(C₁-C₈)-alkyl-CO, (C₆-C₁₂)-bicycloalkyl-CO, (C₆-C₁₂)-bicycloalkyl-(C₁-C₈)-alkyl-CO, (C₆-C₁₂)-tricycloalkyl-CO, (C₆-C₁₂)-tricycloalkyl-(C₁-C₈)-alkyl-CO, optionally substituted (C₆-C₁₄)-aryl-CO, (C₆-C₁₄)-aryl-(C₁-C₈)-alkyl-CO optionally substituted in the aryl radical, optionally substituted heteroaryl-CO, heteroaryl-(C₁-C₈)-alkyl-CO optionally substituted in the heteroaryl radical, (C₁-C₈)-alkyl-S(O)ₙ, (C₃-C₁₂)-cycloalkyl-S(O)ₙ, (C₃-C₁₂)-cycloalkyl-(C₁-C₈)-alkyl-S(O)ₙ, (C₆-C₁₂)-bicycloalkyl-S(O)ₙ, (C₆-C₁₂)-bicycloalkyl-(C₁-C₈)-alkyl-S(O)ₙ, (C₆-C₁₂)-tricycloalkyl-S(O)ₙ, (C₆-C₁₂)-tricycloalkyl-(C₁-C₈)-alkyl-S(O)ₙ, optionally substituted (C₆-C₁₄)-aryl-S(O)ₙ, (C₆-C₁₄)-aryl-(C₁-C₈)-alkyl-S(O)ₙ optionally substituted in the aryl radical, optionally substituted heteroaryl-S(O)ₙ or heteroaryl-(C₁-C₈)-alkyl-S(O)ₙ, optionally substituted in the heteroaryl radical, where n is 1 or 2;
R¹ is x-NH-C (=NH)-(CH₂)ₚ or X¹-NH-(CH₂)ₚ where p is 0, 1, 2 or 3;
X is hydrogen, (C₁-C₆)-alkyl, (C₁-C₆)-alkylcarbonyl, (C₁-C₆)-alkoxycarbonyl, (C₁-C₁₈)-alkylcarbonyloxy-(C₁-C₆)-alkoxycarbonyl, optionally substituted (C₆-C₁₄)-arylcarbonyl, optionally substituted (C₆-C₁₄)-aryloxycarbonyl, (C₆-C₁₄)-aryl-(C₁-C₆)-alkoxycarbonyl which can also be substituted in the aryl radical, cyano, hydroxyl, (C₁-C₆)-alkoxy, (C₆-C₁₄)-aryl-(C₁-C₆)-alkoxy which can also be substituted in the aryl radical, or amino;
X¹ has one of the meanings of X or is R'-NH-C(=N-R''), where R' and R" independently of one another have the meanings of X;
R² is hydrogen or (C₁-C₈)-alkyl;
R³ is hydrogen, (C₁-C₈)-alkyl, optionally substituted (C₆-C₁₄)-aryl, (C₆-C₁₄)-aryl-(C₁-C₈)-alkyl optionally substituted in the aryl radical, (C₃-C₈)-cycloalkyl, (C₂-C₈)-alkenyl, (C₂-C₈)-alkynyl, (C₂-C₈)-alkenylcarbonyl, (C₂-C₈)-alkynylcarbonyl, pyridyl, R¹¹NH, CON(CH₃)R¹⁴, CONHR¹⁴, CON(CH₃)R¹⁵ or CONHR¹⁵;
R⁵ is optionally substituted (C₆-C₁₄)-aryl, (C₆-C₁₄)-aryl-(C₁-C₈)-alkyl optionally substituted in the aryl radical, a mono- or bicyclic 5- to 12-membered heterocyclic ring which can be aromatic, partially hydrogenated or completely hydrogenated and which can contain one, two or three identical or different heteroatoms from the group consisting of nitrogen, oxygen and sulfur, or a radical R⁶CO-, where the aryl and, independently thereof, the heterocyclic radical, can be mono- or polysubstituted by identical or different radicals from the group consisting of (C₁-C₈)-alkyl, (C₁-C₈)-alkoxy, halogen, nitro, amino or trifluoromethyl;
R⁶ is a natural or unnatural amino acid, imino acid, optionally N-(C₁-C₈)-alkylated or N-((C₆-C₁₄)-aryl-(C₁-C₈)-alkylated) azaamino acid or a dipeptide radical which can also be substituted in the aryl radical, and their esters and amides, where free functional groups can be protected by protective groups customary in peptide chemistry;
R¹⁰ is hydroxyl, (C₁-C₁₈)-alkoxy, (C₆-C₁₄)-aryl-(C₁-C₈)-alkoxy which can also be substituted in the aryl radical, optionally substituted (C₆-C₁₄)-aryloxy, amino or mono- or di-((C₁-C₁₈)-alkyl)amino;
R¹¹ is R¹²CO, optionally substituted (C₆-C₁₄)-aryl-S(O)₂ or (C₁-C₁₈)-alkyl-S (O)₂;
R¹² is hydrogen, (C₁-C₁₈) (C₂-C₈)-alkenyl, (C₂-C₈)-alkynyl, optionally substituted (C₆-C₁₄)-aryl, (C₁-C₁₈)-alkoxy, (C₆-C₁₄)-aryl-(C₁-C₈)-alkoxy which can also be substituted in the aryl radical or optionally substituted (C₆-C₁₄)-aryloxy;
R¹³ is hydrogen or (C₁-C₄)-alkyl;
R¹⁴ is(C₁-C₁₀)-alkyl which can optionally be mono-or polysubstituted by identical or different radicals from the group consisting of hydroxyl, hydroxycarbonyl, aminocarbonyl, mono- or di-((C₁-C₁₈)-alkyl) amino-carbonyl, (C₆-C₁₀)-aryl-(C₁-C₈)-alkoxycarbonyl which can also be substituted in the aryl radical, (C₁-C₈)-alkoxy, (C₁-C₈)-alkoxycarbonyl, optionally substituted (C₃-C₈)-cycloalkyl, tetrazolyl-(C₁-C₃)-alkyl, trifluoromethyl and R⁵;
R¹⁵ is R¹⁶-(C₁-C₆)-alkyl or R¹⁶;
R¹⁶ is a 6- to 24-membered bicyclic or tricyclic radical which is saturated or partially unsaturated and which can also contain one to four identical or different heteroatoms from the group consisting of nitrogen, oxygen and sulfur and which can also be substituted by one or more identical or different substituents from the group consisting of (C₁-C₄)-alkyl and oxo;
c and d are 1 and f is 0;
e and h independently of one another are 0 or 1 and g is 0;
in all its stereoisomeric forms and mixtures thereof in all ratios, or its physiologically tolerable salts.

28. A compound of the formula Id as claimed in claim 26 and/or 27, in which the radical by which the group B is substituted is a (C₁-C₈)-alkyl radical, in all its stereoisomeric forms and mixtures thereof in all ratios, or its physiologically tolerable salts.

29. A compound of the formula Id as claimed in one or more of claims 26 to 28 and/or its physiologically tolerable salts for use as a pharmaceutical.

30. A pharmaceutical preparation which comprises one or more compounds of the formula Id as claimed in one or more of claims 26 to 28 and/or their physiologically tolerable salts in addition to pharmaceutically innocuous excipients and/or additives.

31. A compound of the formula Ie 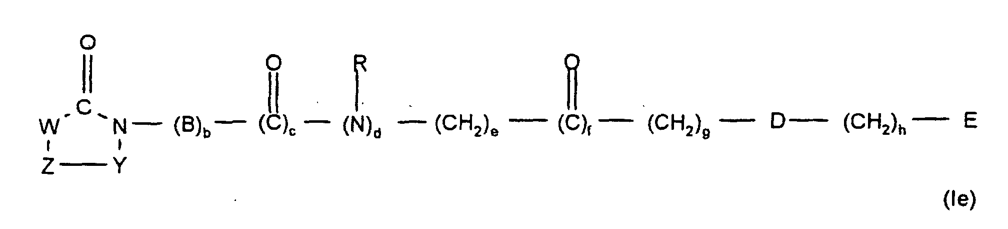 in which
W is R¹-A-C(R¹³) or R¹-A-CH=C;
Y is a carbonyl, thiocarbonyl or methylene group;
Z is N(R⁰), oxygen, sulfur or a methylene group;
A is a bivalent radical from the group consisting of (C₁-C₆)-alkylene, (C₃-C₇)-cycloalkylene, phenylene, phenylene-(C₁-C₆)-alkyl, (C₁-C₆)-alkylenephenyl, phenylene-(C₂-C₆)-alkenyl or a bivalent radical of a 5- or 6-membered saturated or unsaturated ring which can contain 1 or 2 nitrogen atoms and can be mono- or disubstituted by (C₁-C₆)-alkyl or doubly bonded oxygen or sulfur;
B is a bivalent radical from the group consisting of (C₁-C₆)-alkylene, (C₂-C₆)-alkenylene, phenylene, phenylene-(C₁-C₃)-alkyl, (C₁-C₃)-alkylenephenyl;
D is C(R²)(R³), N(R³) or CH=C(R³) ;
E is tetrazolyl, (R⁸O) ₂P (O) , HOS(O) ₂, R⁹NHS(O)₂ or R¹⁰CO;
R is hydrogen, (C₁-C₈)-alkyl, (C₃-C₈)-cycloalkyl, optionally substituted (C₆-C₁₄)-aryl or (C₆-C₁₄)-aryl-(C₁-C₈)-alkyl optionally substituted in the aryl radical;
R⁰ is CHO, (C₁-C₈)-alkyl-CO, (C₃-C₁₂)-cycloalkyl-CO, (C₃-C₁₂)-cycloalkyl-(C₁-C₈)-alkyl-CO, (C₆-C₁₂)-bicycloalkyl-CO, (C₆-C₁₂)-bicycloalkyl-(C₁-C₈)-alkyl-CO, (C₆-C₁₂)-tricycloalkyl-CO, (C₆-C₁₂)-tricycloalkyl-(C₁-C₈)-alkyl-CO, optionally substituted (C₆-C₁₄)-aryl-CO, (C₆-C₁₄)-aryl-C₈)-alkyl-CO, optionally substituted in the aryl radical, optionally substituted heteroaryl-CO, heteroaryl-(C₁-C₈)-alkyl-CO optionally substituted in the heteroaryl radical, (C₁-C₈)-alkyl-S (O)ₙ, (C₃-C₁₂)-cycloalkyl-S (O)ₙ, (C₃-C₁₂)-cycloalkyl-(C₁-C₈)-alkyl-S(O)ₙ, (C₆-C₁₂)-bicycloalkyl-S (O)ₙ, (C₆-C₁₂)-bicycloalkyl-(C₁-C₈)-alkyl-S (O)ₙ, (C₆-C₁₂)-tricycloalkyl-S (O)ₙ, (C₆-C₁₂)-tricycloalkyl-(C₁-C₈)-alkyl-S (O)ₙ, optionally substituted (C₆-C₁₄)-aryl-S (O)ₙ, (C₆-C₁₄)-aryl-(C₁-C₈)-alkyl-S (O)ₙ optionally substituted in the aryl radical, optionally substituted heteroaryl-S(O)ₙ or heteroaryl-(C₁-C₈)-alkyl-S(O)ₙ optionally substituted in the heteroaryl radical, where n is 1 or 2;
R¹ is X-NH-C (=NH)-(CH₂)ₚ or X¹-NH-(CH₂)ₚ, where p is 0, 1, 2 or 3;
X is hydrogen (C₁-C₆)-alkyl, (C₁-C₆)-alkylcarbonyl, (C₁-C₆)-alkoxycarbonyl, (C₁-C₁₈)-alkylcarbonyloxy-(C₁-C₆)-alkoxycarbonyl, optionally substituted (C₆-C₁₄)-arylcarbonyl, optionally substituted (C₆-C₁₄)-aryloxycarbonyl, (C₆-C₁₄)-aryl-(C₁-C₆)-alkoxycarbonyl which can also be substituted in the aryl radical, (R⁸O)₂P(O), cyano, hydroxyl, (C₁-C₆)-alkoxy, (C₆-C₁₄)-aryl-(C₁-C₆)-alkoxy which can also be substituted in the aryl radical, or amino;
X¹ has one of the meanings of X or is R'-NH-C(=N-R''), where R' and R " independently of one another have the meanings of X;
R² is hydrogen, (C₁-C₈)-alkyl, optionally substituted (C₆-C₁₄)-aryl, (C₆-C₁₄)-aryl-(C₁-C₈)-alkyl optionally substituted in the aryl radical or (C₃-C₈)-cycloalkyl;
R³ is hydrogen, (C₁-C₈)-alkyl, optionally substituted (C₆-C₁₄)-aryl, (C₆-C₁₄)-aryl-(C₁-C₈)-alkyl optionally substituted in the aryl radical, (C₃-C₈)-cycloalkyl, (C₂-C₈)-alkenyl, (C₂-C₈)-alkynyl, (C₂-C₈)-alkenylcarbonyl, (C₂-C₈)-alkynylcarbonyl, pyridyl, R¹¹NH, R⁴CO, COOR⁴, CON (CH₃) R¹⁴, CONHR¹⁴, CSNHR¹⁴, COOR¹⁵, CON (CH₃) R¹⁵ or CONHR¹⁵;
R⁴ is hydrogen or (C₁-C₂₈)-alkyl which can optionally be mono- or polysubstituted by identical or different radicals R^{4'};
R^{4'} is hydroxyl, hydroxycarbonyl, aminocarbonyl, mono- or di-((C₁-C₁₈)-alkyl) aminocarbonyl, amino-(C₂-C₁₈)-alkylaminocarbonyl, amino-(C₁-C₃)-alkylphenyl-(C₁-C₃)-alkylaminocarbonyl, (C₁-C₁₈)-alkylcarbonylamino-(C₁-C₃)-alkylphenyl-(C₁-C₃)-alkylaminocarbonyl, (C₁-C₁₈)-alkylcarbonylamino-(C₂-C₁₈)-alkylaminocarbonyl, (C₆-C₁₄)-aryl-(C₁-C₈)-alkoxycarbonyl which can also be substituted in the aryl radical, amino, mercapto, (C₁-C₁₈)-alkoxy, (C₁-C₁₈)-alkoxycarbonyl, optionally substituted (C₃-C₈)-cycloalkyl, halogen, nitro, trifluoromethyl or the radical R⁵;
R⁵ is optionally substituted (C₆-C₁₄)-aryl, (C₆-C₁₄)-aryl-(C₁-C₈)-alkyl optionally substituted in the aryl radical, a mono- or bicyclic 5- to 12-membered heterocyclic ring which can be aromatic, partially hydrogenated or completely hydrogenated and which can contain one, two or three identical or different heteroatoms from the group consisting of nitrogen, oxygen and sulfur, a radical R⁶ or a radical R⁶CO-, where the aryl and, independently thereof, the heterocyclic radical can be mono- or polysubstituted by identical or different radicals from the group consisting of (C₁-C₁₈)-alkyl, (C₁-C₁₈)-alkoxy, halogen, nitro, amino or trifluoromethyl;
R⁶ is R⁷R⁸N, R⁷O or R⁷S or an amino acid side chain, a natural or unnatural amino acid, imino acid, optionally N-(C₁-C₈)-alkylated or N-((C₆-C₁₄)-aryl-(C₁-C₈)-alkylated) azaamino acid or a dipeptide radical which can also be substituted in the aryl radical and/or in which the peptide bond can be reduced to -NH-CH₂- , and their esters and amides, where instead of free functional groups there can optionally be hydrogen or hydroxymethyl and/or where free functional groups can be protected by protective groups customary in peptide chemistry;
R⁷ is hydrogen, (C₁-C₁₈)-alkyl, (C₆-C₁₄)-aryl-(C₁-C₈)-alkyl, (C₁-C₁₈)-alkyl carbonyl, (C₁-C₁₈)-alkoxycarbonyl, (C₆-C₁₄)-arylcarbonyl, (C₆-C₁₄)-aryl-(C₁-C₈)-alkylcarbonyl or (C₆-C₁₄)-aryl-(C₁-C₁₈)- alkyloxycarbonyl, where the alkyl groups can optionally be substituted by an amino group and/or where the aryl radicals can be mono- or polysubstituted, preferably monosubstituted, by identical or different radicals from the group consisting of (C₁-C₈)-alkyl, (C₁-C₈)-alkoxy, halogen, vitro, amino and trifluoromethyl, a natural or unnatural amino acid, imino acid, optionally N-(C₁-C₈)-alkylated or N-((C₆-C₁₄)-aryl-(C₁-C₈)-alkylated) azaamino acid or a dipeptide radical which can also be substituted in the aryl radical and/or in which the peptide bond can be reduced to -NH-CH₂-;
R⁸ is hydrogen, (C₁-C₁₈)-alkyl, optionally substituted (C₆-C₁₄)-aryl or (C₆-C₁₄)-aryl-(C₁-C₈)-alkyl which can also be substituted in the aryl radical;
R⁹ is hydrogen, aminocarbonyl, (C₁-C₁₈)-alkylaminocarbonyl, (C₃-C₈)-cycloalkylaminocarbonyl, optionally substituted (C₆-C₁₄)-arylaminocarbonyl, (C₁-C₁₈)-alkyl, optionally substituted (C₆-C₁₄)-aryl or (C₃-C₈)-cycloalkyl;
R¹⁰ is hydroxyl, (C₁-C₁₈)-alkoxy, (C₆-C₁₄)-aryl-(C₁-C₈)-alkoxy which can also be substituted in the aryl radical, optionally substituted (C₆-C₁₄)-aryloxy, amino or mono- or di-((C₁-C₁₈)-alkyl)amino;
R¹¹ is hydrogen, (C₁-C₁₈)-alkyl, R¹²CO, optionally substituted (C₆-C₁₄)-aryl-S (O) ₂, (C₁-C₁₈)-alkyl-S (O) ₂, (C₆-C₁₄)-aryl-(C₁-C₈)-alkyl optionally substituted in the aryl radical or R⁹NHS(O) ₂;
R¹² is hydrogen, (C₁-C₁₈)-alkyl, (C₂-C₈)-alkenyl, (C₂-C₈)-alkynyl, optionally substituted (C₆-C₁₄)-aryl, (C₁-C₁₈)-alkoxy, (C₆-C₁₄)-aryl-(C₁-C₈)-alkoxy which can also be substituted in the aryl radical, optionally substituted (C₆-C₁₄)-aryloxy, amino or mono- or di-((C₁-C₁₈)-alkyl) amino;
R¹³ is hydrogen, (C₁-C₆)-alkyl, (C₆-C₁₄)-aryl-(C₁-C₈)-alkyl optionally substituted in the aryl radical or (C₃-C₈)-cycloalkyl;
R¹⁴ is hydrogen or (C₁-C₂₈)-alkyl which can optionally be mono- or polysubstituted by identical or different radicals from the group consisting of hydroxyl, hydroxycarbonyl, aminocarbonyl, mono- or di-((C₁-C₁₈)-alkyl)-aminocarbonyl, amino-(C₂-C₁₈)-alkylamino-carbonyl, amino-(C₁-C₃)-alkylphenyl-(C₁-C₃)-alkylaminocarbonyl, (C₁-C₁₈)-alkylcarbonylamino-(C₁-C₃)-alkylphenyl-(C₁-C₃)-alkylaminocarbonyl, (C₁-C₁₈)-alkylcarbonylamino-(C₂-C₁₈)-alkylamino-carbonyl, (C₆-C₁₄)-aryl-(C₁-C₈)-alkoxycarbonyl which can also be substituted in the aryl radical, amino, mercapto, (C₁-C₁₈)-alkoxy, (C₁-C₁₈)-alkoxycarbonyl, optionally substituted (C₃-C₈)-cycloalkyl, HOS (O)₂-(C₁-C₃)-alkyl, R⁹NHS (O)₂-(C₁-C₃)-alkyl, (R⁸O)₂P (O)-(C₁-C₃)-alkyl, tetrazolyl-(C₁-C₃)-alkyl, halogen, nitro, trifluoromethyl and R⁵;
R¹⁵ is R¹⁶-(C₁-C₆)-alkyl or R¹⁶;
R¹⁶ is a 6- to 24-membered bicyclic or tricyclic radical which is saturated or partially unsaturated and which can also contain one to four identical or different heteroatoms from the group consisting of nitrogen, oxygen and sulfur and which can also be substituted by one or more identical or different substituents from the group consisting of (C₁-C₄)-alkyl and oxo;
b, c, d and f independently of one another are 0 or 1, but cannot all simultaneously be 0;
e, g and h independently of one another are 0, 1, 2, 3, 4, 5 or 6;
in all its stereoisomeric forms and mixtures thereof in all ratios, or its physiologically tolerable salts.

32. A compound of the formula Ie as claimed in claim 31 and/or its physiologically tolerable salts for use as a pharmaceutical.

33. A pharmaceutical preparation which comprises one or more compounds of the formula Ie as claimed in claim 31 and/or their physiologically tolerable salts in addition to pharmaceutically innocuous excipients and/or additives.

## Revendications

1. Utilisation de composés de formule I 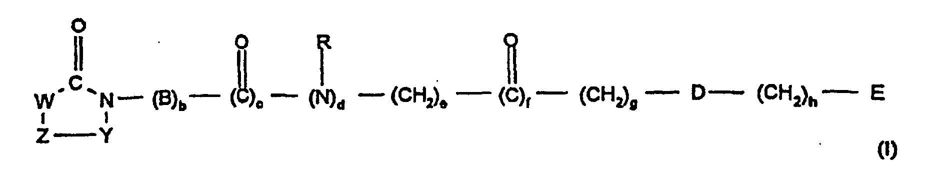 dans laquelle
W représente un groupe R'-A-C(R¹³) ou R¹-A-CH=C;
Y représente un groupe carbonyle, thiocarbonyle ou méthylène;
Z représente N(R⁰),l'oxygène, le soufre ou un groupe méthylène;
A représente un reste divalent de la série des restes alkylène en C₁-C₆, cycloalkylène en C₃-C₇, phénylène, phénylène-(alkyle en C₁-C₆), (alkylène en C₁-C₆)-phényle et phénylène-(alcényle en C₂-C₆), ou un reste divalent d'un cycle saturé ou insaturé de 5 ou 6 chaînons pouvant contenir 1 ou 2 atomes d'azote et être substitué une où deux fois par alkyle en C₁-C₆ ou par de l'oxygène ou du soufre doublement lié;
B représente un reste divalent de la série des restes alkylène en C₁-C₆, alcénylène en C₂-C₆, phénylène, phénylène-(alkyle en C₁-C₃) et (alkylène en C₁-C₃)-phényle, le reste alkylène en C₁-C₆ divalent pouvant être non substitué ou substitué par un reste de la série des restes alkyle en C₁-C₈, alcényle en C₂-C₈, alcynyle en C₂-C₈, cycloalkyle en C₃-C₁₀, (cycloalkyl en C₃-C₁₀)-(alkyle en C₁-C₆), aryle en C₆-C₁₄ éventuellement substitué, (aryl en C₆-C₁₄)-(alkyle en C₁-C₆) éventuellement substitué sur le reste aryle, hétéroaryle éventuellement substitué et hétéroaryl-(alkyle en C₁-C₆) éventuellement substitué sur le reste hétéroaryle;
D représente un groupe C(R²)(R³)_{,} N(R³) ou CH=C(R³);
E représente un groupe tétrazolyle, (R⁸O)₂P(O), HOS(O)₂, R⁹NHS(O)₂ ou R¹⁰CO;
R représente un atome d'hydrogène ou un reste alkyle en C₁-C₈, cycloalkyle en C₃-C₈, aryle en C₆-C₁₄ éventuellement substitué ou (aryl en C₆-C₁₄)-(alkyle en C₁-C₈) éventuellement substitué sur le reste aryle;
R⁰ représente un atome d'hydrogène ou un reste alkyle en C₁-C₈, cycloalkyle en C₃-C₁₂, (cycloalkyl en C₃-C₁₂)-(alkyle en C₁-C₈), bicycloalkyle en C₆-C₁₂ (bicycloalkyl en C₆-C₁₂)-(alkyle en C₁-C₈), tricycloalkyle en C₆-C₁₂, (tricycloalkyl en C₆-C₁₂)-(alkyle en C₁-C₈), aryle en C₆-C₁₄ éventuellement substitué, (aryl en C₆-C₁₄)-(alkyle en C₁-C₈) éventuellement substitué sur le reste aryle, hétéroaryle éventuellement substitué, hétéroaryl-(alkyle en C₁-C₈) éventuellement substitué sur le reste hétéroaryle, CHO, (alkyl en C₁-C₈)-CO_{,} (cyclaalkyl en C₃-C₁₂)-CO, (cycloalkyl en C₃-C₁₂)-(alkyl en C₁-C₈)-CO, (bicycloalkyl en C₆-C₁₂)-CO, (bicycloalkyl en C₆-C₁₂)-(alkyl en C₁-C₈)-CO, (tricycloalkyl en C₆-C₁₂)-CO, (tricycloalkyl en C₆-C₁₂)-(alkyl en C₁-C₈)-CO, (aryl en C₆-C₁₄)-CO éventuellement substitué, (aryl en C₆-C₁₄)-(alkyl en C₁-C₈)-CO éventuellement substitué sur le reste aryle, hétéroaryl-CO éventuellement substitué, hétéroaryl-(alkyl en C₁-C₈)-CO éventuellement substitué sur le reste hétéroaryle, (alkyl en C₁-C₈)-S(O)ₙ, (cycloalkyl en C₃-C₁₂)-S(O)ₙ, (cycloalkyl en C₃-C₁₂)-(alkyl en C₁-C₈)-S(O)ₙ, (bicycloalkyl en C₆-C₁₂)-S(O)ₙ, (bicycloalkyl en C₆-C₁₂)-(alkyl en C₁-C₈)-S(O)ₙ, (tricycloalkyl en C₆-C₁₂)-S(O)ₙ, (tricycloalkyl en C₆-C₁₂)-(alkyl en C₁-C₈)-S(O)ₙ, (aryl en C₆-C₁₄)-S(O)ₙ éventuellement substitué, (aryl en C₆-C₁₄)-(alkyl en C₁-C₈)-S(O)ₙ éventuellement substitué sur le reste aryle, hétéroaryl-S(O)ₙ éventuellement substitué ou hétéroaryl-(alkyl en C₁-C₈)-S(O)ₙ éventuellement substitué sur le reste hétéroaryle, n étant égal 1 ou 2;
R¹ représente un groupe X-NH-C(=NH)-(CH₂)ₚ ou X¹-NH-(CH₂)ₚ, où p est 0, 1, 2 ou 3;
X représente un atome d'hydrogène ou un reste alkyle en C₁-C₆, (alkyl en C₁-C₆)carbonyle, (alcoxy en C₁-C₆)carbonyle, (alkyl en C₁-C₁₈)carbonyloxy-(alcoxy en C₁-C₆)carbonyle, (aryl en C₆-C₁₄)carbonyle éventuellement substitué, (aryloxy en C₆-C₁₄)carbonyle éventuellement substitué, (aryl en C₆-C₁₄)-(alcoxy en C₁-C₆)carbonyle pouvant aussi être substitué sur le reste aryle, (R⁸O)₂P(O), cyano, hydroxy, alcoxy en C₁-C₆, (aryl en C₆-C₁₄)-(alcoxy en C₁-C₆) pouvant aussi être substitué sur le reste aryle, ou amino;
X¹ a l'une des significations de X ou représente un groupe R'-NH-C(=N-R"), dans lequel R' et R" ont indépendamment l'un de l'autre les significations de X;
R² représente un atome d'hydrogène ou un reste alkyle en C₁-C₈, aryle en C₆-C₁₄ éventuellement substitué, (aryl en C₆-C₁₄)-(alkyle en C₁-C₈) éventuellement substitué sur le reste aryle, ou cycloalkyle en C₃-C₈;
R³ représente un atome d'hydrogène ou un reste alkyle en C₁-C₈, aryle en C₆-C₁₄ éventuellement substitué, (aryl en C₆-C₁₄)-(alkyle en C₁-C₈) éventuellement substitué sur le reste aryle, cycloalkyle en C₃-C₈, alcényle en C₂-C₈, alcynyle en C₂-C₈, (alcényl en C₂-C₈)carbonyle, (alcynyl en C₂-C₈)carbonyle, pyridyle, R¹¹NH, R⁴CO, COOR⁴, CON(CH₃)R¹⁴, CONHR¹⁴, CSNHR¹⁴_{,} COOR¹⁵, CON(CH₃)R¹⁵ ou CONHR¹⁵;
R⁴ représente un atome d'hydrogène ou un reste alkyle en C₁-C₂₈ pouvant éventuellement être substitué une ou plusieurs fois par des restes R^{4'} identiques ou différents;
R^{4'} représente un reste hydroxy, hydroxycarbonyle, aminocarbonyle, mono- ou di(alkyl en C₁-C₁₈)aminocarbonyle, amino-(alkyl en C₂-C₁₈)aminocarbonyle, amino(alkyl en C₁-C₃)phényl-(alkyl en C₁-C₃)aminocarbonyle, (alkyl en C₁-C₁₈)carbonylamino(alkyl en C₁-C₃)phényl-(alkyl en C₁-C₃)aminocarbonyle, (alkyl en C₁-C₁₈)carbonylamino-(alkyl en C₂-C₁₈)aminocarbonyle, (aryl en C₆-C₁₄)-(alcoxy en C₁-C₈)carbonyle pouvant aussi être substitué sur le reste aryle, amino, mercapto, alcoxy en C₁-C₁₈, (alcoxy en C₁-C₁₈)carbonyle, cycloalkyle en C₃-C₈ éventuellement substitué, halogéno, nitro, trifluorométhyle ou le reste R⁵,
R⁵ représente un reste aryle en C₆-C₁₄ éventuellement substitué ou (aryl en C₆-C₁₄)-(alkyle en C₁-C₈) éventuellement substitué sur le reste aryle, un cycle hétérocyclique mono- ou bicyclique de 5 à 12 chaînons pouvant être aromatique, partiellement hydrogéné ou entièrement hydrogéné et pouvant contenir un, deux ou trois hétéroatomes identiques ou différents de la série de l'azote, de l'oxygène et du soufre, un reste R⁶ ou un reste R⁶-CO, le reste aryle et le reste hétérocyclique pouvant être indépendamment substitués une ou plusieurs fois par des restes identiques ou différents de la série des restes alkyle en C₁-C₁₈, alcoxy en C₁-C₁₈, halogéno, nitro, amine ou trifluorométhyle;
R⁶ représente un groupe R⁷R⁸N, R⁷O ou R⁷S ou une chaîne latérale d'aminoacide, un reste d'aminoacide naturel ou synthétique, d'iminoacide, d'aza-aminoacide éventuellement N-substitué par un reste alkyle en C₁-C₈ ou N-substitué par un reste (aryl en C₆-C₁₄)-(alkyle en C₁-C₈) ou de dipeptide, qui peut aussi être substitué sur le reste aryle et/ou dont la liaison peptidique peut être réduite en - NH-CH₂-, et leurs esters et amides, et où, à la place de groupes fonctionnels libres, il peut éventuellement y avoir de l'hydrogène ou un groupe hydroxyméthyle et/ou des groupes fonctionnels libres peuvent être protégés par des groupes protecteurs classiques dans la chimie des peptides;
R⁷ représente un atome d'hydrogène, un reste alkyle en C₁-C₁₈, (aryl en C₆-C₁₄)-(alkyle en C₁-C₈), (alkyl en C₁-C₁₈)carbonyle, (alcoxy en C₁-C₁₈)carbonyle, (aryl en C₆-C₁₄)carbonyle, (aryl en C₆-C₁₄)-(alkyl en C₁-C₈)carbonyle ou (aryl en C₆-C₁₄)-(alkyl en C₁-C₁₈)oxycarbonyle, les groupes alkyle pouvant éventuellement être substitués par un groupe amino et/ou les restes aryle pouvant être substitués une ou plusieurs fois, de préférence une fois, par des restes identiques ou différents de la série des restes alkyle en C₁-C₈, alcoxy en C₁-C₈, halogéno, nitro, amino et trifluorométhyle, ou un reste d'aminoacïde naturel ou synthétique, d'iminoacide, d'aza-aminoacide éventuellement N-substitué par un reste alkyle en C₁-C₈ ou N-substitué par un reste (aryl en C₆-C₁₄)-(alkyle en C₁-C₈) ou de dipeptide, qui peut aussi être substitué sur le reste aryle et/ou dont la liaison peptidique peut être réduite en -NH-CH₂-;
R⁸ représente un atome d'hydrogène ou un reste alkyle en C₁-C₁₈, aryle en C₆-C₁₄ éventuellement substitué ou (aryl en C₆-C₁₄)-(alkyle en C₁-C₈) pouvant aussi être substitué sur le reste aryle;
R⁹ représente un atome d'hydrogène ou un reste aminocarbonyle, (alkyl en C₁-C₁₈)aminocarbonyle, (cycloalkyl en C₃-C₈)aminocarbonyle, (aryl en C₆-C₁₄)aminocarbonyle éventuellement substitué, alkyle en C₁-C₁₈, aryle en C₆-C₁₄ éventuellement substitué ou cycloalkyle en C₃-C₈;
R¹⁰ représente un reste hydroxy, alcoxy en C₁-C₁₈, (aryl en C₆-C₁₄)-(alcoxy en C₁-C₈) pouvant aussi être substitué sur le reste aryle, aryloxy en C₆-C₁₄ éventuellement substitué, amino ou mono- ou di(alkyl en C₁-C₁₈)amino;
R¹¹ représente un atome d'hydrogène ou un reste alkyle en C₁-C₁₈, R¹²CO, aryl-S(O)₂ en C₆-C₁₄ éventuellement substitué, alkyl-S(O)₂ en C₁-C₁₈, (aryl en C₆-C₁₄)-(alkyle en C₁-C₈) éventuellement substitué sur le reste aryle, ou R⁹NHS(O)₂;
R¹² représente un atome d'hydrogène ou un reste alkyle en C₁-C₁₈, alcényle en C₂-C₈, alcynyle en C₂-C₈, aryle en C₆-C₁₄ éventuellement substitué, alcoxy en C₁-C₁₈, (aryl en C₆-C₁₄)-(alcoxy en C₁-C₈) pouvant aussi être substitué sur le reste aryle, aryloxy en C₆-C₁₄ éventuellement substitué, amino ou mono- ou di(alkyl en C₁-C₁₈)amino;
R¹³ représente un atome d'hydrogène ou un reste alkyle en C₁-C₆, (aryl en C₆-C₁₄)-(alkyle en C₁-C₈) éventuellement substitué sur le reste aryle ou cycloalkyle en C₃-C₈;
R¹⁴ représente un atome d'hydrogène ou un reste alkyle en C₁-C₂₈ éventuellement substitué une ou plusieurs fois par des restes identiques ou différents de la série des restes hydroxy, hydroxycarbonyle, aminocarbonyle, mono- ou di(alkyl en C₁-C₁₈)aminocarbonyle, amino-(alkyl en C₂-C₁₈)aminocarbonyle, amino(alkyl en C₁-C₃)phényl-(alkyl en C₁-C₃)aminocarbonyle, (alkyl en C₁-C₁₈)carbonyl-amino(alkyl en C₁-C₃)phényl-(alkyl en C₁-C₃)aminocarbonyle, (alkyl en C₁-C₁₈)carbonylamino-(alkyl en C₂-C₁₈)aminocarbonyle, (aryl en C₆-C₁₄)-(alcoxy en C₁-C₈)carbonyle pouvant aussi être substitué sur le reste aryle, amine, mercapto, alcoxy en C₁-C₁₈, (alcoxy en C₁-C₁₈)carbonyle, cycloalkyle en C₃-C₈ éventuellement substitué, HOS(O)₂-(alkyle en C₁-C₃), R⁹NHS(O)₂-(alkyle en C₁-C₃), (R⁸O)₂P(O)-(alkyle en C₁-C₃), tétrazolyl-(alkyle en C₁-C₃), halogéno, nitro, trifluorométhyle et R⁵;
R¹⁵ représente un reste R¹⁶-(alkyle en C₁-C₆) ou R¹⁶;
R¹⁶ représente un reste bicyclique ou tricyclique de 6 à 24 chaînons, saturé ou partiellement insaturé, pouvant aussi contenir un à quatre hétéroatomes identiques ou différents de la série de l'azote, de l'oxygène et du soufre et pouvant aussi être substitué par un ou plusieurs substituants identiques ou différents de la série des restes allyle en C₁-C₄ et oxo;
b, c, d et f représentent, indépendamment les uns des autres, 0 ou 1, mais ne peuvent pas tous être 0 en même temps;
e, g et h représentent indépendamment les uns des autres 0, 1, 2, 3, 4, 5 ou 6;
dans toutes leurs formes stéréo-isomères et leurs mélanges en toutes proportions, ainsi que de leurs sels pharmaceutiquement acceptables, pour la préparation de médicaments destinés à inhiber l'adhésion et/ou la migration de leucocytes ou à inhiber le récepteur de VLA-4.

2. Utilisation de composés de formule I selon la revendication 1 dans toutes leurs formes stéréo-isomères et leurs mélanges en toutes proportions, ainsi que de leurs sels physiologiquement acceptables, dans laquelle, dans la formule I,
W représente un groupe R¹-A-C(R¹³) ou R¹-A-CH=C;
Y représente un groupe carbonyle, thiocarbonyle ou méthylène;
Z représente N(R⁰), l'oxygène, le soufre ou un groupe méthylène;
A représente un reste divalent de la série des restes alkylène en C₁-C₆, cycloalkylène en C₃-C₇, phénylène, phénylène-(alkyle en C₁-C₆), (alkylène en C₁-C₆)-phényle et phénylène-(alcényle en C₂-C₆), ou un reste divalent d'un cycle saturé ou insaturé de 5 ou 6 chaînons pouvant contenir 1 ou 2 atomes d'azote et être substitué une ou deux fois par alkyle en C₁-C₆ ou par de l'oxygène ou du soufre doublement lié;
B représente un reste divalent de la série des restes alkylène en C₁-C₆, alcénylène en C₂-C₆, phénylène, phénylène-(alkyle en C₁-C₃) et (alkylène en C₁-C₃)-phényle;
D représente un groupe C(R²)(R³), N(R³) ou CH=C(R³);
E représente un groupe tétrazolyle, (R⁸O)₂P(O), HOS(O)₂, R⁹NHS(O)₂ ou R¹⁰CO;
R et R⁰ représentent indépendamment l'un de l'autre un atome d'hydrogène ou un reste alkyle en C₁-C₈, cycloalkyle en C₃-C₈, aryle en C₆-C₁₄ éventuellement substitué ou (aryl en C₆-C₁₄)-(alkyle en C₁-C₈) éventuellement substitué sur le reste aryle;
R¹ représente un groupe X-NH-C(=NH)-(CH₂)ₚ ou X¹-NH-(CH₂)ₚ, où p est 0, 1, 2 ou 3;
X représente un atome d'hydrogène ou un reste alkyle en C₁-C₆, (alkyl en C₁-C₆)carbonyle, (alcoxy en C₁-C₆)carbonyle, (alkyl en C₁-C₁₈)carbonyloxy-(alcoxy en C₁-C₆)carbonyle, (aryl en C₆-C₁₄)carbonyle éventuellement substitué, (aryloxy en C₆-C₁₄)carbonyle éventuellement substitué, (aryl en C₆-C₁₄)-(alcoxy en C₁-C₆)carbonyle pouvant aussi être substitué sur le reste aryle, (R⁸O)₂P(O), cyano, hydroxy, alcoxy en C₁-C₆, (aryl en C₆-C₁₄)-(alcoxy en C₁-C₆) pouvant aussi être substitué sur le reste aryle, ou amino;
X¹ a l'une des significations de X ou représente un groupe R'-NH-C(=N-R"), dans lequel R' et R" ont indépendamment l'un de l'autre les significations de X;
R² représente un atome d'hydrogène ou un reste alkyle en C₁-C₈, aryle en C₆-C₁₄ éventuellement substitué, (aryl en C₆-C₁₄)-(alkyle en C₁-C₈) éventuellement substitué sur le reste aryle, ou cycloalkyle en C₃-C₈;
R³ représente un atome d'hydrogène ou un reste alkyle en C₁-C₈, aryle en C₆-C₁₄ éventuellement substitué, (aryl en C₆-C₁₄)-(alkyle en C₁-C₈) éventuellement substitué sur le reste aryle, cycloalkyle en C₃-C₈, alcényle en C₂-C₈, alcynyle en C₂-C₈, (alcényl en C₂-C₈)carbonyle, (alcynyl en C₂-C₈)carbonyle, pyridyle, R¹¹NH, R⁴CO, COOR⁴, CON(CH₃)R¹⁴, CONHR¹⁴, CSNHR¹⁴, COOR¹⁵, CON(CH₃)R¹⁵ ou CONHR¹⁵;
R⁴ représente un atome d'hydrogène ou un reste alkyle en C₁-C₂₈ pouvant éventuellement être substitué une ou plusieurs fois par des restes R^{4'} identiques ou différents;
R^{4'} représente un reste hydroxy, hydroxycarbonyle, aminocarbonyle, mono- ou di(alkyl en C₁-C₁₈)aminocarbonyle, amino-(alkyl en C₂-C₁₈)aminocarbonyle, amino(alkyl en C₁-C₃)phényl-(alkyl en C₁-C₃)aminocarbonyle, (alkyl en C₁-C₁₈)carbonylamino(alkyl en C₁-C₃)phényl-(alkyl en C₁-C₃)aminocarbonyle, (alkyl en C₁-C₁₈)carbonylamino-(alkyl en C₂-C₁₈)aminocarbonyle, (aryl en C₆-C₁₄)-(alcoxy en C₁-C₈)carbonyle pouvant aussi être substitué sur le reste aryle, amino, mercapto, alcoxy en C₁-C₁₈, (alcoxy en C₁-C₁₈)carbonyle, cycloalkyle en C₃-C₈ éventuellement substitué, halogéno, nitro, trifluorométhyle ou le reste R⁵,
R⁵ représente un reste aryle en C₆-C₁₄ éventuellement substitué ou (aryl en C₆-C₁₄)-(alkyle en C₁-C₈) éventuellement substitué sur le reste aryle, un cycle hétérocyclique mono- ou bicyclique de 5 à 12 chaînons pouvant être aromatique, partiellement hydrogéné ou entièrement hydrogéné et pouvant contenir un, deux ou trois hétéroatomes identiques ou différents de la série de l'azote, de l'oxygène et du soufre, un reste R⁶ ou un reste R⁶-CO, le reste aryle et le reste hétérocyclique pouvant être indépendamment substitués une ou plusieurs fois par des restes identiques ou différents de la série des restes alkyle en C₁-C₁₈, alcoxy en C₁-C₁₈, halogéno, nitro, amino ou trifluorométhyle;
R⁶ représente un groupe R⁷R⁸N, R⁷O ou R⁷S ou une chaîne latérale d'aminoacide, un reste d'aminoacide naturel ou synthétique; d'iminoacide, d'aza-aminoacide éventuellement N-substitué par un reste alkyle en C₁-C₈ ou N-substitué par un reste (aryl en C₆-C₁₄)-(alkyle en C₁-C₈) ou de dipeptide, qui peut aussi être substitué sur le reste aryle et/ou dont la liaison peptidique peut être réduite en - NH-CH₂-, et leurs esters et amides, et où, à la place de groupes fonctionnels libres, il peut éventuellement y avoir de l'hydrogène ou un groupe hydroxyméthyle et/ou des groupes fonctionnels libres peuvent être protégés par des groupes protecteurs classiques dans la chimie des peptides;
R⁷ représente un atome d'hydrogène, un reste allyle en C₁-C₁₈, (aryl en C₆-C₁₄)-(alkyle en C₁-C₈), (alkyl en C₁-C₁₈)carbonyle, (alcoxy en C₁-C₁₈)carbonyle, (aryl en C₆-C₁₄)carbonyle, (aryl en C₆-C₁₄)-(alkyl en C₁-C₈)carbonyle ou (aryl en C₆-C₁₄)-(alkyl en C₁-C₁₈)oxycarbonyle, les groupes alkyle pouvant éventuellement être substitués par un groupe amino et/ou les restes aryle pouvant être substitués une ou plusieurs fois, de préférence une fois, par des restes identiques ou différents de la série des restes allyle en C₁-C₈, alcoxy en C₁-C₈, halogéno, nitro, amino et trifluorométhyle, ou un reste d'aminoacide naturel ou synthétique, d'iminoacide, d'aza-aminoacide éventuellement N-substitué par un reste alkyle en C₁-C₈ ou N-substitué par un reste (aryl en C₆-C₁₄)-(alkyle en C₁-C₈) ou de dipeptide, qui peut aussi être substitué sur Ie reste aryle et/ou dont la liaison peptidique peut être réduite en NH-CH₂-;
R⁸ représente un atome d'hydrogène ou un reste allyle en C₁-C₁₈, aryle en C₆-C₁₄ éventuellement substitué ou (aryl en C₆-C₁₄)-(alkyle en C₁-C₈) pouvant aussi être substitué sur le reste aryle;
R⁹ représente un atome d'hydrogène ou un reste aminocarbonyle, (alkyl en C₁-C₁₈)aminocarbonyle, (cycloalkyl en C₃-C₈)aminocarbonyle, (aryl en C₆-C₁₄)aminocarbonyle éventuellement substitué, alkyle en C₁-C₁₈, aryle en C₆-C₁₄ éventuellement substitué ou cycloalkyle en C₃-C₈;
R¹⁰ représente un reste hydroxy, alcoxy en C₁-C₁₈, (aryl en C₆-C₁₄)-(alcoxy en C₁-C₈) pouvant aussi être substitué sur le reste aryle, aryloxy en C₆-C₁₄ éventuellement substitué, amino ou mono- ou di(alkyl en C₁-C₁₈)amino;
R¹¹ représente un atome d'hydrogène ou un reste alkyle en C₁-C₁₈, R¹²CO, aryl-S(O)₂ en C₆-C₁₄ éventuellement substitué, alkyl-S(O)₂ en C₁-C₁₈, (aryl en C₆-C₁₄)-(alkyle en C₁-C₈) éventuellement substitué sur le reste aryle, ou R⁹NHS(O)₂;
R¹² représente un atome d'hydrogène ou un reste alkyle en C₁-C₁₈, alcényle en C₂-C₈, alcynyle en C₂-C₈, aryle en C₆-C₁₄ éventuellement substitué, alcoxy en C₁-C₁₈, (aryl en C₆-C₁₄)-(alcoxy en C₁-C₈) pouvant aussi être substitué sur le reste aryle, aryloxy en C₆-C₁₄ éventuellement substitué, amino ou mono- ou di(alkyl en C₁-C₁₈)amino;
R¹³ représente un atome d'hydrogène ou un reste alkyle en C₁-C₆, (aryl en C₆-C₁₄)-(alkyle en C₁-C₈) éventuellement substitué sur le reste aryle ou cycloalkyle en C₃-C₈;
R¹⁴ représente un atome d'hydrogène ou un reste alkyle en C₁-C₂₈ éventuellement substitué une ou plusieurs fois par des restes identiques ou différents de la série des restes hydroxy, hydroxycarbonyle, aminocarbonyle, mono- ou di(alkyl en C₁-C₁₈)aminocarbonyle, amino-(alkyl en C₂-C₁₈)aminocarbonyle, amino(alkyl en C₁-C₃)phényl-(alkyl en C₁-C₃)aminocarbonyle, (alkyl en C₁-C₁₈)carbonylamino(alkyl en C₁-C₃)phényl-(alkyl en C₁-C₃)aminocarbonyle, (alkyl en C₁-C₁₈)carbonylamino-(alkyl en C₂-C₁₈)aminocarbonyle, (aryl en C₆-C₁₄)-(alcoxy en C₁-C₈)carbonyle pouvant aussi être substitué sur le reste aryle, amino, mercapto, alcoxy en C₁-C₁₈, (alcoxy en C₁-C₁₈)carbonyle, cycloalkyle en C₃-C₈ éventuellement substitué, HOS(O)₂-(alkyle en C₁-C₃), R⁹NHS(O)₂-(alkyle en C₁-C₃), (R⁸O)₂P(O)-(alkyle en C₁-C₃), tétrazolyl-(alkyle en C₁-C₃), halogéno, nitro, trifluorométhyle et R⁵;
R¹⁵ représente un reste R¹⁶-(alkyle en C₁-C₆) ou R¹⁶;
R¹⁶ représente un reste bicyclique ou tricyclique de 6 à 24 chaînons, saturé ou partiellement insaturé, pouvant aussi contenir un à quatre hétéroatomes identiques ou différents de la série de l'azote, de l'oxygène et du soufre et pouvant aussi être substitué par un ou plusieurs substituants identiques ou différents de la série des restes allyle en C₁-C₄ et oxo;
b, c, d et f représentent, indépendamment les uns des autres, 0 ou 1, mais ne peuvent pas tous être 0 en même temps;
e, g et h représentent indépendamment les uns des autres 0, 1, 2, 3, 4, 5 ou 6.

3. Utilisation de composés de formule I selon la revendication 1 dans toutes leurs formes stéréo-isomères et leurs mélanges en toutes proportions, ainsi que de leurs sels physiologiquement acceptables, dans laquelle, dans la formule I, R⁰ représente un reste alkyle en C₁-C₈, cycloalkyle en C₃-C₈, (cycloalkyl en C₃-C₈)-(alkyle en C₁-C₄), aryle en C₆-C₁₄ éventuellement substitué ou (aryl en C₆-C₁₄)-(alkyle en C₁-C₈) éventuellement substitué sur le reste aryle, de préférence un reste biphénylylméthyle, naphtylméthyle ou benzyle, non substitué ou substitué une ou plusieurs fois sur le reste aryle.

4. Utilisation de composés de formule I selon la revendication 1 et/ou 3 dans toutes leurs formes stéréo-isomères et leurs mélanges en toutes proportions, ainsi que de leurs sels physiologiquement acceptables, dans laquelle, dans la formule I, même temps
W représente un groupe R¹-A-CH=C dans lequel A représente un reste phénylène, ou W représente un groupe R¹-A-C(R¹³) dans lequel A représente un reste divalent de la série des restes méthylène, éthylène, triméthylène, tétraméthylène, pentaméthylène, cyclohexylène, phénylène, phénylèneméthyle;
B représente un reste divalent de la série des restes méthylène, éthylène, triméthylène, tétraméthylène, vinylène, phénylène ou méthylène ou éthylène substitué;
E représente un groupe R¹⁰CO;
R représente un atome d'hydrogène ou un reste alkyle en C₁-C₆ ou benzyle;
R⁰ représente un reste allyle en C₁-C₈, cycloalkyle en C₃-C₈, aryle en C₆-C₁₄ éventuellement substitué ou (aryl en C₆-C₁₄)-(alkyle en C₁-C₈) éventuellement substitué sur le reste aryle;
R¹ représente un groupe X-NH-C(=NH), X-NH-C(=NX)-NH ou X-NH-CH₂;
X représente un atome d'hydrogène ou un reste (alkyl en C₁-C₆)carbonyle, (alcoxy en C₁-C₆)carbonyle, (alkyl en C₁-C₈)carbonyloxy-(alcoxy en C₁-C₆)carbonyle, (aryl en C₆-C₁₄)-(alcoxy en C₁-C₆)carbonyle ou hydroxy;
R² représente un atome d'hydrogène ou un reste alkyle en C₁-C₈;
R³ représente un reste allyle en C₁-C₈, aryle en C₆-C₁₄ éventuellement substitué, (aryl en C₆-C₁₄)-(alkyle en C₁-C₈), cycloalkyle en C₃-C₈, alcényle en C₂-C₈, alcynyle en C₂-C₈, pyridyle, R¹¹NH, R⁴CO, COOR⁴, CONHR¹⁴, CSNHR¹⁴_{,} COOR¹⁵ et CONHR¹⁵;
et e, g et h représentent indépendamment les uns des autres les nombres 0, 1, 2 ou 3.

5. Utilisation de composés de formule I selon l'une ou plusieurs des revendications 1 à 4 dans toutes leurs formes stéréo-isomères et leurs mélanges en toutes proportions, ainsi que de leurs sels physiologiquement acceptables, dans laquelle, dans la formule I, W représente un groupe R¹-A-C(R¹³) et R¹³ est un reste alkyle en C₁-C₆, (aryl en C₆-C₁₄)-(alkyle en C₁-C₈) éventuellement substitué sur le reste aryle ou cycloalkyle en C₃-C₈.

6. Utilisation de composés de formule I selon l'une ou plusieurs des revendications 1 à 5 dans toutes leurs formes stéréo-isomères et leurs mélanges en toutes proportions, ainsi que de leurs sels physiologiquement acceptables, dans laquelle, dans la formule I, R³ représente un reste aryle en C₆-C₁₄ éventuellement substitué, COOR⁴, R¹¹NH ou CONHR¹⁴, où NHR¹⁴ représente le reste d'un ce-aminoacide, de ses ω-amino-alkylamides en C₂-C₈, de ses esters d'alkyle en C₁-C₈ ou de ses esters d'(aryl en C₆-C₁₄)-(alkyle en C₁-C₄), de préférence le reste des α-aminoacides valine, lysine, phénylglycine, phénylalanine ou tryptophane ou de leurs esters d'alkyle en C₁-C₈ ou de leurs esters d'(aryl en C₆-C₁₄)-(alkyle en C₁-C₄).

7. Utilisation de composés de formule I selon l'une ou plusieurs des revendications 1 et 3 à 6 dans toutes leurs formes stéréo-isomères et leurs mélanges en toutes proportions, ainsi que de leurs sels physiologiquement acceptables, dans laquelle, dans la formule I, en même temps
W représente un groupe R¹-A-C(R¹³);
Y représente un groupe carbonyle;
Z représente N(R⁰);
A représente un groupe éthylène, triméthylène, tétraméthylène, pentaméthylène, cyclohexylène, phénylène ou phénylèneméthyle;
B représente un reste méthylène non substitué ou substitué;
D représente un groupe C(R²)(R³);
E représente un groupe R¹⁰CO;
R représente un atome d'hydrogène ou un reste alkyle en C₁-C₄, en particulier un atome d'hydrogène ou un groupe méthyle ou éthyle;
R⁰ représente un reste alkyle en C₁-C₈, cycloalkyle en C₃-C₈, aryle en C₆-C₁₄ éventuellement substitué ou (aryl en C₆-C₁₄)-(alkyle en C₁-C₈) éventuellement substitué sur le reste aryle;
R¹ représente un groupe H₂N-C(=NH), H₂N-C(=NH)-NH ou H₂N-CH₂;
R² représente un atome d'hydrogène;
R³ représente le reste CONHR¹⁴;
R¹⁰ représente un reste hydroxy ou alcoxy en C₁-C₈, de préférence alcoxy en C₁-C₄;
R¹³ représente un reste alkyle en C₁-C₆, cycloalkyle en C₃-C₇ ou benzyle, en particulier méthyle;
R¹⁴ représente un reste méthyle substitué par un groupe hydroxycarbonyle et par un reste de la série des restes alkyle en C₁-C₄, phényle et benzyle, ou représente un reste méthyle substitué par un reste (alcoxy en C₁-C₈)carbonyle, de préférence (alcoxy en C₁-C₄)carbonyle et par un reste de la série des restes alkyle en C₁-C₄, phényle et benzyle;
b, c et d représentent 1 et e, f et g représentent 0,
h représente 1 ou 2, de préférence 1.

8. Utilisation de composés de formule I selon l'une ou plusieurs des revendications 1 et 3 et 4 dans toutes leurs formes stéréo-isomères et leurs mélanges en toutes proportions, ainsi que de leurs sels physiologiquement acceptables, dans laquelle, dans la formule I, en même temps
W représente un groupe R¹-A-CH=C, dans lequel A représente un reste phénylène, ou W représente un groupe R¹-A-C(R¹³) dans lequel A représente un reste divalent de la série des restes méthylène, éthylène, triméthylène, tétraméthylène, pentaméthylène, cyclohexylène, phénylène ou phénylènanéthyle;
B représente un reste divalent de la série des restes méthylène, éthylène, triméthylène, tétraméthylène, vinylène, phénylène ou un reste méthylène ou éthylène substitué;
E représente un groupe R¹⁰CO;
R représente un atome d'hydrogène ou un reste alkyle en C₁-C₆;
R⁰ représente un reste alkyle en C₁-C₈, cycloalkyle en C₃-C₈, aryle en C₆-C₁₄ éventuellement substitué ou (aryl en C₆-C₁₄)-(alkyle en C₁-C₈) éventuellement substitué sur le reste aryle;
R¹ représente un groupe X-NH-C(=NH), X-NH-C(=NX)-NH ou X-NH-CH₂;
X représente un atome d'hydrogène ou un reste (alkyl en C₁-C₆)carbonyle, (alcoxy en C₁-C₆)carbonyle, (alkyl en C₁-C₈)carbonyloxy-(alcoxy en C₁-C₆)carbonyle, (aryle en C₆-C₁₄)-(alcoxy en C₁-C₆)carbonyle ou hydroxy;
R² représente un atome d'hydrogène ou un reste alkyle en C₁-C₈;
R³ représente un reste CONHR¹⁵ ou CONHR¹⁴, où R¹⁴ représente un reste alkyle en C₁-C₈ non substitué ou substitué par un ou plusieurs restes aryle en C₆-C₁₄;
R¹⁵ représente un reste R¹⁶-(alkyle en C₁-C₆) ou R¹⁶, R¹⁶ étant un reste bicyclique ou tricyclique ponté de 7 à 12 chaînons, saturé ou partiellement insaturé, pouvant aussi contenir un à quatre hétéroatomes identiques ou différents de la série de l'azote, de l'oxygène et du soufre, et pouvant aussi être substitué par un ou plusieurs substituants identiques ou différents de la série des restes allyle en C₁-C₄ et oxo, et R¹⁵ est en particulier un reste adamantyle ou un reste adamantylméthyle;
et e, g et h représentent indépendamment les uns des autres les nombres 0,1, 2 ou 3 et b, c et d représentent 1.

9. Utilisation de composés de formule I selon lone ou plusieurs des revendications 1, 3, 4, 5 et 8 dans toutes leurs formes stéréo-isomères et leurs mélanges en toutes proportions, ainsi que de leurs sels physiologiquement acceptables, dans laquelle, dans la formule I, en même temps
W représente un groupe R¹-A-C(R¹³);
Y représente un groupe carbonyle;
Z représente N(R⁰);
A représente un reste éthylène, triméthylène, tétraméthylène, pentaméthylène, cyclohexylène, phénylène ou phénylèneméthyle;
B représente un reste méthylène non substitué ou substitué;
D représente un groupe C(R²)(R³);
E représente un groupe R¹⁰CO;
R représente un atome d'hydrogène ou un reste alkyle en C₁-C₄, en particulier un atome d'hydrogène ou un groupe méthyle ou éthyle;
R⁰ représente un reste alkyle en C₁-C₈, cycloalkyle en C₃-C₈, aryle en C₆-C₁₄ éventuellement substitué ou (aryl en C₆-C₁₄)-(alkyle en C₁-C₈) éventuellement substitué sur le reste aryle;
R¹ représente un groupe H₂N-C(=NH), H₂N-C(=NH)-NH ou H₂N-CH₂;
R² représente un atome d'hydrogène;
R³ représente un reste CONHR¹⁵ ou CONHR¹⁴, où R¹⁴ représente un reste alkyle en C₁-C₆ non substitué ou substitué par un ou plusieurs restes aryle en C₆-C₁₀;
R¹⁰ représente un groupe hydroxy ou alcoxy en C₁-C₈, de préférence alcoxy en C₁-C₄;
R¹³ représente un reste alkyle en C₁-C₆, cycloalkyle en C₃-C₇ ou benzyle, en particulier méthyle;
R¹⁵ représente un reste adamantyle ou un reste adamantylméthyle;
b, c et d représentent 1 et e, f et g représentent 0;
h représente 1 ou 2, de préférence 1.

10. Utilisation de composés de formule I selon l'une ou plusieurs des revendications 1, 3, 4 et 5 dans toutes leurs formes stéréo-isomères et leurs mélanges en toutes proportions, ainsi que de leurs sels physiologiquement acceptables, dans laquelle, dans la formule I, en même temps
W représente un groupe R¹-A-C(R¹³);
Y représente un groupe carbonyle;
Z représente N(R⁰);
A représente un reste éthylène, triméthylène, tétraméthylène, pentaméthylène, cyclohexylène, phénylène ou phénylèneméthyle;
B représente un reste méthylène ou éthylène non substitué ou substitué;
D représente un groupe C(R²)(R³);
E représente un groupe R¹⁰CO;
R représente un atome d'hydrogène ou un reste alkyle en C₁-C₄, en particulier un atome d'hydrogène ou un groupe méthyle ou éthyle;
R⁰ représente un reste alkyle en C₁-C₈, cycloalkyle en C₃-C₈, aryle en C₆-C₁₄ éventuellement substitué ou (aryl en C₆-C₁₄)-(alkyle en C₁-C₈) éventuellement substitué sur le reste aryle;
R¹ représente un groupe H₂N-C(=NH), H₂N-C(=NH)-NH ou H₂N-CH₂;
R² représente un atome d'hydrogène;
R³ représente un reste phényle ou naphtyle non substitué, un reste phényle ou naphtyle substitué par un, deux ou trois restes identiques ou différents de la série des restes alkyle en C₁-C₄, alcoxy en C₁-C₄, hydroxy, halogéno, trifluorométhyle, nitro, méthylènedioxy, éthylènedioxy, hydroxycarbonyle, (alcoxy en C₁-C₄)carbonyle, aminocarbonyle, cyano, phényle, phénoxy, benzyle et benzyloxy, un reste pyridyle, un reste alkyle en C₁-C₄, un reste alcényle en C₂-C₄, un reste alcynyle en C₂-C₄ ou un reste cycloalkyle en C₅-C₆, et R³ représente en particulier un reste phényle ou naphtyle non substitué ou substitué;
R¹⁰ représente un groupe hydroxy ou alcoxy en C₁-C₈, en particulier alcoxy en C₁-C₄, et R¹⁰ représente de préférence un reste de la série des restes hydroxy, méthoxy, éthoxy, propoxy et isopropoxy;
R¹³ représente un reste alkyle en C₁-C₆, cycloalkyle en C₃-C₇ ou benzyle, en particulier méthyle;
b, c et d représentent 1 et e, f et g représentent 0;
h représente 1 ou 2, de préférence 1.

11. Utilisation de composés de formule I selon l'une ou plusieurs des revendications 1, 3, 4 et 5 dans toutes leurs formes stéréo-isomères et leurs mélanges en toutes proportions, ainsi que de leurs sels physiologiquement acceptables, dans laquelle, dans la formule I, en même temps
W représente un groupe R¹-A-C(R¹³);
Y représente un groupe carbonyle;
Z représente N(R⁰);
A représente un reste éthylène, triméthylène, tétraméthylène, pentaméthylène, cyclohexylène, phénylène ou phénylèneméthyle;
B représente un reste méthylène ou éthylène non substitué ou substitué;
D représente un groupe C(R²)(R³);
E représente un groupe R¹⁰CO;
R représente un atome d'hydrogène ou un reste alkyle en C₁-C₄, en particulier un atome d'hydrogène ou un groupe méthyle ou éthyle;
R⁰ représente un reste alkyle en C₁-C₈, cycloalkyle en C₃-C₈, aryle en C₆-C₁₄ éventuellement substitué ou (aryl en C₆-C₁₄)-(alkyle en C₁-C₈) éventuellement substitué sur le reste aryle;
R¹ représente un groupe H₂N-C(=NH), H₂N-C(=NH)-NH ou H₂N-CH₂;
R² représente un atome d'hydrogène;
R³ représente un groupe R¹¹NH;
R¹⁰ représente un reste hydroxy ou alcoxy en C₁-C₈, en particulier alcoxy en C₁-C₄, et R¹⁰ représente de préférence un reste de la série des restes hydroxy, méthoxy, éthoxy, propoxy et isopropoxy;
R¹³ représente un reste alkyle en C₁-C₆, cycloalkyle en C₃-C₇ ou benzyle, en particulier méthyle;
b, c, d et e représentent 1 et f et g représentent 0;
h représente 0.

12. Utilisation de composés de formule I selon l'une ou plusieurs des revendications 1 et 3 à 11 dans toutes leurs formes stéréo-isomères et leurs mélanges en toutes proportions, ainsi que de leurs sels physiologiquement acceptables, dans laquelle un reste méthylène substitué ou un reste éthylène substitué représenté par le groupe B porte comme substituant un reste de la série des restes alkyle en C₁-C₈, alcényle en C₂-C₆, alcynyle en C₂-C₆, cycloalkyle en C₃-C₈, en particulier cycloalkyle en C₅-C₆, (cycloalkyle en C₃-C₈)-(alkyle en C₁-C₄), en particulier (cycloalkyl en C₅-C₆)-(alkyle en C₁-C₄), aryle en C₆-C₁₀ éventuellement substitué, (aryl en C₆-C₁₀)-alkyle (en C₁-C₄) éventuellement substitué sur le reste aryle, hétéroaryle éventuellement substitué et hétéroaryl-(alkyle en C₁-C₄) éventuellement substitué sur le reste hétéroaryle.

13. Utilisation de composés de formule I selon l'une ou plusieurs des revendications 1 et 3 à 12 dans toutes leurs formes stéréo-isomères et leurs mélanges en toutes proportions, ainsi que de leurs sels physiologiquement acceptables, dans laquelle B représente un reste méthylène non substitué ou un reste méthylène substitué par un reste alkyle en C₁-C₈.

14. Utilisation de composés de formule I selon l'une ou plusieurs des revendications 1 à 13 et/ou de leurs sels physiologiquement acceptables pour la préparation de médicaments anti-inflammatoires.

15. Utilisation de composés de formule I selon l'une ou plusieurs des revendications 1 à 13 et/ou de leurs sels physiologiquement acceptables pour la préparation de médicaments destinés au traitement ou à la prophylaxie de la polyarthrite rhumatoïde, des maladies intestinales inflammatoires, du lupus érythémateux aigu disséminé ou de maladies inflammatoires du système nerveux central.

16. Utilisation de composés de formule I selon l'une ou plusieurs des revendications 1 à 13 et/ou de leurs sels physiologiquement acceptables pour la préparation de médicaments destinés au traitement ou à la prophylaxie de l'asthme ou d'allergies.

17. Utilisation de composés de formule I selon l'une ou plusieurs des revendications 1 à 13 et/ou de leurs sels physiologiquement acceptables pour la préparation de médicaments pour le traitement ou la prophylaxie de maladies cardiovasculaires, de l'artériosclérose, de resténoses ou du diabète, pour inhiber les altérations de greffes d'organes, pour empêcher la croissance tumorale ou la formation de métastases tumorales ou pour la thérapie du paludisme.

18. Utilisation de composés de formule I selon l'une ou plusieurs des revendications 1 à 13 et/ou de leurs sels physiologiquement acceptables pour inhiber l'adhésion et/ou la migration des leucocytes ou pour inhiber le récepteur de VLA-4.

19. Utilisation de composés de formule I selon l'une ou plusieurs des revendications 1 à 13 et/ou de leurs sels physiologiquement acceptables pour le traitement ou la prophylaxie de maladies dans lesquelles l'adhésion des leucocytes et/ou la migration des leucocytes présente un niveau indésirable, ou de maladies dans lesquelles les processus d'adhésion dépendant du VLA-4 jouent un rôle, pour le traitement ou la prophylaxie de la polyarthrite rhumatoïde, des maladies intestinales inflammatoires, du lupus érythémateux aigu disséminé, de maladies inflammatoires du système nerveux central, de l'asthme d'allergies, de maladies cardiovasculaires, de l'artériosclérose, de resténoses, du diabète, pour inhiber les altérations de greffes d'organes, pour empêcher la croissance tumorale ou la formation de métastases tumorales, pour la thérapie du paludisme ou comme anti-inflammatoires.

20. Composés de formule Ib 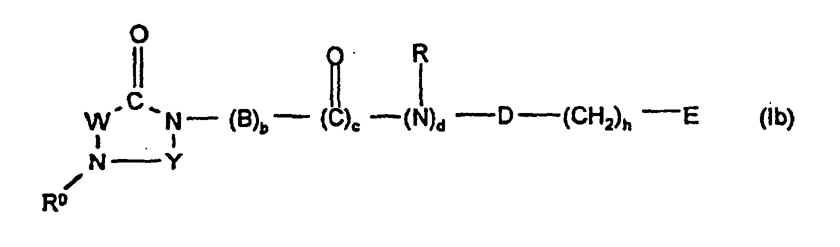 dans laquelle
W représente un groupe R¹-A-CH ou R¹-A-CH=C;
Y représente un groupe carbonyle, thiocarbonyle ou méthylène;
A représente un reste divalent de la série des restes alkylène en C₁-C₆, cycloalkylène en C₃-C₇, phénylène, phénylène-(alkyle en C₁-C₆), (alkylène en C₁-C₆)-phényle et phénylène-(alcényle en C₂-C₆), ou un reste divalent d'un cycle saturé ou insaturé de 5 ou 6 chaînons pouvant contenir 1 ou 2 atomes d'azote et être substitué une ou deux fois par alkyle en C₁-C₆ ou par de l'oxygène ou du soufre doublement lié;
B représente un reste divalent de la série des restes alkylène en C₁-C₆, alcénylène en C₂-C₆, phénylène, phénylène-(alkyle en C₁-C₃) et (alkylène en C₁-C₃)-phényle;
D représente un groupe C(R²)(R³);
E représente un groupe tétrazolyle, (R⁸O)₂P(O), HOS(O)₂, R⁹NHS(O)₂ ou R¹⁰CO;
R représente un atome d'hydrogène ou un reste alkyle en C₁-C₈, cycloalkyle en C₃-C₈, aryle en C₆-C₁₄ éventuellement substitué ou (aryl en C₆-C₁₄)-(alkyle en C₁-C₈) éventuellement substitué sur le reste aryle;
R⁰ représente un reste alkyle en C₇-C₈, cycloalkyle en C₃-C₈, aryle en C₆-C₁₄ éventuellement substitué ou (aryl en C₆-C₁₄)-(alkyle en C₁-C₈) éventuellement substitué sur le reste aryle;
R¹ représente un groupe X-NH-C(=NH)-(CH₂)ₚ ou X¹-NH-(CH₂)ₚ, où p est 0, 1, 2 ou 3;
X représente un atome d'hydrogène ou un reste alkyle en C₁-C₆, (alkyl en C₁-C₆)carbonyle, (alcoxy en C₁-C₆)carbonyle, (alkyl en C₁-C₁₈)carbonyloxy-(alcoxy en C₁-C₆)carbonyle, (aryl en C₆-C₁₄)carbonyle éventuellement substitué, (aryloxy en C₆-C₁₄)carbonyle éventuellement substitué, (aryl en C₆-C₁₄)-(alcoxy en C₁-C₆)carbonyle pouvant aussi être substitué sur le reste aryle, (R⁸O)₂P(O), cyano, hydroxy, alcoxy en C₁-C₆, (aryl en C₆-C₁₄)-(alcoxy en C₁-C₆) pouvant aussi être substitué sur le reste aryle, ou amino;
X¹ a l'une des significations de X ou représente un groupe R'-NH-C(=N-R"), dans lequel R' et R" ont indépendamment l'un de l'autre les significations de X;
R² représente un atome d'hydrogène ou un reste phényle;
R³ représente un atome d'hydrogène ou un reste COOR⁴, CON(CH₃)R⁴ ou CONHR⁴;
R⁴ représente un atome d'hydrogène ou un reste alkyle en C₁-C₂₈ pouvant éventuellement être substitué une ou plusieurs fois par des restes R^{4'} identiques ou différents;
R^{4'} représente un reste hydroxy, hydroxycarbonyle, aminocarbonyle, mono- ou di(alkyl en C₁-C₁₈)aminocarbonyle, amino-(alkyl en C₂-C₁₈)aminocarbonyle, amino(alkyl en C₁-C₃)phényl-(alkyl en C₁-C₃)aminocarbonyle, (alkyl en C₁-C₁₈)carbonylamino(alkyl en C₁-C₃)phényl-(alkyl en C₁-C₃)aminocarbonyle, (alkyl en C₁-C₁₈)carbonylaminl(alkyl en C₂-C₁₈)aminocarbonyle, (aryl en C₆-C₁₄)-(alcoxy en C₁-C₈)carbonyle pouvant aussi être substitué sur le reste aryle, amine, mercapto, alcoxy en C₁-C₁₈, (alcoxy en C₁-C₁₈)carbonyle, cycloalkyle en C₃-C₈ éventuellement substitué, halogéno, nitro, trifluorométhyle ou le reste R⁵,
R⁵ représente un reste aryle en C₆-C₁₄ éventuellement substitué ou (aryl en C₆-C₁₄)-(alkyle en C₁-C₈) éventuellement substitué sur le reste aryle, un cycle hétérocyclique mono- ou bicyclique de 5 à 12 chaînons pouvant être aromatique, partiellement hydrogéné ou entièrement hydrogéné et pouvant contenir un, deux ou trois hétéroatomes identiques ou différents de la série de l'azote, de l'oxygène et du soufre, un reste R⁶ ou un reste R⁶-CO, le reste aryle et le reste hétérocyclique pouvant être indépendamment substitués une ou plusieurs fois par des restes identiques ou différents de la série des restes alkyle en C₁-C₁₈, alcoxy en C₁-C₁₈, halogéno, nitro, amino ou trifluorométhyle;
R⁶ représente un groupe R⁷R⁸N, R⁷O ou R⁷S ou une chaîne latérale d'aminoacide, un reste d'aminoacide naturel ou synthétique, d'iminoacide, d'aza-aminoacide éventuellement N-substitué par un reste alkyle en C₁-C₈ ou N-substitué par un reste (aryl en C₆-C₁₄)-(alkyle en C₁-C₈) ou de dipeptide, qui peut aussi être substitué sur le reste aryle et/ou dont la liaison peptidique peut être réduite en -NH-CH₂-, et leurs esters et amides, et où, à la place de groupes fonctionnels libres, il peut éventuellement y avoir de l'hydrogène ou un groupe hydroxyméthyle et/ou des groupes fonctionnels libres peuvent être protégés par des groupes protecteurs classiques dans la chimie des peptides;
R⁷ représente un atome d'hydrogène, un reste alkyle en C₁-C₁₈, (aryl en C₆-C₁₄)-(alkyle en C₁-C₈), (alkyl en C₁-C₁₈)carbonyle, (alcoxy en C₁-C₁₈)carbonyle, (aryl en C₆-C₁₄)carbonyle, (aryl en C₆-C₁₄)-(alkyl en C₁-C₈)carbonyle ou (aryl en C₆-C₁₄)-(alkyl en C₁-C₁₈)oxycarbonyle, les groupes alkyle pouvant éventuellement être substitués par un groupe amino et/ou les restes aryle pouvant être substitués une ou plusieurs fois, de préférence une fois, par des restes identiques ou différents de la série des restes alkyle en C₁-C₈, alcoxy en C₁-C₈, halogéno, nitro, amino et trifluorométhyle, ou un reste d'aminoacide naturel ou synthétique, d'iminoacide, d'aza-aminoacide éventuellement N-substitué par un reste alkyle en C₁-C₈ ou N-substitué par un reste (aryl en C₆-C₁₄)-(alkyle en C₁-C₈) ou de dipeptide, qui peut aussi être substitué sur le reste aryle et/ou dont la liaison peptidique peut être réduite en NH-CH₂-;
R⁸ représente un atome d'hydrogène ou un reste alkyle en C₁-C₁₈, aryle en C₆-C₁₄ éventuellement substitué ou (aryl en C₆-C₁₄)-(alkyle en C₁-C₈) pouvant aussi être substitué sur le reste aryle;
R⁹ représente un atome d'hydrogène ou un reste aminocarbonyle, (alkyl en C₁-C₁₈)aminocarbonyle, (cycloalkyl en C₃-C₈)aminocarbonyle, (aryl en C₆-C₁₄)aminocarbonyle éventuellement substitué, alkyle en C₁-C₁₈, aryle en C₆-C₁₄ éventuellement substitué ou cycloalkyle en C₃-C₈;
R¹⁰ représente un reste hydroxy, alcoxy en C₁-C₁₈, (aryl en C₆-C₁₄)-(alcoxy en C₁-C₈) pouvant aussi être substitué sur le reste aryle, aryloxy en C₆-C₁₄ éventuellement substitué, amine ou mono- ou di(alkyl en C₁-C₁₈)amino;
b, c et d représentent, indépendamment les uns des autres, 0 ou 1, mais ne peuvent pas tous être 0 en même temps;
h représente les nombres 0, 1, 2, 3, 4, 5 ou 6;
dans toutes leurs formes stéréo-isomères et leurs mélanges en toutes proportions, ainsi que leurs sels pharmaceutiquement acceptables.

21. Composés de formule Ib selon la revendication 20 et/ou leurs sels physiologiquement acceptables, à utiliser comme médicaments.

22. Préparation pharmaceutique, **caractérisée en ce qu'**elle contient un ou plusieurs composés de formule selon la revendication 20 et/ou leurs sels physiologiquement acceptables en plus de supports et/ou d'additifs pharmaceutiquement acceptables.

23. Composés de formule Ic 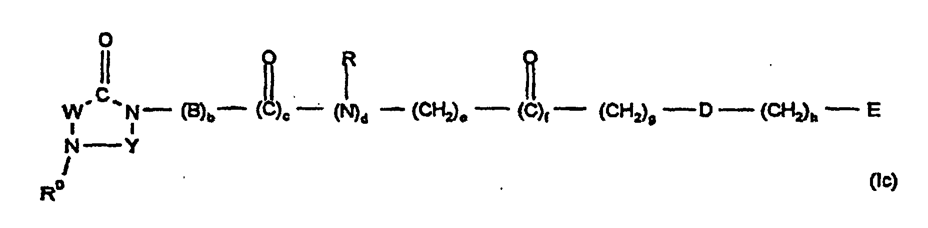 dans laquelle
W représente un groupe R¹-A-C(R¹³);
Y représente un groupe carbonyle, thiocarbonyle ou méthylène;
A représente un reste phénylène;
B représente un reste divalent de la série des restes alkylène en C₁-C₆, alcénylène en C₂-C₆, phénylène, phénylène-(alkyle en C₁-C₃) et (alkylène en C₁-C₃)-phényle;
D représente un groupe C(R²)(R³), N(R³) ou CH=C(R³);
E représente un groupe tétrazolyle, (R⁸O)₂P(O), HOS(O)₂, R⁹NHS(O)₂ ou R¹⁰CO;
R représente un atome d'hydrogène ou un reste alkyle en C₁-C₈, cycloalkyle en C₃-C₈, aryle en C₆-C₁₄ éventuellement substitué ou (aryl en C₆-C₁₄)-(alkyle en C₁-C₈) éventuellement substitué sur le reste aryle;
R⁰ représente un reste (aryl en C₆-C₁₄)-(alkyle en C₁-C₈) éventuellement substitué sur le reste aryle,
R¹ représente un groupe X-NH-C(NH)-(CH₂)ₚ ou X¹-NH-(CH₂)ₚ, où p est 0, 1, 2 ou 3;
X représente un atome d'hydrogène ou un reste alkyle en C₁-C₆, (alkyl en C₁-C₆)carbonyle, (alcoxy en C₁-C₆)carbonyle, (alkyl en C₁-C₁₈)carbonyloxy-(alcoxy en C₁-C₆)carbonyle, (aryl en C₆-C₁₄)carbonyle éventuellement substitué, (aryloxy en C₆-C₁₄)carbonyle éventuellement substitué, (aryl en C₆-C₁₄)-(alcoxy en C₁-C₆)carbonyle pouvant aussi être substitué sur le reste aryle, (R⁹O)₂P(O), cyano, hydroxy, alcoxy en C₁-C₆, (aryl en C₆-C₁₄)-(alcoxy en C₁-C₆) pouvant aussi être substitué sur le reste aryle, ou amino;
X¹ a l'une des significations de X ou représente un groupe R'-NH-C(=N-R"), dans lequel R¹ et R" ont indépendamment l'un de l'autre les significations de X;
R² représente un atome d'hydrogène ou un reste alkyle en C₁-C₈, aryle en C₆-C₁₄ éventuellement substitué, (aryl en C₆-C₁₄)-(alkyle en C₁-C₈) éventuellement substitué sur le reste aryle, ou cycloalkyle en C₃-C₈;
R³ représente un atome d'hydrogène ou un reste alkyle en C₁-C₈, aryle en C₆-C₁₄ éventuellement substitué, (aryl en C₆-C₁₄)-(alkyle en C₁-C₈) éventuellement substitué sur le reste aryle, cycloalkyle en C₃-C₈, alcényle en C₂-C₈, alcynyle en C₂-C₈, (alcényl en C₂-C₈)carbonyle, (alcynyl en C₂-C₈)carbonyle, pyridyle, R¹¹NH_{,} R⁴CO, COOR⁴, CON(CH₃)R¹⁴, CONHR¹⁴, CSNHR¹⁴, COOR¹⁵, CON(CH₃)R¹⁵ ou CONHR¹⁵;
R⁴ représente un atome d'hydrogène ou un reste alkyle en C₁-C₂₈ pouvant éventuellement être substitué une ou plusieurs fois par des restes R^{4'} identiques ou différents;
R^{4'} représente un reste hydroxy, hydroxycarbonyle, aminocarbonyle, mono- ou di(alkyl en C₁-C₁₈)aminocarbonyle, amino-(alkyl en C₂-C₁₈)aminocarbonyle, amino(alkyl en C₁-C₃)phényl-(alkyl en C₁-C₃)aminocarbonyle, (alkyl en C₁-C₁₈)carbonylamino(alkyl en, C₁-C₃)phényl-(alkyl en C₁-C₃)aminocarbonyle, (alkyl en C₁-C₁₈)carbonylamino-(alkyl en C₂-C₁₈)aminocarbonyle, (aryl en C₆-C₁₄)-(alcoxy en C₁-C₈)carbonyle pouvant aussi être substitué sur le reste aryle, amino, mercapto, alcoxy en C₁-C₁₈, (alcoxy en C₁-C₁₈)carbonyle, cycloalkyle en C₃-C₈ éventuellement substitué, halogéno, nitro, trifluorométhyle ou le reste R⁵,
R⁵ représente un reste aryle en C₆-C₁₄ éventuellement substitué ou (aryl en C₆-C₁₄)-(alkyle en C₁-C₈) éventuellement substitué sur le reste aryle, un cycle hétérocyclique mono- ou bicyclique de 5 à 12 chaînons pouvant être aromatique, partiellement hydrogéné ou entièrement hydrogéné et pouvant contenir un, deux ou trois hétéroatomes identiques ou différents de la série de l'azote, de l'oxygène et du soufre, un reste R⁶ ou un reste R⁶-CO, le reste aryle et le reste hétérocyclique pouvant être indépendamment substitués une ou plusieurs fois par des restes identiques ou différents de la série des restes alkyle en C₁-C₁₈, alcoxy en C₁-C₁₈, halogéno, nitro, amino ou trifluorométhyle;
R⁶ représente un groupe R⁷R⁸N, R⁷O ou R⁷S ou une chaîne latérale d'aminoacide, un reste d'aminoacide naturel ou synthétique, d'iminoacide, d'aza-aminoacide éventuellement N-substitué par un reste alkyle en C₁-C₈ ou N-substitué par un reste (aryl en C₆-C₁₄)-(alkyle en C₁-C₈) ou de dipeptide, qui peut aussi être substitué sur le reste aryle et/ou dont la liaison peptidique peut être réduite en -NH-CH₂-, et leurs esters et amides, et où, à la, place de groupes fonctionnels libres, il peut éventuellement y avoir de l'hydrogène ou un groupe hydroxyméthyle et/ou des groupes fonctionnels libres peuvent être protégés par des groupes protecteurs classiques dans la chimie des peptides;
R⁷ représente un atome d'hydrogène, un reste alkyle en C₁-C₁₈, (aryl en C₆-C₁₄)-(alkyle en C₁-C₈), (alkyl en C₁-C₁₈)carbonyle, (alcoxy en C₁-C₁₈)carbonyle, (aryl en C₆-C₁₄)carbonyle, (aryl en C₆-C₁₄)-(alkyl en C₁-C₈)carbonyle ou (aryl en C₆-C₁₄)-(alkyl en C₁-C₁₈)oxycarbonyle, les groupes alkyle pouvant éventuellement être substitués par un groupe amino et/ou les restes aryle pouvant être substitués une ou plusieurs fois, de préférence un fois, par des restes identiques ou différents de la série des restes allyle en C₁-C₈, alcoxy en C₁-C₈, halogéno, nitro, amine et trifluorométhyle, ou un reste d'aminoacide naturel ou synthétique, d'iminoacide, d'aza-aminoacide éventuellement N-substitué par un reste alkyle en C₁-C₈ ou N-substitué par un reste (aryl en C₆-C₁₄)-(alkyle en C₁-C₈) ou de dipeptide, qui peut aussi être substitué sur le reste aryle et/ou dont la liaison peptidique peut être réduite en NH-CH₂-;
R⁸ représente un atome d'hydrogène ou un reste alkyle en C₁-C₁₈, aryle en C₆-C₁₄ éventuellement substitué ou (aryl en C₆-C₁₄)-(alkyle en C₁-C₈) pouvant aussi être substitué sur le reste aryle;
R⁹ représente un atome d'hydrogène ou un reste aminocarbonyle, (alkyl en C₁-C₁₈)aminocarbonyle, (cycloalkyl en C₃-C₈)aminocarbonyle, (aryl en C₆-C₁₄)aminocarbonyle éventuellement substitué, alkyle en C₁-C₁₈, aryle en C₆-C₁₄ éventuellement substitué ou cycloalkyle en C₃-C₈;
R¹⁰ représente un reste hydroxy, alcoxy en C₁-C₁₈, (aryl en C₆-C₁₄)-(alcoxy en C₁-C₈) pouvant aussi être substitué sur le reste aryle, aryloxy en C₆-C₁₄ éventuellement substitué, amino ou mono- ou di(alkyl en C₁-C₁₈)amino;
R¹¹ représente un atome d'hydrogène ou un reste alkyle en C₁-C₁₈, R¹²CO, aryl-S(O)₂ en C₆-C₁₄ éventuellement substitué, alkyl-S(O)₂ en C₁-C₁₈, (aryl en C₆-C₁₄)-(alkyle en C₁-C₈) éventuellement substitué sur le reste aryle, ou R⁹NHS(O)₂;
R¹² représente un atome d'hydrogène ou un reste alkyle en C₁-C₁₈, alcényle en C₂-C₈, alcynyle en C₂-C₈, aryle en C₆-C₁₄ éventuellement substitué, alcoxy en C₁-C₁₈, (aryl en C₆-C₁₄)-(alcoxy en C₁-C₈) pouvant aussi être substitué sur le reste aryle, aryloxy en C₆-C₁₄ éventuellement substitué, amino ou mono- ou di(alkyl en C₁-C₁₈)amino;
R¹³ représente un reste alkyle en C₁-C₆, (aryl en C₆-C₁₄)-(alkyle en C₁-C₈) éventuellement substitué sur le reste aryle ou cycloalkyle en C₃-C₈;
R¹⁴ représente un atome d'hydrogène ou un reste alkyle en C₁-C₂₈ éventuellement substitué une ou plusieurs fois par des restes identiques ou différents de la série des restes hydroxy, hydroxycarbonyle, aminocarbonyle, mono- ou di(alkyl en C₁-C₁₈)aminocarbonyle, amino-(alkyl en C₂-C₁₈)aminocarbonyle, amino(alkyl en C₁-C₃)phényl-(alkyl en C₁-C₃)aminocarbonyle, (alkyl en C₁-C₁₈)carbonyl-amino(alkyl en C₁-C₃)phényl-(alkyl en C₁-C₃)aminocarbonyle, (alkyl en C₁-C₁₈)carbonylamino-(alkyl en C₂-C₁₈)aminocarbonyle, (aryl en C₆-C₁₄)-(alcoxy en C₁-C₈)carbonyle pouvant aussi être substitué sur le reste aryle, amino, mercapto, alcoxy en C₁-C₁₈, (alcoxy en C₁-C₁₈)carbonyle, cycloalkyle en C₃-C₈ éventuellement substitué, HOS(O)₂-(alkyle en C₁-C₃), R⁹NHS(O)₂-(alkyle en C₁-C₃), (R⁸O)₂P(O)-(alkyle en C₁-C₃), tétrazolyl-(alkyle en C₁-C₃), halogéno, nitro, trifluorométhyle et R⁵;
R¹⁵ représente un reste R¹⁶-(alkyle en C₁-C₆) ou R¹⁶;
R¹⁶ représente un reste bicyclique ou tricyclique de 6 à 24 chaînons, saturé ou partiellement insaturé, pouvant aussi contenir un à quatre hétéroatomes identiques ou différents de la série de l'azote, de l'oxygène et du soufre et pouvant aussi être substitué par un ou plusieurs substituants identiques ou différents de la série des restes alkyle en C₁-C₄ et oxo;
b, c, d et f représentent, indépendamment les uns des autres, 0 ou 1, mais ne peuvent pas tous être 0 en même temps;
e, g et h représentent indépendamment les uns des autres 0, 1, 2, 3, 4, 5 ou 6;
dans toutes leurs formes stéréo-isomères et leurs mélanges en toutes proportions, ainsi que leurs sels pharmaceutiquement acceptables.

24. Composés de formule Ic selon la revendication 23 et/ou leurs sels physiologiquement acceptables, à utiliser comme médicaments.

25. Préparation pharmaceutique, **caractérisée en ce qu'**elle contient un ou plusieurs composés de formule Ic selon la revendication 23 et/ou leurs sels physiologiquement acceptables en plus de supports et/ou d'additifs pharmaceutiquement acceptables.

26. Composés de formule Id 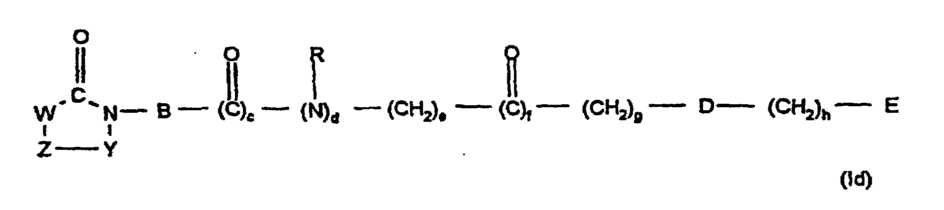 dans laquelle
W représente un groupe R¹-A-C(R¹³) ou R¹-A-CH=C;
Y représente un groupe carbonyle, thiocarbonyle ou méthylène;
Z représente N(R⁰);
A représente un reste divalent de la série des restes alkylène en C₁-C₆, cycloalkylène en C₃-C₇, phénylène, phénylène-(alkyle en C₁-C₆), (alkylène en C₁-C₆)-phényle et phénylène-(alcényle en C₂-C₆), ou un reste divalent d'un cycle saturé ou insaturé de 5 ou 6 chainons pouvant contenir 1 ou 2 atomes d'azote et être substitué une ou deux fois par alkyle en C₁-C₆ ou par de l'oxygène ou du soufre doublement lié;
B représente un reste divalent alkylène en C₁-C₆ pouvant être non substitué ou substitué par un reste de la série des restes allyle en C₁-C₈, alcényle en C₂-C₈, alcynyle en C₂-C₈, cycloalkyle en C₃-C₁₀, (cycloalkyl en C₃-C₁₀)-(alkyle en C₁-C₆), aryle en C₆-C₁₄ éventuellement substitué, (aryl en C₆-C₁₄)-(alkyle en C₁-C₆) éventuellement substitué sur le reste aryle, hétéroaryle éventuellement substitué et hétéroaryl-(alkyle en C₁-C₆) éventuellement substitué sur le reste hétéroaryle;
D représente un groupe C(R²)(R³), N(R³) ou CH=C(R³);
E représente un groupe tétrazolyle, (R⁸O)₂P(O), HOS(O)₂, R⁹NHS(O)₂ ou R¹⁰CO;
R représente un atome d'hydrogène ou un reste alkyle en C₁-C₈, cycloalkyle en C₃-C₈, aryle en C₆-C₁₄ éventuellement substitué ou (aryl en C₆-C₁₄)-(alkyle en C₁-C₈) éventuellement substitué sur le reste aryle;
R⁰ représente un atome d'hydrogène ou un reste alkyle en C₁-C₈, cycloalkyle en C₃-C₁₂, (cycloalkyl en C₃-C₁₂)-(alkyle en C₁-C₈), bicycloalkyle en C₆-C₁₂, (bicycloalkyl en C₆-C₁₂)-(alkyle en C₁-C₈), tricycloalkyle en C₆-C₁₂ (tricycloalkyl en C₆-C₁₂)-(alkyle en C₁-C₈), aryle en C₆-C₁₄ éventuellement substitué, (aryl en C₆-C₁₄)-(alkyle en C₁-C₈) éventuellement substitué sur le reste aryle, hétéroaryle éventuellement substitué, hétéroaryl-(alkyle en C₁-C₈) éventuellement substitué sur le reste hétéroaryle, CHO, (alkyl en C₁-C₈)-CO, (cycloalkyl en C₃-C₁₂)-CO, (cycloalkyl en C₃-C₁₂)-(alkyl en C₁-C₈)-CO, (bicycloalkyl en C₆-C₁₂)-CO, (bicycloalkyl en C₆-C₁₂)-(alkyl en C₁-C₈)-CO, (tricycloalkyl en C₆-C₁₂)-CO, (tricycloalkyl en C₆-C₁₂)-(alkyl en C₁-C₈)-CO, (aryl en C₆-C₁₄)-CO éventuellement substitué, (aryl en C₆-C₁₄)-(alkyl en C₁-C₈)-CO éventuellement substitué sur le reste aryle, hétéroaryl-CO éventuellement substitué, hétéroaryl-(alkyl en C₁-C₈)-CO éventuellement substitué sur le reste hétéroaryle, (alkyl en C₁-C₈)-S(O)ₙ, (cycloalkyl en C₃-C₁₂)-S(O)ₙ, (cycloalkyl en C₃-C₁₂)-(alkyl en C₁-C₈)-S(O)ₙ, (bicycloalkyl en C₆-C₁₂)-S(O)ₙ, (bicycloalkyl en C₆-C₁₂)-(alkyl en C₁-C₈)-S(O)ₙ, (tricycloalkyl en C₆-C₁₂)-S(O)ₙ, (tricycloalkyl en C₆-C₁₂)-(alkyl en C₁-C₈)-S(O)ₙ, (aryl en C₆-C₁₄)-S(O)ₙ éventuellement substitué, (aryl en C₆-C₁₄)-(alkyl en C₁-C₈)-S(O)ₙ éventuellement substitué sur le reste aryle, hétéroaryl-S(O)ₙ éventuellement substitué ou hétéroaryl-(alkyl en C₁-C₈)-S(O)ₙ éventuellement substitué sur le reste hétéroaryle, n étant égal à 1 ou 2;
R¹ représente un groupe X-NH-C(=NH)-(CH₂)ₚ ou X¹-NH-(CH₂)ₚ, où p est 0, 1, 2 ou 3;
X représente un atome d'hydrogène ou un reste alkyle en C₁-C₆, (alkyl en C₁-C₆)carbonyle, (alcoxy en C₁-C₆)carbonyle, (alkyl en C₁-C₁₈)carbonyloxy-(alcoxy en C₁-C₆)carbonyle, (aryl en C₆-C₁₄)carbonyle éventuellement substitué, (aryloxy en C₆-C₁₄)carbonyle éventuellement substitué, (aryl en C₆-C₁₄)-(alcoxy en C₁-C₆)carbonyle pouvant aussi être substitué sur le reste aryle, (R⁸O)₂P(O), cyano, hydroxy, alcoxy en C₁-C₆, (aryl en C₆-C₁₄)-(alcoxy en C₁-C₆) pouvant aussi être substitué sur le reste aryle, ou amino;
X¹ a l'une des significations de X ou représente un groupe R'-NH-C(=N-R"), dans lequel R' et R" ont indépendamment l'un de l'autre les significations de X;
R² représente un atome d'hydrogène ou un reste alkyle en C₁-C₈, aryle en C₆-C₁₄ éventuellement substitué, (aryl en C₆-C₁₄)-(alkyle en C₁-C₈) éventuellement substitué sur le reste aryle, ou cycloalkyle en C₃-C₈;
R³ représente un atome d'hydrogène ou un reste alkyle en C₁-C₈, aryle en C₆-C₁₄ éventuellement substitué, (aryl en C₆-C₁₄)-(alkyle en C₁-C₈) éventuellement substitué sur le reste aryle, cycloalkyle en C₃-C₈, alcényle en C₂-C₈, alcynyle en C₂-C₈, (alcényl en C₂-C₈)carbonyle, (alcynyl en C₂-C₈)carbonyle, pyridyle, R¹¹NH, R⁴CO, COOR⁴_{,} CON(CH₃)R¹⁴, CONHR¹⁴, CSNHR¹⁴, COOR¹⁵, CON(CH₃)R¹⁵ ou CONHR¹⁵;
R⁴ représente un atome d'hydrogène ou un reste alkyle en C₁-C₂₈ pouvant éventuellement être substitué une ou plusieurs fois par des restes R^{4'} identiques ou différents;
R^{4'} représente un reste hydroxy, hydroxycarbonyle, aminocarbonyle, mono- ou di(alkyl en C₁-C₁₈)aminocarbonyle, amino-(alkyl en C₂-C₁₈)aminocarbonyle, amino(alkyl en C₁-C₃)phényl-(alkyl en C₁-C₃)aminocarbonyle, (alkyl en C₁-C₁₈)carbonylamino(alkyl en C₁-C₃)phényl-(alkyl en C₁-C₃)aminocarbonyle, (alkyl en C₁-C₁₈)carbonylamino-(alkyl en C₂-C₁₈)aminocarbonyle, (aryl en C₆-C₁₄)-(alcoxy en C₁-C₈)carbonyle pouvant aussi être substitué sur le reste aryle, amino, mercapto, alcoxy en C₁-C₁₈, (alcoxy en C₁-C₁₈)carbonyle, cycloalkyle en C₃-C₈ éventuellement substitué, halogéno, nitro, trifluorométhyle ou le reste R⁵,
R⁵ représente un reste aryle en C₆-C₁₄ éventuellement substitué ou (aryl en C₆-C₁₄)-(alkyle en C₁-C₈) éventuellement substitué sur le reste aryle, un cycle hétérocyclique mono- ou bicyclique de 5 à 12 chaînons pouvant être aromatique, partiellement hydrogéné ou entièrement hydrogéné et pouvant contenir un, deux ou trois hétéroatomes identiques ou différents de la série de l'azote, de l'oxygène et du soufre, un reste R⁶ ou un reste R⁶-CO, le reste aryle et le reste hétérocyclique pouvant être indépendamment substitués une ou plusieurs fois par des restes identiques ou différents de la série des restes alkyle en C₁-C₁₈, alcoxy en C₁-C₁₈, halogéno, nitro, amino ou trifluorométhyle;
R⁶ représente un groupe R⁷R⁸N, R⁷O ou R⁷S ou une chaîne latérale d'aminoacide, un reste d'aminoacide naturel ou synthétique, d'iminoacide, d'aza-aminoacide éventuellement N-substitué par un reste alkyle en C₁-C₈ ou N-substitué par un reste (aryl en C₆-C₁₄)-(alkyle en C₁-C₈) ou de dipeptide, qui peut aussi être substitué sur le reste aryle et/ou dont la liaison peptidique peut être réduite en -NH-CH₂-, et leurs esters et amides, et où, à la place de groupes fonctionnels libres, il peut éventuellement y avoir de l'hydrogène ou un groupe hydroxyméthyle et/ou des groupes fonctionnels libres peuvent être protégés par des groupes protecteurs classiques dans la chimie des peptides;
R⁷ représente un atome d'hydrogène, un reste alkyle en C₁-C₁₈, (aryl en C₆-C₁₄)-(alkyle en C₁-C₈), (alkyl en C₁-C₁₈)carbonyle, (alcoxy en C₁-C₁₈)carbonyle, (aryl en C₆-C₁₄)carbonyle, (aryl en C₆-C₁₄)-(alkyl en C₁-C₈)carbonyle ou (aryl en C₆-C₁₄)-(alkyl en C₁-C₁₈)oxycarbonyle, les groupes alkyle pouvant éventuellement être substitués par un groupe amino et/ou les restes aryle pouvant être substitués une ou plusieurs fois, de préférence une fois, par des restes identiques ou différents de la série des restes alkyle en C₁-C₈, alcoxy en C₁-C₈, halogéno, nitro, amino et trifluorométhyle, ou un reste d'aminoacide naturel ou synthétique, d'iminoacide, d'aza-aminoacide éventuellement N-substitué par un reste alkyle en C₁-C₈ ou N-substitué par un reste (aryl en C₆-C₁₄)-(alkyle en C₁-C₈) ou de dipeptide, qui peut aussi être substitué sur le reste aryle et/ou dont la liaison peptidique peut être réduite en NH-CH₂-;
R⁸ représente un atome d'hydrogène ou un reste alkyle en C₁-C₁₈, aryle en C₆-C₁₄ éventuellement substitué ou (aryl en C₆-C₁₄)-(alkyle en C₁-C₈) pouvant aussi être substitué sur le reste aryle;
R⁹ représente un atome d'hydrogène ou un reste aminocarbonyle, (alkyl en C₁-C₁₈)aminocarbonyle, (cycloalkyl en C₃-C₈)aminocarbonyle, (aryl en C₆-C₁₄)aminocarbonyle éventuellement substitué, alkyle en C₁-C₁₈, aryle en C₆-C₁₄ éventuellement substitué ou cycloalkyle en C₃-C₈;
R¹⁰ représente un reste hydroxy, alcoxy en C₁-C₁₈, (aryl en C₆-C₁₄)-(alcoxy en C₁-C₈), pouvant aussi être substitué sur le reste aryle, aryloxy en C₆-C₁₄ éventuellement substitué, amino ou mono- ou di(alkyl en C₁-C₁₈)amino;
R¹¹ représente un atome d'hydrogène ou un reste alkyle en C₁-C₁₈, R¹²CO, aryl-S(O)₂ en C₆-C₁₄ éventuellement substitué, alkyl-S(O)₂ en C₁-C₁₈, (aryl en C₆-C₁₄)-(alkyle en C₁-C₈) éventuellement substitué sur le reste aryle, ou R⁹NHS(O)₂;
R¹² représente un atome d'hydrogène ou un reste alkyle en C₁-C₁₈, alcényle en C₂-C₈, alcynyle en C₂-C₈, aryle en C₆-C₁₄ éventuellement substitué, alcoxy en C₁-C₁₈, (aryl en C₆-C₁₄)-(alcoxy en C₁-C₈) pouvant aussi être substitué sur le reste aryle, aryloxy en C₆-C₁₄ éventuellement substitué, amino ou mono- ou di(alkyl en C₁-C₁₈)amino;
R¹³ représente un atome d'hydrogène ou un reste alkyle en C₁-C₆, (aryl en C₆-C₁₄)-(alkyle en C₁-C₈) éventuellement substitué sur le reste aryle ou cycloalkyle en C₃-C₈;
R¹⁴ représente un atome d'hydrogène ou un reste alkyle en C₁-C₂₈ éventuellement substitué une ou plusieurs fois par des restes identiques ou différents de la série des restes hydroxy, hydroxycarbonyle, aminocarbonyle, mono- ou di(alkyl en C₁-C₁₈)aminocarbonyle, amino-(alkyl en C₂-C₁₈)aminocarbonyle, amino(alkyl en C₁-C₃)phényl-(alkyl en C₁-C₃)aminocarbonyle, (alkyl en C₁-C₁₈)carbonyl-amino(alkyl en C₁-C₃)phényl-(alkyl en C₁-C₃)aminocarbonyle, (alkyl en C₁-C₁₈)carbonylamino-(alkyl en C₂-C₁₈)aminocarbonyle, (aryl en C₆-C₁₄)-(alcoxy en C₁-C₈)carbonyle pouvant aussi être substitué sur le reste aryle, amino, mercapto, alcoxy en C₁-C₁₈, (alcoxy en C₁-C₁₈)carbonyle, cycloalkyle en C₃-C₈ éventuellement substitué, HOS(O)₂-(alkyle en C₁-C₃), R⁹NHS(O)₂-(alkyle en C₁-C₃), (R⁸O)₂P(O)-(alkyle en C₁-C₃), tétrazolyl-(alkyle en C₁-C₃), halogéno, nitro, trifluorométhyle et R⁵;
R¹⁵ représente un reste R¹⁶-(alkyle en C₁-C₆) ou R¹⁶;
R¹⁶ représente un reste bicyclique ou tricyclique de 6 à 24 chaînons, saturé ou partiellement insaturé, pouvant aussi contenir un à quatre hétéroatomes identiques ou différents de la série de l'azote, de l'oxygène et du soufre et pouvant aussi être substitué par un ou plusieurs substituants identiques ou différents de la série des restes alkyle en C₁-C₄ et oxo;
c, d et f représentent, indépendamment les uns des autres, 0 ou 1, mais ne peuvent pas tous être 0 en même temps;
e, g et h représentent indépendamment les uns des autres 0, 1, 2, 3, 4, 5 ou 6;
dans toutes leurs formes stéréo-isomères et leurs mélanges en toutes proportions, ainsi que leurs sels pharmaceutiquement acceptables.

27. Composés de formule Id selon la revendication 26, dans laquelle, en même temps,
W représente un groupe R¹-A-C(R¹³);
Y représente un groupe carbonyle;
Z représente N(R⁰);
A représente un reste divalent de la série des restes cycloalkylène en C₃-C₇, phénylène, phénylène-(alkyle en C₁-C₆), (alkylène en C₁-C₆)-phényle, ou un reste divalent d'un cycle saturé ou insaturé de 5 ou 6 chaînons pouvant contenir 1 ou 2 atomes d'azote et être substitué une ou deux fois par alkyle en C₁-C₆ ou par de l'oxygène ou du soufre doublement lié;
B représente un reste divalent méthylène ou éthylène substitué par un reste de la série des restes alkyle en C₁-C₈, alcényle en C₂-C₈, alcynyle en C₂-C₈, cycloalkyle en C₃-C₁₀, (cycloalkyl en C₃-C₁₀)-(alkyle en C₁-C₆), aryle en C₆-C₁₄ éventuellement substitué, (aryl en C₆-C₁₄)-(alkyle en C₁-C₆) éventuellement substitué sur le reste aryle, hétéroaryle éventuellement substitué et hétéroaryl-(alkyle en C₁-C₆) éventuellement substitué sur le reste hétéroaryle;
D représente un groupe C(R²)(R³);
E représente un groupe tétrazolyle ou R¹⁰CO;
R représente un atome d'hydrogène ou un reste alkyle en C₁-C₈;
R⁰ représente un atome d'hydrogène ou un reste alkyle en C₁-C₈, cycloalkyle en C₃-C₁₂, (cycloalkyl en C₃-C₁₂)-(alkyle en C₁-C₈), bicycloalkyle en C₆-C₁₂, (bicycloalkyl en C₆-C₁₂)-(alkyle en C₁-C₈), tricycloalkyle en C₆-C₁₂, (tricycloalkyl en C₆-C₁₂)-(alkyle en C₁-C₈), aryle en C₆-C₁₄ éventuellement substitué, (aryl en C₆-C₁₄)-(alkyle en C₁-C₈) éventuellement substitué sur le reste aryle, hétéroaryle éventuellement substitué, hétéroaryl-(alkyle en C₁-C₈) éventuellement substitué sur le reste hétéroaryle, CHO, (alkyl en C₁-C₈)-CO, (cycloalkyl en C₃-C₁₂)-CO, (cycloalkyl en C₃-C₁₂)-(alkyl en C₁-C₈)-CO, (bicycloalkyl en C₆-C₁₂)-CO, (bicycloalkyl en C₆-C₁₂)-(alkyl en C₁-C₈)-CO, (tricycloalkyl en C₆-C₁₂)-CO, (tricycloalkyl en C₆-C₁₂)-(alkyl en C₁-C₈)-CO, (aryl en C₆-C₁₄)-CO éventuellement substitué, (aryl en C₆-C₁₄)-(alkyl en C₁-C₈)-CO éventuellement substitué sur le reste aryle, hétéroaryl-CO éventuellement substitué, hétéroaryl-(alkyl en C₁-C₈)-CO éventuellement substitué sur le reste hétéroaryle, (alkyl en C₁-C₈)-S(O)ₙ, (cycloalkyl en C₃-C₁₂)-S(O)ₙ, (cycloalkyl en C₃-C₁₂)-(alkyl en C₁-C₈)-S(O)ₙ, (bicycloalkyl en C₆-C₁₂)-S(O)ₙ, (bicycloalkyl en C₆-C₁₂)-(alkyl en C₁-C₈)-S(O)ₙ, (tricycloalkyl en C₆-C₁₂)-S(O)ₙ, (tricycloalkyl en C₆-C₁₂)-(alkyl en C₁-C₈)-S(O)ₙ, (aryl en C₆-C₁₄)-S(O)ₙ éventuellement substitué, (aryl en C₆-C₁₄)-(alkyl en C₁-C₈)-S(O)ₙ éventuellement substitué sur le reste aryle, hétéroaryl-S(O)ₙ éventuellement substitué ou hétéroaryl-(alkyl en C₁-C₈)-S(O)ₙ éventuellement substitué sur le reste hétéroaryle, n étant égal à 1 ou 2;
R¹ représente un groupe X-NH-C(=NH)-(CH₂)ₚ ou X¹ NH-(CH₂)ₚ, où p est 0, 1, 2 ou 3;
X représente un atome d'hydrogène ou un reste alkyle en C₁-C₆, (alkyl en C₁-C₆)carbonyle, (alcoxy en C₁-C₆)carbonyle, (alkyl en C₁-C₁₈)carbonyloxy-(alcoxy en C₁-C₆)carbonyle, (aryl en C₆-C₁₄)carbonyle éventuellement substitué, (aryloxy en C₆-C₁₄)carbonyle éventuellement substitué, (aryl en C₆-C₁₄)-(alcoxy en C₁-C₆)carbonyle pouvant aussi être substitué sur le reste aryle, cyano, hydroxy, alcoxy en C₁-C₆, (aryl en C₆-C₁₄)-(alcoxy en C₁-C₆) pouvant aussi être substitué sur le reste aryle, ou amino;
X¹ a l'une des significations de X ou représente un groupe R'-NH-C(=N-R"), dans lequel R' et R" ont indépendamment l'un de l'autre les significations de X;
R² représente un atome d'hydrogène ou un reste alkyle en C₁-C₈;
R³ représente un atome d'hydrogène ou un reste alkyle en C₁-C₈, aryle en C₆-C₁₄ éventuellement substitué, (aryl en C₆-C₁₄)-(alkyle en C₁-C₈) éventuellement substitué sur le reste aryle, cycloalkyle en C₃-C₈, alcényle en C₂-C₈, alcynyle en C₂-C₈, (alcényl en C₂-C₈)carbonyle, (alcynyl en C₂-C₈)carbonyle, pyridyle, R¹¹NH, CON(CH₃)R¹⁴, CONHR¹⁴, CON(CH₃)R¹⁵ ou CONHR¹⁵;
R⁵ représente un reste aryle en C₆-C₁₄ éventuellement substitué ou (aryl en C₆-C₁₄)-(alkyle en C₁-C₈) éventuellement substitué sur le reste aryle, un cycle hétérocyclique mono- ou bicyclique de 5 à 12 chaînons pouvant être aromatique, partiellement hydrogéné ou entièrement hydrogéné et pouvant contenir un, deux ou trois hétéroatomes identiques ou différents de la série de l'azote, de l'oxygène et du soufre, un reste R⁶ ou un reste R⁶-CO, le reste aryle et le reste hétérocyclique pouvant être indépendamment substitués une ou plusieurs fois par des restes identiques ou différents de la série des restes alkyle en C₁-C₁₈, alcoxy en C₁-C₁₈, halogéno, nitro, amino ou trifluorométhyle;
R⁶ représente un reste d'aminoacide naturel ou synthétique, d'iminoacide, d'aza-aminoacide éventuellement N-substitué par un reste alkyle en C₁-C₈ ou N-substitué par un reste (aryl en C₆-C₁₄)-(alkyle en C₁-C₈) ou de dipeptide, qui peut aussi être substitué sur le reste aryle, et leurs esters et amides, des groupes fonctionnels libres pouvant être protégés par des groupes protecteurs classiques dans la chimie des peptides;
R¹⁰ représente un reste hydroxy, alcoxy en C₁-C₁₈, (aryl en C₆-C₁₄)-(alcoxy en C₁-C₈) pouvant aussi être substitué sur le reste aryle, aryloxy en C₆-C₁₄ éventuellement substitué, amino ou mono- ou di(alkyl en C₁-C₁₈)amino;
R¹¹ représente un reste R¹²CO, aryl-S(O)₂ en C₆-C₁₄ éventuellement substitué ou alkyl-S(O)₂ en C₁-C₁₈;
R¹² représente un atome d'hydrogène ou un reste alkyle en C₁-C₁₈, alcényle en C₂-C₈, alcynyle en C₂-C₈, aryle en C₆-C₁₄ éventuellement substitué, alcoxy en C₁-C₁₈, (aryl en C₆-C₁₄)-(alcoxy en C₁-C₈) pouvant aussi être substitué sur le reste aryle ou aryloxy en C₆-C₁₄ éventuellement substitué;
R¹³ représente un atome d'hydrogène ou un reste alkyle en C₁-C₄;
R¹⁴ représente un reste alkyle en C₁-C₁₀ éventuellement substitué une ou plusieurs fois par des restes identiques ou différents de la série des restes hydroxy, hydroxycarbonyle, aminocarbonyle, mono- ou di(alkyl en C₁-C₁₈)aminocarbonyle, (aryl en C₆-C₁₄)-(alcoxy en C₁-C₈)carbonyle pouvant aussi être substitué sur le reste aryle, alcoxy en C₁-C₈, (alcoxy en C₁-C₈)carbonyle, cycloalkyle en C₃-C₈ éventuellement substitué, tétrazolyl-(alkyle en C₁-C₃), trifluorométhyle et R⁵;
R¹⁵ représente un reste R¹⁶-(alkyle en C₁-C₆) ou R¹⁶;
R¹⁶ représente un reste bicyclique ou tricyclique de 6 à 24 chaînons, saturé ou partiellement insaturé, pouvant aussi contenir un à quatre hétéroatomes identiques ou différents de la série de l'azote, de l'oxygène et du soufre et pouvant aussi être substitué par un ou plusieurs substituants identiques ou différents de la série des restes alkyle en C₁-C₄ et oxo;
c et d représentent 1, et f est 0;
e et h représentent indépendamment l'un de l'autre 0 ou 1 et g est 0;
dans toutes leurs formes stéréo-isomères et leurs mélanges en toutes proportions, ainsi que leurs sels pharmaceutiquement acceptables.

28. Composés de formule Id selon la revendication 26 et/ou 27, dans lesquels le reste par lequel est substitué le groupe B est un reste alkyle en C₁-C₈, dans toutes leurs formes stéréo-isomères et leurs mélanges en toutes proportions, ainsi que leurs sels pharmaceutiquement acceptables.

29. Composés de formule Id selon l'une ou plusieurs des revendications 26 à 28 et/ou leurs sels physiologiquement acceptables, à utiliser comme médicaments.

30. Préparation pharmaceutique, **caractérisée en ce qu'**elle contient un ou plusieurs composés de formule Id selon l'une ou plusieurs des revendications 26 à 28 et/ou leurs sels physiologiquement acceptables en plus de supports et/ou d'additifs pharmaceutiquement acceptables.

31. Composés de formule Ie 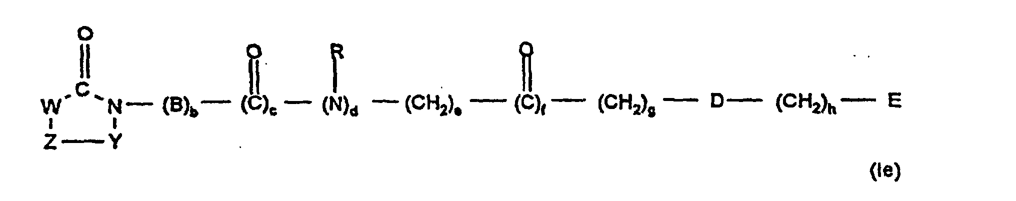 dans laquelle
W représente un groupe R¹-A-C(R¹³) ou R¹-A-CH=C;
Y représente un groupe carbonyle, thiocarbonyle ou méthylène;
Z représente N(R⁰), l'oxygène, le soufre ou un groupe méthylène;
A représente un reste divalent de la série des restes alkylène en C₁-C₆, cycloalkylène en C₃-C₇, phénylène, phénylène-(alkyle en C₁-C₆), (alkylène en C₁-C₆)-phényle et phénylène-(alcényle en C₂-C₆), ou un reste divalent d'un cycle saturé ou insaturé de 5 ou 6 chaînons pouvant contenir 1 ou 2 atomes d'azote et être substitué une ou deux fois par alkyle en C₁-C₆ ou par de l'oxygène ou du soufre doublement lié;
B représente un reste divalent de la série des restes allcylène en C₁-C₆, alcénylène en C₂-C₆, phénylène, phénylène-(alkyle en C₁-C₃) et (alkylène en C₁-C₃)-phényle;
D représente un groupe C(R²)(R³), N(R³) ou CH=C(R³);
E représente un groupe tétrazolyle, (R⁸O)₂P(O), HOS(O)₂, R⁹NHS(O)₂ ou R¹⁰CO;
R représente un atome d'hydrogène ou un reste alkyle en C₁-C₈, cycloalkyle en C₃-C₈, aryle en C₆-C₁₄ éventuellement substitué ou (aryl en C₆-C₁₄)-(alkyle en C₁-C₈) éventuellement substitué sur le reste aryle;
R⁰ représente un reste CHO, (alkyl en C₁-C₈)-CO, (cycloalkyl en C₃-C₁₂)-CO, (cycloalkyl en C₃-C₁₂)-(alkyl en C₁-C₈)-CO, (bicycloalkyl en C₆-C₁₂)-CO, (bicycloalkyl en C₆-C₁₂)-(alkyl en C₁-C₈)-CO, (tricycloalkyl en C₆-C₁₂)-CO, (tricycloalkyl en C₆-C₁₂)-(alkyl en C₁-C₈)-CO, (aryl en C₆-C₁₄)-CO éventuellement substitué, (aryl en C₆-C₁₄)-(alkyl en C₁-C₈)-CO éventuellement substitué sur le reste aryle, hétéroaryl-CO éventuellement substitué, hétéroaryl-(alkyl en C₁-C₈)-CO éventuellement substitué sur le reste hétéroaryle, (alkyl en C₁-C₈)-S(O)ₙ, (cycloalkyl en C₃-C₁₂)-S(O)ₙ, (cycloalkyl en C₃-C₁₂)-(alkyl en C₁-C₈)-S(O)ₙ, (bicycloalkyl en C₆-C₁₂)-S(O)ₙ, (bicycloalkyl en C₆-C₁₂)-(alkyl en C₁-C₈)-S(O)ₙ, (tricycloalkyl en C₆-C₁₂)-S(O)ₙ, (tricycloalkyl en C₆-C₁₂)-(alkyl en C₁-C₈)-S(O)ₙ, (aryl en C₆-C₁₄)-S(O)ₙ éventuellement substitué, (aryl en C₆-C₁₄)-(alkyl en C₁-C₈)-S(O)ₙ éventuellement substitué sur le reste aryle, hétéroaryl-S(O)ₙ éventuellement substitué ou hétéroaryl-(alkyl en C₁-C₈)-S(O)ₙ éventuellement substitué sur le reste hétéroaryle, n étant égal à 1 ou 2;
R¹ représente un groupe X-NH-C(=NH)-(CH₂)ₚ ou X¹-NH-(CH₂)ₚ, aù p est 0, 1, 2 ou 3;
X représente un atome d'hydrogène ou un reste alkyle en C₁-C₆, (alkyl en C₁-C₆)carbonyle, (alcoxy en C₁-C₆)carbonyle, (alkyl en C₁-C₁₈)carbonyloxy-(alcoxy en C₁-C₆)carbonyle, (aryl en C₆-C₁₄)carbonyle éventuellement substitué, (aryloxy en C₆-C₁₄)carbonyle éventuellement substitué, (aryl en C₆-C₁₄)-(alcoxy en C₁-C₆)carbonyle pouvant aussi être substitué sur le reste aryle, (R⁸O)₂P(O), cyano, hydroxy, alcoxy en C₁-C₆, (aryl en C₆-C₁₄)-(alcoxy en C₁-C₆) pouvant aussi être substitué sur le reste aryle, ou amino;
X¹ a l'une des significations de X ou représente un groupe R'-NH-C(=N-R"), dans lequel R' et R" ont indépendamment l'un de l'autre les significations de X;
R² représente un atome d'hydrogène ou un reste alkyle en C₁-C₈, aryle en C₆-C₁₄ éventuellement substitué, (aryl en C₆-C₁₄)-(alkyle en C₁-C₈) éventuellement substitué sur le reste aryle, ou cycloalkyle en C₃-C₈;
R³ représente un atome d'hydrogène ou un reste alkyle en C₁-C₈, aryle en C₆-C₁₄ éventuellement substitué, (aryl en C₆-C₁₄)-(alkyle en C₁-C₈) éventuellement substitué sur le reste aryle, cycloalkyle en C₃-C₈, alcényle en C₂-C₈, alcynyle en C₂-C₈, (alcényl en C₂-C₈)carbonyle, (alcynyl en C₂-C₈)carbonyle, pyridyle, R¹¹NH, R⁴CO, COOR⁴, CON(CH₃)R¹⁴, CONHR¹⁴, CSNHR¹⁴, COOR¹⁵, CON(CH₃)R¹⁵ ou CONHR¹⁵;
R⁴ représente un atome d'hydrogène ou un reste alkyle en C₁-C₂₈ pouvant éventuellement être substitué une ou plusieurs fois par des restes R^{4'} identiques ou différents;
R^{4'} représente un reste hydroxy, hydroxycarbonyle, aminocarbonyle, mono- ou di(alkyl en C₁-C₁₈)aminocarbonyle, amino-(alkyl en C₂-C₁₈)aminocarbonyle, amino(alkyl en C₁-C₃)phényl-(alkyl en C₁-C₃)aminocarbonyle, (alkyl en C₁-C₁₈)carbonylamino(alkyl en C₁-C₃)phényl-(alkyl en C₁-C₃)aminocarbonyle, (alkyl en C₁-C₁₈)carbonylamino-(alkyl en C₂-C₁₈)aminocarbonyle, (aryl en C₆-C₁₄)-(alcoxy en C₁-C₈)carbonyle pouvant aussi être substitué sur le reste aryle, amino, mercapto, alcoxy en C₁-C₁₈, (alcoxy en C₁-C₁₈)carbonyle, cycloalkyle en C₃-C₈ éventuellement substitué, halogéno, nitro, trifluorométhyle ou le reste R⁵,
R⁵ représente un reste aryle en C₆-C₁₄ éventuellement substitué ou (aryl en C₆-C₁₄)-(alkyle en C₁-C₈) éventuellement substitué sur le reste aryle, un cycle hétérocyclique mono- ou bicyclique de 5 à 12 chaînons pouvant être aromatique, partiellement hydrogéné ou entièrement hydrogéné et pouvant contenir un, deux ou trois hétéroatomes identiques ou différents de la série de l'azote, de l'oxygène et du soufre, un reste R⁶ ou un reste R⁶-CO, le reste aryle et le reste hétérocyclique pouvant être indépendamment substitués une ou plusieurs fois par des restes identiques ou différents de la série des restes alkyle en C₁-C₁₈, alcoxy en C₁-C₁₈, halogéno, nitro, amino ou trifluorométhyle;
R⁶ représente un groupe R⁷R⁸N, R⁷O ou R⁷S ou une chaîne latérale d'aminoacide, un reste d'aminoacide naturel ou synthétique, d'iminoacide, d'aza-aminoacide éventuellement N-substitué par un reste alkyle en C₁-C₈ ou N-substitué par un reste (aryl en C₆-C₁₄)-(alkyle en C₁-C₈) ou de dipeptide, qui peut aussi être substitué sur le reste aryle et/ou dont la liaison peptidique peut être réduite en -NH-CH₂-, et leurs esters et amides, et où, à la place de groupes fonctionnels libres, il peut éventuellement y avoir de l'hydrogène ou un groupe hydroxyméthyle et/ou des groupes fonctionnels libres peuvent être protégés par des groupes protecteurs classiques dans la chimie des peptides;
R⁷ représente un atome d'hydrogène, un reste alkyle en C₁-C₁₈, (aryl en C₆-C₁₄)-(allyle en C₁-C₈), (alkyl en C₁-C₁₈)carbonyle, (alcoxy en C₁-C₁₈)carbonyle, (aryl en C₆-C₁₄)carbonyle, (aryl en C₆-C₁₄)-(alkyl en C₁-C₈)carbonyle ou (aryl en C₆-C₁₄)-(alkyl en C₁-C₁₈)oxycarbonyle, les groupes alkyle pouvant éventuellement être substitués par un groupe amino et/ou les restes aryle pouvant être substitués une ou plusieurs fois, de préférence une fois, par des restes identiques ou différents de la série des restes alkyle en C₁-C₈, alcoxy en C₁-C₈, halogéno, nitro, amino et trifluorométhyle, ou un reste d'aminoacide naturel ou synthétique, d'iminoacide, d'aza-aminoacide éventuellement N-substitué par un reste alkyle en C₁-C₈ ou N-substitué par un reste (aryl en C₆-C₁₄)-(alkyle en C₁-C₈) ou de dipeptide, qui peut aussi être substitué sur le reste aryle et/ou dont la liaison peptidique peut être réduite en NH-CH₂-;
R₈ représente un atome d'hydrogène ou un reste alkyle en C₁-C₁₈, aryle en C₆-C₁₄ éventuellement substitué ou (aryl en C₆-C₁₄)-(alkyle en C₁-C₈) pouvant aussi être substitué sur le reste aryle;
R⁹ représente un atome d'hydrogène ou un reste aminocarbonyle, (alkyl en C₁-C₁₈)aminocarbonyle, (cycloalkyl en C₃-C₈)aminocarbonyle, (aryl en C₆-C₁₄)aminocarbonyle éventuellement substitué, alkyle en C₁-C₁₈, aryle en C₆-C₁₄ éventuellement substitué ou cycloalkyle en C₃-C₈;
R¹⁰ représente un reste hydroxy, alcoxy en C₁-C₁₈, (aryl en C₆-C₁₄)-(alcoxy en C₁-C₈) pouvant aussi être substitué sur le reste aryle, aryloxy en C₆-C₁₄ éventuellement substitué, amino ou mono- ou di(alkyl en C₁-C₁₈)amino;
R¹¹ représente un atome d'hydrogène ou un reste alkyle en C₁-C₁₈, R¹²CO, aryl-S(O)₂ en C₆-C₁₄ éventuellement substitué, alkyl-S(O)₂ en C₁-C₁₈, (aryl en C₆-C₁₄)-(alkyle en C₁-C₈) éventuellement substitué sur le reste aryle, ou R⁹NHS(O)₂;
R¹² représente un atome d'hydrogène ou un reste alkyle en C₁-C₁₈, alcényle en C₂-C₈, alcynyle en C₂-C₈, aryle en C₆-C₁₄ éventuellement substitué, alcoxy en C₁-C₁₈, (aryl en C₆-C₁₄)-(alcoxy en C₁-C₈) pouvant aussi être substitué sur le reste aryle, aryloxy en C₆-C₁₄ éventuellement substitué, amino ou mono- ou di(alkyl en C₁-C₁₈)amino;
R¹³ représente un atome d'hydrogène ou un reste alkyle en C₁-C₆, (aryl en C₆-C₁₄)-(alkyle en C₁-C₈) éventuellement substitué sur le reste aryle ou cycloalkyle en C₃-C₈;
R¹⁴ représente un atome d'hydrogène ou un reste alkyle en C₁-C₂₈ éventuellement substitué une ou plusieurs fois par des restes identiques ou différents de la série des restes hydroxy, hydroxycarbonyle, aminocarbonyle, mono- ou di(alkyl en C₁-C₁₈)aminocarbonyle, amino-(alkyl en C₂-C₁₈)aminocarbonyle, amino(alkyl en C₁-C₃)phényl-(alkyl en C₁-C₃)aminocarbonyle, (alkyl en C₁-C₁₈)carbonyl-amino(alkyl en C₁-C₃)phényl-(alkyl en C₁-C₃)aminocarbonyle, (alkyl en C₁-C₁₈)carbonylamino-(alkyl en C₂-C₁₈)aminocarbonyle, (aryl en C₆-C₁₄)-(alcoxy en C₁-C₈)carbonyle pouvant aussi être substitué sur le reste aryle, amino, mercapto, alcoxy en C₁-C₁₈, (alcoxy en C₁-C₁₈)carbonyle, cycloalkyle en C₃-C₈ éventuellement substitué, HOS(O)₂-(alkyle en C₁-C₃), R⁹NHS(O)₂-(alkyle en C₁-C₃), (R⁸O)₂P(O)-(alkyle en C₁-C₃), tétrazolyl-(alkyle en C₁-C₃), halogéno, nitro, trifluorométhyle et R⁵;
R¹⁵ représente un reste R¹⁶-(alkyle en C₁-C₆) ou R¹⁶;
R¹⁶ représente un reste bicyclique ou tricyclique de 6 à 24 chaînons, saturé ou partiellement insaturé, pouvant aussi contenir un à quatre hétéroatomes identiques ou différents de la série de l'azote, de l'oxygène et du soufre et pouvant aussi être substitué par un ou plusieurs substituants identiques ou différents de la série des restes alkyle en C₁-C₄ et oxo;
b, c, d et f représentent, indépendamment les uns des autres, 0 ou 1, mais ne peuvent pas tous être 0 en même temps;
e, g et h représentent indépendamment les uns des autres 0, 1, 2, 3, 4, 5 ou 6;
dans toutes leurs formes stéréo-isomères et leurs mélanges en toutes proportions, ainsi que leurs sels pharmaceutiquement acceptables.

32. Composés de formule Ie selon la revendication 31 et/ou leurs sels physiologiquement acceptables, à utiliser comme médicaments.

33. Préparation pharmaceutique, **caractérisée en ce qu'**elle contient un ou plusieurs composés de formule le selon la revendication 31 et/ou leurs sels physiologiquement acceptables en plus de supports et/ou d'additifs pharmaceutiquement acceptables.
